# EUROPEAN PATENT SPECIFICATION

(11) **EP 4 630 412 B1**
(45) Date of publication and mention of the grant of the patent: **16.09.2026**
(21) Application number: 23818040.0
(22) Date of filing: 05.12.2023
(51) Int. Cl.: C07D 241/38, C07D 265/36, C07D 267/02, C07D 279/16, A61K 31/536, A61P 21/00

(54) **COMPOUNDS FOR THE TREATMENT OF NEUROMUSCULAR DISORDERS**
VERBINDUNGEN ZUR BEHANDLUNG NEUROMUSKULÄRER ERKRANKUNGEN
COMPOSÉS POUR LE TRAITEMENT DE TROUBLES NEUROMUSCULAIRES

(30) Priority: 05.12.2022 EP 22211390
(43) Date of publication of application: 15.10.2025
(73) Proprietor: NMD Pharma A/S, 8200 Århus N (DK)
(72) Inventor: KNUTSEN, Lars J.S., 8200 Aarhus N (DK); KELLY, Nicholas Michael, 8200 Aarhus N (DK); SARASWAT, Neerja, 8200 Aarhus N (DK); HAMLYN, Richard, J., 8200 Aarhus N (DK)
(74) Representative: Høiberg P/S
(86) International application number: PCT/EP2023/084299
(87) International publication number: WO 2024/121130

(56) References cited:
- EP-A1- 3 753 560
- DALLACKER FRANZ ET AL: "Über eine durch Benzo-und Naphtho[1.3]dioxolcarbonsäuren an Mäusen erzeugbare Krampfwirkung / Spasm-Activity Caused by Interaction of Benzo-and Naphtho-1.3-Dioxole-carboxylic Acids of Mice", ZEITSCHRIFT FUER NATURFORSCHUNG. C, A JOURNAL OF BIOSCIENCES., vol. 35, no. 1, 1 February 1980 (1980-02-01), DE, pages 49 - 56, XP093032923, ISSN: 0939-5075, DOI: 10.1515/znc-1980-1-211

## Description

### Technical field

The present disclosure relates to compounds and their use in treating, ameliorating and/or preventing neuromuscular disorders, including the reversal of drug-induced neuromuscular blockade. The compounds as defined herein can inhibit the CIC-1 ion channel. The disclosure further relates to methods of treating, preventing and/or ameliorating neuromuscular disorders, by administering said composition to a person in need thereof.

### Background

Walking, breathing, and eye movement are examples of essential everyday physiological activities that are powered by the contractile activity of skeletal muscle. Skeletal muscles are inherently in a resting state and contractile activity occurs exclusively in response to commands from the central nervous system (CNS). Such neuronal commands take the form of action potentials that travel from the brain to the muscle fibres in several steps. The neuromuscular junction (NMJ) is a highly specialized membrane area on muscle fibres where motor neurons come into close contact with the muscle fibres, and it is at the NMJ where neuronal action potentials are transmitted to muscular action potentials in a one-to-one fashion *via* synaptic transmission.

Unfortunately, none of the currently employed drug regimens for treating neuromuscular disorders, such as myasthenia gravis, Lambert-Eaton Syndrome, Charcot-Marie Tooth (CMT) disease, amyotrophic lateral sclerosis (ALS), spinal muscular atrophy (SMA) and sarcopenia, have been able to fully restore muscle function in compromised patients.

The CIC-1 ion channel (Pedersen, T.H., Riisager, A., Vincenzo de Paoli, F., Chen, T-Y, Nielsen, O.B. Role of physiological CIC-1 Cl- ion channel regulation for the excitability and function of working skeletal muscle. J. Gen. Physiol. 2016, 147, 291 - 308) is emerging as a target for improving muscle function in patients having a neuromuscular disfunction.

### Summary

The present disclosure comprises a series of compounds that can alleviate disorders of the neuromuscular junction through inhibition of CIC-1 channels.

It has been found that compounds that inhibit CIC-1 ion channels are capable of restoring neuromuscular transmission, as evidenced by the data generated by investigation of the compound set in biological models described herein. These compounds thus constitute a group of potential drugs that can be used to treat and/or ameliorate muscle weakness and/or muscle fatigue in neuromuscular junction disorders caused by disease or by neuromuscular blocking agents.

The present disclosure is directed to CIC-1 ion channel inhibitors with application in the treatment of a range of conditions, such as reversal of neuromuscular block, SMA, CMT, ALS and myasthenic conditions, in which muscle activation by the nervous system is compromised and symptoms of weakness and fatigue are prominent.

In one aspect, the disclosure concerns a compound of Formula (I): 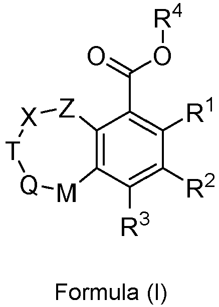 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
- only one of M, Q, T, X and Z is O; and
- when M is O, Q and T are CH₂, X is absent, Z is NH, R¹ is Cl, R² is H and R³ is H then R⁴ is not H.

In another aspect, the disclosure concerns a compound as defined herein for use in treating, ameliorating and/or preventing a neuromuscular disorder, and/or for use in reversing and/or ameliorating a neuromuscular blockade. In yet another aspect, the disclosure concerns a composition comprising a compound as defined herein.

The invention is defined by the claims. Any subject matter falling outside the scope of the claims is provided for information purposes only.

### Definitions

The terms "C₁₋₃ alkyl" and "C₁₋₅ alkyl" refers to a branched or unbranched alkyl group having from one to three or one to five carbon atoms respectively, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl and pent-3-yl.

The term "alkanediyl" refers to the corresponding derivative of an alkyl group having two bonding sites. Thus, a C₁₋₃ alkanediyl refers to -(CH₂)ₙ-, wherein n is a positive integer from 1 to 3, i.e. including -CH₂-, -CH₂CH₂-, and -CH₂CH₂CH₂-.

The term "C₂₋₃ alkenyl" and "C₂₋₅ alkenyl" refers to a branched or unbranched alkenyl group having from two to three or two to five carbon atoms respectively, two of which are connected by a double bond, including but not limited to ethenyl, propenyl, isopropenyl, butenyl, isobutenyl, pentenyl and isopentenyl.

The term "C₂₋₅ alkynyl" refers to a branched or unbranched alkynyl group having from two to five carbon atoms, two of which are connected by a triple bond, including but not limited to ethynyl, prop-1-ynyl, prop-2-ynyl, but-1-ynyl, but-2-ynyl, but-3-ynyl, buta-1,3-diynyl, pent-1-ynyl, pent-2-ynyl, pent-3-ynyl, pent-4-ynyl, penta-2,4-diynyl and penta-1,3-diynyl.

The term "C₃₋₅ cycloalkyl" and "C₃₋₆ cycloalkyl" refers to a group having three to five or three to six carbon atoms respectively including a monocyclic or bicyclic carbocycle, including but not limited to cyclopropyl, cyclobutyl, cyclopentyl and cyclohexyl.

The term "heterocycle" as used herein refers to a monocyclic or bicyclic, heterocyclic ring which is either saturated, unsaturated, or aromatic, and which contains from 1 to 4 heteroatoms independently selected from nitrogen, oxygen and sulfur. The term "C₅ heterocycle" as used herein refers to a 5-membered heterocycle.

The term "5- to 10-membered heteroaryl" refers to a monovalent, aromatic heterocyclic group having one or more heteroatoms, preferably one to three heteroatoms, selected from the group consisting of a nitrogen atom, an oxygen atom, and a sulfur atom. The term "5- to 10-membered heteroaryl" encompasses an aromatic heterocyclic group condensed with an aromatic or non-aromatic carbocyclic ring or an aromatic or non-aromatic heterocyclic ring to form a bicyclic group, and also encompasses an aromatic carbocyclic group condensed with an aromatic or non-aromatic heterocyclic ring to form a bicyclic group. Binding to the heteroaryl may be via a heteroatom or via a carbon atom of the heteroaryl.

In some embodiments, the 5- to 10-membered heteroaryl is a 5-membered heteroaryl. The term "5-membered heteroaryl" refers to a monovalent, aromatic ring system having 5 ring atoms and which contains at least one heteroatom which may be identical or different, said heteroatom being oxygen, nitrogen or sulfur. 5-membered heteroaryls include but are not limited to oxazolyl, thiazolyl, thiadiazolyl, oxadiazolyl, triazolyl, pyrrolyl, thienyl, furanyl, diazolyl, imidazolyl, pyrazolyl, isoxazolyl, isothiazolyl, and tetrazolyl. In one embodiment, the 5-membered heteroaryl is furanyl, such as furan-2-yl. In one embodiment, the 5-membered heteroaryl is thienyl, such as thien-2-yl or thien-3-yl. In one embodiment, the 5-membered heteroaryl is thiazolyl, such as thiazol-2-yl. In one embodiment, the 5-membered heteroaryl is oxazolyl, such as oxazol-2-yl.

In some embodiments, the 5- to 10-membered heteroaryl is a 6-membered heteroaryl. The term "6-membered heteroaryl" refers to a monovalent, aromatic ring system having 6 ring atoms and which contains at least one heteroatom which may be identical or different, said heteroatom being oxygen, nitrogen or sulfur. 6-membered heteroaryls include but are not limited to pyridinyl, pyridazinyl, pyrimidinyl, pyrazinyl, and triazinyl.

In some embodiments, the 5- to 10-membered heteroaryl is a 8-membered heteroaryl. The term "8-membered heteroaryl" refers to a monovalent, aromatic ring system having 8 ring atoms and which contains at least one heteroatom which may be identical or different, said heteroatom being oxygen, nitrogen or sulfur. 8-membered heteroaryls include but are not limited to groups encompassing an aromatic heterocyclic group condensed with an aromatic or non-aromatic carbocyclic ring or an aromatic or non-aromatic heterocyclic ring to form a bicyclic group, and also groups encompassing an aromatic carbocyclic group condensed with an aromatic or non-aromatic heterocyclic ring to form a bicyclic group. 8-membered heteroaryls include but are not limited to 1,4-dihydropyrrolo[3,2-b]pyrrolyl, 1,6-dihydropyrrolo[2,3-b]pyrrolyl, 6*H*-furo[2,3-b]pyrrole, 4*H*-furo[2,3-b]pyrrolyl, 6*H*-thieno[2,3-b]pyrrolyl and 4*H*-thieno[2,3-b]pyrrolyl.

In some embodiments, the 5- to 10-membered heteroaryl is a 9-membered heteroaryl. The term "9-membered heteroaryl" refers to a monovalent, aromatic ring system having 9 ring atoms and which contains at least one heteroatom which may be identical or different, said heteroatom being oxygen, nitrogen or sulfur. 9-membered heteroaryls include but are not limited to groups encompassing an aromatic heterocyclic group condensed with an aromatic or non-aromatic carbocyclic ring or an aromatic or non-aromatic heterocyclic ring to form a bicyclic group, and also groups encompassing an aromatic carbocyclic group condensed with an aromatic or non-aromatic heterocyclic ring to form a bicyclic group. 9-membered heteroaryls include but are not limited to indolyl, isoindolyl, indolizinyl, indazolyl, benzimidazolyl, azaindolyl, azaindazolyl, pyrazolo[1,5-a]pyrimidinyl, purinyl, benzofuranyl, benzo[b]thiophenyl, benzisoxazolyl, benzthiazolyl, benzisothiazolyl, and benzo[c][1,2,5]thiadiazolyl.

In some embodiments, the 5- to 10-membered heteroaryl is a 10-membered heteroaryl. The term "10-membered heteroaryl" refers to a monovalent, aromatic ring system having 10 ring atoms and which contains at least one heteroatom which may be identical or different, said heteroatom being oxygen, nitrogen or sulfur. 10-membered heteroaryls include but are not limited to groups encompassing an aromatic heterocyclic group condensed with an aromatic or non-aromatic carbocyclic ring or an aromatic or non-aromatic heterocyclic ring to form a bicyclic group, and also groups encompassing an aromatic carbocyclic group condensed with an aromatic or non-aromatic heterocyclic ring to form a bicyclic group. 10-membered heteroaryls include but are not limited to quinolinyl, isoquinolinyl, 4*H*-quinolizinyl, quinoxalinyl, phthalazinyl, quinazolinyl, cinnolinyl, 1,8-naphthyridinyl, pyrido[3,2-d]pyrimidinyl, pyrido[4,3-d]pyrimidinyl, pyrido[3,4-b]pyrazinyl, pyrido[2,3-b]pyrazinyl and pteridinyl.

The term "spiro compound" as used herein refers to a compound having one atom (usually a quaternary carbon) as the only common member of two rings.

The term "half-life" as used herein is the time it takes for the compound to lose one-half of its pharmacologic activity. The term "plasma half-life" is the time that it takes the compound to lose one-half of its pharmacologic activity in the blood plasma.

The term "treatment" refers to the combating of a disease or disorder. "Treatment" or "treating," as used herein, includes any desirable effect on the symptoms or pathology of a disease or condition as described herein, and may include even minimal changes or improvements in one or more measurable markers of the disease or condition being treated. "Treatment" or "treating" does not necessarily indicate complete eradication or cure of the disease or condition, or associated symptoms thereof. In some embodiments, the term "treatment" encompasses amelioration and prevention.

The term "amelioration" refers to moderation in the severity of the symptoms of a disease or condition. Improvement in a patient's condition, or the activity of making an effort to correct, or at least make more acceptable, conditions that are difficult to endure related to patient's conditions is considered "ameliorative" treatment.

The term "prevent" or "preventing" refers to precluding, averting, obviating, forestalling, stopping, or hindering something from happening, especially by advance action.

The term "reversal" or "reversing" refers to the ability of a compound to restore nerve-stimulated force in skeletal muscle exposed either *ex vivo* or *in vivo* to a non-depolarizing neuromuscular blocking agent or another pharmaceutical that is able to depress neuromuscular transmission

The term "non-depolarizing blockers" refers to pharmaceutical agents that antagonize the activation of acetylcholine receptors at the post-synaptic muscle fibre membrane by blocking the acetylcholine binding site on the receptor. These agents are used to block neuromuscular transmission and induce muscle paralysis in connection with surgery.

The term "ester hydrolysing reagent" refers to a chemical reagent which is capable of converting an ester functional group to a carboxylic acid with elimination of the alcohol moiety of the original ester, including but not limited to acid, base, a fluoride source, PBr₃, PCl₃ and lipase enzymes.

The term "total membrane conductance (Gm)" is the electrophysiological measure of the ability of ions to cross the muscle fibre surface membrane. It reflects the function of ion channels that are active in resting muscle fibres of which CIC-1 is known to contribute around 80 % in most animal species.

### Detailed description

### Compounds

It is within the scope of the present disclosure to provide a compound for use in treating, ameliorating and/or preventing neuromuscular disorders that reduce neuromuscular function. As disclosed herein, inhibition of CIC-1 improves or restores neuromuscular function. The compounds of the present disclosure comprise compounds capable of inhibiting the CIC-1 channel thereby improving or restoring neuromuscular function.

In one aspect, the disclosure concerns a compound of Formula (I): 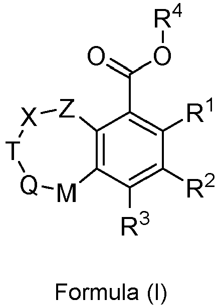 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R¹ is selected from the group consisting of H; F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; C₁₋₃ alkyl optionally substituted with one or more, identical or different, substituents R¹⁰ and OC₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;

when R⁵ and R⁶ are individually C₁₋₅ alkyl optionally substituted with one or more, identical or different, substituents R¹⁰, then R⁵ and R⁶ are optionally joined together to form a ring;
or a pharmaceutically acceptable salt, hydrate, polymorph, tautomer, or solvate thereof.

In one aspect, the disclosure concerns a compound of Formula (I): 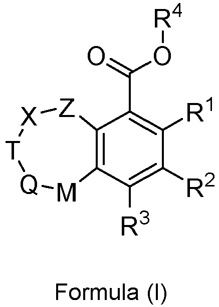 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R¹ is selected from the group consisting of H; F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; C₁₋₃ alkyl optionally substituted with one or more, identical or different, substituents R¹⁰ and OC₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heterocycle optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heterocycle optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, nitro, cyano, Cl, Br, I, and F;

when R⁵ and R⁶ are individually C₁₋₅ alkyl optionally substituted with one or more, identical or different, substituents R¹⁰, then R⁵ and R⁶ are optionally joined together to form a ring;
or a pharmaceutically acceptable salt, hydrate, polymorph, tautomer, or solvate thereof.

In one embodiment, only one of M, Q, T, X and Z is O. In one embodiment, when M is O, Q and T are CH₂, X is absent, Z is NH, R¹ is Cl, R² is H and R³ is H then R⁴ is not H. In one embodiment, the compound is not 6-chloro-3,4-dihydro-2H-1,4-benzoxazine-5-carboxylic acid.

In one aspect, the disclosure concerns a compound of Formula (I): 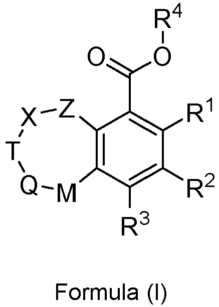 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
- only one of M, Q, T, X and Z is O; and
- when M is O, Q and T are CH₂, X is absent, Z is NH, R¹ is Cl, R² is H and R³ is H then R⁴ is not H.

In one aspect, the disclosure concerns a compound of Formula (I): 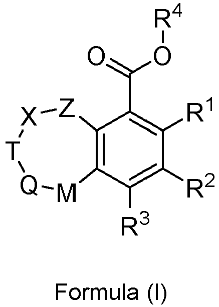 wherein:
- M is NR⁷, O or S;
- Q is CR⁵R⁶;
- T is CR⁵R⁶;
- X is absent or CR⁵R⁶;
- Z is NR⁹, O or S;
- R¹ is selected from the group consisting of F; Cl; Br; C₁₋₃ alkyl optionally substituted with one or more, identical or different, substituents7 R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
- only one of M, Q, T, X and Z is O; and
- when M is O, Q and T are CH₂, X is absent, Z is NH, R¹ is Cl, R² is H and R³ is H then R⁴ is not H.

In one aspect, the disclosure concerns a compound of Formula (II): 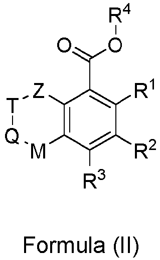
- M is CR⁵R⁶, NR⁷, O or S;
- Q is CR⁵R⁶, NR⁸, O or S;
- T is CR⁵R⁶, NR⁸, O or S;
- Z is CR⁵R⁶, NR⁹, O or S;
- two or more of M, Q, T and Z are NR⁷, NR⁸, NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
- only one of M, Q, T and Z is O; and
- when M is O, Q and T are CH₂, Z is NH, R¹ is Cl, R² is H and R³ is H then R⁴ is not H.

In one aspect, the disclosure concerns a compound of Formula (II): 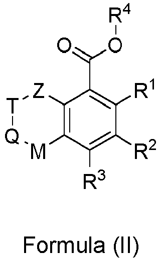
- M is NR⁷, O or S;
- Q is CR⁵R⁶;
- T is CR⁵R⁶;
- Z is NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
- only one of M and Z is O; and
- when M is O, Q and T are CH₂, Z is NH, R¹ is Cl, R² is H and R³ is H then R⁴ is not H.

In one aspect, the disclosure concerns a compound of Formula (I): 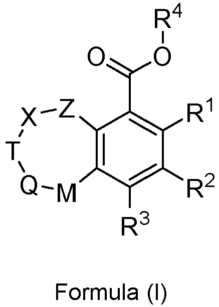
- M is CR⁵R⁶, NR⁷, O or S;
- Q is CR⁵R⁶, NR⁸, O or S;
- T is CR⁵R⁶, NR⁸, O or S;
- X is CR⁵R⁶, NR⁸, O or S;
- Z is CR⁵R⁶, NR⁹, O or S;
- two or more of M, Q, T, X and Z are NR⁷, NR⁸, NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
   - - only one of M, Q, T, X and Z is O.

In one aspect, the disclosure concerns a compound of Formula (I): 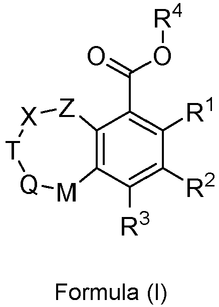
- M is NR⁷, O or S;
- Q is CR⁵R⁶;
- T is CR⁵R⁶;
- X is absent, CR⁵R⁶;
- Z is NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof provided that:
- only one of M and Z is O.

In one embodiment, M is CR⁵R⁶. In one embodiment, M is NR⁷. In one embodiment, M is O. In one embodiment, M is S.

In one embodiment, Q is CR⁵R⁶. In one embodiment, Q is C(H)₂. In one embodiment, Q is NR⁸. In one embodiment, Q is O. In one embodiment, Q is S.

In one embodiment, T is CR⁵R⁶. In one embodiment, T is C(H)₂. In one embodiment, T is C(H)(OMe). In one embodiment, T is C(CH₃)(CH₃). In one embodiment, T is

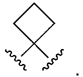

In one embodiment, T is NR⁸. In one embodiment, T is O. In one embodiment, T is S.

In one embodiment, X is absent. In one embodiment, X is CR⁵R⁶. In one embodiment, X is C(H)₂. In one embodiment, X is NR⁸. In one embodiment, X is O. In one embodiment, X is S.

In one embodiment, Z is CR⁵R⁶. In one embodiment, Z is NR⁹. In one embodiment, Z is O. In one embodiment, Z is S.

In one embodiment, M is NR⁷; Q is CR⁵R⁶; T is CR⁵R⁶; X is absent; and Z is O. In one aspect, the disclosure concerns a compound of Formula (III): 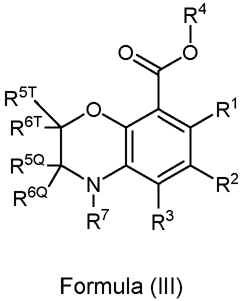 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q} and R^{5T} are independently selected from the group consisting of H, deuterium, F and C₁₋₅ alkyl optionally substituted with one or more, identical or different, substituents R¹⁰;
- R^{6Q} and R^{6T} are independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof.

In one embodiment, M is NR⁷; Q is CR⁵R⁶; T is CR⁵R⁶; X is absent; and Z is S. In one aspect, the disclosure concerns a compound of Formula (IV): 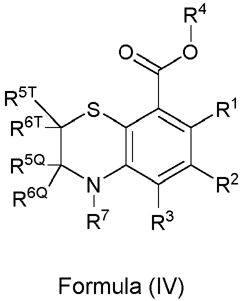
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q} and R^{5T} are independently selected from the group consisting of H, deuterium, F and C₁₋₅ alkyl optionally substituted with one or more, identical or different, substituents R¹⁰;
- R^{6Q} and R^{6T} are independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or a pharmaceutically acceptable salt, hydrate, polymorph, tautomer, or solvate thereof.

In one embodiment, M is NR⁷; Q is CR⁵R⁶; T is CR⁵R⁶; X is absent; and Z is NR⁹. In one aspect, the disclosure concerns a compound of Formula (V): 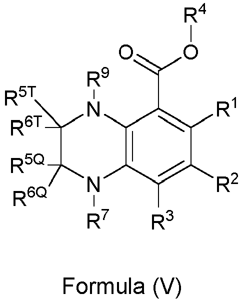 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q} and R^{5T} are independently selected from the group consisting of H, deuterium, F and C₁₋₅ alkyl optionally substituted with one or more, identical or different, substituents R¹⁰;
- R^{6Q} and R^{6T} are independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or a pharmaceutically acceptable salt, hydrate, polymorph, tautomer, or solvate thereof.

In one embodiment, M is O; Q is CR⁵R⁶; T is CR⁵R⁶; X is absent; and Z is NR⁹. In one aspect, the disclosure concerns a compound of Formula (VI): 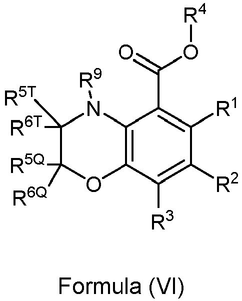
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q} and R^{5T} are independently selected from the group consisting of H, deuterium, F and C₁₋₅ alkyl optionally substituted with one or more, identical or different, substituents R¹⁰;
- R^{6Q} and R^{6T} are independently selected from the group consisting of H, deuterium, F and C₁₋₅ alkyl optionally substituted with one or more, identical or different, substituents R¹⁰;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or a pharmaceutically acceptable salt, hydrate, polymorph, tautomer, or solvate thereof provided that:
- when R¹ is Cl, R², R³, R^{5Q}, R^{5T}, R^{6Q}, R^{6T} and R⁹ are H then R⁴ is not H.

In one embodiment, M is NR⁷; Q is CR⁵R⁶; T is CR⁵R⁶; X is CR⁵R⁶; and Z is O. In one aspect, the disclosure concerns a compound of Formula (VII): 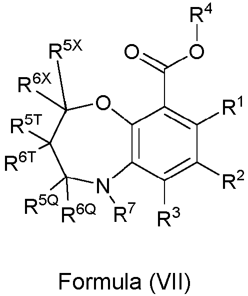 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q}, R^{5T} and R^{5X} are independently selected from the group consisting of H, deuterium, F and C₁₋₅ alkyl optionally substituted with one or more, identical or different, substituents R¹⁰;
- R^{6Q}, R^{6T} and R^{6X} are independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5X} and R^{6X} are individually C₁₋₅ alkyl optionally substituted with one or more, identical or different, substituents R¹⁰, then R^{5X} and R^{6X} are optionally joined together to form a ring;
   or a pharmaceutically acceptable salt, hydrate, polymorph, tautomer, or solvate thereof.

In one embodiment, M is S; Q is CR⁵R⁶; T is CR⁵R⁶; X is absent; and Z is NR⁹. In one aspect, the disclosure concerns a compound of Formula (VIII): 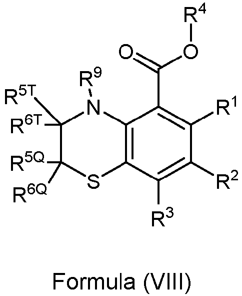 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q} and R^{5T} are independently selected from the group consisting of H, deuterium, F and C₁₋₅ alkyl optionally substituted with one or more, identical or different, substituents R¹⁰;
- R^{6Q} and R^{6T} are independently selected from the group consisting of H, deuterium, F and C₁₋₅ alkyl optionally substituted with one or more, identical or different, substituents R¹⁰;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or a pharmaceutically acceptable salt, hydrate, polymorph, tautomer, or solvate thereof.

In one embodiment, M is S; Q is CR⁵R⁶; T is CR⁵R⁶; X is absent; and Z is S. In one aspect, the disclosure concerns a compound of Formula (IX): 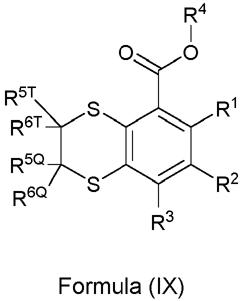 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of **H,** F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q} and R^{5T} are independently selected from the group consisting of H, deuterium, F and C₁₋₅ alkyl optionally substituted with one or more, identical or different, substituents R¹⁰;
- R^{6Q} and R^{6T} are independently selected from the group consisting of H, deuterium, F and C₁₋₅ alkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or a pharmaceutically acceptable salt, hydrate, polymorph, tautomer, or solvate thereof.

In one embodiment, M is NR⁷; Q is CR⁵R⁶; T is CR⁵R⁶; X is CR⁵R⁶; and Z is S. In one aspect, the disclosure concerns a compound of Formula (X): 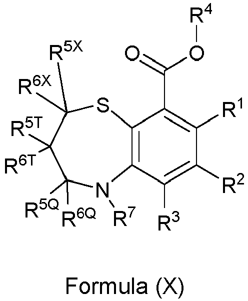 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of **H,** F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q}, R^{5T} and R^{5X} are independently selected from the group consisting of **H,** deuterium, F and C₁₋₅ alkyl optionally substituted with one or more, identical or different, substituents R¹⁰;
- R^{6Q}, R^{6T} and R^{6X} are independently selected from the group consisting of **H,** deuterium, F and C₁₋₅ alkyl optionally substituted with one or more, identical or different, substituents R¹⁰;
- R⁷ is independently selected from the group consisting of **H,** C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5X} and R^{6X} are individually C₁₋₅ alkyl optionally substituted with one or more, identical or different, substituents R¹⁰, then R^{5X} and R^{6X} are optionally joined together to form a ring;
   or a pharmaceutically acceptable salt, hydrate, polymorph, tautomer, or solvate thereof.

In one embodiment, M is NR⁷; Q is CR⁵R⁶; T is CR⁵R⁶; X is CR⁵R⁶; and Z is NR⁹. In one aspect, the disclosure concerns a compound of Formula (XI): 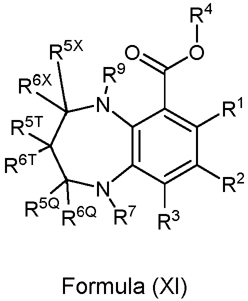 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of **H,** F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q}, R^{5T} and R^{5X} are independently selected from the group consisting of **H,** deuterium, F and C₁₋₅ alkyl optionally substituted with one or more, identical or different, substituents R¹⁰;
- R^{6Q}, R^{6T} and R^{6X} are independently selected from the group consisting of **H,** deuterium, F and C₁₋₅ alkyl optionally substituted with one or more, identical or different, substituents R¹⁰;
- R⁷ is independently selected from the group consisting of **H,** C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁹ is independently selected from the group consisting of **H,** C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R⁵° and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5X} and R^{6X} are individually C₁₋₅ alkyl optionally substituted with one or more, identical or different, substituents R¹⁰, then R^{5X} and R^{6X} are optionally joined together to form a ring;
   or a pharmaceutically acceptable salt, hydrate, polymorph, tautomer, or solvate thereof.

In one embodiment, M is O; Q is CR⁵R⁶; T is CR⁵R⁶; X is CR⁵R⁶; and Z is NR⁹. In one aspect, the disclosure concerns a compound of Formula (XII): 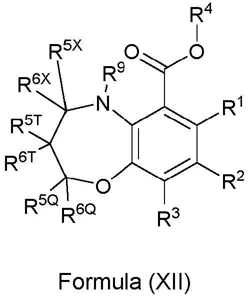 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q}, R^{5T} and R^{5X} are independently selected from the group consisting of H, deuterium, F and C₁₋₅ alkyl optionally substituted with one or more, identical or different, substituents R¹⁰;
- R^{6Q}, R^{6T} and R^{6X} are independently selected from the group consisting of H, deuterium, F and C₁₋₅ alkyl optionally substituted with one or more, identical or different, substituents R¹⁰;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5X} and R^{6X} are individually C₁₋₅ alkyl optionally substituted with one or more, identical or different, substituents R¹⁰, then R^{5X} and R^{6X} are optionally joined together to form a ring;
   or a pharmaceutically acceptable salt, hydrate, polymorph, tautomer, or solvate thereof.

In one embodiment, M is S; Q is CR⁵R⁶; T is CR⁵R⁶; X is CR⁵R⁶; and Z is NR⁹. In one aspect, the disclosure concerns a compound of Formula (XIII): 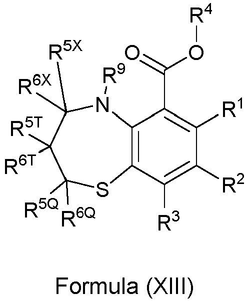 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q}, R^{5T} and R^{5X} are independently selected from the group consisting of H, deuterium, F and C₁₋₅ alkyl optionally substituted with one or more, identical or different, substituents R¹⁰;
- R^{6Q}, R^{6T} and R^{6X} are independently selected from the group consisting of H, deuterium, F and C₁₋₅ alkyl optionally substituted with one or more, identical or different, substituents R¹⁰;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5Q} and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5X} and R^{6X} are individually C₁₋₅ alkyl optionally substituted with one or more, identical or different, substituents R¹⁰, then R^{5X} and R^{6X} are optionally joined together to form a ring;
   or a pharmaceutically acceptable salt, hydrate, polymorph, tautomer, or solvate thereof.

In one embodiment, M is CR⁵R⁶; Q is NR^{8Q}; T is CR⁵R⁶; X is CR⁵R⁶; and Z is O. In one aspect, the disclosure concerns a compound of Formula (XIV): 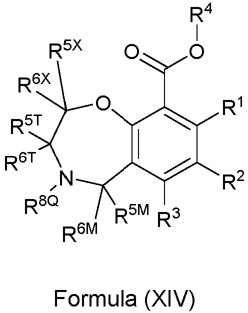 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5M}, R^{5T} and R^{5X} are independently selected from the group consisting of H, deuterium, F and C₁₋₅ alkyl optionally substituted with one or more, identical or different, substituents R¹⁰;
- R^{6M}, R^{6T} and R^{6X} are independently selected from the group consisting of H, deuterium, F and C₁₋₅ alkyl optionally substituted with one or more, identical or different, substituents R¹⁰;
- R^{8Q} is independently selected from benzyl optionally substituted with one or more, identical or different, substituents R¹¹;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R^{5M} and R^{6M} are individually C₁₋₅ alkyl optionally substituted with one or more, identical or different, substituents R¹⁰, then R^{5M} and R^{6M} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5X} and R^{6X} are individually C₁₋₅ alkyl optionally substituted with one or more, identical or different, substituents R¹⁰, then R^{5X} and R^{6X} are optionally joined together to form a ring;
   or a pharmaceutically acceptable salt, hydrate, polymorph, tautomer, or solvate thereof.

In one embodiment, M is O; Q is CR⁵R⁶; T is CR⁵R⁶; X is NR^{8X}; and Z is CR⁵R⁶. In one aspect, the disclosure concerns a compound of Formula (XV): 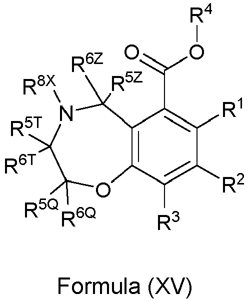 wherein:
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of **H,** F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R^{5Q}, R^{5T} and R^{5Z} are independently selected from the group consisting of H, deuterium, F and C₁₋₅ alkyl optionally substituted with one or more, identical or different, substituents R¹⁰;
- R^{6Q}, R^{6T} and R^{6Z} are independently selected from the group consisting of H, deuterium, F and C₁₋₅ alkyl optionally substituted with one or more, identical or different, substituents R¹⁰;
- R^{8X} is independently selected from benzyl optionally substituted with one or more, identical or different, substituents R¹¹;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵⁰ and R^{6Q} are individually C₁₋₅ alkyl optionally substituted with one or more, identical or different, substituents R¹⁰, then R^{5Q} and R^{6Q} are optionally joined together to form a ring;
   or when R^{5T} and R^{6T} are individually C₁₋₅ alkyl optionally substituted with one or more, identical or different, substituents R¹⁰, then R^{5T} and R^{6T} are optionally joined together to form a ring;
   or when R^{5Z} and R^{6Z} are individually C₁₋₅ alkyl optionally substituted with one or more, identical or different, substituents R¹⁰, then R^{5Z} and R^{6Z} are optionally joined together to form a ring;
   or a pharmaceutically acceptable salt, hydrate, polymorph, tautomer, or solvate thereof.

In one embodiment, R^{5M} is R⁵. In one embodiment, R⁵⁰ is R⁵. In one embodiment, R^{5T} is R⁵. In one embodiment, R^{5X} is R⁵. In one embodiment, R^{5Z} is R⁵. In one embodiment, R^{6M} is R⁶. In one embodiment, R^{6Q} is R⁶. In one embodiment, R^{6T} is R⁶. In one embodiment, R^{6X} is R⁶. In one embodiment, R^{6Z} is R⁶. In one embodiment, R^{8Q} is R⁸. In one embodiment, R^{8X} is R⁸.

In one embodiment, R' is F. In one embodiment, R¹ is Cl. In one embodiment, R¹ is Br. In one embodiment, R¹ is C₁₋₃ alkyl such as Me or Et. In one embodiment, R¹ is C₁₋₃ alkyl optionally substituted with one or more, identical or different, substituents R¹⁰, wherein R¹⁰ is independently selected from the group consisting of deuterium, F, Cl and -OC₁₋₃ alkyl. In one embodiment, R¹ is C₂₋₃ alkenyl. In one embodiment, R¹ is C₂₋₃ alkenyl optionally substituted with one or more, identical or different, substituents R¹⁰, wherein R¹⁰ is independently selected from the group consisting of deuterium, F, Cl and -OC₁₋₃ alkyl. In one embodiment, R¹ is -OC₁₋₃ alkyl, such as OMe, OEt or OiPr. In one embodiment, R¹ is -OC₁₋₃ alkyl, such as OMe. In one embodiment, R¹ is -OC₁₋₃ alkyl optionally substituted with one or more, identical or different, substituents R¹⁰, wherein R¹⁰ is independently selected from the group consisting of deuterium, F, Cl and -OC₁₋₃ alkyl. In one embodiment, R¹ is -OC₃₋₅ cycloalkyl optionally substituted with one or more, identical or different, substituents R'°, wherein R¹⁰ is independently selected from the group consisting of deuterium, F, Cl and -OC₁₋₃ alkyl. In one embodiment, R¹ is -SC₁₋₃ alkyl, such as SMe. In one embodiment, R¹ is -SC₁₋₃ alkyl optionally substituted with one or more, identical or different, substituents R'°, wherein R¹⁰ is independently selected from the group consisting of deuterium, F, Cl and -OC₁₋₃ alkyl. In one embodiment, R¹ is -SC₃₋₅ cycloalkyl optionally substituted with one or more, identical or different, substituents R¹⁰, wherein R¹⁰ is independently selected from the group consisting of deuterium, F, Cl and -OC₁₋₃ alkyl. In one embodiment, R¹ is selected from the group consisting of F, Cl, Me, OMe, OEt, OCHMe₂ and SMe. In one embodiment, R¹ is selected from the group consisting of F, Cl and OMe.

In one embodiment, R² is H. In one embodiment, R² is F. In one embodiment, R² is CI. In one embodiment, R² is Br. In one embodiment, R² is C₁₋₃ alkyl, such as Me. In one embodiment, R² is C₁₋₃ alkyl substituted with one or more, identical or different substituents R'°, wherein R¹⁰ is independently selected from the group consisting of deuterium, F, Cl and -OC₁₋₃ alkyl. In one embodiment, R² is selected from the group consisting of H, F, Cl and Me. In one embodiment, R² is selected from the group consisting of F and CI.

In one embodiment, R¹ is OMe and R² is F or Cl. In one embodiment, R¹ is F and R² is Cl.

In one embodiment, R³ is H. In one embodiment, R³ is F. In one embodiment, R³ is CI.

In one embodiment, R⁴ is H. In one embodiment, R⁴ is C₁₋₅ alkyl. In one embodiment, R⁴ is C₁₋₅ alkyl substituted with one or more, identical or different substituents R¹⁰, wherein R¹⁰ is independently selected from the group consisting of deuterium, F, Cl and -OC₁₋₃ alkyl. In one embodiment, R⁴ is C₂₋₅ alkenyl. In one embodiment, R⁴ is C₂₋₅ alkenyl substituted with one or more, identical or different substituents R¹⁰, wherein R¹⁰ is independently selected from the group consisting of deuterium, F, Cl and -OC₁₋₃ alkyl. In one embodiment, R⁴ is C₂₋₅ alkynyl. In one embodiment, R⁴ is C₂₋₅ alkynyl substituted with one or more, identical or different substituents R'°, wherein R¹⁰ is independently selected from the group consisting of deuterium, F, Cl and -OC₁₋₃ alkyl. In one embodiment, R⁴ is C₃₋₆ cycloalkyl. In one embodiment, R⁴ is C₃₋₆ cycloalkyl substituted with one or more, identical or different substituents R'°, wherein R¹⁰ is independently selected from the group consisting of deuterium, F, Cl and -OC₁₋₃ alkyl. In one embodiment, R⁴ is phenyl. In one embodiment, R⁴ is phenyl substituted with one or more, identical or different substituents R¹¹, wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁴ is benzyl. In one embodiment, R⁴ is benzyl substituted with one or more, identical or different substituents R¹¹, wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F.

In one embodiment, R⁵ is H. In one embodiment, R⁵ is deuterium. In one embodiment, R⁵ is F. In one embodiment, R⁵ is C₁₋₅ alkyl, such as Me, Et or "Pr. In one embodiment, R⁵ is C₁₋₅ alkyl substituted with one or more, identical or different, substituents R¹⁰, wherein R¹⁰ is independently selected from the group consisting of deuterium, F, Cl and -OC₁₋₃ alkyl.

In one embodiment, R⁶ is H. In one embodiment, R⁶ is deuterium. In one embodiment, R⁶ is F. In one embodiment, R⁶ is C₁₋₅ alkyl, such as Me, Et or "Pr. In one embodiment, R⁶ is C₁₋₅ alkyl substituted with one or more, identical or different, substituents R¹⁰, wherein R¹⁰ is independently selected from the group consisting of deuterium, F, Cl and -OC₁₋₃ alkyl.

In one embodiment, R⁵ is C₁₋₅ alkyl substituted with one or more, identical or different, substituents R'°, and R⁶ is C₁₋₅ alkyl substituted with one or more, identical or different, substituents R¹⁰, and R⁵ and R⁶ are joined together to form a ring. When R⁵ and R⁶ are joined together to form a ring, R⁵ and R⁶ are C₁₋₅ alkanediyls, substituted with one or more, identical or different, substituents R¹⁰.

In one embodiment, R⁵ is C₁ alkyl substituted with one or more, identical or different, substituents R'°, and R⁶ is C₁ alkyl substituted with one or more, identical or different, substituents R¹⁰, and R⁵ and R⁶ are joined together to form a ring. In one embodiment, -R⁵-R⁶- is -C(R¹⁰)₂C(R¹⁰)₂-, such as -CH₂CH₂-.

In one embodiment, R⁵ is C₂ alkyl substituted with one or more, identical or different, substituents R'°, and R⁶ is C₁ alkyl substituted with one or more, identical or different, substituents R¹⁰, and R⁵ and R⁶ are joined together to form a ring. In one embodiment, -R⁵-R⁶- is -C(R¹⁰)₂C(R¹⁰)₂C(R¹⁰)₂-, such as -CH₂CH₂CH₂-.

In one embodiment, R⁵ is C₂ alkyl substituted with one or more, identical or different, substituents R'°, and R⁶ is C₂ alkyl substituted with one or more, identical or different, substituents R¹⁰, and R⁵ and R⁶ are joined together to form a ring. In one embodiment, -R⁵-R⁶- is -C(R¹⁰)₂C(R¹⁰)₂C(R¹⁰)₂C(R¹⁰)₂-, such as -CH₂CH₂CH₂CH₂-.

In one embodiment, R⁵ and R⁶ are bound to the same carbon atom and joined together to form a ring, thus forming a spirocyclic ring of Formula (XVI):

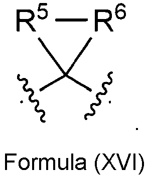

In one embodiment, M is CR⁵R⁶ wherein said R⁵ and R⁶ are joined together to form a spriocyclic ring, and -R⁵-R⁶- is -C(R¹⁰)₂C(R¹⁰)₂-, such
as -CH₂CH₂-, -C(R¹⁰)₂C(R¹⁰)₂C(R¹⁰)₂-, such as -CH₂CH₂CH₂-,
or -C(R¹⁰)₂C(R¹⁰)₂C(R¹⁰)₂C(R¹⁰)₂-, such as -CH₂CH₂CH₂CH₂-.

In one embodiment, R^{5M} and R^{6M} are joined together to form a spriocyclic ring, and - R^{5m}-R ^{6M}- is -C(R¹⁰)₂C(R¹⁰)₂-, such as -CH₂CH₂-. In one embodiment, R^{5M} and R^{6M} are joined together to form a spriocyclic ring, and -R^{5M}-R^{6M}- is -C(R¹⁰)₂C(R¹⁰)₂C(R¹⁰)₂-, such as -CH₂CH₂CH₂-. In one embodiment, R^{5M} and R^{6M} are joined together to form a spriocyclic ring, and -R^{5m}-R^{6M}- is -C(R¹⁰)₂C(R¹⁰)₂C(R¹⁰)₂C(R¹⁰)₂-, such as -CH₂CH₂CH₂CH₂-.

In one embodiment, Q is CR⁵R⁶ wherein said R⁵ and R⁶ are joined together to form a spriocyclic ring, and -R⁵-R⁶- is -C(R¹⁰)₂C(R¹⁰)₂-, such
as -CH₂CH₂-, -C(R¹⁰)₂C(R¹⁰)₂C(R¹⁰)₂-, such as -CH₂CH₂CH₂-,
or -C(R¹⁰)₂C(R¹⁰)₂C(R¹⁰)₂C(R¹⁰)₂-, such as -CH₂CH₂CH₂CH₂-.

In one embodiment, R^{5Q} and R^{6Q} are joined together to form a spriocyclic ring, and - R^{5Q}-R^{6Q}- is -C(R¹⁰)₂C(R¹⁰)₂-, such as -CH₂CH₂-. In one embodiment, R^{5Q} and R^{6Q} are joined together to form a spriocyclic ring, and -R^{5Q}-R^{6Q}- is -C(R¹⁰)₂C(R¹⁰)₂C(R¹⁰)₂-, such as -CH₂CH₂CH₂-. In one embodiment, R^{5Q} and R^{6Q} are joined together to form a spriocyclic ring, and -R^{5Q}-R^{6Q}- is -C(R¹⁰)₂C(R¹⁰)₂C(R¹⁰)₂C(R¹⁰)₂-, such as -CH₂CH₂CH₂CH₂-.

In one embodiment, T is CR⁵R⁶ wherein said R⁵ and R⁶ are joined together to form a spriocyclic ring, and -R⁵-R⁶- is -C(R¹⁰)₂C(R¹⁰)₂-, such
as -CH₂CH₂-, -C(R¹⁰)₂C(R¹⁰)₂C(R¹⁰)₂-, such as -CH₂CH₂CH₂-,
or -C(R¹⁰)₂C(R¹⁰)₂C(R¹⁰)₂C(R¹⁰)₂-, such as -CH₂CH₂CH₂CH₂-.

In one embodiment, R^{5T} and R^{6T} are joined together to form a spriocyclic ring, and -R^{5T}-R^{6T}- is -C(R¹⁰)₂C(R¹⁰)₂-, such as -CH₂CH₂-. In one embodiment, R^{5T} and R^{6T} are joined together to form a spriocyclic ring, and -R^{5T}-R^{6T}- is -C(R¹⁰)₂C(R¹⁰)₂C(R¹⁰)₂-, such as -CH₂CH₂CH₂-. In one embodiment, R^{5T} and R^{6T} are joined together to form a spriocyclic ring, and -R^{5T}-R^{6T}- is -C(R¹⁰)₂C(R¹⁰)₂C(R¹⁰)₂C(R¹⁰)₂-, such as -CH₂CH₂CH₂CH₂-.

In one embodiment, X is CR⁵R⁶ wherein said R⁵ and R⁶ are joined together to form a spriocyclic ring, and -R⁵-R⁶- is -C(R¹⁰)₂C(R¹⁰)₂-, such
as -CH₂CH₂-, -C(R¹⁰)₂C(R¹⁰)₂C(R¹⁰)₂-, such as -CH₂CH₂CH₂-,
or -C(R¹⁰)₂C(R¹⁰)₂C(R¹⁰)₂C(R¹⁰)₂-, such as -CH₂CH₂CH₂CH₂-.

In one embodiment, R^{5X} and R^{6X} are joined together to form a spriocyclic ring, and - R^{5X}-R^{6X}- is -C(R¹⁰)₂C(R¹⁰)₂-, such as -CH₂CH₂-. In one embodiment, R^{5X} and R^{6X} are joined together to form a spriocyclic ring, and -R^{5X}-R^{6X}- is -C(R¹⁰)₂C(R¹⁰)₂C(R¹⁰)₂-, such as -CH₂CH₂CH₂-. In one embodiment, R^{5X} and R^{6X} are joined together to form a spriocyclic ring, and -R^{5X}-R^{6X}- is -C(R¹⁰)₂C(R¹⁰)₂C(R¹⁰)₂C(R¹⁰)₂-, such as -CH₂CH₂CH₂CH₂-.

In one embodiment, Z is CR⁵R⁶ wherein said R⁵ and R⁶ are joined together to form a spriocyclic ring, and -R⁵-R⁶- is -C(R¹⁰)₂C(R¹⁰)₂-, such
as -CH₂CH₂-, -C(R¹⁰)₂C(R¹⁰)₂C(R¹⁰)₂-, such as -CH₂CH₂CH₂-,
or -C(R¹⁰)₂C(R¹⁰)₂C(R¹⁰)₂C(R¹⁰)₂-, such as -CH₂CH₂CH₂CH₂-.

In one embodiment, R^{5Z} and R^{6Z} are joined together to form a spriocyclic ring, and -R^{5Z}-R^{6Z}- is -C(R¹⁰)₂C(R¹⁰)₂-, such as -CH₂CH₂-. In one embodiment, R^{5Z} and R^{6Z} are joined together to form a spriocyclic ring, and -R^{5Z}-R^{6Z}- is -C(R¹⁰)₂C(R¹⁰)₂C(R¹⁰)₂-, such as -CH₂CH₂CH₂-. In one embodiment, R^{5Z} and R^{6Z} are joined together to form a spriocyclic ring, and -R^{5Z}-R^{6Z}- is -C(R¹⁰)₂C(R¹⁰)₂C(R¹⁰)₂C(R¹⁰)₂-, such as -CH₂CH₂CH₂CH₂-.

In one embodiment, R⁷ is H. In one embodiment, R⁷ is C₁₋₅ alkyl, such as Me, Et or "Pr. In one embodiment, R⁷ is C₁₋₅ alkyl substituted with one or more, identical or different, substituents R'°, wherein R¹⁰ is independently selected from the group consisting of F; Cl; and -OC₁₋₃ alkyl.

In one embodiment, R⁷ is C₁₋₃ alkyl substituted with phenyl. In one embodiment, R⁷ is benzyl. In one embodiment, R⁷ is phenylethyl. In one embodiment, R⁷ is phenylpropyl. In one embodiment, R⁷ is C₁₋₃ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁷ is C₁ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁷ is benzyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁷ is C₂ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁷ is phenylethyl substituted with one or more, identical or different, substituents R¹¹, wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁷ is C₃ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁷ is phenylpropyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F.

In one embodiment, R⁷ is C₁₋₃ alkyl substituted with a C₅ heteroaryl. In one embodiment, R⁷ is C₁₋₃ alkyl substituted with a C₅ heteroaryl wherein the C₅ heteroaryl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁷ is (thiophen-2-yl)meth-1-yl. In one embodiment, R⁷ is (thiophen-3-yl)meth-1-yl. In one embodiment, R⁷ is (furan-2-yl)meth-1-yl. In one embodiment R⁷ is (furan-3-yl)meth-1-yl. In one embodiment, R⁷ is (1,3-thiazol-2-yl)meth-1-yl. In one embodiment, R⁷ is(1,3-oxazol-2-yl)meth-1-yl. In one embodiment, R⁷ is (thiophen-2-yl)eth-2-yl. In one embodiment, R⁷ is (thiophen-3-yl)eth-2-yl. In one embodiment, R⁷ is (furan-2-yl)eth-2-yl. In one embodiment, R⁷ is (furan-3-yl)eth-2-yl. In one embodiment, R⁷ is (1,3-thiazol-2-yl)eth-2-yl. In one embodiment, R⁷ is (1,3-oxazol-2-yl)eth-2-yl. In one embodiment, R⁷ is (thiophen-2-yl)prop-3-yl. In one embodiment, R⁷ is (thiophen-3-yl)prop-3-yl. In one embodiment, R⁷ is (furan-2-yl)prop-3-yl. In one embodiment, R⁷ is (furan-3-yl)prop-3-yl. In one embodiment, R⁷ is (1,3-thiazol-2-yl)prop-3-yl. In one embodiment, R⁷ is (1,3-oxazol-2-yl)prop-3-yl.

In one embodiment, R⁷ is C₁₋₃ alkyl substituted with C₃₋₅ cycloalkyl. In one embodiment, R⁷ is C₁₋₃ alkyl substituted with C₃₋₅ cycloalkyl substituted with one or more, identical or different, substituents R¹⁰, wherein R¹⁰ is independently selected from the group consisting of F; Cl; and -OC₁₋₃ alkyl. In one embodiment, R⁷ is cyclopropylmethyl. In one embodiment, R⁷ is cyclopropylethyl. In one embodiment, R⁷ is cyclobutylmethyl.

In one embodiment, R⁷ is C₃₋₅ cycloalkyl. In one embodiment, R⁷ is C₃₋₅ cycloalkyl optionally substituted with one or more, identical or different, substituents R'°, wherein R¹⁰ is independently selected from the group consisting of deuterium, F, Cl and -OC₁₋₃ alkyl. In one embodiment, R⁷ is cyclopropyl. In one embodiment, R⁷ is cyclobutyl. In one embodiment, R⁷ is cyclopentyl.

In one embodiment, R³ is benzyl. In one embodiment, R⁸ is benzyl substituted with one or more, identical or different, substituents R¹¹, wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F.

In one embodiment, R⁹ is H. In one embodiment, R⁹ is C₁₋₅ alkyl, such as Me, Et or "Pr. In one embodiment, R⁹ is C₁₋₅ alkyl substituted with one or more, identical or different, substituents R'°, wherein R¹⁰ is independently selected from the group consisting of F; Cl; and -OC₁₋₃ alkyl.

In one embodiment, R⁹ is C₁₋₃ alkyl substituted with C₃₋₅ cycloalkyl. In one embodiment, R⁹ is C₁₋₃ alkyl substituted with C₃₋₅ cycloalkyl optionally substituted with one or more, identical or different, substituents R'°, wherein R¹⁰ is independently selected from the group consisting of F; Cl; and -OC₁₋₃ alkyl. In one embodiment, R⁹ is cyclopropylmethyl. In one embodiment, R⁹ is cyclopropylethyl. In one embodiment, R⁷ is cyclobutylmethyl.

In one embodiment, R⁹ is C₁₋₃ alkyl substituted with phenyl, such as benzyl, phenylethyl or phenylpropyl. In one embodiment, R⁹ is C₁₋₃ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is C₁ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is benzyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is C₂ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is phenylethyl substituted with one or more, identical or different, substituents R¹¹, wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁹ is C₃ alkyl substituted with phenyl, wherein the phenyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁹ is phenylpropyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F.

In one embodiment, R⁹ is C₁₋₃ alkyl substituted with naphthyl, such as (1-naphthyl)methyl, (2-naphthyl)methyl, (1-naphthyl)ethyl, (2-naphthyl)ethyl, (1-naphthyl)propyl, (2-naphthyl)propyl. In one embodiment, R⁹ is C₁₋₃ alkyl substituted with naphthyl, wherein the naphthyl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is (1-naphthyl)methyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is (2-naphthyl)methyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is (1-naphthyl)ethyl substituted with one or more, identical or different, substituents R¹¹, wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁹ is (2-naphthyl)ethyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is (1-naphthyl)propyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is (2-naphthyl)propyl substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F.

In one embodiment, R⁹ is C₁₋₃ alkyl substituted with a C₅ heterocycle. In one embodiment, R⁹ is C₁₋₃ alkyl substituted with a C₅ heterocycle wherein the C₅ heterocycle is substituted with one or more, identical or different, substituents R¹¹ wherein R¹¹ is independently selected from the group consisting of deuterium, methoxy, nitro, cyano, CI, Br, I, and F. In one embodiment, R⁹ is C₁₋₃ alkyl substituted with a 5- to 10-membered heteroaryl. In one embodiment, R⁹ is C₁₋₃ alkyl substituted with a 5- to 10-membered heteroaryl wherein the 5- to 10-membered heteroaryl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, Cl, Br, I, and F. In one embodiment, R⁹ is C₁₋₃ alkyl substituted with a 5- to 10-membered heteroaryl selected from the group consisting of oxazolyl, thiazolyl, thiadiazolyl, oxadiazolyl, triazolyl, pyrrolyl, thienyl, furanyl, diazolyl, imidazolyl, pyrazolyl, isoxazolyl, isothiazolyl, and tetrazolyl, pyridinyl, pyridazinyl, pyrimidinyl, pyrazinyl, triazinyl, 1,4-dihydropyrrolo[3,2-b]pyrrolyl, 1,6-dihydropyrrolo[2,3-b]pyrrolyl, 6H-furo[2,3-b]pyrrole, 4H-furo[2,3-b]pyrrolyl, 6H-thieno[2,3-b]pyrrolyl, 4H-thieno[2,3-b]pyrrolyl, indolyl, isoindolyl, indolizinyl, indazolyl, benzimidazolyl, azaindolyl, azaindazolyl, pyrazolo[1,5-a]pyrimidinyl, purinyl, benzofuranyl, benzo[b]thiophenyl, benzisoxazolyl, benzthiazolyl, benzisothiazolyl, benzo[c][1,2,5]thiadiazolyl, quinolinyl, isoquinolinyl, 4H-quinolizinyl, quinoxalinyl, phthalazinyl, quinazolinyl, cinnolinyl, 1,8-naphthyridinyl, pyrido[3,2-d]pyrimidinyl, pyrido[4,3-d]pyrimidinyl, pyrido[3,4-b]pyrazinyl, pyrido[2,3-b]pyrazinyl and pteridinyl. In one embodiment, R⁹ is C₁₋₃ alkyl substituted with a 5-membered heteroaryl wherein the 5-membered heteroaryl is substituted with one or more, identical or different, substituents R¹¹ wherein R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F. In one embodiment, R⁹ is (thiophen-2-yl)meth-1-yl. In one embodiment, R⁹ is (thiophen-3-yl)meth-1-yl. In one embodiment, R⁹ is (furan-2-yl)meth-1-yl. In one embodiment, R⁹ is (furan-3-yl)meth-1-yl. In one embodiment, R⁹ is (1,3-thiazol-2-yl)meth-1-yl. In one embodiment, R⁹ is (1,3-oxazol-2-yl)meth-1-yl. In one embodiment, R⁹ is (thiophen-2-yl)eth-2-yl. In one embodiment, R⁹ is (thiophen-3-yl)eth-2-yl. In one embodiment, R⁹ is (furan-2-yl)eth-2-yl. In one embodiment, R⁹ is (furan-3-yl)eth-2-yl. In one embodiment, R⁹ is (1,3-thiazol-2-yl)eth-2-yl. In one embodiment, R⁹ is (1,3-oxazol-2-yl)eth-2-yl. In one embodiment, R⁹ is (thiophen-2-yl)prop-3-yl. In one embodiment, R⁹ is (thiophen-3-yl)prop-3-yl. In one embodiment, R⁹ is (furan-2-yl)prop-3-yl. In one embodiment, R⁹ is (furan-3-yl)prop-3-yl. In one embodiment, R⁹ is (1,3-thiazol-2-yl)prop-3-yl. In one embodiment, R⁹ is (1,3-oxazol-2-yl)prop-3-yl.

In one embodiment, R⁹ is C₃₋₅ cycloalkyl optionally substituted with one or more, identical or different, substituents R'°, wherein R¹⁰ is independently selected from the group consisting of deuterium, F, Cl and -OC₁₋₃ alkyl. In one embodiment, R⁹ is C₃₋₅ cycloalkyl. In one embodiment, R⁹ is cyclopropyl. In one embodiment, R⁹ is cyclobutyl. In one embodiment, R⁹ is cyclopentyl.

In one embodiment, R¹⁰ is deuterium. In one embodiment, R¹⁰ is F. In one embodiment, R¹⁰ is CI. In one embodiment, R¹⁰ is -OC₁₋₃ alkyl such as OMe.

In one embodiment, R¹¹ is deuterium. In one embodiment, R¹¹ is methoxy. In one embodiment, R¹¹ is -OCF₃. In one embodiment, R¹¹ is Me. In one embodiment, R¹¹ is CF₃. In one embodiment, R¹¹ is CF₂Cl. In one embodiment, R¹¹ is CF₂H. In one embodiment, R¹¹ is CFH₂. In one embodiment, R¹¹ is CD₃. In one embodiment, R¹¹ is cyclopropyl. In one embodiment, R¹¹ is NH₂. In one embodiment, R¹¹ is -NHAc. In one embodiment, R¹¹ is -C(=O)-NH₂. In one embodiment, R¹¹ is nitro. In one embodiment, R¹¹ is cyano. In one embodiment, R¹¹ is Cl. In one embodiment, R¹¹ is Br. In one embodiment, R¹¹ is I. In one embodiment, R¹¹ is F.

In one embodiment, the compound is selected from the group consisting of:
6-chloro-7-methoxy-4-methyl-3,4-dihydro-2H-1,4-benzoxazine-8-carboxylic acid;
7-chloro-6-fluoro-4-methyl-3,4-dihydro-2H-1,4-benzoxazine-5-carboxylic acid;
7-chloro-6-fluoro-4-methyl-3,4-dihydro-2H-1,4-benzoxazine-8-carboxylic acid;
5-chloro-7-fluoro-3,4-dihydro-2H-1,4-benzoxazine-8-carboxylic acid;
6-chloro-7-fluoro-3,4-dihydro-2H-1,4-benzoxazine-8-carboxylic acid;
6-chloro-7-fluoro-4-methyl-3,4-dihydro-2H-1,4-benzoxazine-8-carboxylic acid;
7-chloro-6-fluoro-1,4-dimethyl-1,2,3,4-tetrahydroquinoxaline-5-carboxylic acid;
6-chloro-7-fluoro-2,2,4-trimethyl-3,4-dihydro-2H-1,4-benzoxazine-8-carboxylic acid;
7-chloro-6-methoxy-4-methyl-3,4-dihydro-2H-1,4-benzoxazine-5-carboxylic acid;
7-chloro-6-fluoro-3,4-dihydro-2H-1,4-benzoxazine-5-carboxylic acid;
7-chloro-8-methoxy-5-methyl-2,3,4,5-tetrahydro-1,5-benzoxazepine-9-carboxylic acid;
7-chloro-8-fluoro-5-methyl-2,3,4,5-tetrahydro-1,5-benzoxazepine-9-carboxylic acid;
6-chloro-7-methoxy-3,4-dihydro-2H-1,4-benzoxazine-8-carboxylic acid;
7-chloro-8-fluoro-2,3,4,5-tetrahydro-1,5-benzoxazepine-9-carboxylic acid;
7-chloro-8-ethoxy-5-methyl-2,3,4,5-tetrahydro-1,5-benzoxazepine-9-carboxylic acid;
6-chloro-7-fluoro-4-(3-methylbutyl)-3,4-dihydro-2H-1,4-benzoxazine-8-carboxylic acid;
6-chloro-4-ethyl-7-fluoro-3,4-dihydro-2H-1,4-benzoxazine-8-carboxylic acid;
6-chloro-7-fluoro-4-methyl-3,4-dihydro-2H-1,4-benzothiazine-8-carboxylic acid;
6-chloro-4-(cyclopropylmethyl)-7-fluoro-3,4-dihydro-2H-1,4-benzoxazine-8-carboxylic acid;
6-chloro-7-fluoro-4-propyl-3,4-dihydro-2H-1,4-benzoxazine-8-carboxylic acid;
4-benzyl-6-chloro-7-fluoro-3,4-dihydro-2H-1,4-benzoxazine-8-carboxylic acid;
6-chloro-7-fluoro-4-[3-(4-fluorophenyl)propyl]-3,4-dihydro-2H-1,4-benzoxazine-8-carboxylic acid;
6-chloro-4-methyl-7-(propan-2-yloxy)-3,4-dihydro-2H-1,4-benzoxazine-8-carboxylic acid;
6-chloro-7-fluoro-4-[2-(4-fluorophenyl)ethyl]-3,4-dihydro-2H-1,4-benzoxazine-8-carboxylic acid;
6-chloro-4-methyl-7-(methylsulfanyl)-3,4-dihydro-2H-1,4-benzoxazine-8-carboxylic acid;
6-chloro-7-fluoro-4-(3-methoxypropyl)-3,4-dihydro-2H-1,4-benzoxazine-8-carboxylic acid;
6-chloro-4-ethyl-7-methoxy-3,4-dihydro-2H-1,4-benzoxazine-8-carboxylic acid;
6-chloro-7-methoxy-4-methyl-3,4-dihydro-2H-1,4-benzothiazine-8-carboxylic acid;
6-fluoro-7-methoxy-4-methyl-3,4-dihydro-2H-1,4-benzoxazine-8-carboxylic acid;
7-methoxy-4,6-dimethyl-3,4-dihydro-2H-1,4-benzoxazine-8-carboxylic acid;
6-chloro-7-methoxy-4-(propan-2-yl)-3,4-dihydro-2H-1,4-benzoxazine-8-carboxylic acid;
6-chloro-7-methoxy-4-(2-phenylethyl)-3,4-dihydro-2H-1,4-benzoxazine-8-carboxylic acid;
6-chloro-7-methoxy-4-[2-(4-methoxyphenyl)ethyl]-3,4-dihydro-2H-1,4-benzoxazine-8-carboxylic acid;
6-chloro-7-methoxy-2-(methoxymethyl)-4-methyl-3,4-dihydro-2H-1,4-benzoxazine-8-carboxylic acid;
6-chloro-7-methoxy-4-[(thiophen-3-yl)methyl]-3,4-dihydro-2H-1,4-benzoxazine-8-carboxylic acid;
6-chloro-7-methoxy-2,2,4-trimethyl-3,4-dihydro-2H-1,4-benzoxazine-8-carboxylic acid;
6-chloro-7-fluoro-4-(2-methoxyethyl)-3,4-dihydro-2H-1,4-benzoxazine-8-carboxylic acid;
6-chloro-7-methoxy-4-methyl-3,4-dihydrospiro[1,4-benzoxazine-2,1'-cyclobutane]-8-carboxylic acid;
6-chloro-4-[3-(furan-2-yl)propyl]-7-methoxy-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(1,3-thiazol-2-yl)methyl]-3,4-dihydro-2H-1,4-benzoxazine-8-carboxylic acid;
6-chloro-5-fluoro-7-methoxy-4-methyl-3,4-dihydro-2H-1,4-benzoxazine-8-carboxylic acid;
4-benzyl-7-chloro-6-fluoro-3,4-dihydro-2H-1,4-benzoxazine-5-carboxylic acid; 6-chloro-4-(cyclobutylmethyl)-7-fluoro-3,4-dihydro-2H-1,4-benzoxazine-8-carboxylic acid;
6-chloro-7-methoxy-4-[3-(1,3-thiazol-2-yl)propyl]-3,4-dihydro-2H-1,4-benzoxazine-8-carboxylic acid;
6-chloro-4,7-dimethyl-3,4-dihydro-2H-1,4-benzoxazine-8-carboxylic acid;
6-chloro-7-methoxy-4-[(4-methoxyphenyl)methyl]-3,4-dihydro-2H-1,4-benzoxazine-8-carboxylic acid;
6-chloro-7-methoxy-4-[(1,3-oxazol-2-yl)methyl]-3,4-dihydro-2H-1,4-benzoxazine-8-carboxylic acid;
7-chloro-6-fluoro-4-(3-methylbutyl)-3,4-dihydro-2H-1,4-benzoxazine-5-carboxylic acid;
4-benzyl-6-chloro-7-methoxy-3,4-dihydro-2H-1,4-benzoxazine-8-carboxylic acid;
6-chloro-4-[2-(3,5-difluorophenyl)ethyl]-7-methoxy-3,4-dihydro-2H-1,4-benzoxazine-8-carboxylic acid;
7-chloro-6-fluoro-4-[(thiophen-3-yl)methyl]-3,4-dihydro-2H-1,4-benzoxazine-5-carboxylic acid;
4-benzyl-6-fluoro-7-methoxy-3,4-dihydro-2H-1,4-benzoxazine-8-carboxylic acid;
methyl 6-chloro-7-methoxy-3,4-dihydro-2H-1,4-benzoxazine-8-carboxylate;
ethyl 6-chloro-7-fluoro-3,4-dihydro-2H-1,4-benzoxazine-8-carboxylate;
methyl 7-chloro-6-fluoro-3,4-dihydro-2H-1,4-benzoxazine-5-carboxylate;
methyl 6-chloro-7-fluoro-3,4-dihydro-2H-1,4-benzoxazine-8-carboxylate;
7-chloro-6-fluoro-4-propyl-3,4-dihydro-2H-1,4-benzoxazine-5-carboxylic acid;
7-chloro-6-fluoro-4-methyl-1,2,3,4-tetrahydro-5-quinoxalinecarboxylic acid;
7-chloro-6-fluoro-4-[(p-methoxyphenyl)methyl]-3,4-dihydro-2H-1,4-benzoxazine-5-carboxylic acid;
1-benzyl-7-chloro-6-fluoro-4-methyl-1,2,3,4-tetrahydro-5-quinoxalinecarboxylic acid;
7-chloro-6-fluoro-4-[(p-tolyl)methyl]-3,4-dihydro-2H-1,4-benzoxazine-5-carboxylic acid;
7-chloro-6-fluoro-4-[(1-naphthyl)methyl]-3,4-dihydro-2H-1,4-benzoxazine-5-carboxylic acid;
7-chloro-6-fluoro-4-[(o-tolyl)methyl]-3,4-dihydro-2H-1,4-benzoxazine-5-carboxylic acid; and
4-benzyl-6,7-dichloro-3,4-dihydro-2H-1,4-benzoxazine-8-carboxylic acid.

In one embodiment, the compound or the compound for use according to the present disclosure has been modified in order to increase its half-life when administered to a patient, in particular its plasma half-life. In one embodiment, the compound or the compound for use according to the present disclosure further comprises a moiety conjugated to said compound, thus generating a moiety-conjugated compound. In one embodiment, said moiety-conjugated compound has a plasma and/or serum half-life being longer than the plasma and/or serum half-life of the non-moiety conjugated compound. In one embodiment, the moiety conjugated to the compound or compound for use according to the present disclosure, is one or more type(s) of moieties selected from the group consisting of albumin, fatty acids, polyethylene glycol (PEG), acylation groups, antibodies and antibody fragments.

In one embodiment, the compound has activity on the CIC-1 receptor. In one embodiment, the compound is an inhibitor of the CIC-1 ion channel.

In one embodiment, the compound is capable of improving the recovered force in isolated rat soleus muscles after exposure to tubocurarine. In one embodiment, the recovery of force in muscles with neuromuscular dysfunction is >5%, for example >10%, for example >15%, for example >20%, for example >25%, for example >30% and for example >35%.

### Neuromuscular disorders

The compound or compound for use of the present disclosure may be used for treating, ameliorating and/or preventing a neuromuscular disorder, or reversing neuromuscular blockade.

The inventors of the present disclosure have shown that inhibition of CIC-1 channels strengthens neuromuscular transmission. CIC-1 function may therefore contribute to muscle weakness in conditions of compromised neuromuscular transmission.

Thus, in one embodiment of the present disclosure, the compound or the compound for use as described herein inhibits CIC-1 channels. Thus, it is appreciated that compounds and/or compounds for use of Formula (I) inhibit CIC-1 channels.

The neuromuscular disorder may also include neuromuscular dysfunctions.

Neuromuscular disorders include for example disorders with symptoms of muscle weakness and fatigue. Such disorders may include conditions with reduced neuromuscular transmission safety factor. In one embodiment the neuromuscular disorders are motor neuron disorders. Motor neuron disorders are disorders with reduced safety in the neuromuscular transmission. In one embodiment motor neuron disorders are selected from the group consisting of amyotrophic lateral sclerosis (ALS) (Killian JM, Wilfong AA, Burnett L, Appel SH, Boland D. Decremental motor responses to repetitive nerve stimulation in ALS. Muscle Nerve, 1994, 17, 747-754), spinal muscular atrophy (SMA) (Wadman RI, Vrancken AF, van den Berg LH, van der Pol WL. Dysfunction of the neuromuscular junction in spinal muscular atrophy types 2 and 3. Neurology, 2012, 79, 2050-2055), Charcot-Marie Tooth disease (Bansagi B, Griffin H, Whittaker RG, Antoniadi T, Evangelista T, Miller J, Greenslade M, Forester N, Duff J, Bradshaw A, Kleinle S, Boczonadi V, Steele H, Ramesh V, Franko E, Pyle A, Lochmüller H, Chinnery PF, Horvath R. Genetic heterogeneity of motor neuropathies. Neurology, 2017, 28;88(13):1226-1234), X-linked spinal and bulbar muscular atrophy (Yamada, M., Inaba, A., Shiojiri, T. X-linked spinal and bulbar muscular atrophy with myasthenic symptoms. Journal of the Neurological Sciences, 1997, 146, 183-185), Kennedy's disorder (Stevic, Z., Peric, S., Pavlovic, S., Basta, I., Lavrnic, D., Myasthenic symptoms in a patient with Kennedy's disorder. Acta Neurologica Belgica, 2014, 114, 71-73), multifocal motor neuropathy (Roberts, M., Willison, H.J., Vincent, A., Newsom-Davis, J. Multifocal motor neuropathy human sera block distal motor nerve conduction in mice. Ann Neurol. 1995, 38, 111-118), Guillain-Barré syndrome (Ansar, V., Valadi, N. Guillain-Barré Syndrome Prim. Care, 2015, 42, 189-193); poliomyelitis (Trojan, D.A., Gendron, D., Cashman, N.R. Electrophysiology and electrodiagnosis of the post-polio motor unit. Orthopedics, 1991, 14, 1353-1361, and Birk T.J. Poliomyelitis and the post-polio syndrome: exercise capacities and adaptation - current research, future directions, and widespread applicability. Med. Sci. Sports Exerc., 1993, 25, 466-472), post-polio syndrome (Garcia, C.C., Potian, J.G., Hognason, K., Thyagarajan, B., Sultatos, L.G., Souayah, N., Routh, V.H., McArdle, J.J. Acetylcholinesterase deficiency contributes to neuromuscular junction dysfunction in type 1 diabetic neuropathy. Am. J. Physiol. Endocrinol. Metab., 2012, 15, E551 - 561) and sarcopenia (Gilmore K.J., Morat T., Doherty T.J., Rice C.L., Motor unit number estimation and neuromuscular fidelity in 3 stages of sarcopenia. 2017 55(5):676-684). In one embodiment, the neuromuscular disorder is diabetic polyneuropathy. In one embodiment, the neuromuscular disorder is sarcopenia. In one embodiment, the neuromuscular disorder is Kennedy's disorder. In one embodiment, the neuromuscular disorder is multifocal motor neuropathy.

Thus, in one embodiment of the present disclosure the neuromuscular disorder is amyotrophic lateral sclerosis (ALS). In another embodiment the neuromuscular disorder is spinal muscular atrophy (SMA). In another embodiment the neuromuscular disorder is Charcot-Marie tooth disease (CMT). In another embodiment the neuromuscular disorder is sarcopenia. In yet another embodiment, the neuromuscular disorder is critical illness myopathy (CIM).

As stated above the neuromuscular disorders include for example disorders with symptoms of muscle weakness and fatigue. Such disorder may for example include diabetes (Am. J. Physiol. Endocrinol. Metab., 2012, 15, E551 - 561).

In another embodiment the neuromuscular disorders is chronic fatigue syndrome. Chronic fatigue syndrome (CFS) (Fletcher, S.N., Kennedy, D.D., Ghosh, I.R., Misra, V.P., Kiff, K., Coakley, J.H., Hinds, C.J. Persistent neuromuscular and neurophysiologic abnormalities in long-term survivors of prolonged critical illness. Crit. Care Med. 2003, 31, 1012 - 1016) is the common name for a medical condition characterized by debilitating symptoms, including fatigue that lasts for a minimum of six months in adults. CFS may also be referred to as systemic exertion intolerance disorder (SEID), myalgic encephalomyelitis (ME), post-viral fatigue syndrome (PVFS), chronic fatigue immune dysfunction syndrome (CFIDS), or by several other terms. Symptoms of CFS include malaise after exertion; unrefreshing sleep, widespread muscle and joint pain, physical exhaustion, and muscle weakness.

In a further embodiment the neuromuscular disorder is a critical illness polyneuropathy (Angelini C. Spectrum of metabolic myopathies. Biochim. Biophys. Acta., 2015, 1852, 615 - 621) or CIM (Latronico, N., Bolton, C.F. Critical illness polyneuropathy and myopathy: a major cause of muscle weakness and paralysis. Lancet Neurol. 2011, 10, 931-941). Critical illness polyneuropathy and CIM are overlapping syndromes of widespread muscle weakness and neurological dysfunction developing in critically ill patients.

The neuromuscular disorder may also include metabolic myopathy (Milone, M., Wong, L.J. Diagnosis of mitochondrial myopathies. Mol. Genet. Metab., 2013, 110, 35 - 41) and mitochondrial myopathy (Srivastava, A., Hunter, J.M. Reversal of neuromuscular block. Br. J. Anaesth. 2009, 103, 115 - 129). Metabolic myopathies result from defects in biochemical metabolism that primarily affects muscle. These may include glycogen storage disorders, lipid storage disorder and 3-phosphocreatine stores disorder. Mitochondrial myopathy is a type of myopathy associated with mitochondrial disorder. Symptoms of mitochondrial myopathies include muscular and neurological problems such as muscle weakness, exercise intolerance, hearing loss and trouble with balance and coordination. Thus, in one embodiment of the present disclosure the neuromuscular disorder is metabolic myopathy.

In another embodiment the neuromuscular disorder is periodic paralysis, in particular hypokalemic periodic paralysis which is a disorder of skeletal muscle excitability that presents with recurrent episodes of weakness, often triggered by exercise, stress, or carbohydrate-rich meals (Wu, F., Mi, W., Cannon, S.C., Neurology, 2013, 80, 1110-1116 and Suetterlin, K. et at, Current Opinion Neurology, 2014, 27, 583-590) or hyperkalemic periodic paralysis which is an inherited autosomal dominant disorder that affects sodium channels in muscle cells and the ability to regulate potassium levels in the blood (Ammat, T. et at, Journal of General Physiology, 2015, 146, 509-525).

In an embodiment the neuromuscular disorder is a myasthenic condition. Myasthenic conditions are characterized by muscle weakness and neuromuscular transmission failure. Congenital myasthenic syndromes (Finlayson, S., Beeson, D., Palace, J. Congenital myasthenic syndromes: an update. Pract. Neurol., 2013, 13, 80 - 91) is an inherited neuromuscular disorder caused by defects of several types at the neuromuscular junction.

Myasthenia gravis and Lambert-Eaton syndrome (Titulaer MJ, Lang B, Verschuuren JJ. Lambert-Eaton myasthenic syndrome: from clinical characteristics to therapeutic strategies. Lancet Neurol. 2011, 10, 1098-107) are examples of myasthenic conditions. Myasthenia gravis is either an autoimmune or congenital neuromuscular disorder that leads to fluctuating muscle weakness and fatigue. In the most common cases, muscle weakness is caused by circulating antibodies that block ACh receptors at the postsynaptic neuromuscular junction, inhibiting the excitatory effects of the neurotransmitter ACh on nicotinic ACh-receptors at neuromuscular junctions (Gilhus, N.E., Owe, J.F., Hoff, J.M., Romi, F., Skeie, G.O., Aarli, J.A. Myasthenia Gravis: A Review of Available Treatment Approaches, Autoimmune Diseases, 2011, Article ID 84739). Lambert-Eaton myasthenic syndrome (also known as LEMS, Lambert-Eaton syndrome, or Eaton-Lambert syndrome) is a rare autoimmune disorder that is characterized by muscle weakness of the limbs. It is the result of an autoimmune reaction in which antibodies are formed against presynaptic voltage-gated calcium channels, and likely other nerve terminal proteins, in the neuromuscular junction. Thirty to fifty percent of patients with acetylcholine receptor (AChR) antibody-negative myasthenia gravis (MG) have antibodies to muscle specific kinase (MuSK) and are referred to as having MuSK-MG (Borges, L.S., Richman, D.P., Muscle-Specific Kinase Myasthenia Gravis, Frontiers in Immunology, 2020, 11:707). . In one embodiment of the present disclosure the neuromuscular disorder is myasthenia gravis. In another embodiment the neuromuscular disorder is autoimmune myasthenia gravis. In another embodiment the neuromuscular disorder is MuSK-MG. In another embodiment the neuromuscular disorder is Lambert-Eaton syndrome. In another embodiment the neuromuscular disorder is seronegative myasthenia gravis. In another embodiment the neuromuscular disorder is congenital myasthenia gravis. In one embodiment the neuromuscular disorder is congenital myasthenic syndrome.

X-linked myotubular myopathy is a part of a group of centronuclear myopathies where cell nuclei are abnormally located in the centre of muscle cells instead of their normal location at the periphery. It is one of the severest congenital muscle diseases and is characterized by marked muscle weakness, hypotonia and feeding and breathing difficulties (Dowling JJ, Lawlor MW, Das S. X-Linked Myotubular Myopathy. 2002 Feb 25 [Updated 2018 Aug 23]. In: Adam MP, Ardinger HH, Pagon RA, et al., editors. GeneReviews® [Internet]. Seattle (WA): University of Washington, Seattle; 1993-2022. Available from: https://www.ncbi.nlm.nih.gov/books/NBK1432/). In one embodiment the neuromuscular disorder is myotubular myopathy.

Duchenne muscular dystrophy is a severe type of muscular dystrophy that primarily affects boys resulting initially in fatigue and muscle weakness (Angelini C, Tasca E, Fatigue in muscular dystrophies, Neuromuscular Disorders, 2012, 22 Suppl 3: S214-20. doi:10.1016/j.nmd.2012.10.010). In one embodiment the neuromuscular disorder is Duchenne muscular dystrophy.

Multiple sclerosis (MS) is the most common demyelinating disease, in which the insulating covers of nerve cells in the brain and spinal cord are damaged. This damage disrupts the ability of parts of the nervous system to transmit signals, resulting in a range of signs and symptoms, including physical, mental, and sometimes psychiatric problems. It has been shown that people with multiple sclerosis typically experience greater levels of exercise-induced fatigue compared with healthy individuals (Brotherton et al, J. Neuorophysiol. 2022, 128, 105-117). There are four types of MS in what is known as the Lublin classification: clinically isolated syndrome (CIS), relapsing-remitting MS (RRMS), primary progressive MS (PPMS) and secondary progressive MS (SPMS). In one embodiment, the neuromuscular disorder is selected from the group consisting of multiple sclerosis (MS), clinically isolated syndrome (CIS), relapsing-remitting MS (RRMS), primary progressive MS (PPMS) and secondary progressive MS (SPMS).

Neuromuscular blockade is used in connection with surgery under general anaesthesia. Reversing agents are used for more rapid and safer recovery of muscle function after such blockade. Complications with excessive muscle weakness after blockade during surgery can result in delayed weaning from mechanical ventilation and respiratory complications after the surgery. These complications can have pronounced effects on outcome of the surgery and future quality of life of patients, there is a need for improved reversing agents (Murphy GS, Brull SJ. Residual neuromuscular block: lessons unlearned. Part I: definitions, incidence, and adverse physiologic effects of residual neuromuscular block. Anesth Analg. 2010 111(1):120-8). Thus, in one embodiment, the neuromuscular disorder has been induced by a neuromuscular blocking agent. In one particular embodiment the neuromuscular disorder is muscle weakness caused by neuromuscular blockade after surgery. In another embodiment of the present disclosure the compound or the compound for use is used for reversing and/or ameliorating neuromuscular blockade after surgery. In one embodiment, the neuromuscular blockade is drug induced. In one embodiment, the neuromuscular blockade is caused by non-depolarizing neuromuscular blocker or antibiotic agent. In one embodiment the neuromuscular blockade is induced by an antibiotic. In one embodiment the neuromuscular blockade is induced by a non-depolarizing neuromuscular blocker.

In one embodiment the compound or the compound for use of the present disclosure is used to prevent a neuromuscular disorder. The compound or the compound for use may for example be used prophylactically against nerve gas that is known to cause symptoms of muscle weakness and fatigue (Kawamura, Y., Kihara, M., Nishimoto, K., Taki, M. Efficacy of a half dose of oral pyridostigmine in the treatment of chronic fatigue syndrome: three case reports. Pathophysiology, 2003, 9, 189-194). In one embodiment the compound or the compound for use of the present disclosure may be used in the treatment of muscle weakness (such as drooping eyelids, loss of facial expression, constipation, muscle weakness in arms, muscle weakness in legs and dyspnoea) caused by botulism poisoning. In one embodiment the compound or the compound for use of the present disclosure may be used in the treatment of snake bites where the snake toxin, such as α-neurotoxin or myotoxin, is known to cause symptoms of muscle weakness and fatigue.

In one embodiment, the neuromuscular disorder is selected from the group consisting of myasthenia gravis, autoimmune myasthenia gravis, congenital myasthenic syndrome, seronegative myasthenia gravis, muscle specific kinase myasthenia gravis (MuSK-MG), Lambert-Eaton Syndrome, amyotrophic lateral sclerosis (ALS), spinal muscular atrophy (SMA), critical illness myopathy (CIM), Charcot-Marie Tooth disease, diabetic polyneuropathy, periodic paralysis, hypokalemic periodic paralysis, hyperkalemic periodic paralysis, myotubular myopathy, Duchenne muscular dystrophy, Guillain-Barré syndrome, poliomyelitis, post-polio syndrome, chronic fatigue syndrome, critical illness polyneuropathy, metabolic myopathy, Kennedy's disorder, multiple sclerosis and multifocal motor neuropathy.

### Pharmaceutical formulations

In one aspect, the present invention relates to a composition comprising the compound as disclosed herein. The composition according to the present disclosure may be used for treating, ameliorating and/or preventing a neuromuscular disorder, and/or for use in reversing and/or ameliorating a neuromuscular blockade. Thus, the compositions and compounds described herein can be pharmaceutically acceptable. In one embodiment the composition as described herein is in the form of a pharmaceutical formulation. In one embodiment, the composition as described herein further comprises a pharmaceutically acceptable carrier. In one aspect, the present invention concerns a composition comprising the compound as defined herein and a pharmaceutically acceptable carrier.

### Combination therapy

The composition of the present disclosure may comprise further active ingredients/agents or other components to increase the efficiency of the composition. Thus, in one embodiment the composition further comprises at least one further active agent. It is appreciated that the active agent can be suitable for treating, preventing or ameliorating said neuromuscular disorder.

The active agent in certain embodiments can be an acetylcholine esterase inhibitor. Said acetylcholine esterase inhibitor may for example be selected from the group consisting of delta-9-tetrahydrocannabinol, carbamates, physostigmine, neostigmine, pyridostigmine, ambenonium, demecarium, rivastigmine, phenanthrene derivatives, galantamine, piperidines, donepezil, tacrine, edrophonium, huperzine, ladostigil, ungeremine and lactucopicrin.

In certain embodiments, the acetylcholine esterase inhibitor is selected from the group consisting of neostigmine, physostigmine and pyridostigmine. In certain embodiments, the acetylcholine esterase inhibitor is neostigmine or pyridostigmine.

The active agent may also be an immunosuppressive drug. Immunosuppressive drugs are drugs that suppress or reduce the strength of the body's immune system. Immunosuppressive drugs include but are not limited to glucocorticoids, corticosteroids, cytostatics, antibodies and drugs acting on immunophilins. In one embodiment the active agent is prednisone.

The active agent may also be an agent that is used in anti-myotonic treatment. Such agents include for example blockers of voltage gated Na⁺ channels, and aminoglycosides.

The active agent may also be an agent for reversing a neuromuscular blockade after surgery. Such agents include for example neostigmine or sugammadex (Org 25969, tradename Bridion).

The active agent may also be an agent for increasing ACh release by blocking voltage-gated K⁺ channels in the pre-synaptic terminal. Such agent includes 3,4-diaminopyridine (Amifampridine; tradename Firdapse).

The active agent may also be an agent for increasing the levels of survival motor neuron (SMN) protein that are produced. For example, by alternating the splicing of the SMN2 gene in order to increase the expression of full-length SMN protein from SMN2 (Zanetta C, Nizzardo M, Simone C, Monguzzi E, Bresolin N, Comi GP, Corti S, "Molecular therapeutic strategies for spinal muscular atrophies: current and future clinical trials". Clinical Therapeutics, 2014, 36 (1): 128-40). Such agents include antisense oligonucleotides such as Nusinersen (tradename Spinraza) or small molecules such as Risdiplam (tradename Evrysdi).

The active agent may be a gene therapy, for example by using viral vectors to deliver the SMN1 transgene to the affected motor neurons, where it leads to an increase in SMN protein production. Such gene therapies include onasemnogene abeparvovec (tradename Zolgensma). Such gene therapies include nusinersen (tradename Spinraza), risdiplam (tradename Evrysdi) and Branaplam.

The active agent may be a small molecule that increases expression of the SMN2 gene, thus increasing the amount of full-length SMN protein available. Such therapies include salbutamol (also, called albuterol; tradename Ventolin),

The active agent may also be an agent for increasing muscle reactivity. Such agents include skeletal troponin activators such as Tirasemtiv and Reldesemtiv (CK-2127107) (Hwee, D.T., Kennedy, A.R., Hartman, J.J., Ryans, J., Durham, N., Malik, F.I., Jasper, J.R. The small-molecule fast skeletal troponin activator, CK-2127107, improves exercise tolerance in a rat model of heart failure. Journal of Pharmacology and Experimental Therapeutics, 2015, 353, 159 - 168). Such agents may also be antibodies that block the activation of the skeletal muscle protein myostatin, such as Apitegromab (SRK-015) or GYM329 (RO7204239),

The active agent may also be an agent that disrupts or blocks the IgG-FcRn interaction thereby reducing the overall IgG recycling. Such agents may be antibodies, such as the aglycosylated immunoglobulin (Ig)G1 monoclonal antibody Nipocalimab, or IgG1 Fc fragment such as Efgartigimod alfa (tradename Vyvgart).

The active agent may also be an agent that is an inhibitor of the complement component C5a. The active agent may also be an agent that downregulates the overexpression of PMP22 protein, leading to improvement of neuronal signalling in dysfunctional peripheral nerves. Such agents may be combination drugs such as PXT3003. Alternatively, the active agent may also be an agent that binds to the protein complement component 5 (C5) and inhibits its cleavage into C5a and C5b. Such agents may be Zilucoplan (RA101495).

### Methods

In one aspect, the present invention relates to a compound as defined herein for use as a medicament.

In one aspect, the present invention relates to a compound as defined herein for use in treating, ameliorating and/or preventing a neuromuscular disorder.

In one aspect, the present invention relates to a compound as defined herein for use in the treatment of an indication selected from the group consisting of myasthenia gravis, autoimmune myasthenia gravis, congenital myasthenic syndrome, seronegative myasthenia gravis, muscle specific kinase myasthenia gravis (MuSK-MG), Lambert-Eaton Syndrome, amyotrophic lateral sclerosis (ALS), spinal muscular atrophy (SMA), critical illness myopathy (CIM), Charcot-Marie Tooth disease, diabetic polyneuropathy, periodic paralysis, hypokalemic periodic paralysis, hyperkalemic periodic paralysis, myotubular myopathy, Duchenne muscular dystrophy, Guillain-Barré syndrome, poliomyelitis, post-polio syndrome, chronic fatigue syndrome, critical illness polyneuropathy, metabolic myopathy, Kennedy's disorder, multiple sclerosis and multifocal motor neuropathy. In one aspect, the present invention relates to a compound as defined herein for use in the symptomatic treatment of sarcopenia.

In one aspect, the present invention relates to a compound as defined herein for use in reversing and/or ameliorating a neuromuscular blockade.

In one aspect, the disclosure concerns a compound of Formula (I): 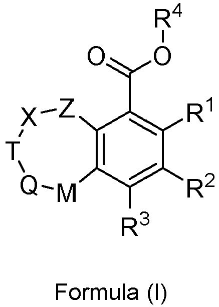 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R' is selected from the group consisting of H; F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; C₁₋₃ alkyl optionally substituted with one or more, identical or different, substituents R¹⁰ and -OC₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
   when R⁵ and R⁶ are individually C₁₋₅ alkyl optionally substituted with one or more, identical or different, substituents R¹⁰, then R⁵ and R⁶ are optionally joined together to form a ring;
   or a pharmaceutically acceptable salt, hydrate, polymorph, tautomer, or solvate thereof
   for use in treating, ameliorating and/or preventing a neuromuscular disorder, and/or for use in reversing and/or ameliorating a neuromuscular blockade.

In one embodiment, the compound for use is selected from the group consisting of:
7-chloro-2,3-dihydro-1,4-benzodioxine-5-carboxylic acid;
7-chloro-6-methoxy-2,3-dihydro-1,4-benzodioxine-5-carboxylic acid;
7-chloro-6-methyl-2,3-dihydro-1,4-benzodioxine-5-carboxylic acid;
7,8-dichloro-6-methyl-2,3-dihydro-1,4-benzodioxine-5-carboxylic acid;
6-chloro-7-methoxy-4-methyl-3,4-dihydro-2H-1,4-benzoxazine-8-carboxylic acid;
7-chloro-6-fluoro-4-methyl-3,4-dihydro-2H-1,4-benzoxazine-5-carboxylic acid;
7-chloro-6-fluoro-4-methyl-3,4-dihydro-2H-1,4-benzoxazine-8-carboxylic acid;
5-chloro-7-fluoro-3,4-dihydro-2H-1,4-benzoxazine-8-carboxylic acid;
6-chloro-7-fluoro-3,4-dihydro-2H-1,4-benzoxazine-8-carboxylic acid;
7-chloro-6-methoxy-4-methyl-3,4-dihydro-2H-1,4-benzoxazine-8-carboxylic acid;
6-chloro-7-fluoro-4-methyl-3,4-dihydro-2H-1,4-benzoxazine-8-carboxylic acid;
7-chloro-6-fluoro-1,4-dimethyl-1,2,3,4-tetrahydroquinoxaline-5-carboxylic acid;
6-chloro-7-fluoro-2,2,4-trimethyl-3,4-dihydro-2H-1,4-benzoxazine-8-carboxylic acid;
7-chloro-6-methoxy-4-methyl-3,4-dihydro-2H-1,4-benzoxazine-5-carboxylic acid;
7-chloro-6-fluoro-3,4-dihydro-2H-1,4-benzoxazine-5-carboxylic acid;
7-chloro-8-methoxy-5-methyl-2,3,4,5-tetrahydro-1,5-benzoxazepine-9-carboxylic acid;
7-chloro-8-fluoro-5-methyl-2,3,4,5-tetrahydro-1,5-benzoxazepine-9-carboxylic acid;
6-chloro-7-methoxy-3,4-dihydro-2H-1,4-benzoxazine-8-carboxylic acid;
7-chloro-8-fluoro-2,3,4,5-tetrahydro-1,5-benzoxazepine-9-carboxylic acid;
7-chloro-8-ethoxy-5-methyl-2,3,4,5-tetrahydro-1,5-benzoxazepine-9-carboxylic acid;
6-chloro-7-fluoro-4-(3-methylbutyl)-3,4-dihydro-2H-1,4-benzoxazine-8-carboxylic acid;
6-chloro-4-ethyl-7-fluoro-3,4-dihydro-2H-1,4-benzoxazine-8-carboxylic acid;
6-chloro-7-fluoro-4-methyl-3,4-dihydro-2H-1,4-benzothiazine-8-carboxylic acid;
6-chloro-4-(cyclopropylmethyl)-7-fluoro-3,4-dihydro-2H-1,4-benzoxazine-8-carboxylic acid;
6-chloro-7-fluoro-4-propyl-3,4-dihydro-2H-1,4-benzoxazine-8-carboxylic acid;
4-benzyl-6-chloro-7-fluoro-3,4-dihydro-2H-1,4-benzoxazine-8-carboxylic acid;
7-fluoro-6-methoxy-4-methyl-3,4-dihydro-2H-1,4-benzoxazine-8-carboxylic acid;
6-chloro-7-fluoro-4-[3-(4-fluorophenyl)propyl]-3,4-dihydro-2H-1,4-benzoxazine-8-carboxylic acid;
6-chloro-4-methyl-7-(propan-2-yloxy)-3,4-dihydro-2H-1,4-benzoxazine-8-carboxylic acid;
6-chloro-7-fluoro-4-[2-(4-fluorophenyl)ethyl]-3,4-dihydro-2H-1,4-benzoxazine-8-carboxylic acid;
6-chloro-4-methyl-7-(methylsulfanyl)-3,4-dihydro-2H-1,4-benzoxazine-8-carboxylic acid;
6-chloro-7-fluoro-4-(3-methoxypropyl)-3,4-dihydro-2H-1,4-benzoxazine-8-carboxylic acid;
6-chloro-4-ethyl-7-methoxy-3,4-dihydro-2H-1,4-benzoxazine-8-carboxylic acid;
6-chloro-7-methoxy-4-methyl-3,4-dihydro-2H-1,4-benzothiazine-8-carboxylic acid;
6-fluoro-7-methoxy-4-methyl-3,4-dihydro-2H-1,4-benzoxazine-8-carboxylic acid;
7-methoxy-4,6-dimethyl-3,4-dihydro-2H-1,4-benzoxazine-8-carboxylic acid;
6-chloro-7-methoxy-4-(propan-2-yl)-3,4-dihydro-2H-1,4-benzoxazine-8-carboxylic acid;
6-chloro-7-methoxy-4-(2-phenylethyl)-3,4-dihydro-2H-1,4-benzoxazine-8-carboxylic acid;
6-chloro-7-methoxy-4-[2-(4-methoxyphenyl)ethyl]-3,4-dihydro-2H-1,4-benzoxazine-8-carboxylic acid;
6-chloro-7-methoxy-2-(methoxymethyl)-4-methyl-3,4-dihydro-2H-1,4-benzoxazine-8-carboxylic acid;
6-chloro-7-methoxy-4-[(thiophen-3-yl)methyl]-3,4-dihydro-2H-1,4-benzoxazine-8-carboxylic acid;
6-chloro-7-methoxy-2,2,4-trimethyl-3,4-dihydro-2H-1,4-benzoxazine-8-carboxylic acid;
6-chloro-7-methoxy-2-(methoxymethyl)-4-methyl-3,4-dihydro-2H-1,4-benzoxazine-5-carboxylic acid;
6-chloro-7-fluoro-4-(2-methoxyethyl)-3,4-dihydro-2H-1,4-benzoxazine-8-carboxylic acid;
6-chloro-7-methoxy-4-methyl-3,4-dihydrospiro[1,4-benzoxazine-2,1'-cyclobutane]-8-carboxylic acid;
6-chloro-4-[3-(furan-2-yl)propyl]-7-methoxy-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(1,3-thiazol-2-yl)methyl]-3,4-dihydro-2H-1,4-benzoxazine-8-carboxylic acid;
6-chloro-5-fluoro-7-methoxy-4-methyl-3,4-dihydro-2H-1,4-benzoxazine-8-carboxylic acid;
4-benzyl-7-chloro-6-fluoro-3,4-dihydro-2H-1,4-benzoxazine-5-carboxylic acid;
6-chloro-4-(cyclobutylmethyl)-7-fluoro-3,4-dihydro-2H-1,4-benzoxazine-8-carboxylic acid;
6-chloro-7-methoxy-4-[3-(1,3-thiazol-2-yl)propyl]-3,4-dihydro-2H-1,4-benzoxazine-8-carboxylic acid;
6-chloro-4,7-dimethyl-3,4-dihydro-2H-1,4-benzoxazine-8-carboxylic acid;
6-chloro-7-methoxy-4-[(4-methoxyphenyl)methyl]-3,4-dihydro-2H-1,4-benzoxazine-8-carboxylic acid;
6-chloro-7-methoxy-4-[(1,3-oxazol-2-yl)methyl]-3,4-dihydro-2H-1,4-benzoxazine-8-carboxylic acid;
7-chloro-6-fluoro-4-(3-methylbutyl)-3,4-dihydro-2H-1,4-benzoxazine-5-carboxylic acid;
4-benzyl-6-chloro-7-methoxy-3,4-dihydro-2H-1,4-benzoxazine-8-carboxylic acid;
6-chloro-4-[2-(3,5-difluorophenyl)ethyl]-7-methoxy-3,4-dihydro-2H-1,4-benzoxazine-8-carboxylic acid;
7-chloro-6-fluoro-4-[(thiophen-3-yl)methyl]-3,4-dihydro-2H-1,4-benzoxazine-5-carboxylic acid;
4-benzyl-6-fluoro-7-methoxy-3,4-dihydro-2H-1,4-benzoxazine-8-carboxylic acid;
methyl 6-chloro-7-methoxy-3,4-dihydro-2H-1,4-benzoxazine-8-carboxylate;
ethyl 6-chloro-7-fluoro-3,4-dihydro-2H-1,4-benzoxazine-8-carboxylate;
methyl 7-chloro-6-fluoro-3,4-dihydro-2H-1,4-benzoxazine-5-carboxylate;
methyl 6-chloro-7-fluoro-3,4-dihydro-2H-1,4-benzoxazine-8-carboxylate;
7-chloro-6-fluoro-4-propyl-3,4-dihydro-2H-1,4-benzoxazine-5-carboxylic acid;
7-chloro-6-fluoro-4-methyl-1,2,3,4-tetrahydro-5-quinoxalinecarboxylic acid;
7-chloro-6-fluoro-4-[(p-methoxyphenyl)methyl]-3,4-dihydro-2H-1,4-benzoxazine-5-carboxylic acid;
1-benzyl-7-chloro-6-fluoro-4-methyl-1,2,3,4-tetrahydro-5-quinoxalinecarboxylic acid;
7-chloro-6-fluoro-4-[(p-tolyl)methyl]-3,4-dihydro-2H-1,4-benzoxazine-5-carboxylic acid;
7-chloro-6-fluoro-4-[(1-naphthyl)methyl]-3,4-dihydro-2H-1,4-benzoxazine-5-carboxylic acid;
7-chloro-6-fluoro-4-[(o-tolyl)methyl]-3,4-dihydro-2H-1,4-benzoxazine-5-carboxylic acid; and
4-benzyl-6,7-dichloro-3,4-dihydro-2H-1,4-benzoxazine-8-carboxylic acid.

The references to methods of treatment in this description are to be interpreted as references to the compounds, pharmaceutical compositions and medicaments of the present invention for use in a method for the treatment of the human (or animal) body by therapy (or for diagnosis).

In one aspect, the present disclosure relates to a method of treating, preventing and/or ameliorating a neuromuscular disorder, said method comprising administering a therapeutically effective amount of the compound or the compound for use as defined herein to a person in need thereof.

In one aspect, the present disclosure relates to a method of reversing and/or ameliorating a neuromuscular blockade, said method comprising administering a therapeutically effective amount of the compound or the compound for use as defined herein to a person in need thereof.

In one aspect, the present disclosure relates to a method for recovery of neuromuscular transmission, said method comprising administering a therapeutically effective amount of the compound or the compound for use as defined herein to a person in need thereof.

The person in need thereof may be a person having a neuromuscular disorder or a person at risk of developing a neuromuscular disorder or a person having symptoms of muscle weakness and/or fatigue. In another embodiment the person in need thereof is a person with reduced neuromuscular transmission safety with prolonged recovery after neuromuscular blockade. Types of neuromuscular disorders are defined herein above. In an embodiment the person has amyotrophic lateral sclerosis, spinal muscular atrophy, myasthenia gravis or Lambert-Eaton syndrome.

A therapeutically effective amount is an amount that produces a therapeutic response or desired effect in the person taking it. Administration routes, formulations and dosages can be optimized by persons of skill in the art.

The method of treatment may be combined with other methods that are known to treat, prevent and/or ameliorate neuromuscular disorders. The treatment method may for example be combined with administration of any of the agents mentioned herein above. In one embodiment the treatment is combined with administration of acetylcholine esterase inhibitor such as for example neostigmine or pyridostigmine.

In one aspect, the invention relates to a method for recovery of force in muscles with neuromuscular dysfunction, said method comprising administering a compound or a composition as defined herein to a subject in need thereof. The term "recovery of force in muscles with neuromuscular dysfunction" as used herein refers to the ability of a compound to recover contractile force in nerve-stimulated healthy rat muscle after exposure to submaximal concentration (115 nM) of tubocurarine for 90 minutes. Recovery of force is quantified as the percentage of the force prior to tubocurarine that is recovered after addition of the compound. In one embodiment, said recovery of force is >5%, such as >10%, such as >15%, such as >20%, such as >25%, such as >30%, such as >35%.

In one aspect, the present invention relates to a method of treating botulism poisoning, snake bites or nerve gas poisoning or preventing nerve gas poisoning, said method comprising administering a therapeutically effective amount of the compound as defined herein to a person in need thereof.

Another aspect of the disclosure relates to use of a compound as defined herein, for the manufacture of a medicament for the treatment, prevention and/or amelioration of a neuromuscular disorder.

Another aspect relates to use of a compound as defined herein, for the manufacture of a medicament or a reversal agent for reversing and/or ameliorating a neuromuscular blockade after surgery.

In one aspect, the present invention related to use of a compound as defined herein for the manufacture of a medicament for the treatment of botulism poisoning, snake bites or nerve gas poisoning or prevention of nerve gas poisoning.

### Method of manufacturing

In one aspect, the present disclosure relates to methods of manufacturing compounds or compounds for use according to formula (I).

Compounds according to the present invention may be prepared according to any conventional methods of chemical synthesis known by the skilled person, e.g. those described in the working examples. The starting materials for the processes described in the present application are known or may readily be prepared by conventional methods known by the skilled artisan from commercially available chemicals.

The end products of the reactions described herein may be isolated by conventional technique such as extraction, crystallisation, distillation, chromatography etc.

The compounds of this invention may exist in unsolvated as well as in solvated forms with pharmaceutically acceptable solvents such as water, ethanol and the like. In general, the solvated forms are considered equivalent to the unsolvated forms for the purposes of this invention.

### Examples

### General synthetic strategies

General methods for the synthesis of carboxylic acids and substitution on aromatic rings are featured in literature sources such as: March's Advanced Organic Chemistry: Reactions, Mechanisms, and Structure, 8th Edition, Michael B. Smith, Ed.; ISBN: 978-1-119-37179-3; John Wiley, 2020.

### Materials and methods

### Chemicals

Compounds for testing were obtained from different suppliers including Enamine, Vitas, and CanAm Bioresearch. For synthesis of particular compounds please see below.

### NMR Spectra

¹H-NMR and ¹⁹F-NMR spectra were either recorded on a Bruker AM-300 spectrometer and were calibrated using residual nondeuterated solvent as internal reference. Spectra were processed using Spinworks version 4.0 (developed by Dr. Kirk Marat, Department of Chemistry, University of Manitoba).

Otherwise ¹H, ¹³C and ¹⁹F NMR analyses were conducted Jeol 400 using deuterated chloroform or deuterated dimethyl sulfoxide as solvent. The shift (d) of each signal was measured in parts per million (ppm) relative the residual solvent peak, and the multiplicity reported together with the associated coupling constant (J), where applicable.

### LC/MS System

Samples were analysed my direct inject on a Waters Acquity QDa Mass Detector with a Waters 2695 HPLC. Mass spectra were recorded in ESI scan mode (negative/positive).

### HPLC method

The product was analysed by Waters 2695 HPLC consisting of a Waters 996 photodiode array detector, Kromasil Eternity C18, 5 µm, 4.6 X 150 mm column. Flow rate: 1 mL/minute, run time 20 minutes. Solvent A: methanol; solvent B: 0.1% formic acid in water. Gradient 0-100 % Solvent B over 15 minutes with monitoring at 280 nm.

### UPLC-MS method

UPLC-MS analysis was carried out on a Waters Acquity UPLC system consisting of an Acquity I-Class Sample Manager-FL, Acquity I-Class Binary Solvent Manager and an Acquity UPLC Column Manager. UV detection was afforded using an Acquity UPLC PDA detector (scanning from 210 to 400 nm), whilst mass detection was achieved using an Acquity QDa detector (mass scanning from 100-1250 Da; positive and negative modes simultaneously), and ELS detection was achieved using an Acquity UPLC ELS Detector.

**Samples** were prepared by dissolution (with or without sonication) into 1 mL of 50% (v/v) MeCN in water. The resulting solutions were then filtered through a 0.2 □m syringe filter before submitting for analysis. All of the solvents, including formic acid and 36% ammonia solution, were purchased as HPLC grade.

### Acidic 2 min UPLC-MS method

Eluent A: 0.1% v/v formic acid in 10mM ammonium formate
Eluent B: 0.1% v/v formic acid in MeCN
Flow rate 0.8mL/min; column oven 50°C; sample manager 20°C; injection volume 2 µL. 1.5 minutes equilibration time between samples on a Waters Acquity UPLC BEH C18 column (2.1 × 50 mm, 1.7 µm).
Gradient as given in table below.

| **Time (min)** | **Eluent A (%)** | **Eluent B (%)** |
|---|---|---|
| 0.00 | 95 | 5 |
| 0.25 | 95 | 5 |
| 1.25 | 5 | 95 |
| 1.55 | 5 | 95 |
| 1.65 | 95 | 5 |
| 2.00 | 95 | 5 |

### Acidic 4 min UPLC-MS method

Eluent A: 0.1% v/v formic acid in 10mM ammonium formate
Eluent B: 0.1% v/v formic acid in MeCN
Flow rate 0.8mL/min; column oven 50°C; sample manager 20°C; injection volume 2 µL. 1.5 minutes equilibration time between samples on a Waters Acquity UPLC BEH C18 column (2.1 × 50 mm, 1.7 µm).
Gradient as given in table below.

| **Time (min)** | **Eluent A (%)** | **Eluent B (%)** |
|---|---|---|
| 0.00 | 95 | 5 |
| 0.25 | 95 | 5 |
| 2.75 | 5 | 95 |
| 3.25 | 5 | 95 |
| 3.35 | 95 | 5 |
| 4.00 | 95 | 5 |

### Acidic 6 min UPLC-MS method

Eluent A: 0.1% v/v formic acid in 10mM ammonium formate
Eluent B: 0.1% v/v formic acid in MeCN
Flow rate 0.8mL/min; column oven 50°C; sample manager 20°C; injection volume 2 µL. 1.5 minutes equilibration time between samples on a Waters Acquity UPLC BEH C18 column (2.1 × 50 mm, 1.7 µm).
Gradient as given in table below.

| **Time (min)** | **Eluent A (%)** | **Eluent B (%)** |
|---|---|---|
| 0.00 | 95 | 5 |
| 0.30 | 95 | 5 |
| 6.00 | 5 | 95 |
| 6.10 | 95 | 5 |
| 7.00 | 95 | 5 |

### Basic 2 min UPLC-MS method

Eluent A: 0.1% ammonia in water
Eluent B: 0.1% ammonia in MeCN
Flow rate 0.8mL/min; column oven 50°C; sample manager 20°C; injection volume 2 µL. 1.5 minutes equilibration time between samples on a Waters Acquity UPLC BEH C18 column (2.1 × 50 mm, 1.7 µm).
Gradient as given in table below.

| **Time (min)** | **Eluent A (%)** | **Eluent B (%)** |
|---|---|---|
| 0.00 | 95 | 5 |
| 0.25 | 95 | 5 |
| 1.25 | 5 | 95 |
| 1.55 | 5 | 95 |
| 1.65 | 95 | 5 |
| 2.00 | 95 | 5 |

### Basic 4 min UPLC-MS method

Eluent A: 0.1% ammonia in water
Eluent B: 0.1% ammonia in MeCN
Flow rate 0.8mL/min; column oven 50°C; sample manager 20°C; injection volume 2 µL. 1.5 minutes equilibration time between samples on a Waters Acquity UPLC BEH C18 column (2.1 × 50 mm, 1.7 µm).
Gradient as given in table below.

| **Time (min)** | **Eluent A (%)** | **Eluent B (%)** |
|---|---|---|
| 0.00 | 95 | 5 |
| 0.25 | 95 | 5 |
| 2.75 | 5 | 95 |
| 3.25 | 5 | 95 |
| 3.35 | 95 | 5 |
| 4.00 | 95 | 5 |

### Basic 6 min UPLC-MS method

Eluent A: 0.1% ammonia in water
Eluent B: 0.1% ammonia in MeCN
Flow rate 0.8mL/min; column oven 50°C; sample manager 20°C; injection volume 2 µL. 1.5 minutes equilibration time between samples on a Waters Acquity UPLC BEH C18 column (2.1 × 50 mm, 1.7 µm).
Gradient as given in table below.

| **Time (min)** | **Eluent A (%)** | **Eluent B (%)** |
|---|---|---|
| 0.00 | 95 | 5 |
| 0.30 | 95 | 5 |
| 6.00 | 5 | 95 |
| 6.10 | 95 | 5 |
| 7.00 | 95 | 5 |

### Preparative HPLC purification

Preparative HPLC purification was carried out either on a Teledyne ISCO ACCQPrep^{®} HP150 system or on a Waters Mass-directed PrepLC system. All masses were detected with electrospray ionisation (ESI).

Prep HPLC Columns:
- C1 XBridge BEH C18. Dimensions: 19 mm x 150 mm 5 µm.
- C2 XBridge BEH C18. Dimensions: 19 mm x 150 mm 5 µm.

Solvents:
A: [10 mM NH₄HCO₃ in H₂O + 0.1% NH₃] or [H₂O + 0.1% NH₃]
B: [10 mM NH₄HCO₃ in MeCN + 0.1% NH₃] or [MeCN + 0.1% NH₃]

Flow rate: 21.0 mL/min

### Example 1: 7-Chloro-6-methoxy-2,3-dihydro-1,4-benzodioxine-5-carboxylic acid (A-1)

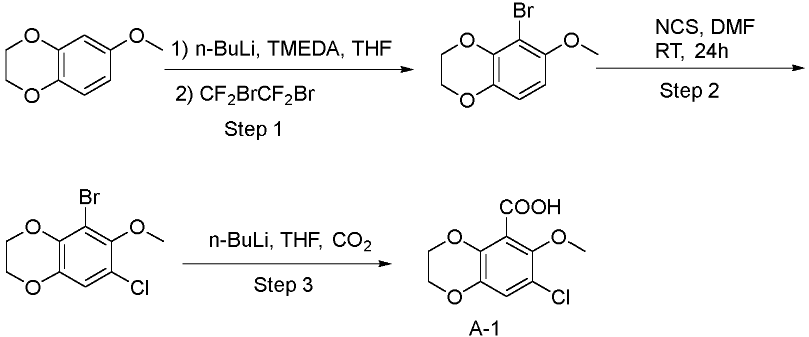

### Step 1: 5-Bromo-6-methoxy-2,3-dihydrobenzo[b][1,4]dioxine

To a stirred solution of 6-methoxy-2,3-dihydrobenzo[b][1,4]dioxine (3.32 g, 20 mmol) in anhydrous THF (20 mL), TMEDA (18.1 mL, 120 mmol) and n-BuLi (2.5 M in hexane, 24 mL, 60 mmol) were added at -50°C under an nitrogen atmosphere. The mixture was stirred at the same temperature for 2 h and 1,2-dibromotetrafluoroethane (7.15 mL, 60 mmol) was added. Stirring at -50°C was continued for 1 h and then the reaction was allowed to warm to ambient temperature. The reaction mixture was quenched with water (25 mL), extracted with EtOAc (2 x 50 mL) then the combined extracts were washed with brine (50 mL), dried (Na₂SO₄) and evaporated. The residue was purified by chromatography on silica gel eluting with hexane/EtOAc (0-20%) to provide the title compound (4.7 g, 96%) as a yellow oil.

¹H NMR (300 MHz, CDCl₃) δ ppm 6.85 (d, 1H); 6.49 (d, 1H); 4.43-4.37 (m, 2H); 4.29-4.22 (m, 2H); 3.88 (s, 3H); ES-MS: 246 [M+1].

### Step 2: 5-Bromo-7-chloro-6-methoxy-2,3-dihydrobenzo[b][1,4]dioxine

A mixture of 5-bromo-6-methoxy-2,3-dihydrobenzo[b][1,4]dioxine (380 mg, 1.55 mmol) and NCS (207 mg, 1.55 mmol) in DMF (5 mL) was stirred at ambient temperature for 24 h. The reaction mixture was quenched with water (20 mL) and extracted with EtOAc (2 x 100 mL). the combined extracts were washed with brine (50 mL), dried (Na₂SO₄) and evaporated. The residue was purified by chromatography on silica gel eluting with hexane/EtOAc (0-10%) to provide the title compound (373 mg, 86%) as an oil.

¹H NMR (300 MHz, CDCl₃) δ ppm 6.91 (s, 1H); 4.38-4.32 (m, 2H); 4.26-4.20 (m, 2H); 3.83 (s, 3H); ES-MS: 280 [M+1].

### Step 3: 7-Chloro-6-methoxy-2,3-dihydro-1,4-benzodioxine-5-carboxylic acid

To a solution of 5-bromo-7-chloro-6-methoxy-2,3-dihydrobenzo[b][1,4]dioxine (373 mg, 1.33 mmol) in THF (7 mL) at -78°C, n-BuLi (0.58 mL, 1.46 mmol, [1.6 M]) was added, and the mixture was stirred at ambient temperature for 1 h. The reaction mixture was cooled to 0°C and CO₂ gas was bubbled through it for 45 min. followed by quenching with water (25 mL) and washing with EtOAc (2 x 25 mL). The aqueous layer was acidified to pH ~2 with 1.0 N HCl and extracted with EtOAc (2 x 25 mL). The combined extracts were dried (Na₂SO₄), concentrated under reduced pressure and the crude product was purified by semi-prep HPLC (10 to100% acetonitrile /0.1% formic acid in water) to afford the title acid (250 mg, 76%) as an off-white solid.

¹H NMR (300 MHz, CDCl₃) δ ppm 7.26 (s, 1H); 4.65-4.45 (m, 4H); 4.16 (s, 3H) ES-MS: 243 [M-1].

HPLC Retention Time: 8.62 min., purity >98% at 280 nm.

### Example 2: 6-Chloro-7-methoxy-4-methyl-3,4-dihydro-2H-1,4-benzoxazine-8-carboxylic acid (A-2)

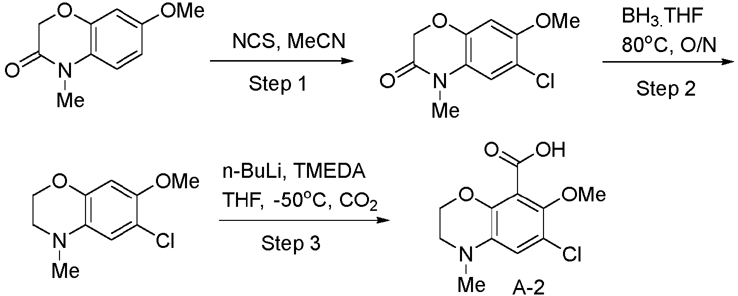

### Step 1: 6-Chloro-7-methoxy-4-methyl-2,4-dihydro-1,4-benzoxazin-3-one

A mixture of 7-methoxy-4-methyl-2,4-dihydro-1,4-benzoxazin-3-one (1240 mg, 6.42 mmol) and NCS (900 mg, 6.74 mmol) in DMF (5 mL) was stirred at 65°C for 16 h. The reaction mixture was quenched with water (20 mL) and extracted with EtOAc (2 x 30 mL). The combined extracts were washed with brine (30 mL), dried (Na₂SO₄) and concentrated. The resultant residue was purified by chromatography on silica gel eluting with hexane/EtOAc (0-5%) to provide the title compound (1.40 g, 95%).

¹H NMR (300 MHz, CDCl₃) δ 7.26 (s, 1H), 6.90 (s, 1H), 4.80 (s, 2H), 3.14 (s, 3H), 3.60 (s, 3H) ppm.

### Step 2: 6-Chloro-7-methoxy-4-methyl-3,4-dihydro-2H-1,4-benzoxazine

A mixture of 6-chloro-7-methoxy-4-methyl-2,4-dihydro-1,4-benzoxazin-3-one (1.00 g, 4.393 mmol) and 1N BH₃-THF solution (17.6 mL, 17.572 mmol) was stirred at 80°C for 16 h. The reaction mixture was quenched with MeOH (20 mL), evaporated, then 1N NaOH (50 mL) was added and the product was extracted with DCM (3 x 50 mL). The combined extracts were washed with brine (30 mL), dried (Na₂SO₄) and concentrated. The resultant residue was purified by chromatography on silica gel eluting with hexane/EtOAc (0-5%) to provide the title compound (840 mg, 89%).

¹H NMR (300 MHz, CDCl₃) δ 6.45 (s, 1H), 6.90 (s, 1H), 4.37 (m, 2H), 3.79 (s, 3H), 3.18 (m, 2H), 2.80 (s, 3H) ppm.

### Step 3: 6-Chloro-7-methoxy-4-methyl-3,4-dihydro-2H-1,4-benzoxazine-8-carboxylic acid

To a solution of 6-chloro-7-methoxy-4-methyl-3,4-dihydro-2H-1,4-benzoxazine (200 mg, 0.936 mmol) in THF (4 mL) and TMEDA (0.9 mL, 5.616 mmol) at -78°C and n-BuLi (2.5 M in hexane) (1.12 mL, 2.808 mmol) was introduced. The reaction mixture was stirred at that temperature for 2 h and and CO₂ (g) was bubbled through it for 10 min. The reaction mixture was gradually brought to 0°C and quenched with 1NNaOH to pH ~11. Following washing with EtOAc (2 x 10 mL) the aqueous layer was separated and acidified with 1N HCl to pH ~1 prior to extraction with EtOAc (3 x 50 mL). The combined extracts were dried (Na₂SO₄) and concentrated to afford the title compound (162 mg, 76%).

¹H NMR (300 MHz, CD₃OD) δ 6.73 (s, 1H), 4.31 (t, 2H), 3.81 (s, 3H), 3.37 (t, 2H), 2.80 (t, 2H),) ppm.

ES-MS: *m*/*z* 256.9 (M-1).

HPLC: Retention time 9.26 min., purity >98 % at 280 nm.

### Example 3: 7-Chloro-6-fluoro-4-methyl-3,4-dihydro-2H-1,4-benzoxazine-5-carboxylic acid (A-3)

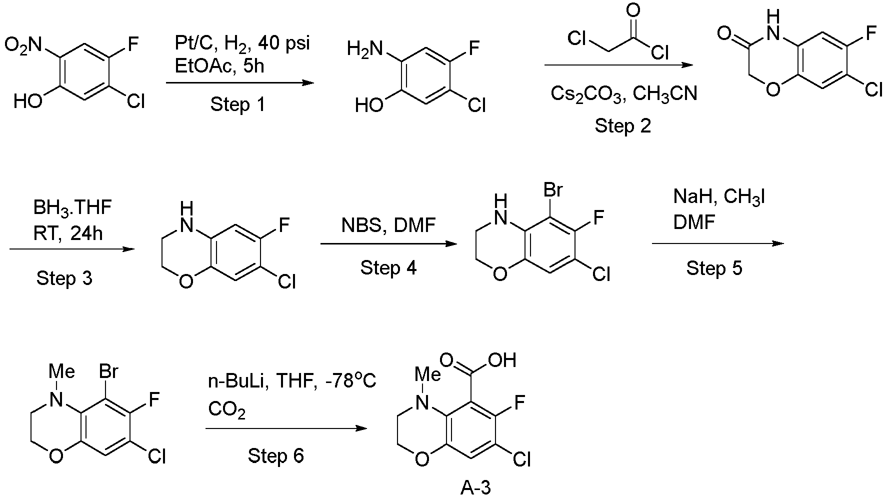

### Step 1: 2-Amino-5-chloro-4-fluorophenol

To a solution of 5-chloro-4-fluoro-2-nitrophenol (10.0 g, 52.2 mmol) in EtOAc (120 mL), 5% Pt/C (1.00 g) was added. The reaction mixture was hydrogenated at 20 psi for 16 h. The reaction mixture was filtered through a pad of celite, washed with EtOAc (120 mL) and concentrated under reduced pressure to obtain the title compound (8.28 g, 98%) as a dark brown solid.

¹H NMR (300 MHz, DMSO-d₆) δ 9.40 (s, 1H), 6.65 (d, 1H), 6.52 (d, 1H), 4.90 (br s, 2H).

### Step 2: 7-Chloro-6-fluoro-2,4-dihydro-1,4-benzoxazin-3-one

To a solution of 2-amino-5-chloro-4-fluorophenol (8.40 g, 52.0 mmol) in acetonitrile (125 mL) at 0°C, Cs₂CO₃ (42.4 g, 130 mmol) was introduced, followed by the addition of 2-chloroacetyl chloride (4.4 mL, 54.2 mmol). The mixture was stirred at ambient temperature for 16 h before filtration through a celite pad, and the filter pad was washed with EtOAc (150 mL). The solvent was removed under reduced pressure and the residue treated with EtOAc (250 mL) and water (250 mL). The layers were separated, the aqueous layer was extracted with EtOAc (2 x 150 mL). The combined organic layers were dried (Na₂SO₄), concentrated under reduced pressure and triturated with diethyl ether (3 x 40 mL) to obtain the title compound (4.92 g, 47%).

¹H NMR (300 MHz, DMSO-d₆) δ 10.9 (s, 1H), 7.23 (d, 1H), 6.86 (d, 1H), 4.60 (s, 2H).

### Step 3: 7-Chloro-6-fluoro-2,3-dihydro-1,4-benzoxazine

To a solution of 7-chloro-6-fluoro-2,4-dihydro-1,4-benzoxazin-3-one (6.00g, 29.8 mmol) in THF (100 mL), at 0°C 1M BH₃.THF (60 mL, 59.3 mmol) was added. The reaction mixture was stirred at ambient temperature for 24 h. The reaction mixture was cooled to 0 °C and carefully quenched with 1.0 N NaOH (120 mL). The reaction mixture was then diluted with water (120 mL) and the solvent was removed under reduced pressure. The residue was extracted with EtOAc (2 x 250 mL). The combined organic layers were dried (Na₂SO₄) and concentrated under reduced pressure to provide the title compound (4.92 g, 97%).

¹H NMR (300 MHz, CDCl₃) δ 6.77 (d, 1H), 6.36 (d, 1H), 4.19 (t, 2H), 3.84 (br s, 1H), 3.40 (t, 2H).

### Step 4: 5-Bromo-7-chloro-6-fluoro-2,3-dihydro-1,4-benzoxazine

NBS (0.59 g, 3.31 mmol) was added to a solution of 7-chloro-6-fluoro-3,4-dihydro-2H-1,4-benzoxazine (0.59 g, 3.14 mmol) in DMF (6.0 mL) and the reaction mixture was stirred at ambient temperature for 16 h. The reaction mixture was quenched with water (20 mL) and extracted with EtOAc (2 x 50 mL). The combined organic layers were dried (Na₂SO₄) and concentrated under reduced pressure. The residue was purified by column chromatography on silica gel eluting with EtOAc/hexane (0-50%) to obtain the title compound (0.30 g, 36%).

¹H NMR (300 MHz, CDCl₃) δ 6.79 (d, 1H), 4.37 (br s, 1H), 4.19 (t, 2H), 3.52 - 3.48 (m, 2H).

### Step 5: 5-Bromo-7-chloro-6-fluoro-4-methyl-2,3-dihydro-1,4-benzoxazine

To a solution of 5-bromo-7-chloro-6-fluoro-3,4-dihydro-2H-1,4-benzoxazine (300 mg, 1.13 mmol) in THF (12 mL) at 0°C, NaH (60% dispersion in mineral oil) (180 mg, 4.50 mmol) was added and the mixture was stirred for 1 h. At that temperature, CH₃I (0.84 mL, 13.5 mmol) was added, and the reaction mixture was stirred at ambient temperature for 16 h. The reaction mixture was cooled to 0°C, quenched with water (10 mL) and extracted with EtOAc (2 x 25 mL). The combined organic layers were dried (Na₂SO₄) and concentrated under reduced pressure. The residue was purified by column chromatography on silica gel eluting with EtOAc/hexane (0-20%) to obtain the title compound (200 mg, 63%).

¹H NMR (300 MHz, CDCl₃) δ 6.91 (d, 1H), 4.13 (t, 2H), 3.12 (t, 2H), 2.17 (s, 3H).

### Step 6: 7-Chloro-6-fluoro-4-methyl-3,4-dihydro-2H-1,4-benzoxazine-5-carboxylic acid

n-BuLi (2.5 M in hexane) (0.32 mL, 0.78 mmol) was added to a solution of 5-bromo-7-chloro-6-fluoro-4-methyl-3,4-dihydro-2H-1,4-benzoxazine (200 mg, 0.71 mmol) in THF (7.0 mL) at -78°C and the reaction mixture was stirred at that temperature for 1 h. CO₂ gas was bubbled for 40 min. The reaction mixture was slowly brought to ambient temperature, quenched with water (15 mL) and extracted with EtOAc (2 x 20 mL). The aqueous layer was acidified to pH ~2.0 using 1.0 N HCl and extracted with EtOAc (2 x 25 mL). The combined organic layers were dried (Na₂SO₄) and concentrated under reduced pressure to afford the title compound (102 mg, 58%).

¹H NMR (300 MHz, CDCl₃) δ 7.08 (d, *J*_{H-F} = 6.9 Hz, 1H), 4.31 (t, 2H), 3.28 (t, 2H), 2.87 (s, 3H).

ES-MS: *m*/*z* 246.0 (M+H).

HPLC: Retention time 9.27 min., purity: 95.6% at 280 nm.

### Example 4: 7-Chloro-6-fluoro-4-methyl-3,4-dihydro-2H-1,4-benzoxazine-8-carboxylic acid (A-4)

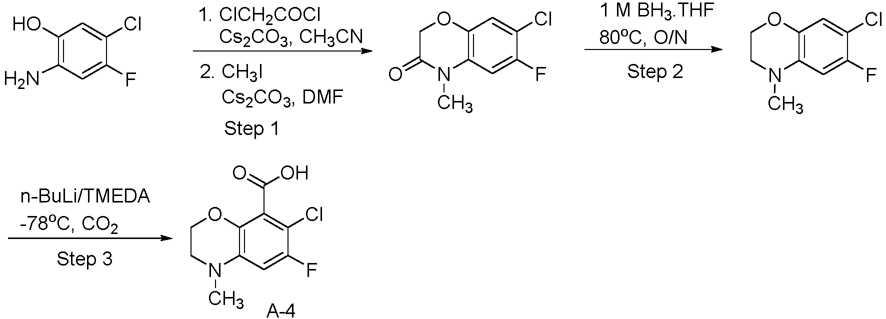

### Step 1: 7-Chloro-6-fluoro-4-methyl-2H-benzo[b][1,4]oxazin-3(4H)-one

To a solution of 2-amino-5-chloro-4-fluorophenol (0.997 g, 6.2 mmol) in acetonitrile (25 mL), Cs₂CO₃ (6.1 g, 18.6 mmol) was added, followed by 2-chloroacetyl chloride (0.732 g, 6.5 mmol) at 0°C. The reaction mixture was stirred at ambient temperature for 16 h. The solvent was removed under reduced pressure and the crude product was filtered through a celite pad with CH₂Cl₂/CH₃OH (5/1) and concentrated. The residue was dissolved in DMF (10 mL) then Cs₂CO₃ (6.1 g, 18.6 mmol) and methyl iodide (2.2 g, 15.5 mmol) was added. The reaction mixture was stirred at ambient temperature for 16 h., solvent was removed under reduced pressure, purified by column chromatography on silica gel eluting with CH₂Cl₂/CH₃OH (0 - 2%) to provide the title compound (0.8 g, 60%) as a gum.

¹H NMR (300 MHz, CD₃OD) δ 7.17 - 7.09 (m, 2H), 4.87 (s, 2H), 3.35 (s, 3H).

¹⁹F NMR (300 MHz, CD₃OD) δ -124.21 ppm.

### Step 2: 7-Chloro-6-fluoro-4-methyl-2,3-dihydro-1,4-benzoxazine

A mixture of 7-chloro-6-fluoro-4-methyl-2H-benzo[b][1,4]oxazin-3(4H)-one (0.800 g, 3.71 mmol), and 1M BH₃.THF (14.84 mL, 14.84 mmol) was heated at reflux for 16 h. The reaction mixture was cooled, carefully quenched with CH₃OH and evaporated. The residue was purified by column chromatography on silica gel eluting with hexane/EtOAc (0-20%) to afford the title compound (0.24 g, 32%) as a brown solid.

¹H NMR (300 MHz, CDCl₃) δ 6.72 (d, 1H), 6.38 (d, 1H), 4.22 (t, 2H), 3.25 (t, 2H), 2.84 (s, 3H).

¹⁹F NMR (300 MHz, CDCl₃) δ -125.12 ppm.

### Step 3: 7-Chloro-6-fluoro-4-methyl-3,4-dihydro-2H-1,4-benzoxazine-8-carboxylic acid

TMEDA (76 mg, 0.655 mmol) and n-BuLi (2.5 M in hexane) (0.262 mL, 0.655 mmol) were added to a solution of 7-chloro-6-fluoro-4-methyl-2,3-dihydro-1,4-benzoxazine (20 mg, 0.595 mmol) in THF (12 mL) at -78°C. The reaction mixture was stirred at that temperature for 1.5 h. and CO₂ gas was bubbled for 10 min. The reaction mixture was stirred at -78°C for 1 h., slowly brought to ambient temperature and quenched with 1M NaOH (5 mL). The reaction mixture was washed with EtOAc (2 x 10 mL), the aqueous layer was acidified to pH ~2.0 using 1.0 N HCl and extracted with EtOAc (2 x 20 mL). The combined organic extracts were dried (Na₂SO₄), concentrated, and crystallised from CH2Cl2/hexane to provide the title compound (55 mg, 38%) as an off-white solid. ¹H NMR (300 MHz, CDCl₃) δ 11.28- 10.06 (br s, 1H), 6.46 (d, *J*_{H-F}=11.3 Hz, 1H), 4.31(t, 2H), 3.33 (t, 2H), 2.89 (s, 3H).

¹⁹F NMR (300 MHz, CDCl₃) δ -122.83 ppm.

ES-MS: *m*/*z* 244.2 (M-H).

HPLC Retention Time: 9.10 min.; purity >98% at 280 nm.

### Example 5: 5-Chloro-7-fluoro-3,4-dihydro-2H-1,4-benzoxazine-8-carboxylic acid (A-5)

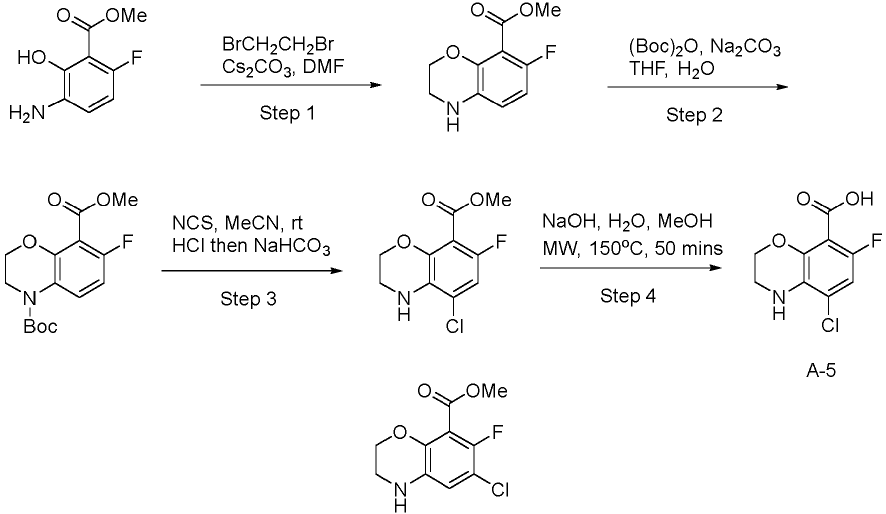

### Step 1: Methyl 7-fluoro-3,4-dihydro-2H-1,4-benzoxazine-8-carboxylate

Cs₂CO₃ (5.266 g, 16.20 mmol) and 1,2-dibromoethane (0.57 mL, 6.48 mmol) were added to a solution of methyl 3-amino-6-fluoro-2-hydroxybenzoate (1.0 g, 5.41 mmol) in DMF (10 mL). The reaction mixture was heated at 70°C for 16 h, cooled to ambient temperature, quenched with water (30 mL) and extracted with EtOAc (3 x 50 mL). The combined extracts were dried (Na₂SO₄) evaporated, and the residue purified by column chromatography on silica gel eluting with hexane/EtOAc (10 - 30%) to give the title compound, which was utilised directly in the next step.

¹H NMR (300 MHz, CDCl₃); δ 6.70 (d, 1H), 6.51 (m, 1H), 4.32 (t, 2H), 3.87 (s, 3H), 3.43 (t, 2H) ppm.

### Step 2: 8-Methyl 4-tert-butyl 7-fluoro-3,4-dihydro-2H-1,4-benzoxazine-4,8-dicarboxylate

Di-tert-butyl dicarbonate (1.136 g, 5.21 mmol) and DMAP (0.029 g, 0.24 mmol) were added to a solution of methyl 7-fluoro-3,4-dihydro-2H-1,4-benzoxazine-8-carboxylate (1.0 g, 4.74 mmol) in THF (15 mL). The reaction mixture was stirred at ambient temperature for 16 h, evaporated, and purified by column chromatography on silica gel eluting with EtOAc/hexane (10-20%) to provide the title compound (230 mg, 16 %) as an oil.

¹H NMR (300 MHz, CDCl₃); δ 7.75 (d, 1H), 6.65 (t 1H), 4.26 (t, 2H), 3.89(s, 3H), 3.82 (t, 2H), 1.49 (s, 9H) ppm.

¹⁹F NMR (300 MHz, CDCl₃); δ -119.15 ppm.

### Step 3: Methyl 5-chloro-7-fluoro-3,4-dihydro-2H-1,4-benzoxazine-8-carboxylate and methyl 6-chloro-7-fluoro-3,4-dihydro-2H-1,4-benzoxazine-8-carboxylate

NCS (0.118 g, 0.89 mmol) was added to a solution of 8-methyl 4*-tert-*butyl 7-fluoro-3,4-dihydro-2H-1,4-benzoxazine-4,8-dicarboxylate (0.230 g, 0.74 mmol) in CH₃CN (8 mL) and stirred at ambient temperature for 16 h. The reaction mixture was quenched with 2N HCl (5 mL), stirred for 15 min, neutralized with saturated Na₂CO₃ solution, and extracted with EtOAc (2 x 30 mL). The combined extracts were washed with brine (30 mL), dried (Na₂SO₄) and evaporated. The residue was purified by column chromatography on silica gel eluting with hexane/EtOAc (10 - 30%) to give methyl 5-chloro-7-fluoro-3,4-dihydro-2H-1,4-benzoxazine-8-carboxylate (15 mg 16%) and methyl 6-chloro-7-fluoro-3,4-dihydro-2H-1,4-benzoxazine-8-carboxylate both as oils

### Methyl 5-chloro-7-fluoro-3,4-dihydro-2H-1,4-benzoxazine-8-carboxylate

¹H NMR (300 MHz, CDCl₃) δ 6.63 (d, 1H), 4.27 (t, 2H), 3.92 1(s, 3H), 3.40 (t, 2H) ppm. ¹⁹F NMR (300 MHz, CD₃OD) δ -129.13.

### Methyl 6-chloro-7-fluoro-3,4-dihydro-2H-1,4-benzoxazine-8-carboxylate

¹H NMR (300 MHz, CDCl₃) δ 6.52 (d, *J*_{H-F}=7.4 Hz, 1H), 4.11 (t, 2H), 3.98 (s, 3H), 3.23(m, 2H) ppm.

### Step 4: 5-Chloro-7-fluoro-3,4-dihydro-2H-1,4-benzoxazine-8-carboxylic acid

1N NaOH (0.06 mL, 0.06 mmol) was added to a solution of methyl 6-chloro-7-fluoro-3,4-dihydro-2H-1,4-benzoxazine-8-carboxylate (15 mg, 0.06 mmol) in MeOH (1 mL) and water (2 mL). The reaction mixture was subjected to microwave conditions at 150°C for 50 min., cooled and evaporated. The product was neutralised with 0.1% formic acid in water and extracted with a mixture of CH₂Cl₂/CH₃OH (20 : 1) (3 x 10 mL), dried (Na₂SO₄) and evaporated to obtain the title acid (13 mg, 91%).

¹H NMR (300 MHz, CD₃OD) δ 7.23 (d, 1H), 4.34 (t, 2H), 3.53 (t, 2H) ppm.

¹⁹F NMR (300 MHz, CD₃OD) δ -121.79.

ES-MS: 230.5 [M-1].

HPLC: Retention time: 7.58 min., purity 97.2 % at 254 nm.

### Example 6: 6-Chloro-7-fluoro-3,4-dihydro-2H-1,4-benzoxazine-8-carboxylic acid (A-6)

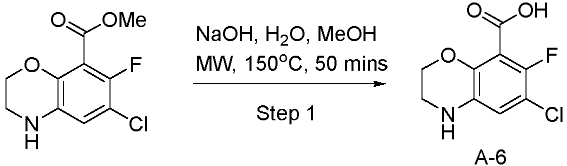

### Step 1: 6-Chloro-7-fluoro-3,4-dihydro-2H-1,4-benzoxazine-8-carboxylic acid

A 1N solution of NaOH (0.136 mL, 0.163 mmol) was added to a solution of methyl 6-chloro-7-fluoro-3,4-dihydro-2H-1,4-benzoxazine-8-carboxylate (0.020 g, 0.0814 mmol) in MeOH (1 mL) and water (2 mL). The reaction mixture was subjected to microwave conditions at 150°C for 50 min., cooled and evaporated. The residue was purified by semi-prep HPLC eluting with 10-100% acetonitrile/0.1% formic acid in water to provide the title compound (5 mg, 26 %) as a solid.

¹H NMR (300 MHz, CDCl₃) δ 6.56 (d, *J*_{H-F}=7.5 Hz, 1H), 4.12 (t, 2H), 3.21(m, 2H).

¹⁹F NMR (300 MHz, CDCl₃) δ 134.52

ES-MS: 232.60 [M+1].

HPLC: Retention Time: 7.57 min., purity 88.3 % at 254 nm.

### Example 7: 6-Chloro-7-fluoro-4-methyl-3,4-dihydro-2H-1,4-benzoxazine-8-carboxylic acid (A-7)

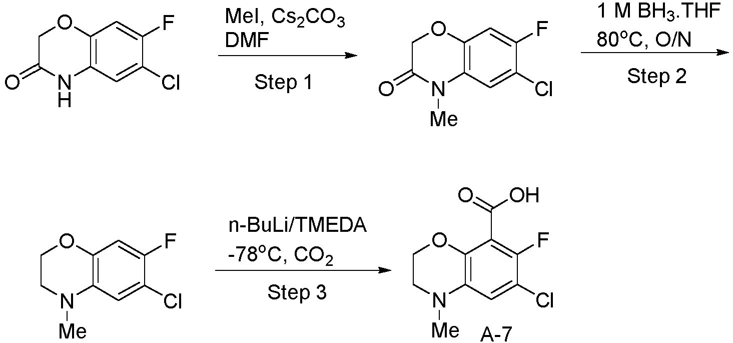

### Step 1: 6-Chloro-7-fluoro-4-methyl-2,4-dihydro-1,4-benzoxazin-3-one

Cs₂CO₃ (4.429 g, 13.6 mmol, 2.0 eq.) and CH₃I (1.27 mL, 20.39 mmol, 3.0 eq.) were added to a solution of 6-chloro-7-fluoro-2,4-dihydro-1,4-benzoxazin-3-one (1.37 g, 6.80 mmol, 1.0 eq.) in DMF (10 mL) the mixture stirred at ambient temperature for 18 h. EtOAc (25 mL) was added and the mixture was washed with sat. NaHCO₃ solution (3 x 50 mL). The organic layer was dried (Na₂SO₄), evaporated, and the residual yellow oil was used in the next step without further purification.

¹H NMR (300 MHz, CDCl₃); δ 7.26 (d, 1H), 7.12 (d, 1H), 4.90 (s, 2H), 3.62 (s, 3H) ppm. ¹⁹F NMR (300 MHz, CDCl₃); δ 119.83 ppm.

### Step 2: 6-Chloro-7-fluoro-4-methyl-3,4-dihydro-2H-1,4-benzoxazine

A mixture of 6-chloro-7-fluoro-4-methyl-2,4-dihydro-1,4-benzoxazin-3-one (400 mg, 1.855 mmol, 1.0 eq.) and 1M BH₃-THF solution (7.42 mL, 7.421 mmol, 4.0 eq.) was stirred at 70°C for 16 h. The reaction mixture was quenched with MeOH (20 mL), evaporated and 1N NaOH (50 mL) was added. Extraction with CH₂Cl₂ (3 x 50 mL) followed by washing of the combined extracts with brine (50 mL) provided an organic layer which was dried (Na₂SO₄). The residue on evaporation was purified by column chromatography on silica gel (0 - 5% EtOAc/hexane) to afford the title compound (320 mg, 85.5 % yield) as a brown solid

¹H NMR (300 MHz, CDCl₃); δ 6.59 (d, 1H), 6.56 (d, 1H), 4.20 (m, 2H), 3.21 (m, 2H), 2.82 (s, 3H) ppm.

¹⁹F NMR (300 MHz, CDCl₃); δ 128.65 ppm.

### Step 3: 6-Chloro-7-fluoro-4-methyl-3,4-dihydro-2H-1,4-benzoxazine-8-carboxylic acid

n-BuLi (2.5 M in hexane) (0.536 mL, 1.339 mmol, 1.5 eq.) was added to a solution of 6-chloro-7-fluoro-4-methyl-3,4-dihydro-2H-1,4-benzoxazine (180 mg, 0.893 mmol, 1.0 eq.) in THF (6 mL) and TMEDA (0.402 mL, 2.679 mmol, 3 eq.) at -78°C. The reaction mixture was stirred at the same temperature for 2 h. CO₂ (g) was bubbled for 10 min., and the reaction mixture was slowly brought to 0°C before being basified with 1M NaOH to pH ~11. It was washed with EtOAc (2 x 20 mL and the aqueous layer was separated and acidified with 1N HCl to pH ~1. The mixture was extracted with EtOAc (3 x 50 mL) and the combined extracts were dried (Na₂SO₄) and concentrated to provide the title compound as a solid (150 mg 68).

¹H NMR (300 MHz, CDCl₃); δ 6.76 (d, *J*_{H-F}=6.9 Hz, 1H), 4.35 (t, 2H), 3.29 (t, 2H), 2.89 (s, 3H) ppm.

¹⁹F NMR (300 MHz, CDCl₃); δ 134.24 ppm.

ES-MS: 244.27 [M-1].

HPLC: Retention Time: 9.26 min., purity >98% at 254 nm.

### Example 8: 7-Chloro-6-fluoro-1,4-dimethyl-1,2,3,4-tetrahydroquinoxaline-5-carboxylic acid (A-8)

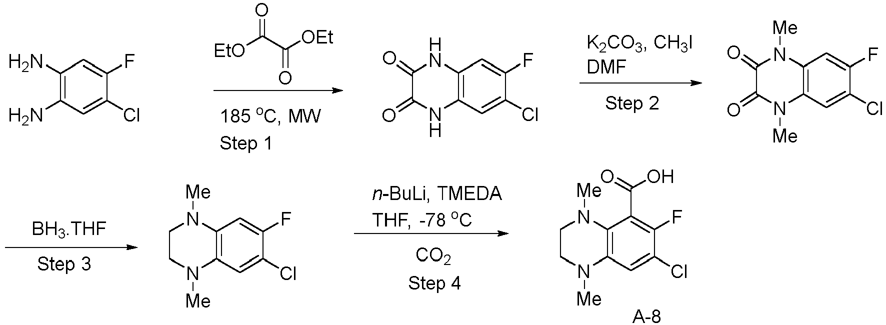

### Step 1: 6-Chloro-7-fluoroquinoxaline-2,3(1H,4H)-dione

A mixture of 4-chloro-5-fluorobenzene-1,2-diamine (2.0 g, 12.45 mmol), and diethyl oxalate (8.5 mL, 62.3 mmol) was heated under microwave conditions at 180°C for 1.5h. The reaction mixture was cooled, hexane (50 mL) was added. The solid was filtered and washed with additional hexane to provide the title compound (1.5 g, 56%) as a dark solid.

¹H NMR (300 MHz, DMSO-d₆) δ 12.04 (br s, 1H), 11.96 (br s, 1H), 7.20 (d, 1H), 7.06 (d, 1H).

¹⁹F NMR (300 MHz, DMSO-d₆) δ -122.97 ppm.

### Step 2: 6-Chloro-7-fluoro-1,4-dimethylquinoxaline-2,3(1H,4H)-dione

K₂CO₃ (2.9 g, 21.0 mmol) and methyl iodide (2.6 g, 18.2 mmol) were added to a solution of 6-chloro-7-fluoroquinoxaline-2,3(1H,4H)-dione (1.5 g, 7.0 mmol), in DMF (12 mL). The reaction mixture was heated at 50°C for 3 h. The solvent was removed under reduced pressure and the residue was purified by column chromatography on silica gel eluting with CH₂Cl₂/CH₃OH (0 -3%) to provide the title compound (0.5 g, 29%) as a brown solid.

¹H NMR (300 MHz, CD₃OD) δ 7.51 (d, 1H), 7.38 (d, 1H), 3.59 (s, 3H), 3.58 (s, 3H).

¹⁹F NMR (300 MHz, CD₃OD) δ -121.80 ppm.

### Step 3: 6-Chloro-7-fluoro-1,4-dimethyl-1,2,3,4-tetrahydroquinoxaline

6-Chloro-7-fluoro-1,4-dimethylquinoxaline-2,3(1H,4H)-dione (0.3 g, 1.24 mmol), and 1M BH₃.THF (4.95 mL, 4.95 mmol) were heated at reflux for 16 h. The reaction mixture was cooled, carefully quenched with CH₃OH, evaporated and the residue purified by column chromatography on silica gel eluting with hexane/EtOAc (0-50%) to obtain the title compound (0.12 g, 45%) as a light yellow solid.

¹H NMR (300 MHz, CDCl₃) δ 6.37 (d, 1H), 6.25 (d, 1H), 3.34 (t, 2H), 3.32(t, 2H), 2.82 (s, 3H), 2.80 (s, 3H).

¹⁹F NMR (300 MHz, CDCl₃) δ -129.20 ppm.

### Step 4: 7-Chloro-6-fluoro-1,4-dimethyl-1,2,3,4-tetrahydroquinoxaline-5-carboxylic acid

n-BuLi (2.5 M in hexane) (0.25 mL, 0.615 mmol) was added to a solution of 6-chloro-7-fluoro-1,4-dimethyl-1,2,3,4-tetrahydroquinoxaline (120 mg, 0.559 mmol) in THF (10 mL) at -78°C, along with TMEDA (71.5 mg, 0.615 mmol). The reaction mixture was stirred at that temperature for 1.5 h., and CO₂ gas was bubbled for 10 min. The reaction mixture was stirred at -78°C for a further 1 h., slowly brought to ambient temperature and quenched with 1M NaOH (5 mL). The mixture was washed with EtOAc (2 x 10 mL), the aqueous layer was acidified to pH ~2.0 using 1.0 N HCl and concentrated. The residue was purified by semi-prep HPLC eluting 0 - 100% acetonitrile/0.1% formic acid in water to obtain the title compound (5 mg, 4%) as a gum.

¹H NMR (300 MHz, CDCl₃) δ 6.67 (d, *J*_{H-F}=7.5 Hz, 1H), 3.31 - 3.26 (m, 2H), 3.25 - 3.19 (m, 2H), 2.90 (s, 3H), 2.89 (s, 3H).

¹⁹F NMR (300 MHz, CDCl₃) δ -133.88 ppm.

ES-MS: *m*/*z* 259.1 (M-H).

HPLC: Retention time 6.71 min., purity 96.0% at 254nm.

### Example 9: 6-Chloro-7-fluoro-2,2,4-trimethyl-3,4-dihydro-2H-1,4-benzoxazine-8-carboxylic acid (A-9)

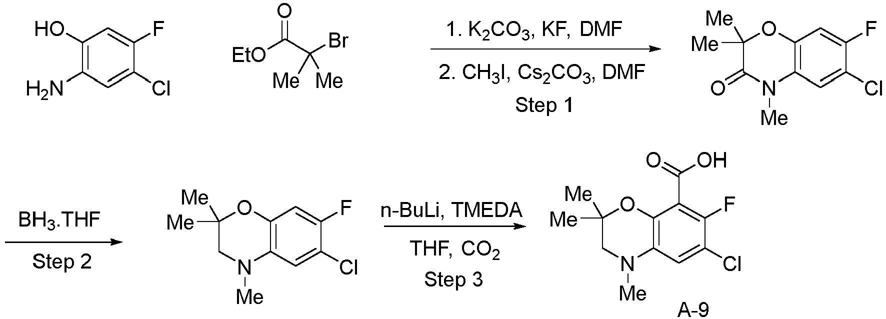

### Step 1: 6-Chloro-7-fluoro-2,2,4-trimethyl-1,4-benzoxazin-3-one

A mixture of KF (0.72 g, 12.4 mmol) and ethyl 2-bromo-2-methylpropanoate (1.21 g, 6.2 mmol) in DMF (5 mL) was added to a solution of 2-amino-4-chloro-5-fluorophenol (0.5 g, 3.1 mmol) and the reaction mixture was stirred at 60°C for 16 h in a sealed tube. The black solution was poured into a mixture of crushed ice and water; a solid formed, which was collected by filtration after the ice had melted. The solid was washed with water (15 mL), dried, and used for next stage without purification. It was dissolved in DMF (10 mL), Cs₂CO₃ (4.04 g, 12.4 mmol), methyl iodide (1.32 g, 9.3 mmol) were added and the reaction mixture stirred at ambient temperature for 16 h. The solvent was removed under reduced pressure and the residue, purified by column chromatography on silica gel eluting with CH₂Cl₂/CH₃OH (0-5%) to obtain the title compound (0.22 g, 29%) as an orange solid.

¹H NMR (300 MHz, CDCl₃) δ 6.90 (d, 1H), 6.76 (d, 1H), 3.30 (s, 3H), 1.47 (s, 6H).

¹⁹F NMR (300 MHz, CD₃OD) δ -120.64 ppm.

### Step 2: 6-Chloro-7-fluoro-2,2,4-trimethyl-3H-1,4-benzoxazine

A mixture of 6-chloro-7-fluoro-2,2,4-trimethyl-1,4-benzoxazin-3-one (0.22 g, 0.9 mmol), and 1M BH₃.THF (3.6 mL, 3.6 mmol) was heated at reflux for 16 h. The reaction mixture was cooled, carefully quenched with CH₃OH and evaporated. The residue was purified by column chromatography on silica gel eluting with hexane/EtOAc (0-20%) to obtain the title compound (0.20 g, 97%) as a colourless oil.

¹H NMR (300 MHz, CDCl₃) δ 6.59 (d, 1H), 6.56 (d, 1H), 2.91 (s, 2H), 2.86 (s, 3H), 1.31 (s, 6H).

¹⁹F NMR (300 MHz, CD₃OD) δ -128.82 ppm.

### Step 3: 6-chloro-7-fluoro-2,2,4-trimethyl-3,4-dihydro-2H-1,4-benzoxazine-8-carboxylic acid

TMEDA (0.11 g, 0.96 mmol) and n-BuLi (2.5 M in hexane) (0.384 mL, 0.96 mmol) were added to a solution of 6-chloro-7-fluoro-2,2,4-trimethyl-3H-1,4-benzoxazine (0.2 g, 0.87 mmol) in THF (10 mL) at -78°C. The reaction mixture was stirred at that temperature for 1.5 h., and CO₂ gas was bubbled for 10 min. The reaction mixture was stirred at - 78°C for 1 h., slowly brought to ambient temperature and quenched with 1M NaOH (5 mL). The reaction mixture was extracted with EtOAc (2 x 10 mL), the aqueous layer was acidified to pH ~2.0 using 1.0 N HCl and extracted with EtOAc (2 x 20 mL). The combined organic layers were dried (Na₂SO₄), concentrated to provide the title compound (0.062 g, 26%) as an orange solid.

¹H NMR (300 MHz, CDCl₃) δ 6.75 (d, *J*_{H-F}=6.9 Hz, 1H), 3.04 (s, 2H), 2.92 (s, 3H), 1.41 (s, 6H).

¹⁹F NMR (300 MHz, CDCl₃) δ -126.67 ppm.

ES-MS: *m*/*z* 272.3 (M-H).

HPLC: Retention time 10.90 min., purity >98% at 254 nm.

### Example 10: 7-Chloro-6-methoxy-4-methyl-3,4-dihydro-2H-1,4-benzoxazine-5-carboxylic acid (A-10)

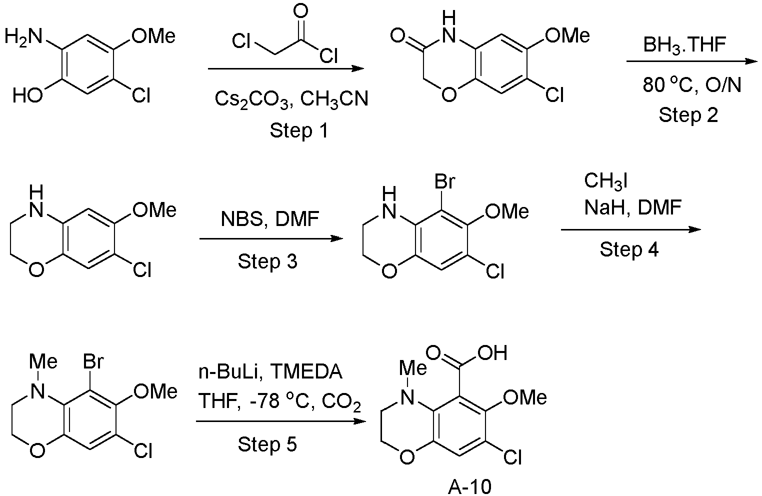

### Step 1: 7-Chloro-6-methoxy-2H-benzoxazin-3(4H)-one

Cs₂CO₃ (11.0 g, 33.9 mmol) and 2-chloroacetyl chloride (1.54 g, 13.6 mmol) were added to a solution of 2-amino-5-chloro-4-methoxyphenol (1.97 g, 11.3 mmol) at 0°C in acetonitrile (25 mL) and the mixture was stirred at ambient temperature for 16 h. The solvent was removed under reduced pressure and the crude product filtered through celite pad using 20% CH₃OH/ CH2Cl2, concentrated to obtain the title compound (2.3 g, 95%) as a brown gum.

¹H NMR (300 MHz, CDCl₃) δ 9.20 (br s, 1H) 7.02 (s, 1H), 6.42 (s, 1H), 4.57 (s, 2H), 3.84 (s, 3H).

### Step 2: 7-Chloro-6-methoxy-3,4-dihydro-2H-benzo[b][1,4]oxazine

A mixture of 7-chloro-6-methoxy-2H-benzo[b][1,4]oxazin-3(4H)-one (2.3 g, 10.77 mmol) and 1M BH₃.THF (43.1 mL, 43.1 mmol) was heated at reflux for 16 h. The mixture was cooled, carefully quenched with CH₃OH, evaporated and purified by column chromatography on silica gel eluting with CH₃OH/CH₂Cl₂ (0-2%) to obtain the title compound (1.2 g, 53%) as a brown solid.

¹H NMR (300 MHz, CDCl₃) δ 6.79 (s, 1H), 6.21 (s, 1H), 4.18 (t, 2H), 3.78 (s, 3H), 3.40 (t, 2H).

### Step 3: 5-Bromo-7-chloro-6-methoxy-3,4-dihydro-2H-benzo[b][1,4]oxazine

NBS (0.71 g, 4.0 mmol) was added to a solution of 7-chloro-6-methoxy-3,4-dihydro-2H-benzo[b][1,4]oxazine (0.8 g, 4.0 mmol) in DMF (5 mL) at ambient temperature. The mixture was stirred at ambient temperature for 16 h. The solvent was removed under reduced pressure and the residue purified by column chromatography on silica gel eluting with EtOAc/hexane (0-50%) to obtain the title compound (0.15 g, 13%) as a light brown solid.

¹H NMR (300 MHz, CDCl₃) δ 6.79 (s, 1H), 4.18 (t, 2H), 3.81 (s, 3H), 3.47 (t, 2H).

### Step 4: 5-Bromo-7-chloro-6-methoxy-4-methyl-3,4-dihydro-2H-benzo[b][1,4]oxazine

NaH (60% dispersion in mineral oil) (0.77 g, 5.4 mmol) and methyl iodide (0.192 g, 1.35 mmol) were added to a solution of 5-bromo-7-chloro-6-methoxy-3,4-dihydro-2H-benzo[b][1,4]oxazine (0.1 g, 0.54 mmol) in DMF (3 mL). The mixture was stirred at ambient temperature for 16 h and the solvent was removed under reduced pressure and the residue purified by column chromatography on silica gel eluting with EtOAc/hexane (0-30%) to obtain the title compound (0.22 g, 29%) as a colourless oil.

¹H NMR (300 MHz, CDCl₃) δ 6.88 (s, 1H), 4.13(t, 2H), 3.83 (s, 3H), 3.11 (t, 2H), 2.85 (s, 3H).

### Step 5: 7-Chloro-6-methoxy-4-methyl-3,4-dihydro-2H-1,4-benzoxazine-5-carboxylic acid

n-BuLi (2.5 M in hexane) (0.15 mL, 0.38 mmol) was added to a solution of 5-bromo-7-chloro-6-methoxy-4-methyl-3,4-dihydro-2H-benzo[b][1,4]oxazine (0.1 g, 0.342 mmol) in THF (6 mL) at -78°C. The mixture was stirred at that temperature for 0.5 h., CO₂ gas was bubbled for 10 min. and the reaction stirred at -78°C for a further 1 h. and slowly brought to ambient temperature before being quenched with 1M NaOH (5 mL). The mixture was washed with EtOAc (2 x 10 mL), the aqueous layer was acidified to pH ~2.0 using 1.0 N HCl and extracted with EtOAc (2 x 20 mL). The combined organic layers were dried (Na₂SO₄), concentrated and the residue crystallised from CH₂Cl₂/hexane to provide the title compound (9 mg, 10%) as an off-white solid.

¹H NMR (300 MHz, CDCl₃) δ 10.02- 9.80 (br s, 1H), 6.97 (s, 1H), 4.21 (t, 2H), 3.87 (s, 3H), 3.24 (t, 2H), 2.88 (s, 3H).

ES-MS: *m*/*z* 244.2 (M-H).

HPLC: Retention time 9.10 min., purity >98% at 280 nm.

### Example 11: 7-Chloro-6-fluoro-3,4-dihydro-2H-1,4-benzoxazine-5-carboxylic acid (A-11)

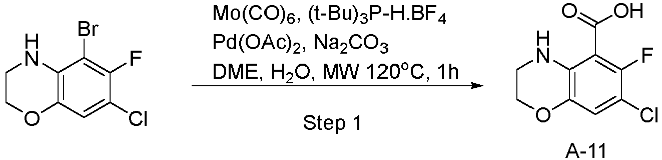

### Step 1: 7-Chloro-6-fluoro-3,4-dihydro-2H-1,4-benzoxazine-5-carboxylic acid

A mixture of 5-bromo-7-chloro-6-fluoro-3,4-dihydro-2H-1,4-benzoxazine (300 mg, 1.13 mmol), Mo(CO)₆ (357 mg,1.36 mmol), (t-Bu)₃-H BF4 (17 mg, 0.06 mmol), Pd(OAc)₂ (13 mg, 0.06 mmol) and Na₂CO₃ (179 mg, 1.68 mmol) in DME (3.0 mL) and water (1.0 mL) was heated at 120°C under microwave conditions for 1 h. The reaction mixture was diluted with water (25 mL) and washed with EtOAc (2 x 25 mL). The aqueous layer was acidified to pH ~2.0 using 1.0 N HCl, extracted with EtOAc (2 x 25 mL) and the combined extracts were dried (Na₂SO₄) and concentrated under reduced pressure to afford the title compound (65 mg, 25%).

¹H NMR (300 MHz, CDCl₃) δ 7.77 (br s, 1H), 6.92 (d, *J*_{H-F} = 7.2 Hz, 1H), 4.17 (t, 2H), 3.54 (t, 2H).

ES-MS: *m*/*z* 230.4 (M-H).

HPLC: Retention time 9.93 min., purity >98% at 254 nm.

### Example 12: 7-Chloro-8-methoxy-5-methyl-2,3,4,5-tetrahydro-1,5-benzoxazepine-9-carboxylic acid (A-12)

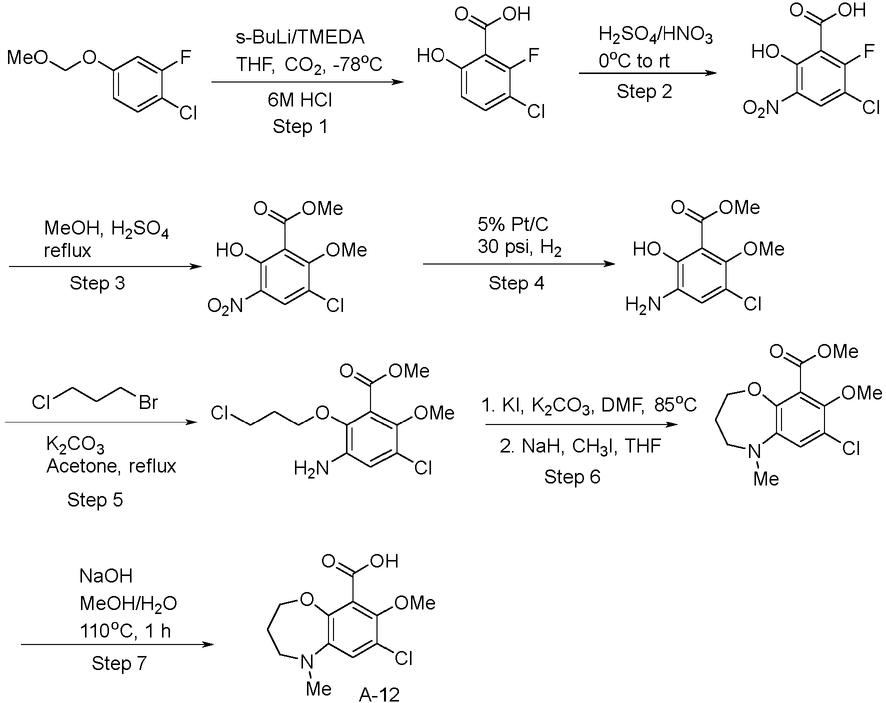

### Step 1: 3-Chloro-2-fluoro-6-hydroxybenzoic acid

s-BuLi (1.4 M in cyclohexane) (8.25 mL, 11.55 mmol) was added to a solution of 1-chloro-2-fluoro-4-(methoxymethoxy)benzene (2.0 g, 10.5 mmol) in THF (25 mL) at - 78°C and TMEDA (1.34 g, 11.55 mmol). The reaction mixture was stirred at that temperature for 1.5 h., and CO₂ gas was bubbled for 10 min. The reaction mixture was stirred for a further hour at -78°C for 1 h., slowly brought to ambient temperature and quenched with 1M NaOH (20 mL). The reaction mixture was washed with EtOAc (2 x 10 mL), the aqueous layer was acidified to pH ~1.0 using 6.0 N HCl (20 mL) and stirred at ambient temperature for 16 h before being extracted with EtOAc (2 x 35 mL). The combined organic extracts were dried (Na₂SO₄) and concentrated to obtain the title compound (1.4 g, 70%) as a white solid.

¹H NMR (300 MHz, CD₃OD) δ 7.56 - 7.46 (m, 1H), 6.79 (dd, 1H).

¹⁹F NMR (300 MHz, CD₃OD) δ -108.56 ppm.

### Step 2: 3-Chloro-2-fluoro-6-hydroxy-5-nitrobenzoic acid

70% HNO₃ (0.46 g, 5.12 mmol) was added to a solution of 3-chloro-2-fluoro-6-hydroxybenzoic acid (0.75 g, 3.94 mmol) in sulphuric acid (20 mL) at 0°C. The reaction mixture was stirred at ambient temperature for 16 h. The solution was poured into a mixture of crushed ice and water and a solid formed, which was collected by filtration after the ice had melted. The solid was washed with water (15 mL) and dried to obtain the title compound (0.70 g, 75%) as a light yellow solid.

¹H NMR (300 MHz, CD₃OD) δ 8.39 (d, 1H).

¹⁹F NMR (300 MHz, CD₃OD) δ -100.38 ppm.

### Step 3: Methyl 3-chloro-6-hydroxy-2-methoxy-5-nitrobenzoate

A solution of 3-chloro-2-fluoro-6-hydroxy-5-nitrobenzoic acid (0.70 g, 2.97 mmol) in CH₃OH (20 mL) and sulphuric acid (2 mL) was heated at reflux for 48 hours. The reaction mixture was cooled, evaporated, and purified by column chromatography on silica gel eluting with CH₃OH/CH₂Cl₂ (0-30%) to obtain the title compound (0.5 g, 64%) as a yellow oil.

¹H NMR (300 MHz, CDCl₃) δ 10.90 (br s, 1H), 8.23 (s, 1H), 4.02 (s, 3H), 3.98 (s, 3H).

### Step 4: Methyl 3-amino-5-chloro-2-hydroxy-6-methoxybenzoate

5% Pt/C catalyst (0.05 g) was added to a solution of methyl 3-chloro-6-hydroxy-2-methoxy-5-nitrobenzoate (0.50 g, 2.97 mmol) in EtOAc (20 mL). The reaction mixture was stirred under a hydrogen atmosphere at 30 psi for 4 h., filtered through a celite pad, washed with CH₃OH (20 mL) and concentrated to obtain the title compound (0.4 g, 90%) as a dark brown solid.

¹H NMR (300 MHz, CD₃OD) δ 6.93 (s, 1H), 3.99 (s, 3H), 3.76 (s, 3H).

### Step 5: Methyl 3-amino-5-chloro-2-(3-chloropropoxy)-6-methoxybenzoate

1-Bromo-3-chloropropane (2.37 g, 15.1 mmol), K₂CO₃ (2.1 g, 15.1 mmol) was added to a solution of methyl 3-amino-5-chloro-2-hydroxy-6-methoxybenzoate (0.35 g, 1.51 mmol) in acetone (20 mL). The reaction mixture was heated at reflux for 16 h., cooled and extracted with EtOAc (2 x 20 mL), evaporated and the residue was purified by column chromatography on silica gel eluting with EtOAc/hexane (0-50%) to obtain the title compound (0.45 g, 97%) as a yellow oil.

¹H NMR (300 MHz, CD₃OD) δ 6.82 (s, 1H), 4.09 (t, 2H), 3.93 (s, 3H), 3.81 (s, 3H), 3.76 (t, 2H), 2.23 - 2.12 (m, 2H).

### Step 6: Methyl 7-chloro-8-methoxy-5-methyl-2,3,4,5-tetrahydrobenzo[b][1,4]oxazepine-9-carboxylate

K₂CO₃ (0.4 g, 2.92 mmol) and KI (0.485 g, 2.92 mmol) were added to a solution of methyl 3-amino-5-chloro-2-(3-chloropropoxy)-6-methoxybenzoate (0.45 g, 1.46 mmol) in DMF (6 mL) and the reaction mixture was heated at 85°C for 48 h., cooled and evaporated. The crude residue in THF (10 mL) was mixed with NaH (60% in mineral oil) (0.234 g, 5.84 mmol) at 0°C and the reaction mixture was stirred for 30 min. Methyl iodide (0.41 g, 2.92 mmol) was added at 0°C and the reaction stirred at ambient temperature for 16 h. The reaction mixture was treated with water (20 mL) and the mixture extracted with EtOAc (2 x 20 mL). The combined extracts were evaporated, and the residue purified by column chromatography on silica gel eluting with EtOAc/hexane (0-50%) to obtain the title compound (0.026 g, 6%) as a yellow oil.

¹H NMR (300 MHz, CD₃OD) δ 6.83 (s, 1H), 4.07 (t, 2H), 3.91 (s, 3H), 3.83 (s, 3H), 3.13 (t, 2H), 2.86 (s, 3H), 2.07 - 1.93 (m, 2H).

### Step 7: 7-Chloro-8-methoxy-5-methyl-2,3,4,5-tetrahydro-1,5-benzoxazepine-9-carboxylic acid

NaOH (0.018 g, 0.46 mmol) was added to a solution of methyl 7-chloro-8-methoxy-5-methyl-2,3,4,5-tetrahydrobenzo[b][1,4]oxazepine-9-carboxylate (0.026 g, 0.091 mmol) in a mixture of MeOH and H₂O (3 and 1 mL). The reaction mixture was heated at 110°C for 1 h. under microwave conditions, cooled and evaporated. The residue was purified by semi-prep HPLC eluting with 10 - 100% acetonitrile/ 0.1% formic acid in water to obtain the title compound (15 mg, 61%) as a colourless liquid.

¹H NMR (300 MHz, CD₃OD) δ 6.92 (s, 1H), 4.07 (t, 2H), 3.84 (s, 3H), 3.13 (t, 2H), 2.87 (s, 3H), 2.08 - 1.99 (m, 2H).

ES-MS: *m*/*z* 270.2 (M-H).

HPLC: Retention time 7.99 min., purity >98% at 254 nm.

### Example 13: 7-Chloro-8-fluoro-5-methyl-2,3,4,5-tetrahydro-1,5-benzoxazepine-9-carboxylic acid (A-13) and 7-chloro-8-ethoxy-5-methyl-2,3,4,5-tetrahydro-1,5-benzoxazepine-9-carboxylic acid (A-16)

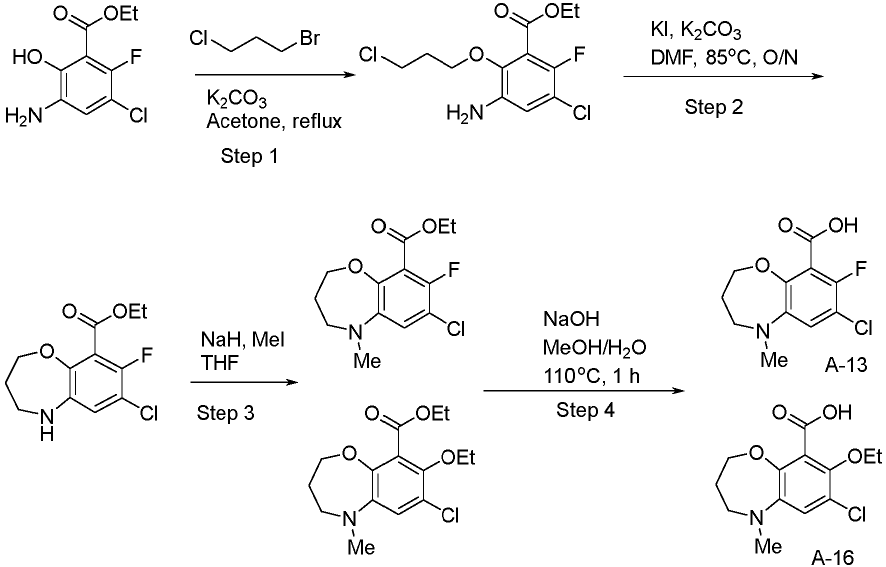

### Step 1: Ethyl 3-amino-5-chloro-2-(3-chloropropoxy)-6-fluorobenzoate

K₂CO₃ (2.96 g, 21.6 mmol), and 1-bromo-3-chloropropane (0.40 g, 2.57 mmol) was added to a solution of ethyl 3-amino-5-chloro-6-fluoro-2-hydroxybenzoate (0.5 g, 2.14 mmol) in acetone (30 mL). The reaction mixture was heated at reflux for 16 h, cooled, treated with water (20 mL) and extracted with EtOAc (2 x 20 mL). The combined extracts were dried (Na₂SO₄), evaporated and the residue purified by column chromatography on silica gel eluting with hexane/EtOAc (0-50%) to provide the title compound (0.45 g, 68%) as a yellow oil.

¹H NMR (300 MHz, CDCl₃) δ 6.81 (d, 1H), 4.41 (q, 2H), 4.10 (t, 2H), 3.76 (t, 2H), 2.25 - 2.08 (m, 2H), 1.38 (t, 3H).

¹⁹F NMR (300 MHz, CDCl₃) δ -128.62 ppm.

### Step 2: Ethyl 7-chloro-8-fluoro-2,3,4,5-tetrahydrobenzo[b][1,4]oxazepine-9-carboxylate

K₂CO₃ (0.393 g, 2.84 mmol), and KI (0.47 g, 2.84 mmol) were added to a solution of ethyl 3-amino-5-chloro-2-(3-chloropropoxy)-6-fluorobenzoate (0.44 g, 1.42 mmol) in DMF (6 mL). The reaction mixture was heated at 85°C for 16 h in a sealed tube, cooled and evaporated. The residue was purified by column chromatography on silica gel eluting with hexane/EtOAc (10 - 50%) to give the title compound (0.38 g, 98%) as a light-yellow gum.

¹H NMR (300 MHz, CDCl₃) δ 6.80 (d, 1H), 4.40 (q, 2H), 4.10 (t, 2H), 3.20 (t, 2H), 2.07 - 1.95 (m, 2H), 1.37 (t, 3H).

¹⁹F NMR (300 MHz, CDCl₃) δ -127.45 ppm.

ES- MS: *m*/*z* 274.6 (M+H).

### Step 3: Ethyl 7-chloro-8-fluoro-5-methyl-2,3,4,5-tetrahydro-1,5-benzoxazepine-9-carboxylate and ethyl 7-chloro-8-ethoxy-5-methyl-2,3,4,5-tetrahydro-1,5-benzoxazepine-9-carboxylate

NaH (60% in mineral oil) (0.222 g, 5.55 mmol) was added to a solution of ethyl 7-chloro-8-fluoro-2,3,4,5-tetrahydrobenzo[b][1,4]oxazepine-9-carboxylate in DMF (4 mL) at 0°C and stirred for 30 min. Methyl iodide (0.24 g, 1.67 mmol) was added at 0°C and the reaction mixture stirred at ambient temperature for 16 h., evaporated and the residue purified by column chromatography on silica gel eluting with hexane/EtOAc (0 - 30%) to afford a mixture of ethyl 7-chloro-8-fluoro-5-methyl-2,3,4,5-tetrahydrobenzo[b][1,4]oxazepine-9-carboxylate and ethyl 7-chloro-8-ethoxy-5-methyl-2,3,4,5-tetrahydrobenzo[b][1,4]oxazepine-9-carboxylate (0.2 g, 50%) as a yellow gum, used in the next step without separation.

¹H NMR (300 MHz, CDCl₃) δ 6.80 (d, 1H), 4.46 - 4.31 (m, 2H), 4.14 - 3.98 (m, 2H), 3.17 - 3.04 (m, 2H), 2.85 (s, 3H), 2.08 - 1.94 (m, 2H), 1.44 - 1.26 (m, 6H).

¹⁹F NMR (300 MHz, CDCl₃) δ -127.45 ppm.

### Step 4: 7-Chloro-8-fluoro-5-methyl-2,3,4,5-tetrahydro-1,5-benzoxazepine-9-carboxylic acid and 7-chloro-8-ethoxy-5-methyl-2,3,4,5-tetrahydro-1,5-benzoxazepine-9-carboxylic acid

NaOH (0.084 g, 2.1 mmol) was added to a solution of 7-chloro-8-fluoro-5-methyl-2,3,4,5-tetrahydrobenzo[b][1,4]oxazepine-9-carboxylic acid and 7-chloro-8-ethoxy-5-methyl-2,3,4,5-tetrahydrobenzo[b][1,4]oxazepine-9-carboxylic acid (0.06 g, 0.21 mmol) in MeOH/H₂O (4/2 mL). The reaction mixture was heated at 110°C for 1 hour under microwave conditions, cooled and evaporated. The resultant residue was separated by semi-prep HPLC eluting with acetonitrile/0.1% formic acid in water 30 - 50% to provide 7-chloro-8-fluoro-5-methyl-2,3,4,5-tetrahydro-1,5-benzoxazepine-9-carboxylic acid (6 mg) as a gum and 7-chloro-8-ethoxy-5-methyl-2,3,4,5-tetrahydro-1,5-benzoxazepine-9-carboxylic acid (8.7 mg) as an off-white solid.

### 7-Chloro-8-fluoro-5-methyl-2,3,4,5-tetrahydro-1,5-benzoxazepine-9-carboxylic acid (A-13)

¹H NMR (300 MHz, CD₃OD) δ 6.91 (d, 1H), 4.10 (t, 2H), 3.13 (t, 2H), 2.87 (s, 3H), 2.09 - 1.97 (m, 2H).

¹⁹F NMR (300 MHz, CD₃OD) δ -130.57 ppm.

ES- MS: *m*/*z* 260.6 (M+H).

HPLC: Retention time 8.86 min., Purity: 98.7% @ 254 nm.

### 7-Chloro-8-ethoxy-5-methyl-2,3,4,5-tetrahydro-1,5-benzoxazepine-9-carboxylic acid (A-16)

¹H NMR (300 MHz, CD₃OD) δ 6.89 (s, 1H), 4.13 - 4.02 (m, 4H), 3.12 (t, 2H), 2.87 (s, 3H), 2.08 - 1.97 (m, 2H) 1.35 (t, 3H).

ES- MS: *m*/*z* 286.6 (M+H).

HPLC: Retention time 9.21 min., Purity: 95.9% @ 254 nm.

### Example 14: 6-Chloro-7-methoxy-2,2,4-trimethyl-3,4-dihydro-2H-1,4-benzoxazine-8-carboxylic acid (A-37)

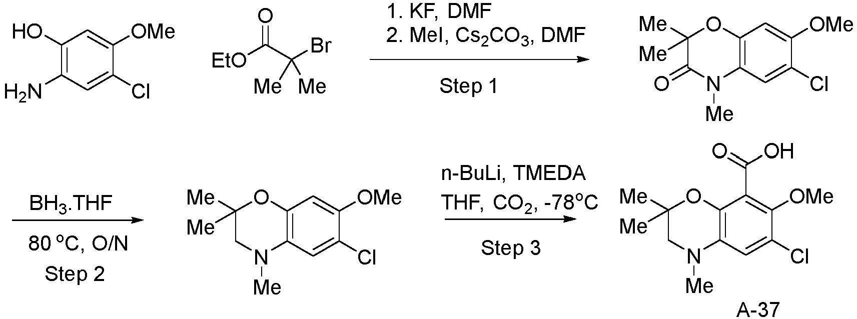

### Step 1: 6-Chloro-7-methoxy-2,2,4-trimethyl-2H-benzo[b][1,4]oxazin-3(4H)-one

KF (0.604 g, 10.4 mmol), and ethyl 2-bromo-2-methylpropanoate (1.0 g, 5.2 mmol) were added to a solution of 2-amino-4-chloro-5-methoxyphenol (0.45 g, 2.6 mmol) in DMF (5 mL) and the reaction mixture was stirred at 60°C for 16 h in a sealed tube. The dark solution was poured on to a mixture of crushed ice and water; a solid formed, which was collected by filtration after the ice had melted. The solid was washed with water (15 mL), dried *in vacuo* and utilised in the next step without purification.

Cs₂CO₃ (3.4 g, 10.4 mmol), and methyl iodide (1.1 g, 7.8 mmol) were added to the solid product from the previous step in DMF (10 mL) and the reaction mixture was stirred at ambient temperature for 16 h. The solvent was removed and the residue was purified by column chromatography on silica gel eluting with hexane/EtOAc (0 - 30%) to obtain the title compound (0.22 g, 33%) as a gum.

¹H NMR (300 MHz, CDCl₃) δ 7.00 (s, 1H), 6.67 (s, 1H), 3.90 (s, 3H), 3.37 (s, 3H), 1.55(s, 6H).

### Step 2: 6-Chloro-7-methoxy-2,2,4-trimethyl-3,4-dihydro-2H-benzo[b][1,4]oxazine

6-Chloro-7-methoxy-2,2,4-trimethyl-2H-benzo[b][1,4]oxazin-3(4H)-one (0.21 g, 0.82 mmol), and 1M BH₃.THF (3.3 mL, 3.3 mmol) were heated at reflux for 16 h. The reaction mixture was cooled, carefully quenched with methanol (10 mL) and evaporated. The resultant residue was purified by column chromatography on silica gel eluting with hexane/EtOAc (0 - 20%) to obtain the title compound (0.20 g, 97%) as an oil.

¹H NMR (300 MHz, CDCl₃) δ 6.70 (s, 1H), 6.50 (s, 1H), 3.85 (s, 3H), 2.94 (s, 2H), 2.89 (s, 3H), 1.39 (s, 6H).

### Step 3: 6-Chloro-7-methoxy-2,2,4-trimethyl-3,4-dihydro-2H-1,4-benzoxazine-8-carboxylic acid

s-BuLi (1.4 M in cyclohexane) (0.52 mL, 0.73 mmol) and TMEDA (0.085 g, 0.73 mmol) were added to a solution of 6-chloro-7-methoxy-2,2,4-trimethyl-3,4-dihydro-2H-benzo[b][1,4]oxazine (0.16 g, 0.66 mmol) in THF (6 mL) at -78°C. The reaction mixture was stirred at that temperature for 1.5 h and CO₂ gas was bubbled for 10 min. The reaction mixture was stirred at -78°C for 1 h., slowly brought to ambient temperature and quenched with 1N NaOH (5 mL). The reaction mixture was washed with EtOAc (2 x 10 mL), the aqueous layer was acidified to pH ~2.0 using 1.0 N HCl and extracted into EtOAc (2 x 20 mL). The combined extracts were dried (Na₂SO₄) and concentrated *in vacuo* to afford the title acid (0.04 g, 21%) as a white solid.

¹H NMR (300 MHz, CD₃OD) δ 6.74 (s, 1H), 3.81 (s, 3H), 3.02 (s, 2H), 2.92 (s, 3H), 1.33 (s, 6H).

ES- MS: *m*/*z* 284.2 (M-H).

HPLC: Retention time 10.39 min., Purity: >99% @ 254 nm.

### Example 15: 6-chloro-7-fluoro-4-methyl-3,4-dihydro-2H-1,4-benzothiazine-8-carboxylic acid (A-19)

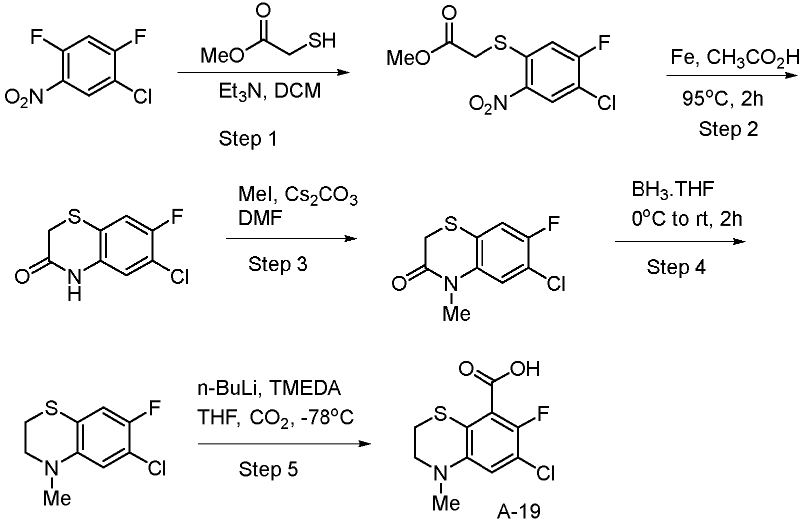

### Step 1: Methyl (4-chloro-5-fluoro-2-nitrophenylthio)acetate

Triethylamine (4.40 mL, 31.0 mmol) was added to a solution of 1-chloro-2,4-difluoro-5-nitrobenzene (5.00 g, 25.8 mmol) and methyl thioglycolate (2.30 mL, 25.8 mmol) in CH₂Cl₂ (40 mL) at 0°C. The reaction mixture was stirred at ambient temperature for 16 h and quenched with 1.0 N HCl (75 mL). The organic layer was dried (Na₂SO₄), concentrated under reduced pressure and the residue was purified by column chromatography on silica gel eluting with CH₂Cl₂ to obtain the title compound (5.06 g, 70%).

¹H NMR (300 MHz, CDCl₃); δ 8.11 (d, 1H), 7.21 (d, 1H), 3.81 (s, 2H), 3.79 (s, 3H).

### Step 2: 6-Chloro-7-fluoro-2,4-dihydro-1,4-benzothiazin-3-one

Fe powder (3.99 g, 71.4 mmol) was added to a solution of methyl (4-chloro-5-fluoro-2-nitrophenylthio)acetate (4.00 g, 17.2 mmol) in AcOH (55.0 mL) at ambient temperature. The reaction mixture was heated at 95°C for 2 h and stirred at ambient temperature for 20 h. The reaction mixture was quenched with 2.0 N HCl (200 mL) and stirred for 1 h. The precipitate was collected by filtration, washed with water (20 mL) and dissolved in EtOAc (60 mL). The solution was washed with water (60 mL) and the organic layer dried (Na₂SO₄), concentrated under reduced pressure and the residue was triturated with hexanes (10 mL) to provide the title compound (0.41 g, 11 %).

¹H NMR (300 MHz, CDCl₃); δ 8.26 (br s, 1H), 7.14 (d, 1H), 6.91 (d, 1H), 3.43 (s, 2H).

### Step 3: 6-Chloro-7-fluoro-4-methyl-2,4-dihydro-1,4-benzothiazin-3-one

CH₃I (0.31 mL, 6.18 mmol) was added to a suspension of 6-chloro-7-fluoro-2,4-dihydro-1,4-benzothiazin-3-one (0.27 g, 1.24 mmol) and Cs₂CO₃ (0.61 g, 1.87 mmol) in DMF (6.0 mL) in a sealed tube at 0°C. The reaction mixture was stirred at ambient temperature for 16 h, quenched with water (20 mL) and extracted with EtOAc (2 x 20 mL). The combined extracts were washed with 1.0 N HCl (2 x 20 mL), dried (Na₂SO₄) and concentrated under reduced pressure to obtain the title compound (180 mg, 63%). ¹H NMR (300 MHz, CDCl₃); δ 7.17 (d, 1H), 7.09 (d, 1H), 3.42 (s, 5H).

### Step 4: 6-Chloro-7-fluoro-4-methyl-3,4-dihydro-2H-1,4-benzothiazine

BH₃-THF (1.0 M solution) (4.0 mL, 5.42 mmol) was added to 6-chloro-7-fluoro-4-methyl-2,4-dihydro-1,4-benzothiazin-3-one (180 mg, 0.78 mmol) at 0°C and the reaction mixture was stirred at ambient temperature for 2 h. The reaction mixture was cooled to 0 °C and quenched with 1.0 N NaOH (3.0 mL). EtOAc (20 mL) and water (20 mL) were added to the mixture, and layers separated. The aqueous layer was extracted with EtOAc (2 x 15 mL) and combined extracts were dried (Na₂SO₄) and concentrated under reduced pressure to obtain the title compound (150 mg, 89 %).

¹H NMR (300 MHz, CDCl₃); δ 6.86 (d, 1H), 6.61 (d, 1H), 3.52 - 3.48 (m, 2H), 3.09 - 3.06 (m, 2H), 2.91 (s, 3H).

Step 5: 6-Chloro-7-fluoro-4-methyl-3,4-dihydro-2H-1,4-benzothiazine-8-carboxylic acid n-BuLi (2.5 M in hexane) (0.33 mL, 0.83 mmol) and TMEDA (0.13 mL, 0.83 mmol) were added to a solution of 6-chloro-7-fluoro-4-methyl-3,4-dihydro-2H-1,4-benzothiazine (150 mg, 0.69 mmol) in THF at -78°C. The reaction mixture was stirred at -78°C for 1 h and CO₂ (g) was bubbled through it for 45 min. The reaction mixture was slowly brought to 0°C, quenched with water (10 mL) and washed with EtOAc (3 x 15 mL). The aqueous layer was acidified with 1.0 N HCl to ~pH 4 and the mixture was extracted with EtOAc (2 x 20 mL). The combined extracts were dried (Na₂SO₄), concentrated and triturated with hexanes (2.0 mL) to afford the title compound (42 mg, 14 %).

¹H NMR (300 MHz, CD₃OD); δ 6.73 (d, 1H), 3.60 (t, 2H), 2.97 (t, 2H), 2.96 (s, 3H) ES-MS: *m*/*z* 262.5 (M+1)

HPLC retention time: 10.2 min., purity: 98.0% at 280 nm.

### Example 16: 6-Chloro-4-methyl-7-(methylsulfanyl)-3,4-dihydro-2H-1,4-benzoxazine-8-carboxylic acid (A-26)

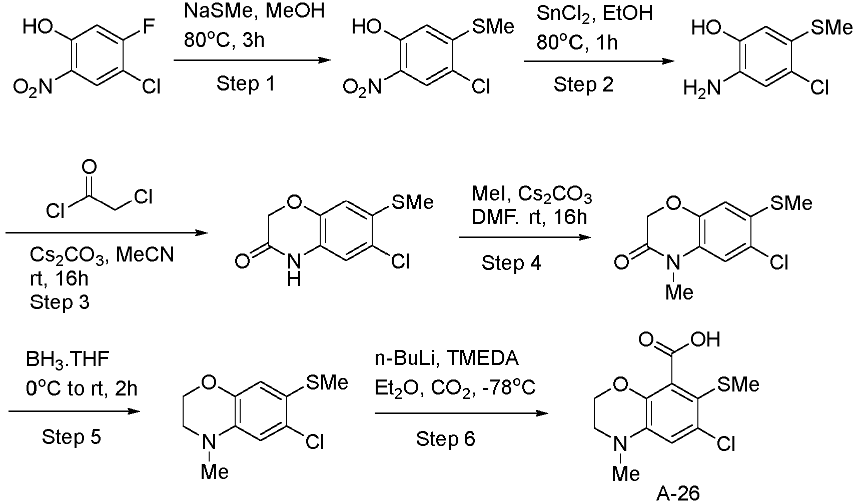

### Step 1: 4-Chloro-5-(methylthio)-2-nitrophenol

NaSCH₃ (2.20 g, 31.4 mmol) was added to a solution of 4-chloro-5-fluoro-2-nitrophenol (3.00 g, 15.7 mmol) in methanol (30 mL) at 0°C in a sealed tube and the reaction mixture was heated at 80°C for 3 h. The reaction was brought to room temperature and the solvent was removed under reduced pressure. The residue was treated with EtOAc (60 mL) and water (60 mL), the layers were separated, and the aqueous layer was extracted further with EtOAc (2 x 60 mL). The combined organic layers were dried (Na₂SO₄), concentrated under reduced pressure and the residue was triturated with ether (2 x 5.0 mL) to obtain the title compound (2.30 g, 67 %).

¹H NMR (300 MHz, CDCl₃); δ 10.7 (s, 1H), 8.01 (s, 1H), 6.82 (s, 1H), 3.53 (s, 3H).

### Step 2: 2-Amino-4-chloro-5-(methylthio)phenol

Tin(II) chloride dihydrate (7.08 g, 31.4 mmol) was added to a solution of 4-chloro-5-(methylthio)-2-nitrophenol (2.30 g, 10.5 mmol) in ethanol (25 mL). The reaction mixture was heated at 80°C for 1 h, solvent was removed under reduced pressure, the pH was adjusted with satd. NaHCO₃ to ~9 and the mixture was extracted with EtOAc (3 x 30 mL). The combined extracts were dried (Na₂SO₄) and concentrated under reduced pressure to obtain the title compound (1.00 g, 50 %).

¹H NMR (300 MHz, CDCl₃); δ 6.80 (s, 1H), 6.73 (s, 1H), 3.72 (br s, 2H), 2.39 (s, 3H).

### Step 3: 6-Chloro-7-(methylthio)-2,4-dihydro-1,4-benzoxazin-3-one

Chloroacetyl chloride (0.46 mL, 5.84 mmol) was added to a suspension of 2-amino-4-chloro-5-(methylthio)phenol (1.00g, 5.27 mmol) and Cs₂CO₃ (4.29 g, 13.2 mmol) at 0°C. The reaction mixture was stirred at ambient temperature for 16 h and the solvent was removed under reduced pressure. EtOAc (30 mL) and water (30 mL) were introduced to the residue, the layers were separated and the aqueous layer was extracted with EtOAc (2 x 30 mL). The combined extracts were dried (Na₂SO₄), concentrated under reduced pressure and the residue was triturated with diethyl ether (3 x 10 mL) to provide the title compound (0.98 g, 81%).

¹H NMR (300 MHz, DMSO-d₆) δ 10.8 (br s, 1H), 6.93 (s, 1H), 6.90 (s, 1H), 4.60 (s, 2H), 2.45 (s, 3H).

### Step 4: 6-Chloro-4-methyl-7-(methylthio)-2,4-dihydro-1,4-benzoxazin-3-one

CH₃I (1.10 mL, 21.3 mmol) was added to a mixture of 6-chloro-7-(methylthio)-2,4-dihydro-1,4-benzoxazin-3-one (0.98 g, 4.27 mmol), Cs₂CO₃ (2.08 g, 6.38 mmol) and DMF (12.0 mL) in a sealed tube at 0°C. The reaction mixture was stirred at ambient temperature for 16 h, quenched with water (20 mL) and extracted into EtOAc (2 x 20 mL). The combined organic layers were washed with 1.0 N HCl (2 x 20 mL), dried (Na₂SO₄) and concentrated under reduced pressure. The residue was purified by column chromatography on silica gel eluting with EtOAc/hexanes (10 - 20%) to afford the title compound (0.70 g, 67%).

¹H NMR (300 MHz, CDCl₃); δ 6.98 (s, 1H), 6.82 (s, 1H), 4.60 (s, 2H), 3.32 (s, 3H), 2.46 (s, 3H).

### Step 5: 6-Chloro-4-methyl-7-(methylthio)-3,4-dihydro-2H-1,4-benzoxazine

BH₃-THF (1.0 M solution) (6.0 mL, 6.15 mmol) was added to 6-chloro-4-methyl-7-(methylthio)-2,4-dihydro-1,4-benzoxazin-3-one (300 mg, 1.23 mmol) at 0°C and the reaction mixture was stirred at room temperature for 2 h. The reaction mixture was cooled to 0°C, quenched with 1.0 N NaOH (3.0 mL) and EtOAc (20 mL) and water (20 mL) were introduced. The layers were separated and the aqueous layer was extracted further with EtOAc (2 x 20 mL). The combined extracts were dried (Na₂SO₄) and concentrated under reduced pressure to obtain the title compound (250 mg, 88 %).

¹H NMR (300 MHz, CDCl₃); δ 6.74 (s, 1H), 6.67 (s, 1H), 4.28 - 4.25 (m, 2H), 3.27 - 3.24 (m, 2H), 2.86 (s, 3H), 2.40 (s, 3H).

### Step 6: 6-Chloro-4-methyl-7-(methylsulfanyl)-3,4-dihydro-2H-1,4-benzoxazine-8-carboxylic acid

n-BuLi (2.5 M in hexane) (1.00 mL, 2.50 mmol) was added to a solution of 6-chloro-4-methyl-7-(methylthio)-3,4-dihydro-2H-1,4-benzoxazine (290 mg, 1.26 mmol) and TMEDA (0.38 mL, 2.52 mmol) in THF at -78°C. The reaction mixture was stirred at that temperature for 1 h and CO₂ was bubbled for 45 min. The reaction mixture was slowly brought to 0°C, quenched with water (15 mL) and washed with EtOAc (3 x 20 mL). The aqueous layer was acidified with 1.0 N HCl to pH ~4 and extracted with EtOAc (2 x 20 mL). The combined organic extracts were dried (Na₂SO₄) and concentrated under reduced pressure to afford the title compound (3.3 mg, 1%).

¹H NMR (300 MHz, CDCl₃); δ 6.74 (s, 1H), 4.31 (t, 2H), 3.34 (t, 2H), 2.92 (s, 3H), 2.35 (s, 3H).

*m*/*z* 272.6 (M-1)

HPLC retention time: 9.55 min., purity: 97.2% at 280 nm.

### Example 17: 6-Fluoro-7-methoxy-4-methyl-3,4-dihydro-2H-1,4-benzoxazine-8-carboxylic acid (A-30)

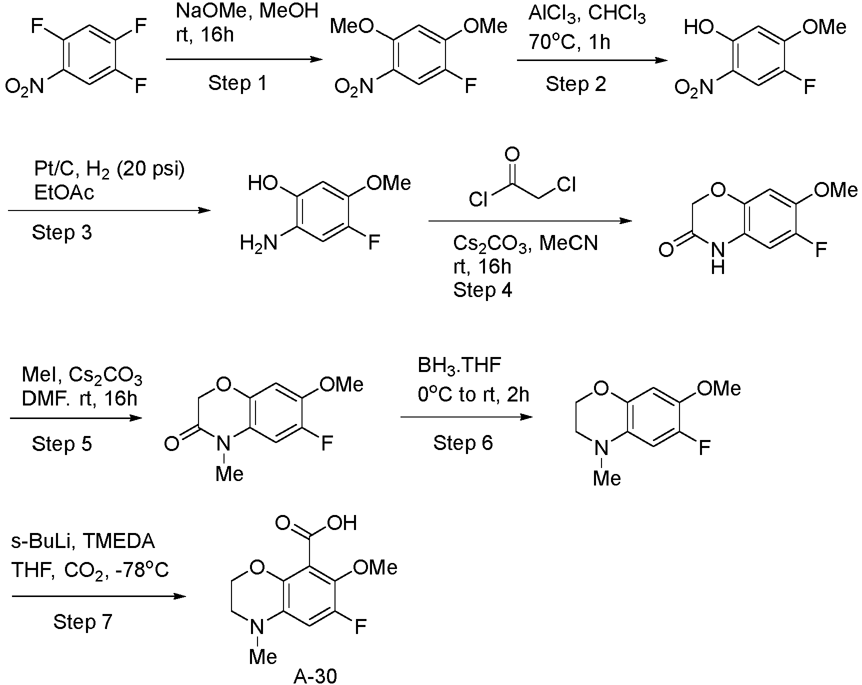

### Step 1: 1-Fluoro-2,4-dimethoxy-5-nitrobenzene

NaOMe (25% in MeOH, 27.0 mL, 124 mmol) was added to a solution of 1,2,4-trifluoro-5-nitrobenzene (10.0 g, 56.4 mmol) in methanol (80 mL) at 0°C. The reaction mixture was allowed to reach ambient temperature and stirred for 16 h. The solvent was removed under reduced pressure, the residue was diluted with EtOAc (200 mL) and washed with 1.0 N HCl (200 mL). The organic layer was dried (Na₂SO₄) and concentrated under reduced pressure. The resultant residue was triturated with hexanes (3 x 75 mL) to give the title compound (10.2 g, 89%) as a white solid.

¹H NMR (300 MHz, CDCl₃); δ 7.83 (d, 1H), 6.60 (s, 1H), 4.00 (s, 3H), 3.98 (s, 3H).

### Step 2: 4-Fluoro-5-methoxy-2-nitrophenol

AlCl₃ (10.1 g, 75.7 mmol) was added to a solution of 1-fluoro-2,4-dimethoxy-5-nitrobenzene (10.2 g, 50.7 mmol) in CHCl₃ (100 mL) at 0°C, the mixture was heated at 70°C for 1 h and was poured into ice water (400 mL) and the mixture was acidified with 1.0 N HCl to pH 2.0. The mixture was extracted with EtOAc (2 x 250 mL), the combined extracts were dried (Na₂SO₄) and evaporated. The residue was triturated with hexanes (2 x 75 mL) to obtain the title compound (9.00 g, 95%) as a yellow solid.

¹H NMR (300 MHz, CDCl₃); δ 10.0 (s, 1H), 7.82 (d, 1H), 6.62 (s, 1H), 3.97 (s, 3H).

### Step 3: 2-Amino-4-fluoro-5-methoxyphenol

5% Pt/C catalyst (20% w/w, 600 mg) was added to a solution of 4-fluoro-5-methoxy-2-nitrophenol (3.00 g, 16.0 mmol) in EtOAc (60 mL). The mixture was stirred for 16 h under a hydrogen atmosphere at 20 psi pressure, filtered through a celite pad and concentrated *in vacuo* to provide the title compound as a brown solid, which was utilised directly in the next step.

¹H NMR (300 MHz, CDCl₃); δ 6.61 (d, 1H), 6.50 (d, 1H), 3.80 (s, 3H).

### Step 4: 6-Fluoro-7-methoxy-2,4-dihydro-1,4-benzoxazin-3-one

Chloroacetyl chloride (2.00 mL, 24.1 mmol) was added to a suspension of 2-amino-4-fluoro-5-methoxyphenol (2.52 g, 16.0 mmol) and Cs₂CO₃ (13.0 g, 39.9 mmol) in acetonitrile (40 mL) at 0°C. The reaction mixture was stirred at ambient temperature for 16 h and the solvent was removed under reduced pressure. EtOAc (60 mL) and water (60 mL) were added to the residue, the layers were separated and the aqueous layer was extracted further with EtOAc (2 x 60 mL). The combined extracts were dried (Na₂SO₄), concentrated under reduced pressure and the residue was triturated with diethyl ether (3 x 15 mL) to give the title compound (2.00 g, 63 %) as a brown solid.

¹H NMR (300 MHz, DMSO-d₆) δ 10.5 (br s, 1H), 6.85 (d, 1H), 6.74 (d, 1H), 4.53 (s, 2H), 3.77 (s, 3H).

### Step 5: 6-Fluoro-7-methoxy-4-methyl-2,4-dihydro-1,4-benzoxazin-3-one

CH₃I (1.80 mL, 35.5 mmol) was added to a suspension of 6-fluoro-7-methoxy-2,4-dihydro-1,4-benzoxazin-3-one (1.40 g, 7.10 mmol) and Cs₂CO₃ (3.47 g, 10.7 mmol) in DMF (20.0 mL) in a sealed tube at 0°C. The reaction mixture was stirred at ambient temperature for 16 h, quenched with water (50 mL) and extracted into EtOAc (2 x 50 mL). The combined extracts were washed with 1.0 N HCl (2 x 50 mL), dried (Na₂SO₄) and concentrated. The residue was purified by column chromatography on silica gel eluting with EtOAc/hexanes (10 - 20%) to afford the title compound (0.54 g, 36 %).

¹H NMR (300 MHz, CDCl₃); δ 6.76 (d, 1H), 6.65 (d, 1H), 4.58 (s, 2H), 3.85 (s, 3H), 3.31 (s, 3H).

### Step 6: 6-Fluoro-7-methoxy-4-methyl-3,4-dihydro-2H-1,4-benzoxazine

BH₃-THF (1.0 M solution) (6.0 mL, 5.98 mmol) was added to 6-fluoro-7-methoxy-4-methyl-2,4-dihydro-1,4-benzoxazin-3-one (0.25 g, 1.18 mmol) at 0°C and the mixture was stirred at ambient temperature for 2 h. The reaction mixture was again cooled to 0°C, quenched with 1.0 N NaOH (3.0 mL) then EtOAc (20 mL) and water (20 mL) were introduced to the mixture. The layers were separated and the aqueous layer was extracted with EtOAc (2 x 20 mL). The combined extracts were dried (Na₂SO₄) and concentrated *in vacuo* to give the title compound (220 mg, 94 %).

¹H NMR (300 MHz, CDCl₃); δ 6.51 - 6.46 (m, 2H), 1.43 (t, 2H), 3.79 (s, 3H), 3.19 (s, 2H), 2.81 (s, 3H).

### Step 7: 6-Fluoro-7-methoxy-4-methyl-3,4-dihydro-2H-1,4-benzoxazine-8-carboxylic acid

s-BuLi (1.4 M in hexane) (2.6 mL, 3.65 mmol) was added to a solution of 6-fluoro-7-methoxy-4-methyl-3,4-dihydro-2H-1,4-benzoxazine (0.24 g, 1.22mmol) and TMEDA (0.55 mL, 3.65 mmol) in THF (6.0 mL) at -78 °C. The reaction mixture was stirred at that temperature for 1 h and CO2 was bubbled through it for 45 min. The mixture was slowly brought to 0°C, quenched with water (15 mL) and washed with EtOAc (3 x 20 mL). The aqueous layer was acidified with 1.0 N HCl to pH ~4 and extracted with EtOAc (2 x 20 mL). The combined organic extracts were dried (Na₂SO₄) and concentrated to afford the title compound (20 mg, 7 %).

¹H NMR (300 MHz, CDCl₃); δ 6.50 (d, 1H), 4.34 (t, 2H), 3.90 (s, 3H), 3.34 - 3.22 (m, 2H), 2.86 (s, 3H).

ES-MS: 240.7 [M-1]

HPLC retention time: 7.92 min, purity: 97.5% at 280 nm.

### Example 18: 6-Chloro-4-methyl-7-(propan-2-yloxy)-3,4-dihydro-2H-1,4-benzoxazine-8-carboxylic acid (A-24)

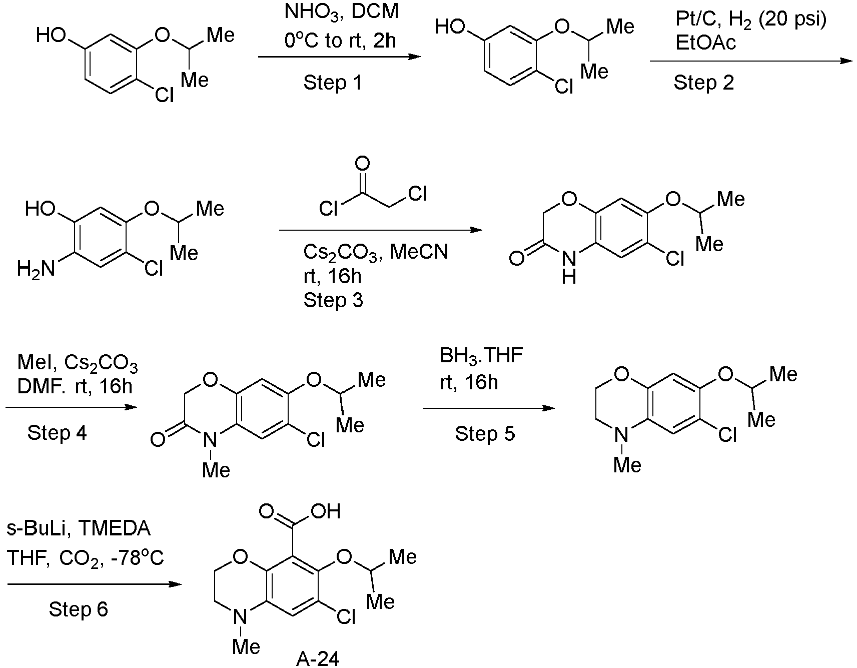

### Step 1: 4-Chloro-5-isopropoxy-2-nitrophenol

Nitric acid (70%) (0.24 mL, 5.36 mmol) was added to a solution of 4-chloro-3-isopropoxyphenol (1.0 g, 5.36 mmol) in CH₂Cl₂ (10 mL) at 0°C. The reaction mixture was stirred at ambient temperature for 2 h. The mixture was poured onto crushed ice and extracted with EtOAc (2 x 50 mL). The combined extracts were dried (Na₂SO₄) and concentrated to afford the title compound (0.78 g, quantitative) which was used directly in the next step.

¹H NMR (300 MHz, CDCl₃) δ 10.87 (s, 1H), 8.07 (s, 1H), 6.53 (s, 1H), 4.72-4.58 (m, 1H), 1.42 (d, 6H).

### Step 2: 2-Amino-4-chloro-5-isopropoxyphenol

Platinum on carbon (5 wt. %, 10% w/w, 78 mg) was added to a solution of 4-chloro-5-isopropoxy-2-nitrophenol (0.78 g, 3.36 mmol) in EtOAc (25 mL). The reaction mixture was hydrogenated at 20 psi at ambient temperature for 16 h. The reaction mixture was filtered through a pad of celite and concentrated to afford the title compound (0.63 g) which was reacted directly in the next step.

¹H NMR (300 MHz, CD₃OD) δ 6.64 (s, 1H), 6.39 (s, 1H), 4.25-4.12 (m, 1H), 1.16 (d, 6H).

### Step 3: 6-Chloro-7-isopropoxy-2,4-dihydro-1,4-benzoxazin-3-one

Cs₂CO₃ (3.05 g, 9.37 mmol) and 2-chloroacetyl chloride (0.53 g, 4.68 mmol) were added to a solution of 2-amino-4-chloro-5-isopropoxyphenol (0.63 g, 3.12 mmol) in acetonitrile (25 mL) at 0°C and the reaction mixture was stirred at ambient temperature for 16 h. The solvent was removed under reduced pressure, the crude product was treated with 20% methanol/CH₂Cl₂, filtered through a celite pad and the filtrate concentrated *in vacuo* to provide the title compound (0.63 g) utilised dire°C ctly in the next step.

¹H NMR (300 MHz, CD₃OD) δ 6.79 (s, 1H), 6.61 (s, 1H), 4.45 (s, 2H), 4.38 (m, 1H), 1.20 (d, 6H).

ES-MS: 242.7 [M+1].

### Step 4: 6-Chloro-7-isopropoxy-4-methyl-2,4-dihydro-1,4-benzoxazin-3-one

Cs₂CO₃ (2.55 g, 7.83 mmol) and CH₃I (0.56 g, 3.91 mmol) were added to a solution of 6-chloro-7-isopropoxy-2,4-dihydro-1,4-benzoxazin-3-one (0.63 g, 2.61 mmol) in DMF (25 mL). The reaction mixture was stirred at ambient temperature for 16 h, diluted with EtOAc (100 mL). The solution was washed with brine (3 x 50 mL), dried (Na₂SO₄), concentrated under reduced pressure and the residue purified by column chromatography on silica gel eluting with EtOAc/hexane (0 - 40%) to obtain the title compound (0.17 g, 25%).

¹H NMR (300 MHz, CDCl₃) δ 6.94 (s, 1H), 6.61 (s, 1H), 4.57(s, 2H), 4.43 (p, 1H), 3.29 (s, 3H), 1.36 (d, 6H).

ES-MS: 256.8 [M+1].

### Step 5: 6-Chloro-7-isopropoxy-4-methyl-3,4-dihydro-2H-1,4-benzoxazine

6-chloro-7-isopropoxy-4-methyl-2,4-dihydro-1,4-benzoxazin-3-one (0.16 g, 0.63 mmol) was added to BH₃.THF (1M in THF) (2.5 mL, 2.5 mmol) and the reaction was stirred at ambient temperature for 16 h. The reaction mixture was quenched with methanol (10 mL), evaporated and purified by column chromatography on silica gel eluting with EtOAc/hexane (5 - 50%) to give the title compound (0.13 g, 87%).

¹H NMR (300 MHz, CDCl₃) δ 6.63 (s, 1H), 6.47 (s, 1H), 4.38-4.23 (m, 3H), 3.17 (t, 2H), 2.80 (s, 3H), 1.31 (d, 6H).

ES-MS: 242.8 [M+1].

### Step 6: 6-chloro-4-methyl-7-(propan-2-yloxy)-3,4-dihydro-2H-1,4-benzoxazine-8-carboxylic acid

s-BuLi (1.4 M in hexane) (0.222 mL, 0.31 mmol) was added to a solution of 6-chloro-7-isopropoxy-4-methyl-3,4-dihydro-2H-1,4-benzoxazine (50 mg, 0.21 mmol) and TMEDA (36 mg, 0.31 mmol) in THF (4 mL) at -78°C. The reaction mixture was stirred at that temperature for 2 h and CO₂ (g) was bubbled for 10 min and slowly brought to 0°C. Quenching with water (20 mL) and washing with CH₂Cl₂ (2 x 20 mL) provided an aqueous layer which was acidified with 1N HCl to pH ~4. Extraction with EtOAc (3 x 25 mL), drying the combined extracts (Na₂SO₄) and concentration under reduced pressure afforded the title compound (27 mg, 46%).

¹H NMR (300 MHz, CDCl₃) δ 6.67 (s, 1H), 4.48 (p, 1H), 4.35 (t, 2H), 3.26 (t, 2H), 2.86 (s, 3H), 1.30 (d, 6H).

ES-MS: 284.6 [M-1].

HPLC: Retention Time: 10.03 min., purity: 95.6% @ 254 nm.

### Example 19: 6-Chloro-7-methoxy-2-(methoxymethyl)-4-methyl-3,4-dihydro-2H-1,4-benzoxazine-8-carboxylic acid (A-35)

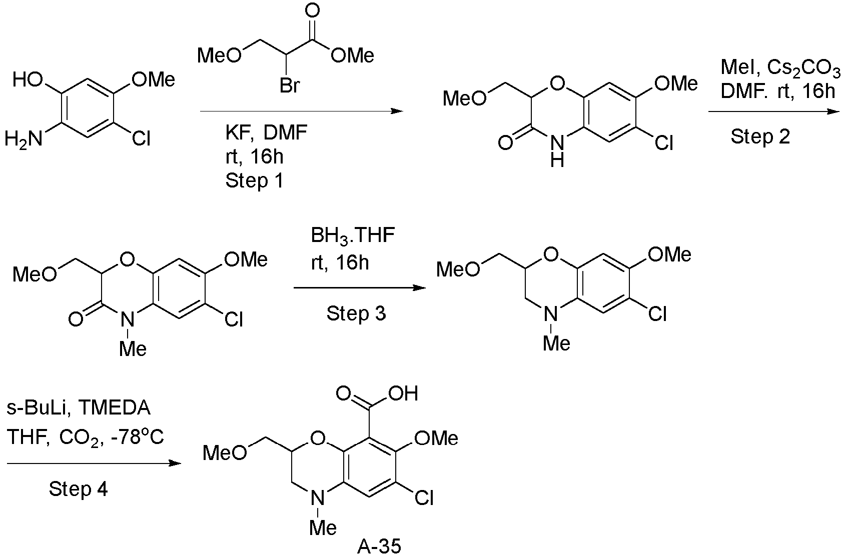

### Step 1: 6-Chloro-7-fluoro-2,2,4-trimethyl-2H-benzo[b][1,4]oxazin-3(4H)-one

KF (0.54 g, 9.22 mmol), and methyl 2-bromo-3-methoxypropionate (0.91 g, 4.61 mmol) were added to a solution of 2-amino-4-chloro-5-methoxyphenol (0.40 g, 2.30 mmol) in DMF (5 mL) and the reaction was stirred at 60°C for 16 h in a sealed tube. The solution was poured into a mixture of crushed ice and water and a solid formed, which was collected by filtration. The solid was washed with water (15 mL) and dried to provide the title compound (0.57 g) which was utilised directly in the next step.

¹H NMR (300 MHz, CDCl₃) δ 6.90 (s, 1H), 6.73 (s, 1H), 4.85-4.79 (m, 1H), 4.0-3.72 (m, 5H), 3.48 (s, 3H).

ES-MS: 258.7 [M+1].

### Step 2: 6-Chloro-7-methoxy-2-(methoxymethyl)-4-methyl-2,4-dihydro-1,4-benzoxazin-3-one

Cs₂CO₃ (2.09 g, 6.40 mmol) and CH₃I (0.45 g, 3.20 mmol) were added to a solution of 6-chloro-7-fluoro-2,2,4-trimethyl-2H-benzo[b][1,4]oxazin-3(4H)-one (0.55 g, 2.13 mmol) in DMF (15 mL). The reaction mixture was stirred at ambient temperature for 16 h, diluted with EtOAc (100 mL), washed with brine solution (3 x 50 mL), dried (Na₂SO₄) and the solution concentrated under reduced pressure and purified by column chromatography on silica gel eluting with EtOAc/hexane (0-40%) to obtain the title compound (0.21 g, 36%).

¹H NMR (300 MHz, CDCl₃) δ 6.97 (s, 1H), 6.72 (s, 1H), 4.76 (t, 1H), 3.94-3.90 (m, 2H), 3.87 (s, 3H), 3.44 (s, 3H). 3.33 (s, 3H).

ES-MS: 272.9 [M+1].

### Step 3: 6-Chloro-7-methoxy-2-(methoxymethyl)-4-methyl-3,4-dihydro-2H-1,4-benzoxazine

BH₃.THF (1M in THF) (2.94 mL, 2.94 mmol) was added to 6-chloro-7-methoxy-2-(methoxymethyl)-4-methyl-2,4-dihydro-1,4-benzoxazin-3-one (0.20 g, 0.74 mmol), and reaction was stirred at ambient temperature for 16 h. The reaction mixture was cooled, and carefully quenched with methanol, evaporated and purified by column chromatography on silica gel eluting with EtOAc/hexane (5-50%) to obtain the title compound (0.134 g, 71%).

¹H NMR (300 MHz, CDCl₃) δ 6.72 (s, 1H), 6.58 (s, 1H), 4.50-4.39 (m, 1H), 3.83 (s, 3H), 3.71-3.53 (m, 2H), 3.47 (s, 3H), 3.21 (dd, 1H), 3.06 (dd, 1H). 2.84 (s, 3H).

ES-MS: 258.7 [M+1].

### Step 4: 6-Chloro-7-methoxy-2-(methoxymethyl)-4-methyl-3,4-dihydro-2H-1,4-benzoxazine-8-carboxylic acid

s-BuLi (1.4 M in hexane) (0.40 mL, 0.56 mmol) was added to a solution of 6-chloro-7-methoxy-2-(methoxymethyl)-4-methyl-3,4-dihydro-2H-1,4-benzoxazine (130 mg, 0.50 mmol) and TMEDA (83.2 mg, 0.56 mmol) in THF (7 mL) at -78°C. The reaction mixture was stirred at that temperature for 1 h and CO₂ (g) was bubbled through for 10 min and slowly brought to 0°C. Quenching with water (20 mL) and washing with CH₂Cl₂ (2 x 20 mL) provided an aqueous layer which was acidified with 1N HCl to pH 4 - 5. The mixture was extracted with EtOAc (3 x 25 mL) then the combined extracts were dried (Na₂SO₄) and concentrated under reduced pressure. The residue product was purified by semi preparative HPLC using 10-100 % CH₃CN/0.1% formic acid in water to provide the title compound (18 mg, 12%).

¹H NMR (300 MHz, CDCl₃) δ 7.91-6.80 (br, 1H), 6.73 (s, 1H), 4.58-4.45 (m, 1H), 3.92 (s, 3H), 3.75-3.57 (m, 2H), 3.48 (s, 3H), 3.35 (dd, 1H), 3.19 (dd, 1H), 2.92 (s, 3H) ES-MS: 300.7 [M-1].

HPLC: Retention Time: 9.14 min., purity: >99 % @ 254 nm.

### Example 20: 6-Chloro-7-methoxy-4-methyl-3,4-dihydrospiro[1,4-benzoxazine-2,1'-cyclobutane]-8-carboxylic acid (A-39)

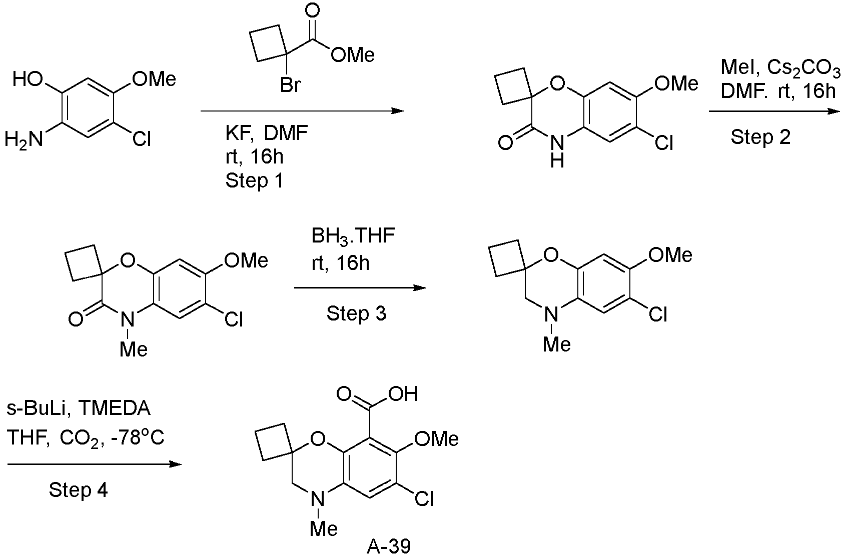

### Step 1: 6-Chloro-7-methoxy-4H-spiro[1,4-benzoxazine-2,1'-cyclobutan]-3-one

KF (0.60 g, 10.36 mmol), and methyl 1-bromocyclobutanecarboxylate (1.0 g, 5.18 mmol) were added to a solution of 2-amino-4-chloro-5-methoxyphenol (0.45 g, 2.59 mmol) in DMF (5 mL), and reaction was stirred at 60°C for 16 h in a sealed tube. The solution was poured into a mixture of crushed ice and water and the resultant solid was collected by filtration. It was washed with water (15 mL), dried to provide the title compound (0.61 g) which was utilised directly in the next step.

¹H NMR (300 MHz, CDCl₃) δ 6.94 (s, 1H), 6.56 (s, 1H), 3.87 (s, 3H), 2.77-2.60 (m, 2H), 2.43-2.22 (m, 2H), 2.10-1.86 (m, 2H).

ES-MS: 254.6 [M+1].

### Step 2: 6-Chloro-7-methoxy-4-methyl-4H-spiro[1,4-benzoxazine-2,1'-cyclobutan]-3-one

Cs₂CO₃ (2.31 g, 7.09 mmol) and CH₃I (0.50 g, 3.55 mmol) were added to a solution of 6-chloro-7-methoxy-4H-spiro[1,4-benzoxazine-2,1'-cyclobutan]-3-one (0.60 g, 2.36 mmol) in DMF (20 mL). The reaction mixture was stirred at ambient temperature for 16 h, diluted with EtOAc (100 mL), washed with brine (3 x 50 mL) and the solution was dried (Na₂SO₄). The residue on evaporation was purified by column chromatography on silica gel eluting with EtOAc/hexane (0 - 40%) to give the title compound (49 mg, 8%).

¹H NMR (300 MHz, CDCl₃) δ 6.99 (s, 1H), 6.72 (s, 1H), 3.92 (s, 3H), 3.37 (s, 3H). 2.73-2.60 (m, 2H), 2.40-2.25 (m, 2H), 2.11-1.92 (m, 2H).

ES-MS: 268.7 [M+1].

### Step 3: 6-Chloro-7-methoxy-4-methyl-3,4-dihydrospiro[1,4-benzoxazine-2,1'-cyclobutane

BH₃.THF (1M in THF) (0.67 mL, 0.67 mmol) was added to 6-chloro-7-methoxy-4-methyl-4H-spiro[1,4-benzoxazine-2,1'-cyclobutan]-3-one (45 mg, 0.17 mmol), and reaction was stirred at ambient temperature for 16 h. The reaction mixture was quenched with methanol, evaporated and purified by column chromatography on silica gel eluting with EtOAc/hexane (5 - 50%) to obtain the title compound (38 mg, 89%). ¹H NMR (300 MHz, CDCl₃) δ 6.70 (s, 1H), 6.53 (s, 1H), 3.85 (s, 3H), 3.11 (s, 2H), 2.91 (s, 3H), 2.39-2.24 (m, 2H), 2.21-2.08 (m, 2H), 2.05-1.68 (m, 2H).

ES-MS: 254.7 [M+1].

### Step 4: 6-Chloro-7-methoxy-4-methyl-3,4-dihydrospiro[1,4-benzoxazine-2,1'-cyclobutane]-8-carboxylic acid

s-BuLi (1.4 M in hexane) (0.11 mL, 0.14 mmol) was added to a solution of 6-chloro-7-methoxy-4-methyl-3,4-dihydrospiro[1,4-benzoxazine-2,1'-cyclobutane] (35 mg, 0.15 mmol) and TMEDA (17.6 mg, 0.15 mmol) in THF (4 mL) at -78°C. The reaction mixture was stirred at that temperature for 1 h and CO₂ (g) was bubbled for 10 min and slowly brought to 0°C. Quenching with water (20 mL) and washing with CH₂Cl₂ (2 x 20 mL) provided an aqueous layer which was acidified with 1N HCl to pH ~4. The mixture was extracted with EtOAc (3 x 25 mL), the combined extracts were dried (Na₂SO₄) and concentrated *in vacuo* to provide the title compound (12 mg 29%).

¹H NMR (300 MHz, CD₃OD) δ 6.70 (s, 1H), 3.81 (s, 3H), 3.20 (s, 2H), 2.93 (s, 3H), 2.35-2.20 (m, 2H), 2.18-2.05 (m, 2H), 2.01-1.70 (m, 2H).

ES-MS: 296.6 [M-1].

HPLC: Retention Time: 10.790 min., purity: 98.5 % @ 254 nm.

### Example 21: 6-chloro-4,7-dimethyl-3,4-dihydro-2H-1,4-benzoxazine-8-carboxylic acid (A-48)

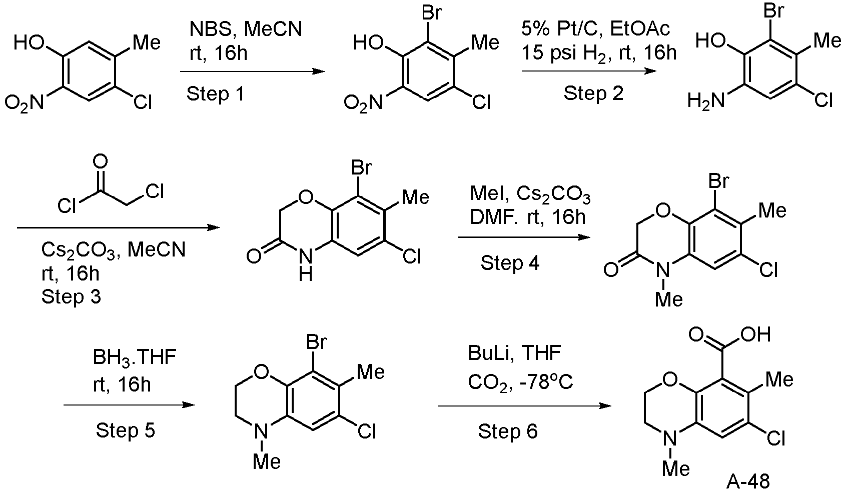

### Step 1: 2-Bromo-6-chloro-4-nitro-3-cresol:

NBS (1.89 g, 10.66 mmol) was added to a solution of 6-chloro-4-nitro-3-cresol (2.0 g, 10.66 mmol) in acetonitrile (25 mL) and the reaction mixture was stirred at ambient temperature for 16 h. The reaction was quenched with water (100 mL), extracted with EtOAc (2 x 150 mL), dried (Na₂SO₄) and concentrated. The resultant residue was purified by column chromatography on silica gel eluting with hexane/EtOAc (0-20%) to give the title compound (1.12 g, 39.0 %).

¹H NMR (300 MHz, CDCl₃) δ 11.37 (s, 1H), 8.34 (s, 1H), 2.83 (s, 3H).

### Step 2: 4-Amino-2-bromo-6-chloro-3-cresol

Platinum on carbon (5 wt. %, 10% w/w, 50 mg) was added to a solution of 2-bromo-6-chloro-4-nitro-3-cresol (0.50 g, 1.88 mmol) in EtOAc (15 mL). The reaction mixture was hydrogenated at 15 psi at ambient temperature for 16 h and filtered through a pad of celite. The filtrate was concentrated to afford 4-amino-2-bromo-6-chloro-3-cresol (0.44 g) which was utilised directly in the next step.

¹H NMR (300 MHz, CDCl₃) δ 6.72 (s, 1H), 4.78-3.60 (br s, 2H), 2.38 (s, 3H).

ES-MS: 236.5 [M+1].

### Step 3: 8-Bromo-6-chloro-7-methyl-2,4-dihydro-1,4-benzoxazin-3-one

Cs₂CO₃ (1.82 g, 5.58 mmol) and 2-chloroacetyl chloride (0.23 g, 2.04 mmol) were added to a solution of 4-amino-2-bromo-6-chloro-3-cresol (0.44 g, 1.86 mmol) in acetonitrile (15 mL) at 0°C and the reaction mixture was stirred at ambient temperature for 16 h. The solvent was removed under reduced pressure and the crude product taken up in 20% methanol/CH₂Cl₂ (25 mL), filtered through a celite pad and concentrated to obtain the title compound (0.50 g) which was utilised directly in the next step.

¹H NMR (300 MHz, CDCl₃) δ 6.80 (s, 1H), 4.81 (s, 2H), 2.46 (s, 3H).

ES-MS: 277.5 [M+1].

### Step 4: 8-Bromo-6-chloro-4-methyl-7-methyl-2,4-dihydro-1,4-benzoxazin-3-one

Cs₂CO₃ (1.77 g, 5.43 mmol) and CH₃I (0.39 g, 2.71 mmol) were added to a solution of 8-bromo-6-chloro-7-methyl-2,4-dihydro-1,4-benzoxazin-3-one (0.50 g, 1.81 mmol) in DMF (20 mL). The reaction mixture was stirred at ambient temperature for 16 h, diluted with EtOAc (100 mL) and washed with brine (3 x 50 mL). The organic layer was dried (Na₂SO₄), the solution concentrated *in vacuo* and the residue purified by column chromatography on silica gel eluting with EtOAc/hexane (0 - 40%) to provide the title compound (0.21 g, 41%).

¹H NMR (300 MHz, CDCl₃) δ 7.25 (s, 1H), 4.99 (s, 2H), 3.62 (s, 3H), 2.77 (s, 3H). ES-MS: 291.5 [M+1].

### Step 5: 8-Bromo-6-chloro-4-methyl-7-methyl-3,4-dihydro-2H-1,4-benzoxazine

BH₃.THF (1M in THF) (2.89 mL, 2.89 mmol) was added to 8-bromo-6-chloro-4-methyl-7-methyl-2,4-dihydro-1,4-benzoxazin-3-one (0.21 g, 0.72 mmol) and the reaction mixture was stirred at ambient temperature for 16 h. The reaction mixture was cooled, quenched with methanol (20 mL), evaporated and the residue purified by column chromatography on silica gel eluting with EtOAc/hexane (5-50%) to give the title compound (57 mg, 29%).

¹H NMR (300 MHz, CDCl₃) δ 6.62 (s, 1H), 4.36 (t, 2H), 3.26 (t, 2H), 2.86 (s, 3H), 2.40 (s, 3H).

ES-MS: 278.6 [M+1].

### Step 6: 6-Chloro-4,7-dimethyl-3,4-dihydro-2H-1,4-benzoxazine-8-carboxylic acid

n-BuLi (2.5 M in hexane) (0.088 mL, 0.22 mmol) was added to a solution of 8-bromo-6-chloro-4-methyl-7-methyl-3,4-dihydro-2H-1,4-benzoxazine (55 mg, 0.20 mmol) in THF (5 mL) at -78°C. The reaction mixture was stirred at that temperature for 1 h, CO₂ (g) was bubbled through it for 10 min and slowly brought to 0°C. Quenching with water (20 mL) and washing with CH₂Cl₂ (2 x 20 mL) provided an aqueous layer which was acidified with 1N HCl to pH ~4. The mixture was extracted with EtOAc (3 x 25 mL), the combined extracts were dried (Na₂SO₄) and concentrated under reduced pressure to provide the title compound (28.3 mg, 59%).

¹H NMR (300 MHz, CDCl₃) δ 6.70 (s, 1H), 4.33 (t, 2H), 3.28 (t, 2H), 2.86 (s, 3H), 2.30 (s, 3H).

ES-MS: 240.7 [M-1].

HPLC: Retention Time: 9.42 min., purity: 98.8% @ 254 nm.

### Example 22: 6-Chloro-7-methoxy-4-methyl-3,4-dihydro-2H-1,4-benzothiazine-8-carboxylic acid (A-29)

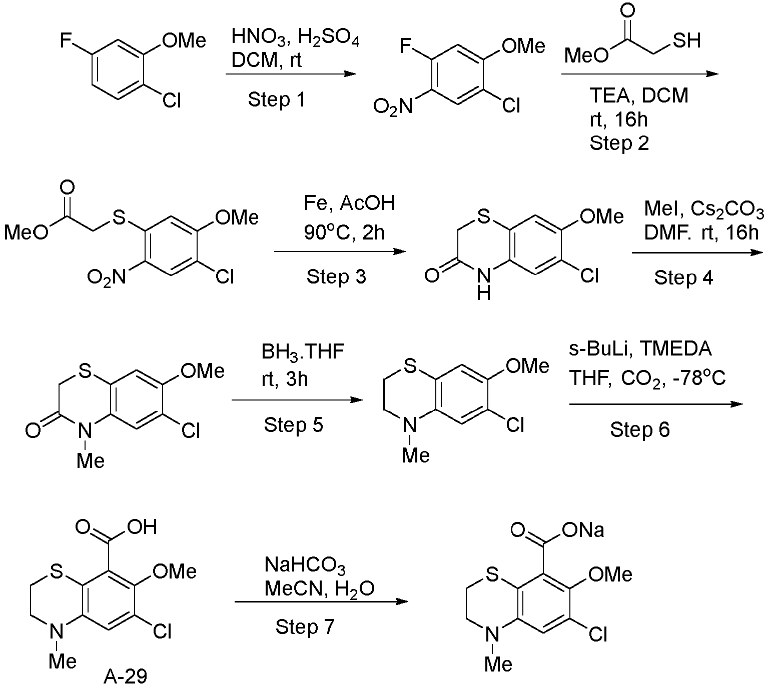

### Step 1: 1-Chloro-4-fluoro-2-methoxy-5-nitrobenzene

A mixture of HNO₃ (4.8 mL, 74.73 mol) and H₂SO₄ (3.98 mL, 74.73 mmol) was added to a solution of 1-chloro-4-fluoro-2-methoxybenzene (10 g, 62.3 mmol) in CH₂Cl₂ (30 mL) at 0°C and the reaction mixture was stirred at room temperature for 16 h. The mixture was poured into ice cold water (50 mL) and diluted with EtOAc (50 mL). The organic layer was extracted, washed with water (2 x 50 mL), dried (Na₂SO₄) and concentrated under reduced pressure. The residue was purified by column chromatography on silica gel eluting with hexanes/EtOAc (5 - 10%) to give the title compound as a yellow solid (4.18 g, 33%).

¹H NMR (300 MHz, CDCl₃) δ 8.04 (d, 1H), 6.79 (d, 1H), 3.97 (s, 3H).

### Step 2: Methyl 2-(4-chloro-5-methoxy-2-nitrophenylthio) acetate

Triethylamine (3.4 mL, 24.41 mmol) and methyl thioglycolate (1.99 mL, 22.4 mmol) were added to a solution of 1-chloro-4-fluoro-2-methoxy-5-nitrobenzene (4.18 gm, 20.34 mmol) in CH₂Cl₂ (32 mL) at 0°C and the reaction mixture was stirred at ambient temperature for 16 h. The mixture was cooled to 0°C, 1N HCl solution was slowly added and the mixture was stirred for 30 min. The organic layer was separated, dried (Na₂SO₄) and concentrated under reduced pressure. The residue was triturated with hexanes to obtain the title compound as a yellow solid (5.0 g, 84%).

¹H NMR (300 MHz, CDCl₃); δ 8.42 (s, 1H), 7.26 (s, 1H), 4.13 (s, 3H), 3.87 (s, 3H), 3.82 (s, 2H).

### Step 3: 6-Chloro-7-methoxy-2H-benzo[b][1,4]thiazin-3(4H)-one

Iron (3.83 g, 68.56 mmol) was added to a mixture of methyl 2-(4-chloro-5-methoxy-2-nitrophenylthio) acetate (5 g, 17.14 mmol) in acetic acid (55 mL). The reaction mixture was heated at 90°C for 2 h, and then stirred at room temperature for 16 h. The mixture was cooled to 0°C followed by slow addition of 1N HCl (90 mL) and stirred for 1 h at 0°C. The precipitate was filtered and dissolved in EtOAc (100 mL). The solution was washed with water (2 x 50 mL) and the organic layer was dried (Na₂SO₄) and concentrated under reduced pressure. The resultant residue was triturated with hexane to obtain the title compound as a white solid (5.0 g, 84%).

¹H NMR (300 MHz, CDCl₃) δ 8.78 (br, 1H), 6.94 (s, 1H), 6.87 (s, 1H), 3.87 (s, 3H), 3.43 (s, 2H).

### Step 4: 6-Chloro-7-methoxy-4-methyl-2H-benzo[b][1,4]thiazin-3(4H)one

Cs₂CO₃ (3.19 g, 9.79 mmol) and CH₃I (3.29 mL, 65.31 mmol) were added to a solution of 6-chloro-7-methoxy-2H-benzo[b][1,4]thiazin-3(4H)-one (1.5 g, 6.53 mmol) in DMF (15 mL) and the mixture stirred at ambient temperature for 16 h. The mixture was diluted with EtOAc (50 mL), washed with water (2 x 50 mL), dried (Na₂SO₄), and concentrated under reduced pressure. The residue was purified by column chromatography on silica gel eluting with hexane/EtOAc (5 - 10%) to provide the title compound as a white solid (1.34 gm, 84 %).

¹H NMR (300 MHz, CDCl₃) δ 7.26 (s, 1H), 7.07 (s, 1H), 4.04 (s, 3H), 3.57-3.56 (m, 5H).

### Step 5: 6-Chloro-7-methoxy-4-methyl-3,4-dihydro-2H-benzo[b]1[1,4]thiazinine

BH₃.THF (28 mL, 27.45 mmol) was added to a solution of 6-chloro-7-methoxy-4-methyl-2H-benzo[b][1,4]thiazin-3(4H)-one (1.34 g, 5.49 mmol) in THF (5 mL) at 0°C and the mixture was stirred at ambient temperature for 3 h, cooled to 0°C before 1N NaOH was added slowly. The mixture was extracted with EtOAc (2 x 50 mL), dried (Na₂SO₄) and concentrated under reduced pressure. The residue was triturated with hexanes to obtain the title compound as a white solid (1.2 gm, 96 %).

¹H NMR (300 MHz, CDCl₃) δ 6.67-6.66 (m, 2H), 3.78 (s, 3H), 3.43-3.40 (m, 2H), 3.08-3.04 (m, 2H), 2.86 (s, 3H).

### Step 6: 6-Chloro-7-methoxy-4-methyl-3,4-dihydro-2H-1,4-benzothiazine-8-carboxylic acid

TMEDA (2.5 mL, 16.88 mmol) and s-BuLi (12.0 mL, 16.88 mmol) were added to a solution of 6-chloro-7-methoxy-4-methyl-3,4-dihydro-2H-benzo[b][1,4]thiazinine (970 mg, 4.22 mmol) in THF (20.0 mL) at -78°C. The reaction mixture was stirred at that temperature for 1 h, CO₂ was bubbled for 45 mins at -78°C. The mixture was slowly brought to room temperature, quenched with water (25 mL) and washed with EtOAc (30 mL). The aqueous layer was acidified to pH ~2.0 using 1.0 N HCl and the product was extracted using EtOAc (2 x 30 mL). The combined organic layers were dried (Na₂SO₄) and concentrated to afford the title compound as a brown oil (577 mg, 49%) which was utilised directly in the next step.

¹H NMR (300 MHz, CDCl₃) δ 11.41-11.14 (br s, 1H), 6.68 (s, 1H), 3.84 (s, 3H), 3.57-3.53 (m, 2H), 2.96-2.92 (m, 5H).

ES-MS: 272.6 [M-1]

### Step 7: Sodium 6-chloro-7-methoxy-4-methyl-3,4-dihydro-2H-1,4-benzothiazine-8-carboxylate

NaHCO₃ (141.64 mg, 1.69 mmol) was added to a solution of 6-chloro-7-methoxy-4-methyl-3,4-dihydro-2H-1,4-benzothiazine-8-carboxylic acid (577 mg, 2.11 mmol) in acetonitrile (10 mL) and water (10 mL). The mixture was stirred for 1 h at ambient temperature, the solvents were removed under vacuum and the residue was washed with EtOAc (30 mL). The aqueous layer was concentrated under reduced pressure and the residue was triturated with chloroform to afford the title compound as a brown solid (300 mg, 48%).

¹H NMR (300 MHz, CD₃OD) δ 6.59 (s, 1H), 3.83 (s, 3H), 3.54-3.51 (m, 2H), 3.02-2.98 (m, 2H), 2.92 (s, 3H).

ES-MS: 272.6 [M-1]

HPLC: Retention time 11.51 min., Purity: 96.5% @ 254 nm.

### Example 23: Methyl 6-chloro-7-methoxy-3,4-dihydro-2H-1,4-benzoxazine-8-carboxylate

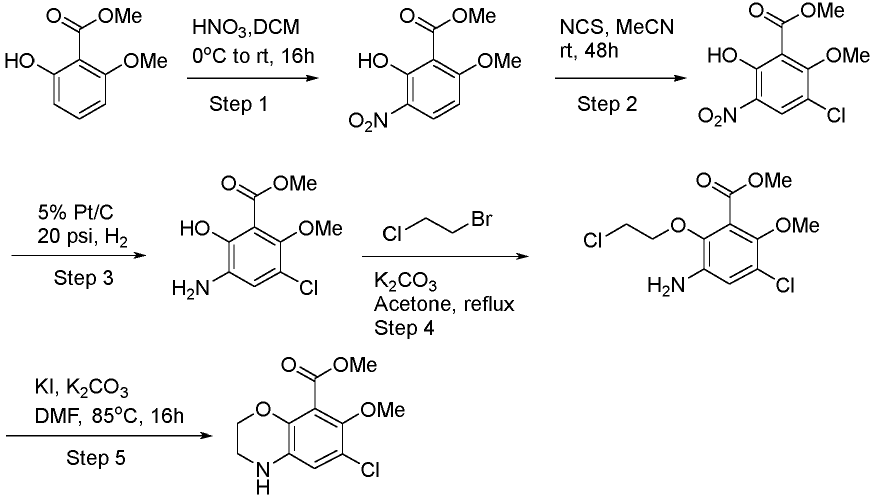

### Step 1: Methyl 2-hydroxy-6-methoxy-3-nitrobenzoate

Nitric acid (70%) (4.91 mL, 82.34 mmol) was added to a solution of methyl 2-hydroxy-6-anisate (15.0 g, 82.34 mmol) in DCM (100 mL) at 0°C. The reaction mixture was stirred at ambient temperature for 16 hours. The mixture was poured into ice cold water and extracted with EtOAc (2 x 50 mL), the combined extracts were dried (Na₂SO₄) and concentrated. The yellow residue was purified by column chromatography on silica gel eluting with hexane/EtOAc (0 - 15%) to provide the title compound as a yellow solid (5.70 g, 31% yield).

¹H NMR (300 MHz, CDCl₃); δ 11.12 (s, 1H), 8.18 (d, 1H), 6.61 (d, 1H), 3.95 (s, 6H) ppm.

### Step 2: Methyl 5-chloro-2-hydroxy-6-methoxy-3-nitrobenzoate

NCS (3.858 g, 28.89 mmol) was added to a solution of methyl 2-hydroxy-6-methoxy-3-nitrobenzoate (5.47 g, 24.08 mmol) in CH₃CN (25 mL) and stirred at ambient temperature for 48 h. The reaction mixture was quenched with 1N HCl solution, extracted with EtOAc (3 x 50 mL), then the combined extracts were washed with brine (50 mL), dried (Na₂SO₄) and evaporated. The residue was purified by column chromatography on silica gel eluting with hexane/EtOAc (0 - 10%) to give the title compound (5.52 g, 87% yield) as a solid.

¹H NMR (300 MHz, CDCl₃); δ 10.96 (s, 1H), 8.23 (s, 1H), 4.02 (s, 3H), 3.98 (s, 3H) ppm.

### Step 3: Methyl 3-amino-5-chloro-2-hydroxy-6-anisate

Pt/C catalyst 5 wt % (10% w/w, 0.550 g) was added to a solution of methyl 5-chloro-2-hydroxy-6-methoxy-3-nitrobenzoate (5.5 g, 21.02 mmol) in MeOH (100 mL). The reaction mixture was hydrogenated at 20 psi at ambient temperature for 2 h. The reaction mixture was filtered through a pad of celite and concentrated *in vacuo* to afford the title compound (5.20 g), used directly in the next step.

¹H NMR (300 MHz, CDCl₃); δ 6.90 (s, 1H), 4.04 (s, 2H), 4.00 (s, 3H), 3.78 (s, 3H) ppm.

### Step 4: Methyl 3-amino-5-chloro-2-(2-chloroethoxy)-6-anisate

K₂CO₃ (18.617 g, 134.70 mmol) and 1-bromo-2-chloroethane (2.43 mL, 29.187 mmol) were added to a solution of methyl 3-amino-5-chloro-2-hydroxy-6-anisate (5.2 g, 22.45 mmol) in acetone (100 mL). The reaction mixture was heated at reflux for 16 h, cooled, evaporated and the residue extracted with EtOAc (2 x 50 mL). The combined extracts were dried (Na₂SO₄), evaporated and the residue was purified by column chromatography on silica gel eluting with hexane/EtOAc (0-50%) to provide the title ester (2.4 g, 68%) as a yellow liquid.

¹H NMR (300 MHz, CDCl₃); δ 6.80 (s, 1H), 4.23 (m, 2H), 3.93 (s, 3H), 3.81 (s, 3H), 3.87 (m, 2H) ppm.

### Step 5: Methyl 6-chloro-7-methoxy-3,4-dihydro-2H-1,4-benzoxazine-8-carboxylate

K₂CO₃ (4.699 g, 34.0 mmol), and KI (5.644 g, 34.0 mmol) was added to a solution of methyl 3-amino-5-chloro-2-(2-chloroethoxy)-6-anisate (5.0 g, 17.0 mmol) in DMF (30 mL). The reaction mixture was heated at 85°C for 16 h in a sealed tube, cooled and evaporated. The residue was purified by column chromatography on silica gel eluting with hexane/EtOAc (0 - 50%) to give the title ester (1.5 g, 34%) as a light-yellow solid. ¹H NMR (300 MHz, CDCl₃); δ 6.60 (s, 1H), 4.22 (m, 2H), 3.91 (s, 3H), 3.80 (s, 3H), 3.77 (m, 2H) ppm.

ES- MS: *m*/*z* 258.7 (M+H).

### Example 24: Ethyl 6-chloro-7-fluoro-3,4-dihydro-2H-1,4-benzoxazine-8-carboxylate

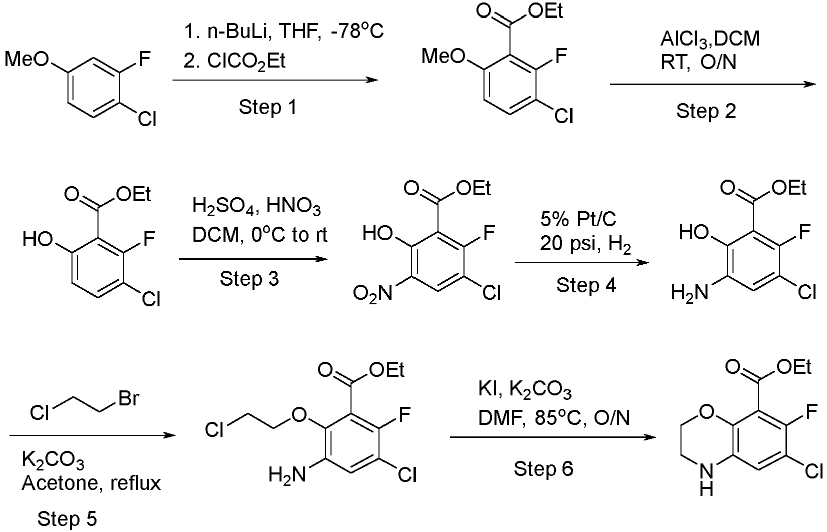

### Step 1: Ethyl 3-chloro-2-fluoro-6-methoxybenzoate

n-BuLi (2.5 M in hexane) (13.2 mL, 33.0 mmol) was added to a solution of 1-chloro-2-fluoro-4-methoxybenzene (5.0 g, 31.1 mmol) in THF (50 mL) at -78°C. The reaction mixture was stirred at same temperature for 1 h. After 1 h, ethyl chloroformate (3.6g, 33.0 mmol) was added dropwise and stirred for 1h at same temperature. The mixture was slowly brought to 0°C, quenched with saturated NH₄Cl solution (10 mL) and washed with CH₂Cl₂ (2 x 20 mL). The aqueous layer was acidified with 1N HCl to pH ~4 and extracted with EtOAc (2 x 75 mL). The combined extracts were dried (Na₂SO₄) and concentrated *in vacuo.* The resultant residue was purified by column chromatography on silica gel eluting with hexane/EtOAc (0 - 10%) to give ethyl 3-chloro-2-fluoro-6-methoxybenzoate as a light-yellow oil (4.2 g, 58 % yield).

¹H NMR (300 MHz, CDCl₃) δ 7.25 (t, 1H), 6.56 (d, 1H), 4.29 (q, 2H), 3.73 (s, 3H), 1.26 (t, 3H).

¹⁹F NMR (300 MHz, CDCl₃) δ -114.99 ppm.

### Step 2: Ethyl 3-chloro-2-fluoro-6-hydroxybenzoate

Aluminium chloride (7.7 mL, 58.0 mmol) was added to a solution of ethyl 3-chloro-2-fluoro-6-methoxybenzoate (4.2 g, 18.0 mmol) in CH₂Cl₂ (50 mL). The reaction mixture was stirred at ambient temperature for 16 h before the mixture was poured into 1N HCl (50 mL) and stirred for 30 min. The aqueous mixture was extracted with CH₂Cl₂ (2 x 75 mL), the combined extracts dried (Na₂SO₄) and concentrated under reduced pressure. The resultant residue was purified by column chromatography on silica gel eluting with hexane/EtOAc (0 - 10%) to afford the title compound as a yellow oil (3.6 g, 92 % yield). ¹H NMR (300 MHz, CDCl₃) δ 11.11 (s, 1H), 7.26 (t, 1H), 6.59 (dd, 1H), 4.30 (q, 2H), 3.37 (s, 3H), 1.27 (t, 3H).

¹⁹F NMR (300 MHz, CDCl₃) δ -105.25 ppm.

### Step 3: Ethyl 3-chloro-2-fluoro-6-hydroxy-5-nitrobenzoate

A premixed solution of sulphuric acid (1.6 mL, 29.6 mmol) and nitric acid (70%) (1.9 mL, 29.6 mmol) was added to a solution of ethyl 3-chloro-2-fluoro-6-hydroxybenzoate (3.6 g, 16.5 mmol) in CH₂Cl₂ (6 mL) at 0°C. The reaction mixture was stirred at ambient temperature for 5 h, poured into crushed ice. After melting the mixture was extracted with EtOAc (2 x 50 mL), dried (Na₂SO₄), and concentrated under reduced pressure to afford the title compound as a light yellow oil (4.2 g, 97 % yield).

¹H NMR (300 MHz, CD₃OD) δ 8.42 (d, 1H), 4.48 (q, 2H), 1.42 (t, 3H).

¹⁹F NMR (300 MHz, CD₃OD) δ -103.50 ppm.

### Step 4: Ethyl 3-amino-5-chloro-6-fluoro-2-hydroxybenzoate

Platinum on carbon (5 wt %) as catalyst (10% w/w), 420 mg) was added to a solution of ethyl 3-chloro-2-fluoro-6-hydroxybenzoate (4.2 mL, 15.9 mmol) in EtOAc (60 mL). The reaction mixture was subjected to 20 psi hydrogen at ambient temperature for 16 h. The reaction mixture was filtered through a pad of celite and concentrated to provide the title amine (3.7 g) as a brown solid, which was utilised directly in the next step.

¹H NMR (300 MHz, CDCl₃) δ 11.68 - 11.18 (br s, 1H), 6.86 (d, 1H), 4.45 (q, 2H), 4.13 - 4.16 (br s, 1H), 1.43 (t, 3H).

¹⁹F NMR (300 MHz, CDCl₃) δ -120.44 ppm.

### Step 5: Ethyl 3-amino-5-chloro-2-(2-chloroethoxy)-6-fluorobenzoate

K₂CO₃ (16.6 g, 120 mmol), and 1-bromo-2-chloroethane (2.1 g, 14.4 mmol) was added to a solution of ethyl 3-amino-5-chloro-6-fluoro-2-hydroxybenzoate (2.8 g, 12.0 mmol) in acetone (50 mL). The reaction mixture was heated at refluxed 16 h., cooled and water added (50 mL). The mixture was extracted with EtOAc (2 x 50 mL) and the combined extracts dried (Na₂SO₄), evaporated and purified by column chromatography on silica gel eluting with hexane/EtOAc (0 -50%) to provide the title compound (2.4 g, 68%) as a yellow liquid.

¹H NMR (300 MHz, CDCl₃) δ 6.82 (d, 1H), 4.40 (q, 2H), 4.23 (t, 2H), 3.80 (t, 2H), 1.38 (t, 3H).

¹⁹F NMR (300 MHz, CDCl₃) δ -128.82 ppm.

### Step 6: Ethyl 6-chloro-7-fluoro-3,4-dihydro-2H-1,4-benzoxazine-8-carboxylate

K₂CO₃ (2.35 g, 17.0 mmol), and KI (2.83 g, 17.0 mmol) were added to a solution of ethyl 3-amino-5-chloro-2-(2-chloroethoxy)-6-fluorobenzoate (2.4 g, 8.5 mmol) in DMF (12 mL). The reaction mixture was heated at 85°C in a sealed tube for 16 h, cooled and evaporated. The residue was purified by column chromatography on silica gel eluting with hexane/ EtOAc (0-50%) to obtain the title ester (1.7 g, 81%) as a light yellow gum. ¹H NMR (300 MHz, CDCl₃) δ 6.65 (d, 1H), 4.39 (q, 2H), 4.27 (t, 2H), 3.41 (t, 2H), 1.37 (t, 3H).

¹⁹F NMR (300 MHz, CDCl₃) δ -128.82 ppm.

ES- MS: *m*/*z* 261.2 (M+H).

### Example 25: Methyl 7-chloro-6-fluoro-3,4-dihydro-2H-1,4-benzoxazine-5-carboxylate

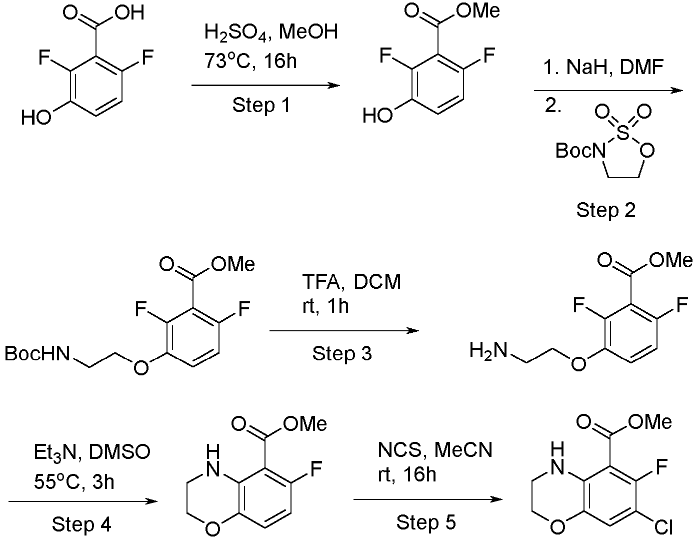

### Step 1: Methyl 2,6-difluoro-3-hydroxybenzoate

Sulphuric acid (1.0 mL) was added to a solution of 2,6-difluoro-3-hydoxybenzoic acid (4.00 g, 23.0 mmol) in methanol (50 mL) at ambient temperature. The reaction mixture was heated at 73 °C for 16 h, cooled and the solvent removed under reduced pressure. The residue was treated with EtOAc (100 mL) and the mixture washed with satd. NaHCO₃ solution (2 x 75 mL). The organic layer was dried (Na₂SO₄) concentrated *in vacuo* to provide the title compound (3.70 g, 86 %).

¹H NMR (300 MHz, CDCl₃); δ 7.12 - 7.05 (m, 1H), 6.89 - 6.83 (m, 1H), 5.12 (d, 1H), 3.96 (s, 3H).

### Step 2: Methyl 2,6-difluoro-3-[2-(tert-butoxycarbonylamino)ethoxy]benzoate

60% NaH (0.82 g, 20.4 mmol) was added to a solution of methyl 2,6-difluoro-3-hydroxybenzoate (3.50 g, 18.6 mmol) in DMF (35 mL) at 0°C. The reaction mixture was stirred at ambient temperature for 15 min, cooled to 0°C, then 2,2-dioxo-[1,2,3]oxathiazolidine-3-carboxylic acid tert-butyl ester (4.57 g, 20.5 mmol) was added and the mixture was stirred at ambient temperature for 2 h. It was cooled to 0°C, quenched with water (100 mL) and extracted with EtOAc (2 x 100 mL). The combined organic layers were washed with water (3 x 100mL), dried (Na₂SO₄) and concentrated under reduced pressure. The residue was triturated with hexanes (3 x 20 mL) to afford the title compound (5.20 g, 84%).

¹H NMR (300 MHz, CDCl₃); δ 7.07 - 7.00 (m, 1H), 6.90 - 6.83 (m, 1H), 5.00 (br s, 1H), 4.06 (t, 2H), 3.96 (s, 3H), 3.53 (q, 2H), 1.45 (s, 9H).

### Step 3: Methyl 3-(2-aminoethoxy)-2,6-difluorobenzoate

TFA (15.0 mL) was added to a solution of methyl 2,6-difluoro-3-[2-(tert-butoxycarbonylamino)ethoxy]benzoate (2.50 g, 7.55 mmol) in CH₂Cl₂ (15.0 mL) at ambient temperature and stirred for 1 h. The solvent was removed under reduced pressure and the residue basified with satd. NaHCO₃ solution before extracted with CH₂Cl₂ (2 x 50 mL). The combined organic layers were dried (Na₂SO₄) and concentrated under reduced pressure to obtain the title compound, which was utilised directly in the next step.

### Step 4: Methyl 6-fluoro-3,4-dihydro-2H-1,4-benzoxazine-5-carboxylate

Et₃N (3.0 mL, 21.4 mmol) was added to a solution of methyl 3-(2-aminoethoxy)-2,6-difluorobenzoate (1.24 g, 5.36 mmol) in DMSO (12 mL) at 0°C. The reaction mixture was heated at 55°C for 3 h and cooled to room temperature. It was quenched with water (50 mL) and extracted with EtOAc (2 x 50 mL). The combined organic layers were washed with 1.0 N HCl (3 x 75 mL) and water (2 x 75 mL). The organic layer was dried (Na₂SO₄) and evaporated *in vacuo* to afford the title compound (0.62 g, 55%).

¹H NMR (300 MHz, CDCl₃); δ 7.47 (br s, 1H), 6.79 (dd, 1H), 6.21 (dd, 1H), 4.15 (t, 2H), 3.89 (s, 3H), 3.51 (t, 2H).

### Step 5: Methyl 7-chloro-6-fluoro-3,4-dihydro-2H-1,4-benzoxazine-5-carboxylate

NCS (0.56 g, 4.93 mmol) was added to a solution of methyl 6-fluoro-3,4-dihydro-2H-1,4-benzoxazine-5-carboxylate (0.98 g, 4.64 mmol) in CH₃CN (20 mL) at ambient temperature and stirred for 16 h. The solvent was removed under reduced pressure and the residue was treated with EtOAc (50 mL) and washed with water (2 x 50 mL). The organic layer was dried (Na₂SO₄) and concentrated under reduced pressure. The residue was triturated with hexanes (15 mL) to afford the title compound (0.77g, 68%). ¹H NMR (300 MHz, CDCl₃); δ 7.46 (br s, 1H), 6.88 (s, 1H), 4.15 (t, 2H), 3.90 (s, 3H), 3.51 (t, 2H).

### Example 26: Methyl 6-chloro-7-fluoro-3,4-dihydro-2H-1,4-benzoxazine-8-carboxylate

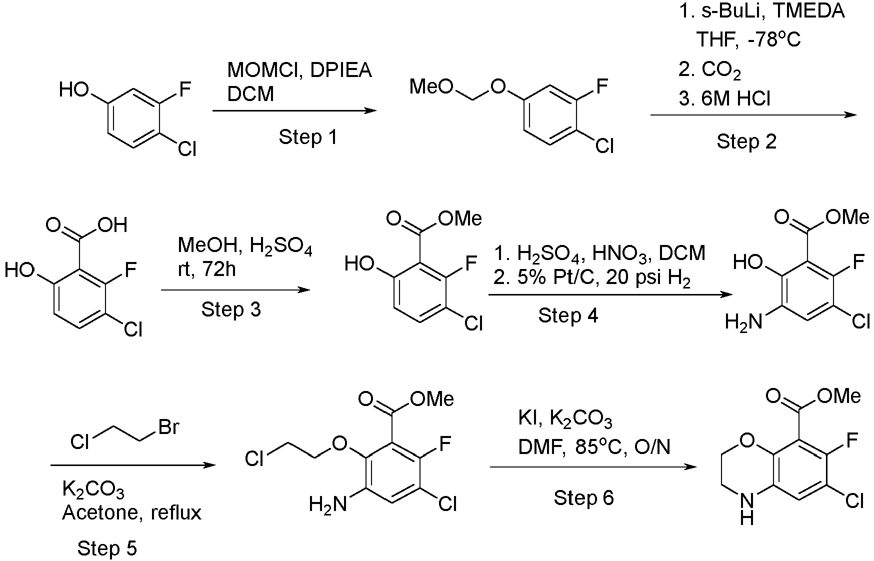

### Step 1: 1-Chloro-2-fluoro-4-(methoxymethoxy)benzene

DIPEA (5.3 mL, 40.96 mmol) and chloromethyl methyl ether (2.42 g, 30.03 mmol) were added to a solution of 4-chloro-3-fluorophenol (4.0 g, 27.3 mmol) in CH₂Cl₂ (50 mL) at 0°C. The reaction mixture was stirred at ambient temperature for 16 h, extracted with CH₂Cl₂ (2 x 75 mL), washed with 1M HCl (30 mL), dried (Na₂SO₄), and concentrated under reduced pressure. The residue was purified by column chromatography on silica gel, eluting with CH₂Cl₂ to provide the title compound as a colourless oil (4.2 g, 81 % yield).

¹H NMR (300 MHz, CDCl₃) δ 7.26 (t, 1H), 6.87 (dd, 1H), 6.80 - 6.74 (m, 1H), 5.14 (s, 2H), 3.17 (s, 3H).

¹⁹F NMR (300 MHz, CDCl₃) δ -113.05 ppm.

### Step 2: 3-Chloro-2-fluoro-6-hydroxybenzoic acid

Sec-BuLi (1.4 M in cyclohexane) (8.25 mL, 11.55 mmol) and TMEDA (1.34 g, 11.55 mmol) were added to a solution of 1-chloro-2-fluoro-4-(methoxymethoxy)benzene (2.0 g, 10.5 mmol) in THF (24 mL) at -78°C. The reaction mixture was stirred at that temperature for 1.5 h, and CO₂ was bubbled through it for 20 min. and the mixture was stirred for 1h at the same temperature before being brought slowly to 0°C and quenched with 1N NaOH solution (10 mL). The basic solution was washed with CH₂Cl₂ (2 x 20 mL) and the aqueous layer was separated and acidified with 6N HCl (30 mL). The mixture was extracted with EtOAc (2 x 75 mL), dried (Na₂SO₄) and concentrated under reduced pressure to provide the title acid (1.4 g, 70% yield).

¹H NMR (300 MHz, CD₃OD) δ 7.52 (t, 1H), 6.79 (dd, 1H).

¹⁹F NMR (300 MHz, CD₃OD) δ -108.56 ppm.

### Step 3: Methyl 3-chloro-2-fluoro-6-hydroxybenzoate

Sulphuric acid (2mL)) was added to a solution of 3-chloro-2-fluoro-6-hydroxybenzoic acid (1.4 g mg, 7.35 mmol) in methanol (20 mL) at ambient temperature and stirred for 72 h. The reaction mixture was extracted with EtOAc (2 x 60 mL) and the combined extracts were washed with saturated NaHCO₃ dried (Na₂SO₄), and evaporated *in vacuo* to obtain the title ester (1.0 g, 67 % yield).

¹H NMR (300 MHz, CDCl₃) δ 11.19 (s, 1H), 7.42 (t, 1H), 6.76 (d, 1H), 3.98 (s, 3H).

¹⁹F NMR (300 MHz, CDCl₃) δ -105.45 ppm.

### Step 4: Methyl 3-amino-5-chloro-6-fluoro-2-hydroxybenzoate

Premixed sulphuric acid (0.605 g, 6.16 mmol) and nitric acid (70%) (0.554 g, 6.16 mmol) was added to a solution of methyl 3-chloro-2-fluoro-6-hydroxybenzoate (0.840 g, 4.11 mmol) in CH₂Cl₂ (3 mL) at 0°C. The reaction mixture was stirred at ambient temperature for 6 h and poured onto crushed ice. After melting the mixture was extracted with EtOAc (2 x 50 mL), dried (Na₂SO₄), and concentrated under reduced pressure. Platinum on carbon (5 wt %, 10% w/w, 84 mg) was added to a solution of the crude product in EtOAc (30 mL). The reaction mixture was hydrogenated at 20 psi at ambient temperature for 16 h. The reaction mixture was filtered through a pad of celite and concentrated to afford the title ester (0.65 g, 72%, crude yield for two steps) which was utilised directly in the next step.

¹H NMR (300 MHz, CDCl₃) δ 11.37 (s, 1H), 6.84 (d, 1H), 3.99(s, 3H), 3.89 - 3.73 (br s, 2H).

¹⁹F NMR (300 MHz, CDCl₃) δ -121.42 ppm.

### Step 5: Methyl 3-amino-5-chloro-2-(2-chloroethoxy)-6-fluorobenzoate

K₂CO₃ (4.09 g, 29.6 mmol), and 1-bromo-2-chloroethane (0.51 g, 3.55 mmol) was added to a solution of methyl 3-amino-5-chloro-6-fluoro-2-hydroxybenzoate (0.65 g, 2.96 mmol) in acetone (30 mL). The reaction mixture was heated at reflux for 16 h., cooled, treated with water (20 mL) and extracted with EtOAc (2 x 30 mL). and dried (Na₂SO₄). The residue on evaporation was purified by column chromatography on silica gel eluting with hexane/EtOAc (0 - 50%) to afford the title compound (0.6 g, 72%) as a yellow oil.

¹H NMR (300 MHz, CDCl₃) δ 6.84 (d, 1H), 4.23 (t, 2H), 4.17 - 3.98 (br s, 2H), 3.93 (s, 3H), 3.80 (t, 2H).

¹⁹F NMR (300 MHz, CDCl₃) δ -128.04 ppm.

### Step 6: Methyl 6-chloro-7-fluoro-3,4-dihydro-2H-1,4-benzoxazine-8-carboxylate

K₂CO₃ (0.59 g, 4.26 mmol), and KI (0.71 g, 4.26 mmol) was added to a solution of methyl 3-amino-5-chloro-2-(2-chloroethoxy)-6-fluorobenzoate (0.6 g, 2.13 mmol) in DMF (6 mL). The reaction mixture was heated at 85°C for 16 h in a sealed tube, cooled and evaporated. The crude product was purified by column chromatography on silica gel eluting with hexane/EtOAc (0 - 50%) to provide the title ester (0.3 g, 57%) as a cream solid.

¹H NMR (300 MHz, CDCl₃) δ 6.66 (d, 1H), 4.28 (t, 2H), 3.92 (s, 3H), 3.41 (t, 2H).

¹⁹F NMR (300 MHz, CDCl₃) δ -128.82 ppm.

ES- MS: *m*/*z* 246.2 (M+H).

### Example 27: 6-Chloro-4-[2-(3,5-difluorophenyl)ethyl]-7-methoxy-3,4-dihydro-2H-1,4-benzoxazine-8-carboxylic acid (A-53)

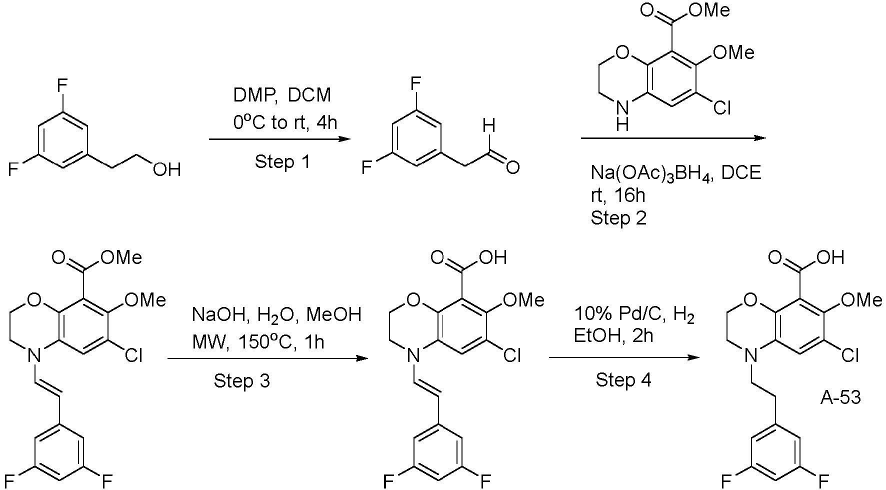

### Step 1: (3,5-Difluorophenyl)acetaldehyde

Dess-Martin periodinane (1.74 g, 4.10 mmol) was added in several portions to a solution of 2-(3,5-difluorophenyl)ethanol (0.54 g, 3.41 mmol) in CH₂Cl₂ (12.0 mL) at 0°C and the reaction mixture was stirred at room temperature for 4 h. The reaction mixture was diluted with CH₂Cl₂ (20 mL), washed with saturated Na₂S₂O₃ (2 x 30 mL) and saturated NaHCO₃ (2 x 30 mL). The organic layer was dried (Na₂SO₄) and concentrated *in vacuo.* The residue was treated with hexanes (3 x 7.0 mL) and the washes were concentrated *in vacuo* to afford the title compound (0.32 g, 60 %).

¹H NMR (300 MHz, CDCl₃); δ 9.76 (s, 1H), 6.79 - 6.74 (m, 3H), 3.70 (s, 2H).

### Step 2: Methyl 6-chloro-4-[(1E)-2-(3,5-difluorophenyl)ethenyl]-7-methoxy-3,4-dihydro-2H-1,4-benzoxazine-8-carboxylate

(3,5-Difluorophenyl)acetaldehyde (115 mg, 0.74 mmol) was added to a solution of methyl 6-chloro-7-methoxy-3,4-dihydro-2H-1,4-benzoxazine-8-carboxylate (63 mg, 0.24 mmol) in 1,2-dichloroethane (3.0 mL) and the reaction mixture was stirred for 30 min. To this mixture, NaBH(OAc)₃ (155 mg, 0.73 mmol) was added and the reaction mixture was stirred at ambient temperature for 16 h. The reaction was diluted with CH₂Cl₂ (15 mL) and washed with saturated NaHCO₃ solution (2 x 15 mL). The organic layer was dried (Na₂SO₄), concentrated *in vacuo* and the product was utilised directly in the next step.

### Step 3: 6-Chloro-4-[(1E)-2-(3,5-difluorophenyl)ethenyl]-7-methoxy-3,4-dihydro-2H-1,4-benzoxazine-8-carboxylic acid

NaOH (59 mg, 1.48 mmol) was added to a solution of methyl 6-chloro-4-[(1E)-2-(3,5-difluorophenyl)ethenyl]-7-methoxy-3,4-dihydro-2H-1,4-benzoxazine-8-carboxylate (98 mg, 0.25 mmol) in a mixture of CH₃OH (2.0 mL) and water (1.0 mL) and the reaction mixture was heated at 150°C under microwave conditions for 1 h. The solvent was removed under reduced pressure, the residue was acidified with 1.0 N HCl to pH ~4 and extracted with EtOAc (2 x 15 mL). The combined organic layers were dried (Na₂SO₄) and concentrated *in vacuo.* The resultant residue was triturated with hexanes (2 x 3.0 mL) to obtain the title compound (60 mg, 63 %).

¹H NMR (300 MHz, CD₃OD); δ 7.63 (d, 1H), 7.19 (s, 1H), 7.01 - 6.98 (m, 2H), 6.61 - 6.56 (m, 1H), 5.80 (d, 1H), 4.29 (t, 2H), 3.88 (s, 3H), 3.69 (t, 2H).

### Step 4: 6-chloro-4-[2-(3,5-difluorophenyl)ethyl]-7-methoxy-3,4-dihydro-2H-1,4-benzoxazine-8-carboxylic acid

10% Pd/C catalyst (50% w/w, 30 mg) was added to a solution of 6-chloro-4-[(1E)-2-(3,5-difluorophenyl)ethenyl]-7-methoxy-3,4-dihydro-2H-1,4-benzoxazine-8-carboxylic acid (60 mg, 0.16 mmol) in ethanol (4.0 mL). The mixture was stirred for 2 h under a hydrogen atmosphere at balloon pressure, filtered through a pad of celite and concentrated *in vacuo* to provide the title compound (20 mg, 33 %).

¹H NMR (300 MHz, CD₃OD); 7.19 (s, 1H), 6.83 - 6.76 (m, 1H), 6.69 - 6.65 (m, 1H), 6.54 - 6.52 (m, 1H), 4.20 (t, 2H), 3.76 (s, 3H), 3.50 (t, 2H), 3.69 (t, 2H), 2.89 (t, 2H).

*m*/*z* 384.7 (M+1)

HPLC retention time: 13.5 min., purity: 95.1 % at 280 nm.

### Example 28: 6-chloro-7-methoxy-4-[(thiophen-2-yl)methyl]-3,4-dihydro-2H-1,4-benzoxazine-8-carboxylic acid (A-42)

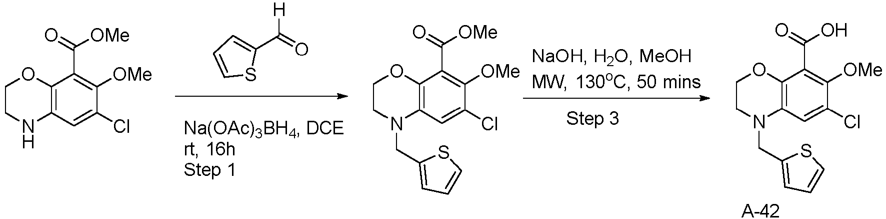

### Step 1: Methyl 6-chloro-7-methoxy-4-thenyl-3,4-dihydro-2H-1,4-benzoxazine-8-carboxylate

Sodium triacetoxyborohydride (197 mg, 0.93 mmol) was added to a solution of methyl 6-chloro-7-methoxy-3,4-dihydro-2H-1,4-benzoxazine-8-carboxylate (80 mg, 0.31 mmol) and 2-thiophenecarboxaldehyde (104 mg, 0.93 mmol) in 1,2-dichloroethane (4 mL). The reaction was stirred at ambient temperature for 16 h, diluted with CH2Cl2 (100 mL), washed with brine (3 x 50 mL), dried (Na₂SO₄) and concentrated under reduced pressure. The residue was purified by column chromatography on silica gel eluting with hexane/EtOAc (0-40%) to give the title compound (107 mg, 97%).

¹H NMR (300 MHz, CDCl₃) δ 7.57 (d, 1H), 7.56 (d, 1H), 7.52 (dd, 1H), 7.11 (s, 1H), 4.83 (s, 2H), 4.54 (t, 2H), 4.21 (s, 3H), 4.11 (s, 3H), 3.61 (t, 2H).

ES-MS: 354.8 [M+1].

### Step 2: 6-Chloro-7-methoxy-4-[(thiophen-2-yl)methyl]-3,4-dihydro-2H-1,4-benzoxazine-8-carboxylic acid

NaOH (35.6 mg, 0.89 mmol) was added to a solution of methyl 6-chloro-7-methoxy-4-thenyl-3,4-dihydro-2H-1,4-benzoxazine-8-carboxylate (105 mg, 0.29 mmol) in methanol (2.0 mL) and water (1.0 mL), and the reaction mixture was heated at 130°C under microwave conditions for 1 h. The solvent was removed under reduced pressure and the residue was diluted with water (15 mL) which was washed with CH₂Cl₂ (2 x 20 mL). The aqueous layer which was acidified with 1N HCl to pH ~4. The mixture was extracted with EtOAc (3 x 25 mL) and the combined extracts were dried (Na₂SO₄) and concentrated *in vacuo* to provide the title compound (63 mg, 62%).

¹H NMR (300 MHz, CDCl₃) δ 7.17 (d, 1H), 6.90 (d, 1H), 6.85 (dd, 1H), 6.77 (s, 1H), 4.50 (s, 2H), 4.12 (t, 2H), 3.67 (s, 3H), 3.23 (t, 2H).

ES-MS: 340.6 [M+1].

HPLC: Retention Time: 10.81 min., purity: 98.0% @ 254 nm.

### Example 29: 6-chloro-7-fluoro-4-(3-methylbutyl)-3,4-dihydro-2H-1,4-benzoxazine-8-carboxylic acid (A-17)

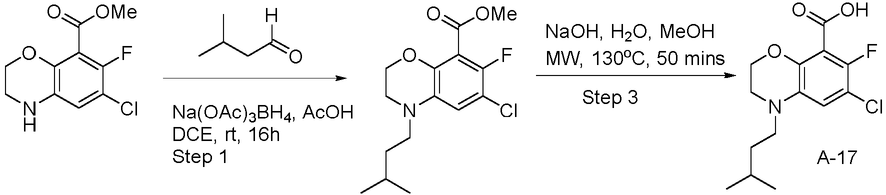

### Step 1: Methyl 6-chloro-7-fluoro-4-isopentyl-2,3-dihydro-1,4-benzoxazine-8-carboxylate

A solution of methyl 6-chloro-7-fluoro-3,4-dihydro-2H-1,4-benzoxazine-8-carboxylate (20 mg, 0.081 mmol), 3-methylbutanal (18 µL, 0.16 mmol) and acetic acid (23 µL, 0.40 mmol) in 1,2-dichloroethane (1 mL) was stirred at ambient temperature for 10 minutes prior to the addition of sodium triacetoxyborohydride (86 mg, 0.41 mmol). The resulting suspension was stirred at ambient temperature for 18 h. The reaction was quenched with NaHCO₃ solution (2 mL) and the biphasic mixture was washed with CH₂Cl₂ (5 mL). The organics eluted were concentrated to dryness to afford the title compound (22 mg, 84%) as a colourless oil.

¹H NMR (400 MHz, CDCl₃) δ 6.60 (d, *J* = 7.1 Hz, 1H), 4.29 - 4.22 (m, 2H), 3.92 (s, 3H), 3.33 - 3.26 (m, 2H), 3.25 - 3.14 (m, 2H), 1.61 (dp, J = 13.2, 6.6 Hz, 1H), 1.50 - 1.39 (m, 2H), 0.96 (d, *J* = 6.6 Hz, 6H).

### Step 2: 6-Chloro-7-fluoro-4-(3-methylbutyl)-3,4-dihydro-2H-1,4-benzoxazine-8-carboxylic acid

To a solution of methyl 6-chloro-7-fluoro-4-isopentyl-2,3-dihydro-1,4-benzoxazine-8-carboxylate (22 mg, 0.070 mmol) in CH₃OH (0.2 mL) was added a solution of NaOH (7.0 mg, 0.17 mmol) in water (0.6 mL). The reaction mixture was stirred at 80 °C overnight. The reaction mixture was concentrated *in vacuo* and diluted with CH₂Cl₂ (10 mL) and water (10 mL). 1 N HCl was added until the pH of the mixture reached 2. The aqueous layer was then extracted with EtOAc (2 x 10 mL). The organic layer was washed with brine (20 mL), dried over Na₂SO₄, filtered then concentrated *in vacuo.* The crude product was suspended in Et₂O, sonicated, and the liquors were removed with a pipette. This process was repeated twice and the solids were dried under reduced pressure to afford the title compound (13 mg, 61%) as a pink solid.

¹H NMR (400 MHz, CDCl₃) δ 6.69 (d, *J* = 7.0 Hz, 1H), 4.38 - 4.32 (m, 2H), 3.38 - 3.32 (m, 2H), 3.27 - 3.18 (m, 2H), 1.63 (dp, *J* = 13.2, 6.6 Hz, 1H), 1.51 - 1.41 (m, 2H), 0.97 (d, *J* = 6.6 Hz, 6H).

UPLC-MS analysis (4 min, basic): rt = 1.30 min, m/z = 302.0 [M+H]⁺, 99% purity.

### Example 30: 4-Benzyl-7-chloro-6-fluoro-3,4-dihydro-2H-1,4-benzoxazine-5-carboxylic acid (A-45)

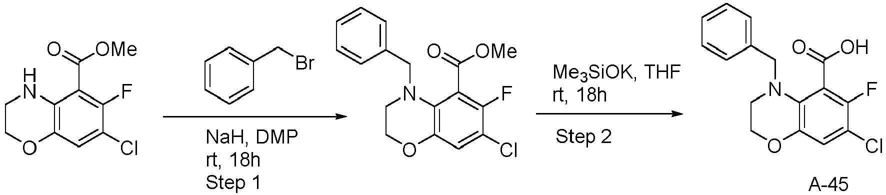

### Step 1: Methyl 4-benzyl-7-chloro-6-fluoro-2,3-dihydro-1,4-benzoxazine-5-carboxylate

To a solution of methyl 7-chloro-6-fluoro-3,4-dihydro-2H-1,4-benzoxazine-5-carboxylate (35 mg, 0.142 mmol) and NaH, 60% in mineral oil (14 mg, 0.35 mmol) in NMP (1.43 mL) stirred at room temperature for 5 mins, was added benzyl bromide (0.025 mL, 0.21 mmol) and the resulting mixture was stirred at room temperature overnight. The reaction was quenched with 1M HCl (10 mL) and extracted with EtOAc (2 x 15 mL). The organic layers were dried over Na₂SO₄, filtered, and concentrated in vacuo. The crude material was purified by column chromatography over silica (4 g cartridge) eluting with a gradient of EtOAc (0% to 50%; v/v) in iso-hexane to afford the title compound (30 mg, 63%).

¹H NMR (400 MHz, CDCl₃) δ 7.39 - 7.28 (m, 5H), 6.95 (dd, *J* = 7.3, 0.6 Hz, 1H), 4.22 (s, 2H), 4.08 - 4.04 (m, 2H), 3.72 (s, 3H), 3.06 (dd, J = 4.9, 3.9 Hz, 2H).

### Step 2: 4-Benzyl-7-chloro-6-fluoro-3,4-dihydro-2H-1,4-benzoxazine-5-carboxylic acid

To a stirred solution of methyl 4-benzyl-7-chloro-6-fluoro-2,3-dihydro-1,4-benzoxazine-5-carboxylate (35 mg, 0.10 mmol) in THF (0.89 mL) was added potassium trimethylsilanolate (0.10 mL, 0.21 mmol, 2M in THF) and the resulting mixture was stirred at room temperature for 2 hours. The reaction mixture was then stirred at 60 °C overnight. 1M HCl (5 mL) was added, and the mixture was extracted with EtOAc (2 x 10 mL). The organics were dried over Na₂SO₄ and concentrated to dryness to afford the title compound (31 mg, 88%) as a brown solid.

¹H NMR (400 MHz, CDCl₃) δ 7.45 - 7.33 (m, 6H), 7.13 (dd, J= 6.8, 1.0 Hz, 1H), 4.29 - 4.22 (m, 2H), 4.16 (d, *J* = 1.6 Hz, 2H), 3.11 (dd, *J* = 5.3, 4.4 Hz, 2H).

UPLC-MS analysis (4 min, basic): rt = 1.08 min, m/z = 322.0 [M+H]⁺, 95% purity.

### Example 31: 7-Chloro-6-fluoro-4-propyl-3,4-dihydro-2H-1,4-benzoxazine-5-carboxylic acid (A-56)

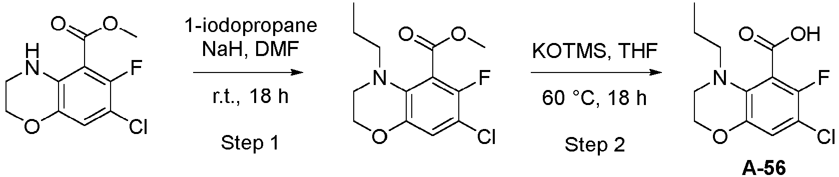

### Step 1: methyl 7-chloro-6-fluoro-4-propyl-2,3-dihydro-1,4-benzoxazine-5-carboxylate

A solution of methyl 7-chloro-6-fluoro-3,4-dihydro-2H-1,4-benzoxazine-5-carboxylate (35 mg, 0.142 mmol) and sodium hydride, 60% in mineral oil (14 mg, 0.356 mmol) in NMP (1.42 mL) was stirred at room temperature for 5 mins, then 1-iodopropane (0.021 mL, 0.214 mmol) was added and the resulting mixture was stirred at room temperature for 18 h.

The reaction mixture was diluted with 1M HCl (10 mL) and extracted with EtOAc (3 x 10 mL). The combined organics were washed with brine (3 × 10 mL), dried over Na₂SO₄ and concentrated to dryness. The crude material was purified by column chromatography over silica (12 g cartridge) eluting with a gradient of EtOAc (0% to 50%; v/v) in isohexane to afford the title compound (30 mg, 70%) as a yellow oil.

¹H NMR (400 MHz, CHLOROFORM-D) δ 6.87 (dd, J = 7.4, 0.6 Hz, 1H), 4.12 - 4.05 (m, 2H), 3.93 (d, J = 0.6 Hz, 3H), 3.24 - 3.17 (m, 2H), 2.97 - 2.90 (m, 2H), 1.62 - 1.53 (m, 2H), 0.87 (t, J = 7.4 Hz, 3H).

### Step 2: 7-Chloro-6-fluoro-4-propyl-3,4-dihydro-2H-1,4-benzoxazine-5-carboxylic acid

To a stirring solution of methyl 7-chloro-6-fluoro-4-propyl-2,3-dihydro-1,4-benzoxazine-5-carboxylate (30 mg, 0.104 mmol) in THF (1.04 mL) was added potassium trimethylsilanolate (0.10 mL, 0.209 mmol) and the reaction mixture was stirred at room temperature for 2 hours.

The reaction mixture was then stirred at 60 °C overnight.

1M HCl (5 mL) was added, and the mixture was extracted with EtOAc (2 x 10 mL). The organics were dried over Na₂SO₄ and concentrated to dryness to give the product as a brown solid.

The crude product was purified by prep HPLC to give the title compound (1.3 mg, 5%) as a colourless solid.

UPLC-MS analysis (4 min, basic): rt = 1.52 min, m/z = 274.1 [M+H]⁺, 99% purity.

¹H NMR (400 MHz, DMSO-D6) δ 6.96 (d, J = 7.4 Hz, 1H), 4.10 - 4.01 (m, 2H), 3.17 - 3.10 (m, 2H), 3.03 - 2.94 (m, 2H), 1.72 - 1.46 (m, 2H), 0.77 (t, J = 7.4 Hz, 3H).

### Example 32: 7-Chloro-6-fluoro-4-[(p-methoxyphenyl)methyl]-3,4-dihydro-2H-1,4-benzoxazine-5-carboxylic acid (A-58)

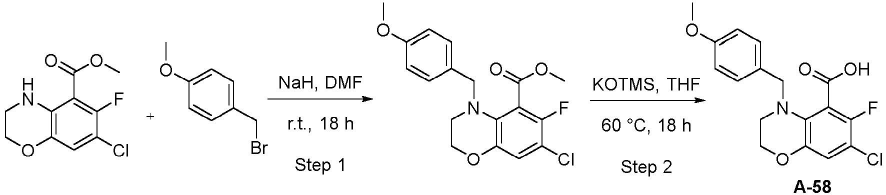

### Step 1: methyl 7-chloro-6-fluoro-4-[(4-methoxyphenyl)methyl]-2,3-dihydro-1,4-benzoxazine-5-carboxylate

To a -10 °C solution of methyl 7-chloro-6-fluoro-3,4-dihydro-2H-1,4-benzoxazine-5-carboxylate (50 mg, 0.199 mmol) in DMF (1.5 mL) was added sodium hydride, 60% in mineral oil (10 mg, 0.259 mmol) and the reaction mixture was stirred at -10 °C for 15 min. A solution of 4-methoxybenzyl bromide (64 mg, 0.319 mmol) in DMF (0.5 mL) was added to a stirred reaction mixture at -10 °C and kept stirring at this temperature for 2 h.

The reaction mixture was quenched with H₂O (10 mL) and extracted with DCM (2 x 20 mL). The organics were washed sequentially with water (2 × 20 mL) and saturated brine solution (20 mL). The organic fraction was dried (Na₂SO₄) and concentrated to dryness. The crude material was purified by column chromatography over silica (12 g cartridge) eluting with a gradient of EtOAc (0% to 100% v/v) in i-Hexane to afford the title compound (55 mg, 64%) as a colourless gum.

¹H NMR (400 MHz, CHLOROFORM-D) δ 7.26 - 7.21 (m, 2H), 6.94 (d, J = 7.3 Hz, 1H), 6.92 - 6.86 (m, 2H), 4.14 (s, 2H), 4.06 - 4.01 (m, 2H), 3.81 (s, 3H), 3.78 (s, 3H), 3.12 - 2.94 (m, 2H).

### Step 2: 7-Chloro-6-fluoro-4-[(p-methoxyphenyl)methyl]-3,4-dihydro-2H-1,4-benzoxazine-5-carboxylic acid

To a solution of methyl 7-chloro-6-fluoro-4-[(4-methoxyphenyl)methyl]-2,3-dihydro-1,4-benzoxazine-5-carboxylate (50 mg, 0.116 mmol) in Et₂O (4 mL) was added potassium tert-butoxide (117 mg, 1.05 mmol) at room temperature. To this slurry water (5 µL) was added and stirred at room temperature for 2 h.

The reaction mixture was diluted with water (20 mL), the aqueous phase was washed with EtOAc (20 mL) and acidified by 1 M HCl to pH ~ 5 and extracted with EtOAc (20 mL). The organic phase was washed with saturated brine solution (20 mL), dried (Na₂SO₄) and concentrated to dryness. The crude material was purified by column chromatography over silica (12 g cartridge) eluting with a gradient of MeOH (0% to 50%; v/v) in EtOAc to afford the impure product.

This crude product was purified by prep-HPLC to afford the title compound (5.5 mg, 13%) as a white solid.

UPLC-MS analysis (4 min, basic): rt = 1.14 min, m/z = 350.0 [M-H]⁻, 100% purity.

¹H NMR (400 MHz, DMSO-D6) δ 7.42 - 7.27 (m, 2H), 7.17 - 7.05 (m, 1H), 6.87 - 6.76 (m, 2H), 4.22 (s, 2H), 4.02 - 3.88 (m, 2H), 3.70 (s, 3H), 2.85 - 2.72 (m, 2H).

### Example 33: 7-Chloro-6-fluoro-4-[(p-tolyl)methyl]-3,4-dihydro-2H-1,4-benzoxazine-5-carboxylic acid (A-60)

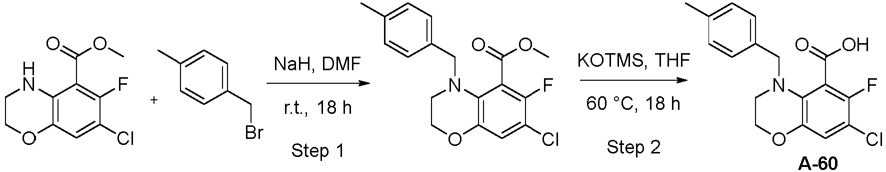

### Step 1: methyl 7-chloro-6-fluoro-4-(p-tolylmethyl)-2,3-dihydro-1,4-benzoxazine-5-carboxylate

To a -15 °C solution of methyl 7-chloro-6-fluoro-3,4-dihydro-2H-1,4-benzoxazine-5-carboxylate (50 mg, 0.199 mmol) in DMF (2 mL) was added sodium hydride, 60% in mineral oil (12 mg, 0.299 mmol) and the resulting suspension was stirred at -13 °C for 15 min. A solution of 4-methylbenzyl bromide (59 mg, 0.319 mmol) in DMF (1 mL) was added to a stirred reaction mixture at -13 °C and kept stirring at this temperature for 2 h.

The reaction mixture was quenched with H₂O (30 mL) and extracted with EtOAc (3 x 20 mL). The organic phase was washed sequentially with water (2 × 20 mL) and saturated brine solution (20 mL). The organic fraction was dried (Na₂SO₄) and concentrated to dryness. The crude product was purified by column chromatography over silica (12 g cartridge) eluting with a gradient of EtOAc (0% to 100% v/v) in i-Hexane to afford the title compound (70 mg, 95%) as a beige solid.

¹H NMR (400 MHz, CHLOROFORM-D) δ 7.23 - 7.12 (m, 4H), 6.94 (d, J = 7.3 Hz, 1H), 4.17 (s, 2H), 4.08 - 3.98 (m, 2H), 3.75 (s, 3H), 3.10 - 3.00 (m, 2H), 2.35 (s, 3H).

### Step 2: 7-Chloro-6-fluoro-4-[(p-tolyl)methyl]-3,4-dihydro-2H-1,4-benzoxazine-5-carboxylic acid

To a solution of methyl 7-chloro-6-fluoro-4-(p-tolylmethyl)-2,3-dihydro-1,4-benzoxazine-5-carboxylate (65 mg, 0.177 mmol) in Et₂O (5 mL) was added potassium tert-butoxide (113 mg, 1.01 mmol) at 0°C for 2 h then at room temperature for 2 h. The reaction mixture was diluted with ether (10 mL) and potassium tert-butoxide (90 mg, 0.802 mmol) was recharged. The reaction mixture was stirred for additional 3 h at room temperature. Water (30 µL) was added and reaction mixture was stirred overnight at room temperature.

The reaction mixture was diluted with water and adjusted pH to ~3 with 1M HCl solution. The product was extracted into EtOAc. The organic phase was washed with saturated brine solution (20 mL), dried (Na₂SO₄) and concentrated to dryness to give the crude oily product which was purified by prep-HPLC to the title compound (24 mg, 39%) as a white solid.

UPLC-MS analysis (4 min, basic): rt = 1.23 min, m/z = 336.1 [M+H]⁺, 97% purity.

¹H NMR (400 MHz, DMSO-D6) δ 7.45 - 7.23 (m, 2H), 7.16 - 7.02 (m, 2H), 6.75 (d, J = 7.3 Hz, 1H), 4.32 (s, 2H), 3.99 - 3.91 (m, 2H), 2.84 - 2.76 (m, 2H), 2.28 (s, 3H).

### Example 34: 7-Chloro-6-fluoro-4-[(1-naphthyl)methyl]-3,4-dihydro-2H-1,4-benzoxazine-5-carboxylic acid (A-61)

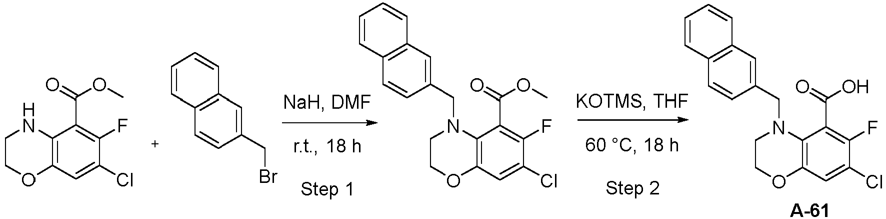

### Step 1: methyl 7-chloro-6-fluoro-4-(2-naphthylmethyl)-2,3-dihydro-1,4-benzoxazine-5-carboxylate

To a -15 °C solution of methyl 7-chloro-6-fluoro-3,4-dihydro-2H-1,4-benzoxazine-5-carboxylate (51 mg, 0.203 mmol) in DMF (2 mL) was added sodium hydride, 60% in mineral oil (12 mg, 0.305 mmol) and the resulting suspension was stirred at -13 °C for 15 min. A solution of 2-(bromomethyl)naphthalene (58 mg, 0.265 mmol) in DMF (1 mL) was added and the solution was kept stirring at this temperature for 3 h. The reaction mixture was quenched with sodium hydroxide 3 M water solution (0.50 mL, 1.50 mmol) and stirred at room temperature for 18 h.

The reaction mixture was diluted with H₂O (30 mL) and extracted with EtOAc (3 x 20 mL). The organic phase was washed sequentially with water (2 × 20 mL) and saturated brine solution (20 mL). The organic fraction was dried (Na₂SO₄) and concentrated to dryness to afford the title compound (70 mg, 71%) as a yellow gum.

¹H NMR (400 MHz, CHLOROFORM-D) δ 7.88 - 7.78 (m, 4H), 7.57 - 7.42 (m, 3H), 6.99 (dd, J = 7.3, 0.5 Hz, 1H), 4.38 (s, 2H), 4.17 - 4.01 (m, 2H), 3.70 (s, 3H), 3.13 - 3.06 (m, 2H).

### Step 2: 7-Chloro-6-fluoro-4-[(1-naphthyl)methyl]-3,4-dihydro-2H-1,4-benzoxazine-5-carboxylic acid

To a solution of methyl 7-chloro-6-fluoro-4-(2-naphthylmethyl)-2,3-dihydro-1,4-benzoxazine-5-carboxylate (70 mg, 0.145 mmol) in Et₂O (5 mL) was added potassium tert-butoxide (179 mg, 1.60 mmol) at 0 °C. To this slurry Water (15 µL) was added at 0 °C and the resulting mixture was stirred at room temperature for 48 h.

The reaction mixture was diluted with water (20 mL), the aqueous phase was washed with EtOAc (2 x 20 mL), acidified by 1 M HCl to pH ~ 2 and extracted with EtOAc. The organics were washed with saturated brine solution (20 mL), dried (Na₂SO₄) and concentrated to dryness. The crude material was purified by column chromatography over C18 (12 g cartridge) eluting with a gradient of MeCN (0.1% NH₃) (10% to 100%; v/v) in water (0.1% NH₃) to afford the title compound (20 mg, 37%) as a white solid. UPLC-MS analysis (4 min, basic): rt = 1.29 min, m/z = 372.1 [M+H]⁺, 100% purity.

¹H NMR (400 MHz, DMSO-D6) δ 7.91 - 7.82 (m, 4H), 7.79 - 7.71 (m, 1H), 7.55 - 7.44 (m, 2H), 6.82 (d, J = 7.2 Hz, 1H), 4.53 (s, 2H), 4.02 (dd, J = 5.0, 3.6 Hz, 2H), 2.94 - 2.62 (m, 2H).

### Example 35: 6-Chloro-4-ethyl-7-fluoro-3,4-dihydro-2H-1,4-benzoxazine-8-carboxylic acid (A-18)

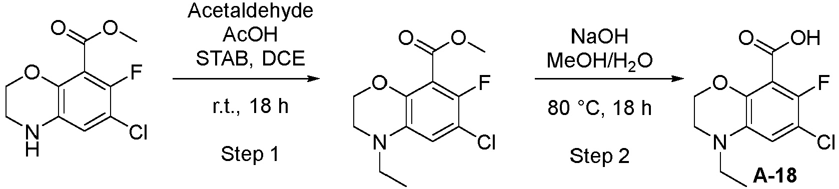

### Step 1: methyl 6-chloro-4-ethyl-7-fluoro-2,3-dihydro-1,4-benzoxazine-8-carboxylate

A solution of methyl 6-chloro-7-fluoro-3,4-dihydro-2H-1,4-benzoxazine-8-carboxylate (20 mg, 0.0814 mmol), acetaldehyde (0.0091 mL, 0.163 mmol) and acetic acid (0.023 mL, 0.407 mmol) in DCE (1 mL) was stirred at room temperature for 10 minutes before the addition of sodium triacetoxyborohydride (86 mg, 0.407 mmol). The resulting suspension was stirred at room temperature for 18 h.

The reaction mixture was quenched with NaHCO₃ solution (2 mL) and the biphasic mixture was placed in a phase separator and washed with DCM (5 mL). The organics eluted were concentrated to dryness to afford the title compound (27 mg, 109%) as a brown oil.

¹H NMR (400 MHz, CHLOROFORM-D) δ 6.65 (d, J = 7.1 Hz, 1H), 4.31 - 4.22 (m, 2H), 3.92 (s, 3H), 3.34 - 3.25 (m, 4H), 1.14 (t, J = 7.1 Hz, 3H).

### Step 2: 6-Chloro-4-ethyl-7-fluoro-3,4-dihydro-2H-1,4-benzoxazine-8-carboxylic acid

To a solution of methyl 6-chloro-4-ethyl-7-fluoro-2,3-dihydro-1,4-benzoxazine-8-carboxylate (27 mg, 0.0987 mmol) in MeOH (0.2 mL) was added a solution of sodium hydroxide (9.9 mg, 0.247 mmol) in water (0.6 mL). The reaction mixture was stirred at 80 °C for 18 h.

The reaction mixture was concentrated *in vacuo* and diluted with DCM (10 mL) and water (10 mL). 1 N HCl was added until the pH of the mixture reached 2. The aqueous layer was then extracted with EtOAc (2 x 10 mL). The organic layer was washed with brine (20 mL), dried over Na₂SO₄, filtered then concentrated *in vacuo.* The crude product was suspended in Et₂O, sonicated, and the liquors were removed with a pipette. This process was renewed twice and the solids were dried under reduced pressure to afford the title compound (10 mg, 38%) as a yellow solid.

UPLC-MS analysis (4 min, basic): rt = 0.89 min, m/z = 260.1 [M+H]⁺, 97% purity.

¹H NMR (400 MHz, CHLOROFORM-D) δ 6.75 (d, J = 7.0 Hz, 1H), 4.42 - 4.35 (m, 2H), 3.39 - 3.28 (m, 4H), 1.16 (t, J = 7.1 Hz, 3H).

### Example 36: 6-Chloro-4-(cyclopropylmethyl)-7-fluoro-3,4-dihydro-2H-1,4-benzoxazine-8-carboxylic acid (A-20)

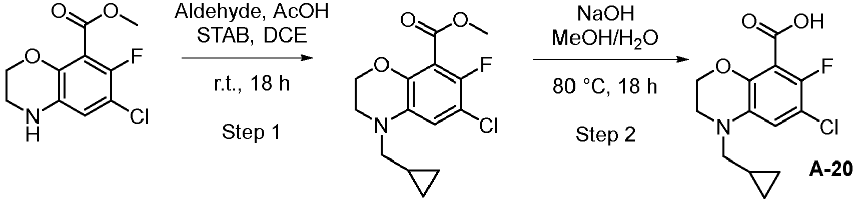

### Step 1: Methyl 6-chloro-4-(cyclopropylmethyl)-7-fluoro-2,3-dihydro-1,4-benzoxazine-8-carboxylate

A solution of cyclopropanecarbaldehyde (0.012 mL, 0.163 mmol), methyl 6-chloro-7-fluoro-3,4-dihydro-2H-1,4-benzoxazine-8-carboxylate (20 mg, 0.0814 mmol) and acetic acid (0.023 mL, 0.407 mmol) in DCE (1 mL) was stirred at room temperature for 10 minutes before the addition of sodium triacetoxyborohydride (86 mg, 0.407 mmol). The resulting mixture was stirred at room temperature for 18 h.

The reaction mixture was quenched with NaHCO₃ solution (2 mL) and the biphasic mixture was placed in a phase separator and washed with DCM (5 mL). The organics eluted were concentrated to dryness to afford the title compound (22 mg, 86%) as a yellow oil.

¹H NMR (400 MHz, CHLOROFORM-D) δ 6.76 - 6.69 (m, 1H), 4.36 - 4.23 (m, 2H), 3.92 (s, 3H), 3.44 - 3.37 (m, 2H), 3.08 (d, J = 6.5 Hz, 2H), 1.06 - 0.92 (m, 1H), 0.65 - 0.52 (m, 2H), 0.28 - 0.17 (m, 2H).

### Step 2: 6-Chloro-4-(cyclopropylmethyl)-7-fluoro-3,4-dihydro-2H-1,4-benzoxazine-8-carboxylic acid

To a solution of methyl 6-chloro-4-(cyclopropylmethyl)-7-fluoro-2,3-dihydro-1,4-benzoxazine-8-carboxylate (22 mg, 0.0734 mmol) in MeOH (0.2 mL) was added a solution of sodium hydroxide (7.3 mg, 0.183 mmol) in water (0.6 mL). The reaction mixture was stirred at 80 °C for 18 h.

The reaction mixture was concentrated *in vacuo* and diluted with DCM (10 mL) and water (10 mL). 1 N HCl was added until the pH of the mixture reached 2. The aqueous layer was then extracted with EtOAc (2 x 10 mL). The organic layer was washed with brine (20 mL), dried over Na₂SO₄, filtered then concentrated *in vacuo* to afford the title compound (7.0 mg, 33%) as an off-white solid.

UPLC-MS analysis (4 min, basic): rt = 1.07 min, m/z = 286.1 [M+H]⁺, 98% purity.

¹H NMR (400 MHz, CHLOROFORM-D) δ 6.81 (d, J = 7.0 Hz, 1H), 4.42 - 4.35 (m, 2H), 3.49 - 3.42 (m, 2H), 3.10 (d, J = 6.5 Hz, 2H), 1.06 - 0.95 (m, 1H), 0.64 - 0.55 (m, 2H), 0.23 (dt, J = 5.9, 4.7 Hz, 2H).

### Example 37: 4-Benzyl-6-chloro-7-fluoro-3,4-dihydro-2H-1,4-benzoxazine-8-carboxylic acid (A-22)

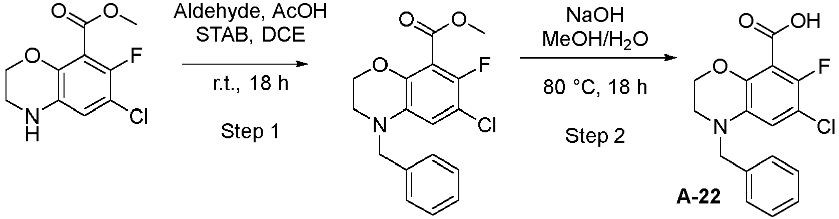

### Step 1: methyl 4-benzyl-6-chloro-7-fluoro-2,3-dihydro-1,4-benzoxazine-8-carboxylate

A solution of methyl 6-chloro-7-fluoro-3,4-dihydro-2H-1,4-benzoxazine-8-carboxylate (20 mg, 0.0814 mmol), benzaldehyde (0.017 mL, 0.163 mmol) and acetic acid (0.023 mL, 0.407 mmol) in DCE (0.81 mL) was stirred at room temperature, then after 10 minutes, sodium triacetoxyborohydride (86 mg, 0.407 mmol) was added and the suspension was stirred at room temperature for 18 h.

The reaction mixture was quenched with NaHCO₃ solution (2 mL) and the biphasic mixture was placed in a phase separator and washed with DCM (5 mL). The organics eluted were concentrated to dryness. The crude material was purified by column chromatography over silica (4 g cartridge) eluting with a gradient of EtOAc (0% to 40%; v/v) in hexane to afford the title compound (23 mg, 60%) as an orange oil.

¹H NMR (400 MHz, CHLOROFORM-D) δ 7.39 - 7.33 (m, 3H), 7.32 - 7.28 (m, 1H), 7.25 - 7.21 (m, 1H), 6.65 (dd, J = 7.1, 3.8 Hz, 1H), 4.71 (d, J = 3.9 Hz, 1H), 4.40 (s, 1H), 4.34 - 4.25 (m, 2H), 3.93 (d, J = 5.0 Hz, 3H), 3.37 - 3.33 (m, 1H), 3.29 (dd, J = 5.6, 4.1 Hz, 1H).

### Step 2: 4-Benzyl-6-chloro-7-fluoro-3,4-dihydro-2H-1,4-benzoxazine-8-carboxylic acid

To a solution of methyl 4-benzyl-6-chloro-7-fluoro-2,3-dihydro-1,4-benzoxazine-8-carboxylate (23 mg, 0.0685 mmol) in MeOH (0.2 mL) was added a solution of sodium hydroxide (5.5 mg, 0.137 mmol) in water (0.6 mL) and the reaction mixture was stirred at 80 °C for 72 h.

The reaction mixture was concentrated *in vacuo* and diluted with DCM (10 mL) and water (10 mL). 1 N HCl was added until the pH of the mixture reached 2. The aqueous layer was then extracted with EtOAc (2 x 10 mL). The organic layer was washed with brine (20 mL), dried over Na₂SO₄, filtered then concentrated *in vacuo.* The crude product was purified by prep-HPLC purification to afford the title compound (2.0 mg, 9%) as a colourless glass.

UPLC-MS analysis (6 min, basic): rt = 1.90 min, m/z = 322.1 [M+H]⁺, 97% purity.

¹H NMR (400 MHz, CHLOROFORM-D) δ 7.31 - 7.13 (m, 5H), 6.50 (s, 1H), 4.34 - 4.17 (m, 4H), 3.21 (s, 2H).

### Example 38: 7-Chloro-6-fluoro-4-[(o-tolyl)methyl]-3,4-dihydro-2H-1,4-benzoxazine-5-carboxylic acid (A-62)

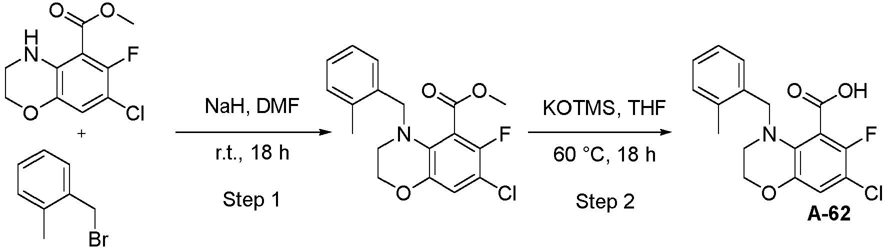

### Step 1: methyl 7-chloro-6-fluoro-4-(o-tolylmethyl)-2,3-dihydro-1,4-benzoxazine-5-carboxylate

To a -15 °C solution of methyl 7-chloro-6-fluoro-3,4-dihydro-2H-1,4-benzoxazine-5-carboxylate (55 mg, 0.219 mmol) in DMF (1.5 mL) was added sodium hydride, 60% in mineral oil (13 mg, 0.329 mmol) at -15 °C and stirred for 20 min. A solution of 1-(bromomethyl)-2-methylbenzene (65 mg, 0.351 mmol) in DMF (0.5 mL) was added to the stirred reaction mixture at -10 °C and kept stirring at the same temperature for 2 h. The reaction mixture was stirred at room temperature for 18 h.

The reaction mixture was quenched with water (10 mL) and extracted into EtOAc (2 x 15 mL). The combined organic layers were washed with water (2 x 10mL), brine (10 mL), dried over Na₂SO₄, and concentrated under reduced pressure. The crude material was purified by column chromatography over silica (12 g cartridge) eluting with a gradient of EtOAc (0% to 100% v/v) in i-Hexane to afford the title compound (52 mg, 64%) as a colourless gum.

¹H NMR (400 MHz, CHLOROFORM-D) δ 7.47 - 7.43 (m, 1H), 7.25 - 7.14 (m, 3H), 6.94 (d, J = 7.3 Hz, 1H), 4.23 (s, 2H), 4.16 - 4.05 (m, 2H), 3.46 (s, 3H), 3.25 - 3.03 (m, 2H), 2.20 (s, 3H).

### Step 2: 7-Chloro-6-fluoro-4-[(o-tolyl)methyl]-3,4-dihydro-2H-1,4-benzoxazine-5-carboxylic acid

To a solution of methyl 7-chloro-6-fluoro-4-(o-tolylmethyl)-2,3-dihydro-1,4-benzoxazine-5-carboxylate (45 mg, 0.122 mmol) in Et₂O (3 mL) was added potassium tert-butoxide (137 mg, 1.22 mmol) at 0 °C. To this slurry, water (10 µL) was added at 0 °C and the reaction mixture was stirred at room temperature for 18 h.

The reaction mixture was diluted with water (20 mL). The aqueous phase was washed with EtOAc (20 mL), acidified by 1 M HCl to pH ~ 2-3 and extracted with EtOAc. The organic phase was washed with saturated brine solution (20 mL), dried (Na₂SO₄), concentrated to dryness. The crude material was purified by prep-HPLC to afford the title compound (15 mg, 35%) as a white solid.

UPLC-MS analysis (4 min, basic): rt = 1.14 min, m/z = 336.0 [M+H]⁺, 95% purity.

¹H NMR (400 MHz, DMSO-D6) δ 7.51 (d, J = 6.2 Hz, 1H), 7.25 - 7.09 (m, 4H), 4.40 (s, 2H), 4.10 - 3.96 (m, 2H), 2.93 - 2.81 (m, 2H), 2.21 (s, 3H).

### Example 39: 6-Chloro-7-fluoro-4-[2-(4-fluorophenyl)ethyl]-3,4-dihydro-2H-1,4-benzoxazine-8-carboxylic acid (A-25)

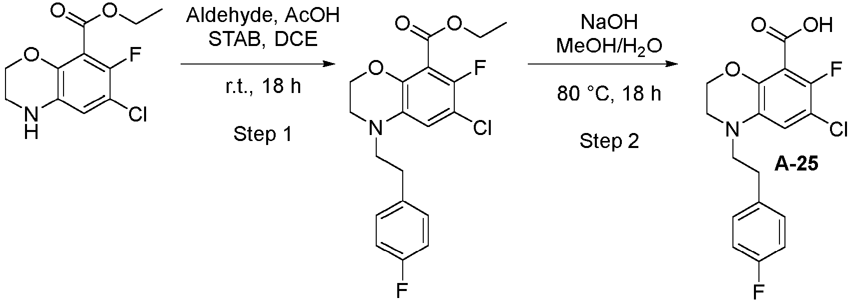

### Step 1: ethyl 6-chloro-7-fluoro-4-[2-(4-fluorophenyl)ethyl]-2,3-dihydro-1,4-benzoxazine-8-carboxylate

Ethyl 6-chloro-7-fluoro-3,4-dihydro-2H-1,4-benzoxazine-8-carboxylate (20 mg, 0.0770 mmol) was dissolved in DCE (1 mL). (4-fluorophenyl)acetaldehyde (21 mg, 0.154 mmol) and acetic acid (0.022 mL, 0.385 mmol) were added and the reaction mixture was stirred at room temperature for 10 minutes. Sodium triacetoxyborohydride (82 mg, 0.385 mmol) was added and the reaction mixture was stirred at room temperature for 24 hours. The reaction mixture was quenched with NaHCO₃ (5 mL) and extracted with DCM (2 x 5 mL). The organic phase was dried over Na₂SO₄, filtered then concentrated *in vacuo.* The crude material was purified by column chromatography over silica (4 g cartridge) eluting with a gradient of dichloromethane (10% to 100%; v/v) in iso-hexane. The residue was re-dissolved in DCE (1 mL) and (4-fluorophenyl)acetaldehyde (8.0 mg, 0.0579 mmol) and acetic acid (0.0088 mL, 0.154 mmol) were added and the reaction mixture was stirred at room temperature for 10 minutes. Sodium triacetoxyborohydride (33 mg, 0.154 mmol) was added and the reaction mixture was stirred at room temperature for 24 hours. The reaction mixture was quenched with NaHCO₃ (5 mL) and extracted with DCM (2 x 5 mL). The organic phase was dried over Na₂SO₄, filtered then concentrated *in vacuo.* The crude material was purified by column chromatography over silica (4 g cartridge) eluting with a gradient of EtOAc (1% to 30%; v/v) in iso-hexane. The fractions were concentrated *in vacuo* to afford the title compound (13 mg, 38%) as a colourless oi.

¹H NMR (400 MHz, CDCl₃) δ 7.18 - 7.12 (m, 2H), 7.00 (td, J = 8.7, 0.8 Hz, 2H), 6.60 (d, J = 7.1 Hz, 1H), 4.45 - 4.36 (m, 2H), 4.20 - 4.11 (m, 2H), 3.44 (t, J = 7.3 Hz, 2H), 3.23 - 3.14 (m, 2H), 2.83 (t, J = 7.1 Hz, 2H), 1.37 (t, J = 7.1 Hz, 3H).

### Step 2: 6-Chloro-7-fluoro-4-[2-(4-fluorophenyl)ethyl]-3,4-dihydro-2H-1,4-benzoxazine-8-carboxylic acid

Ethyl 6-chloro-7-fluoro-4-[2-(4-fluorophenyl)ethyl]-2,3-dihydro-1,4-benzoxazine-8-carboxylate (13 mg, 0.0340 mmol) was dissolved in THF (1 mL). Potassium trimethylsilanolate (0.043 mL, 0.0857 mmol) was added and the reaction mixture was stirred at room temperature for 24 hours.

The reaction mixture was quenched with water (5 mL) and extracted with EtOAc (2 x 10 mL). The aqueous layer was then acidified until the pH reached 2. The aqueous layer was then extracted with EtOAc (2 x 10 mL) and dichloromethane (10 mL). The organic layers were combined, dried over Na₂SO₄, filtered and concentrated *in vacuo.* The crude mixture was purified via prep-HPLC to afford the title compound (1.8 mg, 15%) as a yellow oil.

UPLC-LCMS analysis (4 min, Basic): rt = 1.30 min, m/z = 354.1 [M+H]⁺, 100% purity. ¹H NMR (400 MHz, CD₃OD) δ 7.29 - 7.19 (m, 2H), 7.07 - 6.94 (m, 2H), 6.65 - 6.54 (m, 1H), 4.18 - 4.09 (m, 2H), 3.50 (t, J = 7.1 Hz, 2H), 3.28 - 3.20 (m, 2H), 2.86 (t, J = 7.1 Hz, 2H).

### Example 40: 6-Chloro-7-fluoro-4-propyl-3,4-dihydro-2H-1,4-benzoxazine-8-carboxylic acid (A-21)

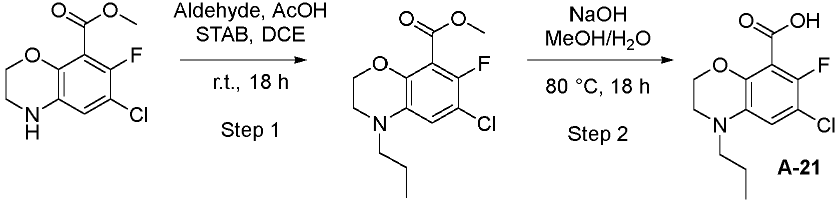

### Step 1: methyl 6-chloro-7-fluoro-4-propyl-2,3-dihydro-1,4-benzoxazine-8-carboxylate

A mixture of methyl 6-chloro-7-fluoro-3,4-dihydro-2H-1,4-benzoxazine-8-carboxylate (20 mg, 0.0814 mmol), propionaldehyde (0.012 mL, 0.163 mmol) and acetic acid (0.023 mL, 0.407 mmol) in DCE (1 mL) was stirred at room temperature for 10 minutes before the addition of sodium triacetoxyborohydride (86 mg, 0.407 mmol). The resulting mixture was stirred at room temperature for 18 h.

The reaction mixture was quenched with NaHCO₃ solution (2 mL) and the biphasic mixture was placed in a phase separator and washed with DCM (5 mL). The organics eluted were concentrated to dryness to afford the title compound (23 mg, 81%) as a yellow oil.

¹H NMR (400 MHz, CHLOROFORM-D) δ 6.61 (d, J = 7.1 Hz, 1H), 4.27 - 4.22 (m, 2H), 3.35 - 3.30 (m, 2H), 3.19 - 3.09 (m, 2H), 1.61 - 1.56 (m, 2H), 1.25 (s, 3H), 0.95 (t, J = 7.4 Hz, 3H).

### Step 2: 6-Chloro-7-fluoro-4-propyl-3,4-dihydro-2H-1,4-benzoxazine-8-carboxylic acid

To a solution of methyl 6-chloro-7-fluoro-4-propyl-2,3-dihydro-1,4-benzoxazine-8-carboxylate (23 mg, 0.0799 mmol) in MeOH (0.2 mL) was added a solution of sodium hydroxide (8.0 mg, 0.200 mmol) in Water (0.6 mL). The reaction mixture was stirred at 80 °C for 18 h.

The reaction mixture was concentrated *in vacuo* and diluted with DCM (10 mL) and water (10 mL). 1 N HCl was added until the pH of the mixture reached 2. The aqueous layer was then extracted with EtOAc (2 x 10 mL). The organic layer was washed with brine (20 mL), dried over Na₂SO₄, filtered then concentrated *in vacuo.* The crude product was purified by prep-HPLC to afford the title compound (6.5 mg, 30%) as an off-white solid.

UPLC-MS analysis (6 min, basic): rt = 1.56 min, m/z = 274.1 [M+H]⁺, 100% purity.

¹H NMR (400 MHz, CHLOROFORM-D) δ 6.63 (d, J = 7.0 Hz, 1H), 4.29 (s, 2H), 3.31 (s, 2H), 3.14 (t, J = 7.6 Hz, 2H), 1.59 (q, J = 7.5 Hz, 2H), 0.95 (t, J = 7.4 Hz, 3H).

### Example 41: 6-Chloro-7-fluoro-4-(3-methoxypropyl)-3,4-dihydro-2H-1,4-benzoxazine-8-carboxylic acid (A-27)

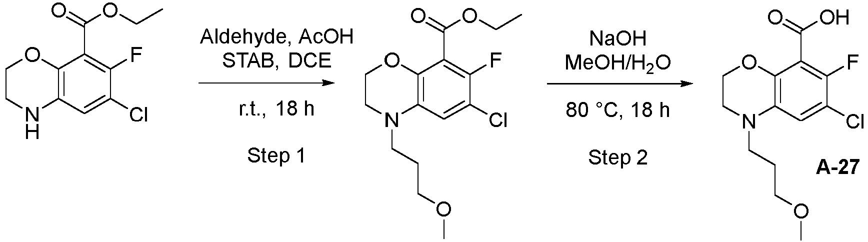

### Step 1: ethyl 6-chloro-7-fluoro-4-(3-methoxypropyl)-2,3-dihydro-1,4-benzoxazine-8-carboxylate

Ethyl 6-chloro-7-fluoro-3,4-dihydro-2H-1,4-benzoxazine-8-carboxylate (50 mg, 0.193 mmol) was dissolved in DCE (2 mL). 3-methoxypropanal (34 mg, 0.385 mmol) and

acetic acid (0.055 mL, 0.963 mmol) were added and the reaction mixture was stirred at room temperature for 10 minutes. Sodium triacetoxyborohydride (204 mg, 0.963 mmol) was added and the reaction mixture was stirred at room temperature for 24 hours, then 50 °C for 1 hour.

The mixture was quenched with NaHCO₃ (8 mL) and extracted with DCM (2 x 10 mL). The organic layers were combined, washed with brine (2 x 10 mL), dried over Na₂SO₄, filtered then concentrated *in vacuo.* The crude material was purified by column chromatography over silica (4 g cartridge) eluting with a gradient of DCM (0% to 100%; v/v) in iso-hexane, followed by MeOH (0% to 29%; v/v) in DCM. The fractions were concentrated *in vacuo* and re-purified by column chromatography over silica (4 g cartridge) eluting with a gradient of EtOAc (10% to 60%; v/v) in iso-hexane to afford the title compound (21 mg, 28%) as a yellow oil.

¹H NMR (400 MHz, CDCl₃) δ 6.70 (d, J = 7.1 Hz, 1H), 4.39 (q, J = 7.1 Hz, 2H), 4.28 - 4.21 (m, 2H), 3.40 (t, J = 5.8 Hz, 2H), 3.36 - 3.27 (m, 7H), 1.86 - 1.73 (m, 2H), 1.37 (t, J = 7.1 Hz, 3H).

### Step 2: 6-Chloro-7-fluoro-4-(3-methoxypropyl)-3,4-dihydro-2H-1,4-benzoxazine-8-carboxylic acid

Ethyl 6-chloro-7-fluoro-4-(3-methoxypropyl)-2,3-dihydro-1,4-benzoxazine-8-carboxylate (21 mg, 0.0633 mmol) was dissolved in THF (1 mL). Potassium trimethylsilanolate (0.079 mL, 0.158 mmol) was added dropwise and the reaction mixture was stirred at room temperature for 72 hours.

The reaction mixture was concentrated *in vacuo* and the residue was taken up in water (5 mL) and EtOAc (5 mL). The aqueous layer was then extracted with EtOAc (2 x 10 mL). 1 N HCl was added to the aqueous layer until the pH reached 1. The aqueous layer was then extracted with EtOAc (2 x 10 mL), dried over Na₂SO₄, filtered and concentrated *in vacuo* to afford the title compound (18 mg, 91%) as a brown solid. UPLC-LCMS analysis (4 min, Acidic): rt = 1.59 min, m/z = 304.0 [M+H]⁺, 98% purity. ¹H NMR (400 MHz, CDCl₃) δ 6.77 (d, J = 7.2 Hz, 1H), 4.43 - 4.25 (m, 2H), 3.43 (t, J = 5.9 Hz, 2H), 3.39 - 3.28 (m, 7H), 1.89 - 1.77 (m, 2H).

### Example 42: 6-chloro-7-fluoro-4-[3-(4-fluorophenyl)propyl]-3,4-dihydro-2H-1,4-benzoxazine-8-carboxylic acid (A-23)

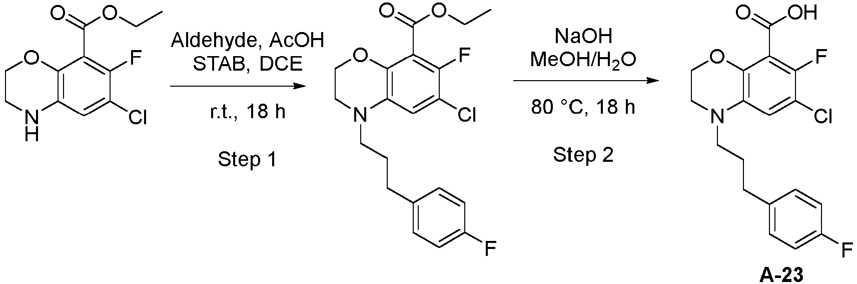

### Step 1: ethyl 6-chloro-7-fluoro-4-[3-(4-fluorophenyl)propyl]-2,3-dihydro-1,4-benzoxazine-8-carboxylate

Ethyl 6-chloro-7-fluoro-3,4-dihydro-2H-1,4-benzoxazine-8-carboxylate (20 mg, 0.0770 mmol) was dissolved in DCE (1 mL). 3-(4-fluorophenyl)propanal (23 mg, 0.154 mmol) and acetic acid (23 mg, 0.385 mmol) were added and the reaction mixture was stirred at room temperature for 10 minutes. Sodium triacetoxyborohydride (82 mg, 0.385 mmol) was added and the reaction mixture was stirred at room temperature for 24 hours.

The reaction mixture was quenched with NaHCO₃ (2 mL) and extracted with DCM (2 x 5 mL). The organic phase was dried over Na₂SO₄, filtered then concentrated *in vacuo.* The crude material was purified by column chromatography over silica (4 g cartridge) eluting with a gradient of dichloromethane (10% to 100%; v/v) in iso-hexane to afford the title compound (21 mg, 65) as a red oil.

¹H NMR (400 MHz, CDCl₃) δ 7.18 - 7.10 (m, 2H), 7.04 - 6.93 (m, 2H), 6.45 (dd, J = 7.1, 0.5 Hz, 1H), 4.39 (q, J = 7.1 Hz, 2H), 4.26 - 4.22 (m, 2H), 3.32 - 3.23 (m, 2H), 3.22 - 3.13 (m, 2H), 2.64 (t, J = 7.6 Hz, 2H), 1.94 - 1.79 (m, 2H), 1.37 (t, J = 7.1 Hz, 3H).

### Step 2: 6-Chloro-7-fluoro-4-[3-(4-fluorophenyl)propyl]-3,4-dihydro-2H-1,4-benzoxazine-8-carboxylic acid

Ethyl 6-chloro-7-fluoro-4-[3-(4-fluorophenyl)propyl]-2,3-dihydro-1,4-benzoxazine-8-carboxylate (21 mg, 0.0531 mmol) was dissolved in MeOH (0.6 mL). Sodium hydroxide (2.5 mg, 0.0637 mmol) in water (0.3 mL) was added and the reaction mixture was stirred at room temperature for 24 hours.

Sodium hydroxide (2.5 mg, 0.0637 mmol) in water (0.3 mL) was added and the reaction mixture was stirred at 60 °C for 24 hours, then 75 °C for 4 days.

The reaction mixture was concentrated *in vacuo* and dissolved in EtOAc (5 mL) and water (5 mL). The aqueous layer was acidified to a pH of 2 by 1 N HCl and extracted with EtOAc (2 x 10 mL). The organic layers were combined, dried over Na₂SO₄, filtered then concentrated *in vacuo.* The residue was purified by prep-HPLC to afford the title compound as a white solid.

UPLC-LCMS analysis (6 min, Basic): rt = 2.36 min, m/z = 368.0 [M+H]⁺, 98% purity.

¹H NMR (400 MHz, d-DMSO) δ 7.33 - 7.20 (m, 2H), 7.15 - 7.02 (m, 2H), 6.63 (d, J = 7.0 Hz, 1H), 4.25 - 4.11 (m, 2H), 3.30 - 3.21 (m, 4H), 2.61 (t, J = 7.6 Hz, 2H), 1.78 (p, J = 7.6 Hz, 2H).

### Example 43: 6-Chloro-7-fluoro-4-(2-methoxyethyl)-3,4-dihydro-2H-1,4-benzoxazine-8-carboxylic acid (A-38)

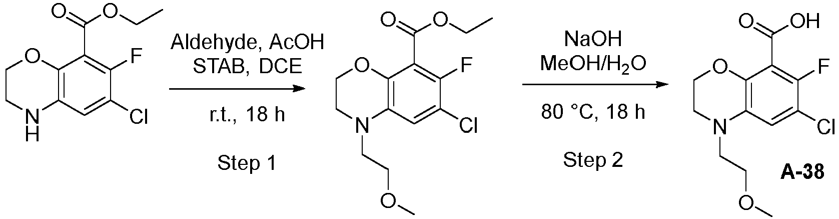

### Step 1: ethyl 7-chloro-6-fluoro-4-(2-methoxyethyl)-2,3-dihydro-1,4-benzoxazine-8-carboxylate

Ethyl 7-chloro-6-fluoro-3,4-dihydro-2H-1,4-benzoxazine-8-carboxylate (39 mg, 0.150 mmol) was dissolved in DCE (1 mL). 2-methoxyacetaldehyde (22 mg, 0.300 mmol) and acetic acid (0.043 mL, 0.751 mmol) were added and the reaction mixture was stirred at room temperature for 15 minutes. Sodium triacetoxyborohydride (159 mg, 0.751 mmol) was added and the reaction mixture was stirred at room temperature for 24 hours, then heated to 45 °C 1 hour.

The reaction mixture was quenched with NaHCO₃ (10 mL) and extracted with DCM (2 x 10 mL). The organic layers were combined, dried over Na₂SO₄, filtered then concentrated *in vacuo.* The crude material was purified by column chromatography over silica (4 g cartridge) eluting with a gradient of EtOAc (0% to 60%; v/v) in iso-hexane to afford the title compound (18 mg, 36%) as a yellow oil.

¹H NMR (400 MHz, CDCl₃) δ 6.64 (dd, J = 7.1, 0.6 Hz, 1H), 4.39 (qd, J = 7.1, 1.0 Hz, 2H), 4.26 - 4.20 (m, 2H), 3.55 (td, J = 5.4, 0.8 Hz, 2H), 3.44 - 3.37 (m, 4H), 3.37 - 3.32 (m, 3H), 1.40 - 1.32 (m, 3H).

### Step 2: 6-Chloro-7-fluoro-4-(2-methoxyethyl)-3,4-dihydro-2H-1,4-benzoxazine-8-carboxylic acid

Ethyl 7-chloro-6-fluoro-4-(2-methoxyethyl)-2,3-dihydro-1,4-benzoxazine-8-carboxylate (18 mg, 0.0567 mmol) was dissolved in THF (1 mL). Potassium trimethylsilanolate (0.071 mL, 0.142 mmol) was added and the reaction mixture was stirred at 45 °C for 24 hours.

The residue was dissolved in water (5 mL) and EtOAc (5 mL). The aqueous phase was washed with EtOAc (2 x 5 mL) then it was acidified with 1 N HCl until the pH reached 1. The mixture was then extracted with EtOAc (10 mL) and DCM (10 mL). The organic layers were dried over Na₂SO₄, filtered then concentrated *in vacuo* to afford the title compound (7.9 mg, 48%) as a brown solid.

UPLC-LCMS analysis (2 min, Basic): rt = 1.52 min, m/z = 290.1 [M+H]⁺, 99% purity.

¹H NMR (400 MHz, CDCl₃) δ 6.75 (d, J = 7.0 Hz, 1H), 4.39 - 4.29 (m, 2H), 3.57 (t, J = 5.4 Hz, 2H), 3.52 - 3.40 (m, 4H), 3.36 (s, 3H).

### Example 44: 6-Chloro-4-(cyclobutylmethyl)-7-fluoro-3,4-dihydro-2H-1,4-benzoxazine-8-carboxylic acid (A-46)

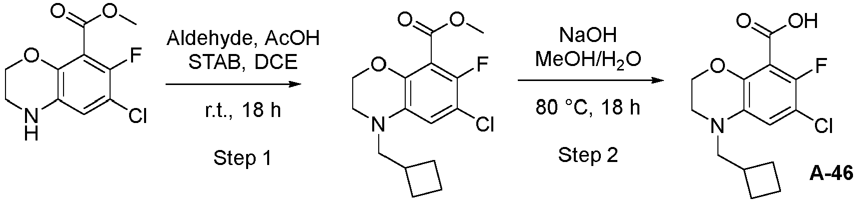

### Step 1: methyl 6-chloro-4-(cyclobutylmethyl)-7-fluoro-2,3-dihydro-1,4-benzoxazine-8-carboxylate

A mixture of methyl 6-chloro-7-fluoro-3,4-dihydro-2H-1,4-benzoxazine-8-carboxylate (20 mg, 0.0814 mmol), cyclobutanecarboxaldehyde (0.012 mL, 0.163 mmol) and acetic acid (0.023 mL, 0.407 mmol) in DCE (1 mL) was stirred at room temperature for 10 minutes before the addition of sodium triacetoxyborohydride (86 mg, 0.407 mmol). The resulting mixture was stirred at room temperature for 18 h.

The reaction mixture was quenched with NaHCO₃ sat. solution (5 mL) for 1 hour then extracted twice with DCM (2 x 5 mL). The organics were washed with brine (10 mL), dried over Na₂SO₄ and concentrated to dryness. The crude mixture was purified by prep-HPLC to afford the title compound (8.0 mg, 31%) as a pale brown oil.

¹H NMR (400 MHz, CHLOROFORM-D) δ 6.60 (dd, J = 7.2, 1.6 Hz, 1H), 4.24 (ddd, J = 5.6, 4.1, 1.7 Hz, 2H), 3.92 (d, J = 1.6 Hz, 3H), 3.34 - 3.27 (m, 2H), 3.19 (dd, J = 7.0, 1.6 Hz, 2H), 2.66 (hept, J = 7.7 Hz, 1H), 2.13 - 2.02 (m, 2H), 1.98 - 1.81 (m, 2H), 1.80 - 1.66 (m, 2H).

### Step 2: 6-Chloro-4-(cyclobutylmethyl)-7-fluoro-3,4-dihydro-2H-1,4-benzoxazine-8-carboxylic acid

To a solution of methyl 6-chloro-4-(cyclobutylmethyl)-7-fluoro-2,3-dihydro-1,4-benzoxazine-8-carboxylate (8.0 mg, 0.0255 mmol) in THF (1 mL) was added potassium trimethylsilanolate (0.013 mL, 0.0255 mmol) and the resulting mixture was stirred at room temperature for 96 h.

The reaction mixture was concentrated to dryness, partitioned between water (10 mL) and EtOAc (10 mL), the aqueous was extracted again with EtOAc (15 mL), then the organics were dried over Na₂SO₄ and concentrated to dryness to afford the title compound (12 mg, 155%) as a brown solid.

UPLC-MS analysis (2 min, basic): rt = 0.82 min, m/z = 298.0-300.0 [M+H]⁺, 99% purity. ¹H NMR (400 MHz, CHLOROFORM-D) δ 6.70 (d, J = 6.9 Hz, 1H), 4.34 (dd, J = 4.8, 4.0 Hz, 2H), 3.40 - 3.33 (m, 2H), 3.22 (d, J = 7.0 Hz, 2H), 2.68 (dt, J = 15.3, 7.6 Hz, 1H), 2.15 - 2.05 (m, 2H), 2.01 - 1.83 (m, 2H), 1.83 - 1.72 (m, 2H).

### Example 45: 7-Chloro-6-fluoro-4-(3-methylbutyl)-3,4-dihydro-2H-1,4-benzoxazine-5-carboxylic acid (A-51)

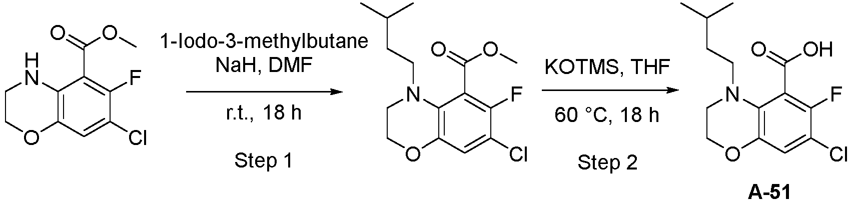

### Step 1: Methyl 7-chloro-6-fluoro-4-isopentyl-2,3-dihydro-1,4-benzoxazine-5-carboxylate

To a solution of methyl 7-chloro-6-fluoro-3,4-dihydro-2H-1,4-benzoxazine-5-carboxylate (35 mg, 0.142 mmol) and sodium hydride, 60% in mineral oil (14 mg, 0.356 mmol) in NMP (1.42 mL) was added 1-iodo-3-methylbutane (0.028 mL, 0.214 mmol) and the resulting mixture was stirred at room temperature for 4 hours, then at 60 °C for 48 hours.

The reaction mixture was quenched with 1M HCl solution (10 mL) and extracted twice with EtOAc (2x10 mL). The organics were washed with water (2 x 10 mL) and brine (15 mL), dried over Na₂SO₄ and concentrated to dryness. The crude material was purified by column chromatography over silica (4 g cartridge) eluting with a gradient of EtOAc (0% to 50%; v/v) in hexane to afford the title compound (9.0 mg, 13%) as a brown oil. UPLC-MS analysis (2 min, basic): rt = 1.34 min, m/z = 316.0 [M+H]⁺, 66% purity.

No NMR data was reported

### Step 2: 7-Chloro-6-fluoro-4-(3-methylbutyl)-3,4-dihydro-2H-1,4-benzoxazine-5-carboxylic acid

A suspension of methyl 7-chloro-6-fluoro-4-isopentyl-2,3-dihydro-1,4-benzoxazine-5-carboxylate (9.0 mg, 0.0285 mmol) and sodium hydroxide (2.3 mg, 0.0570 mmol) in water (0.285 mL) and MeOH (0.285 mL) was stirred at 80 °C for 18 h.

The reaction mixture was cooled and concentrated to dryness. The crude material was purified by column chromatography over C18 (4 g cartridge) eluting with a gradient of MeCN (0.1% NH₃) (5% to 95%; v/v) in water (0.1% NH₃) to afford the title compound (4.0 mg, 44%) as an off-white solid.

UPLC-MS analysis (4 min, basic): rt = 1.10 min, m/z = 302.0 [M+H]⁺, 94% purity.

¹H NMR (400 MHz, DMSO-*D*₆) δ 6.57 (d, J = 6.1 Hz, 1H), 3.97 (q, J = 4.1 Hz, 2H), 3.24 (td, J = 8.3, 3.9 Hz, 2H), 3.05 (q, J = 4.1 Hz, 2H), 1.53 - 1.45 (m, 1H), 1.41 (dq, J = 8.2, 3.7 Hz, 2H), 0.84 (dd, J = 6.6, 3.6 Hz, 6H).

### Example 46: 7-Chloro-6-fluoro-4-[(thiophen-3-yl)methyl]-3,4-dihydro-2H-1,4-benzoxazine-5-carboxylic acid (A-54)

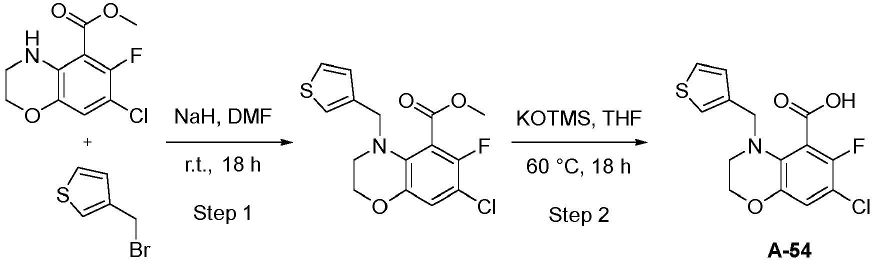

### Step 1: Methyl 7-chloro-6-fluoro-4-(3-thienylmethyl)-2,3-dihydro-1,4-benzoxazine-5-carboxylate

Methyl 7-chloro-6-fluoro-3,4-dihydro-2H-1,4-benzoxazine-5-carboxylate (70 mg, 0.285 mmol) was dissolved in NMP (1.5 mL). 3-(bromomethyl)thiophene (76 mg, 0.427 mmol) and sodium hydride, 60% in mineral oil (17 mg, 0.428 mmol) were added and the reaction mixture was stirred at 80 °C for 2 hours.

The reaction mixture was quenched with NH₄Cl (10 mL) and extracted with EtOAc (10 mL) and DCM (10 mL). The organic layers were combined and dried over Na₂SO₄, filtered then concentrated *in vacuo.* The crude material was purified by column chromatography over silica (12 g cartridge) eluting with a gradient of dichloromethane (20% to 40%; v/v) in iso-hexane to afford the title compound (24.5 mg, 9%) as a yellow oil.

¹H NMR (400 MHz, CHLOROFORM-*D*) δ 7.32 (dd, J = 4.9, 3.0 Hz, 1H), 7.19 (dq, J = 3.0, 1.0 Hz, 1H), 7.03 (dd, J = 4.9, 1.3 Hz, 1H), 6.94 (dd, J = 7.3, 5.1 Hz, 1H), 4.18 (d, J = 1.2 Hz, 2H), 4.07 - 4.02 (m, 2H), 3.79 (s, 3H), 3.11 - 3.08 (m, 2H).

### Step 2: 7-Chloro-6-fluoro-4-[(thiophen-3-yl)methyl]-3,4-dihydro-2H-1,4-benzoxazine-5-carboxylic acid

Methyl 7-chloro-6-fluoro-4-(3-thienylmethyl)-2,3-dihydro-1,4-benzoxazine-5-carboxylate (24.5 mg, 0.0252 mmol) was dissolved in MeOH (1 mL) and water (1 mL). Sodium hydroxide (1.2 mg, 0.0305 mmol) was added and the reaction mixture was stirred at 40 °C for 18 hours. Sodium hydroxide (1.2 mg, 0.0305 mmol) was added then the reaction mixture was stirred at 40 °C for 72 hours. Sodium hydroxide (1.2 mg, 0.0305 mmol) was added and the reaction mixture was stirred at 50 °C for 2.5 hours.

The reaction mixture was concentrated in vacuo. The mixture was dissolved in water (5 mL) and extracted with EtOAc (2 x 5 mL). The aqueous layer was then acidified until the pH reached 1. The mixture was extracted with EtOAc (2 x 10 mL) and DCM (2 x 10 mL). The organic layers were combined, dried over Na₂SO₄, filtered then concentrated *in vacuo.* The residue was purified by prep-HPLC to afford the title compound (1.7 mg, 20%) as a colourless oil.

UPLC-LCMS analysis (4 min, Acidic): rt = 1.89 min, m/z = 328.0 [M+H]⁺, 96% purity. ¹H NMR (400 MHz, DMSO-*d*₆) δ 8.26 (bs, 1H), 7.43 - 7.34 (m, 2H), 7.26 (dd, J = 4.6, 1.5 Hz, 1H), 6.62 (d, J = 7.3 Hz, 1H), 4.34 (s, 2H), 3.88 (dd, J = 5.0, 3.6 Hz, 2H), 2.82 (dd, J = 4.9, 3.7 Hz, 2H).

### Example 47: 7-Chloro-8-fluoro-2,3,4,5-tetrahydro-1,5-benzoxazepine-9-carboxylic acid (A-15)

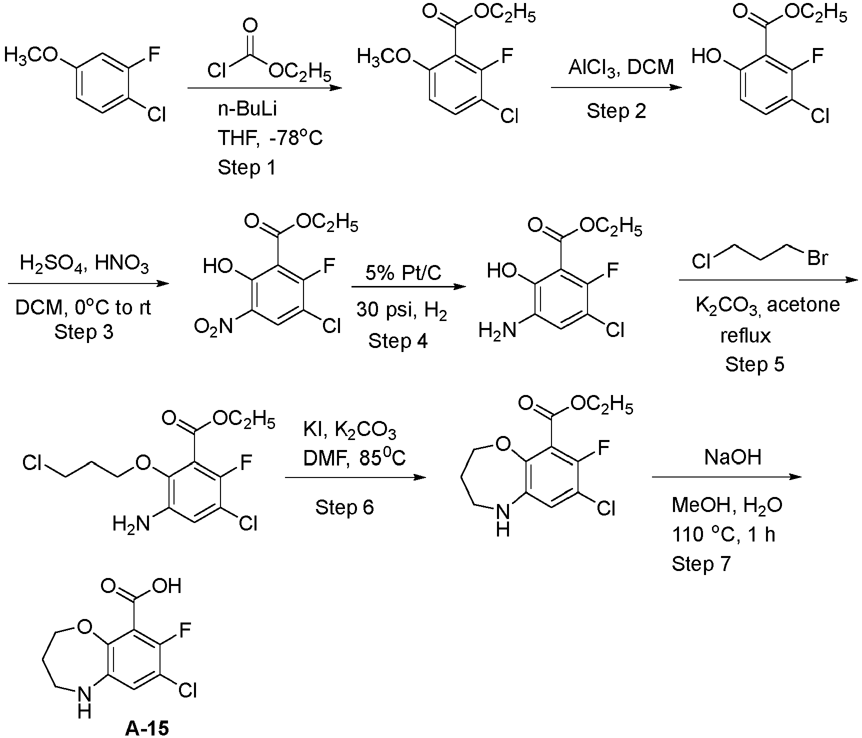

### Step 1: Ethyl 3-chloro-2-fluoro-6-methoxybenzoate

n-BuLi (2.5 M in cyclohexane) (13.2 mL, 33.0 mmol) was added to a solution of 1-chloro-2-fluoro-4-methoxybenzene (5.0 g, 31.1 mmol) in THF (50 mL) at -78°C. The reaction mixture was stirred at that temperature for 1h. and ethyl chloroformate (3.14 mL, 33.0 mmol) in THF (20 mL) was introduced. The reaction mixture was stirred at - 78°C for a further 1 h., slowly brought to ambient temperature and quenched with saturated aqueous NH₄Cl (20 mL) before being extracted into EtOAc (2 x 75 mL). The combined organic layers were dried (Na₂SO₄) and concentrated. The resultant residue was purified by column chromatography on silica gel eluting with hexane/EtOAc (0 - 30%) to give the title compound (4.2 g, 58%) as an oil.

¹H NMR (300 MHz, CDCl₃) δ 7.25 (t, 1H), 6.57 (d, 1H), 4.29 (q, 2H), 3.73 (s, 3H), 1.26 (t, 3H).

¹⁹F NMR (300 MHz, CDCl₃) δ -114.99 ppm.

### Step 2: Ethyl 3-chloro-2-fluoro-6-hydroxybenzoate

Aluminium chloride (7.7 g, 58.0 mmol) was added to a solution of ethyl 3-chloro-2-fluoro-6-methoxybenzoate (4.2 g, 18.0 mmol) in dry CH₂Cl₂ (50 mL). The reaction mixture was stirred at ambient temperature for 16 h, quenched with 1N HCl solution (20 mL) and stirred at ambient temperature for 30 min. The organic layer was separated and the aqueous layer extracted into EtOAc (2 x 50 mL). The combined organic extracts were dried (Na₂SO₄) and evaporated to a residue which was purified by column chromatography on silica gel eluting with hexane/EtOAc (0-10%) to obtain the title compound (3.6 g, 92%) as an oil.

¹H NMR (300 MHz, CDCl₃) δ 11.12 (s, 1H), 7.26 (t, 1H), 6.60 (d, 1H), 4.30 (q, 2H), 1.27 (t, 3H).

¹⁹F NMR (300 MHz, CDCl₃) δ -105.06 ppm.

### Step 3: Ethyl 3-chloro-2-fluoro-6-hydroxy-5-nitrobenzoate

A premixed solution of nitric acid (70%) (1.91 mL, 29.6 mmol), and sulphuric acid (1.58 mL, 29.6 mmol) was added dropwise at 0°C to a solution of ethyl 3-chloro-2-fluoro-6-hydroxybenzoate (3.60 g, 16. 5 mmol) in CH₂Cl₂ (6 mL). The reaction mixture was stirred at ambient temperature for 5 h. The solution was poured into a mixture of crushed ice and H₂O, extracted into EtOAc (3 x 40 mL) after melting then washed with H₂O (3 x 20 mL) and brine (20 mL). The combined extracts were dried (Na₂SO₄) and concentrated *in vacuo* to obtain the title compound (4.2 g, 97%) as an oil, which was utilised directly in the next step.

¹H NMR (300 MHz, CD₃OD) δ 8.42 (d, 1H), 4.48 (q, 2H), 1.42 (t, 3H).

¹⁹F NMR (300 MHz, CD₃OD) δ -103.89 ppm.

### Step 4: Ethyl 3-amino-5-chloro-6-fluoro-2-hydroxybenzoate

5 Wt.% Pt/C (0.42 g, 10%, w/w) was added to a solution of ethyl 3-chloro-2-fluoro-6-hydroxy-5-nitrobenzoate (4.2 g, 15.9 mmol) in EtOAc (60 mL) and the suspension stirred at ambient temperature for 16 h under an atmosphere of H₂ at 30 psi for 16 h. The mixture was filtered through a Celite pad, washed with CH₃OH/EtOAc (20: 80, v/v) and the filtrate concentrated under reduced pressure to yield the title compound (3.7 g) as an oil, which was utilised directly in the next step.

¹H NMR (300 MHz, CDCl₃) δ 11.40 (s, 1H), 6.85 (d, 1H), 4.44 (q, 2H), 4.22 - 3.14 (br s, 1H), 1.42 (t, 3H).

¹⁹F NMR (300 MHz, CDCl₃) δ -120.83 ppm.

### Step 5: Ethyl 3-amino-5-chloro-2-(3-chloropropoxy)-6-fluorobenzoate

K₂CO₃ (2.96 g, 21.4 mmol), and 1-bromo-3-chloropropane (0.404 g, 2.57 mmol) were added to a solution of ethyl 3-amino-5-chloro-6-fluoro-2-hydroxybenzoate (0.5 g, 2.14 mmol) in acetone (30 mL). The reaction mixture was heated at reflux for 16 h in a sealed tube, cooled and extracted into EtOAc (2 x 30 mL), evaporated and the resultant residue purified by column chromatography on silica gel eluting with hexane/EtOAc (0-50%) to give the title compound (0.45 g, 68%) as a yellow liquid.

¹H NMR (300 MHz, CDCl₃) δ 6.81 (d, 1H), 4.41 (q, 2H), 4.10 (t, 2H), 4.02 - 3.81 (br s, 2H), 3.76 (t, 2H), 2.25 - 2.10 (m, 2H), 1.38 (t, 3H).

¹⁹F NMR (300 MHz, CDCl₃) δ -128.43 ppm.

### Step 6: Ethyl 7-chloro-8-fluoro-2,3,4,5-tetrahydrobenzo[b][1,4]oxazepine-9-carboxylate

Solid K₂CO₃ (0.392 g, 2.84 mmol) and KI (0.471 g, 2.84 mmol) were introduced to a solution of ethyl 3-amino-5-chloro-2-(3-chloropropoxy)-6-fluorobenzoate (0.44 g, 1.42 mmol) in DMF (6 mL). The reaction mixture was heated at 85°C for 16 h in a sealed tube, cooled and evaporated. The resultant residue was purified by column chromatography on silica gel eluting with hexane/ EtOAc (10 - 50%) to obtain the title compound (0.38 g, 98%) as an oil.

¹H NMR (300 MHz, CDCl₃) δ 6.80 (d, 1H), 4.40 (q, 2H), 4.10 (t, 2H), 3.21 (t, 2H), 2.07 - 1.95 (m, 2H), 1.37 (t, 3H).

¹⁹F NMR (300 MHz, CDCl₃) δ -127.16 ppm.

### Step 7: 7-Chloro-8-fluoro-2,3,4,5-tetrahydro-1,5-benzoxazepine-9-carboxylic acid

NaOH (0.088 g, 2.19 mmol) was added to a solution of ethyl 7-chloro-8-fluoro-2,3,4,5-tetrahydrobenzo[b][1,4]oxazepine-9-carboxylate (0.06 g, 0.219 mmol) in MeOH/H₂O (3/1 mL). The reaction mixture was heated under microwave conditions at 110°C for 1 h., cooled and evaporated. The resultant residue was purified by semi-prep HPLC eluting with 30 - 100% CH3CN/0.1% formic acid in H₂O to obtain title compound (25 mg, 46%) as a white solid.

¹H NMR (300 MHz, CD₃OD) δ 6.98 (d, 1H), 4.08 (t, 2H), 3.15 (t, 2H), 2.87 (s, 3H), 2.06 - 1.95(m, 2H).

¹⁹F NMR (300 MHz, CDCl₃) δ -131.15 ppm.

ES- MS: *m*/*z* 244.6 (M-H).

HPLC: Retention time 5.93 min., Purity: 95.1% @ 254 nm.

### Example 48: 7-Methoxy-4,6-dimethyl-3,4-dihydro-2H-1,4-benzoxazine-8-carboxylic acid (A-31)

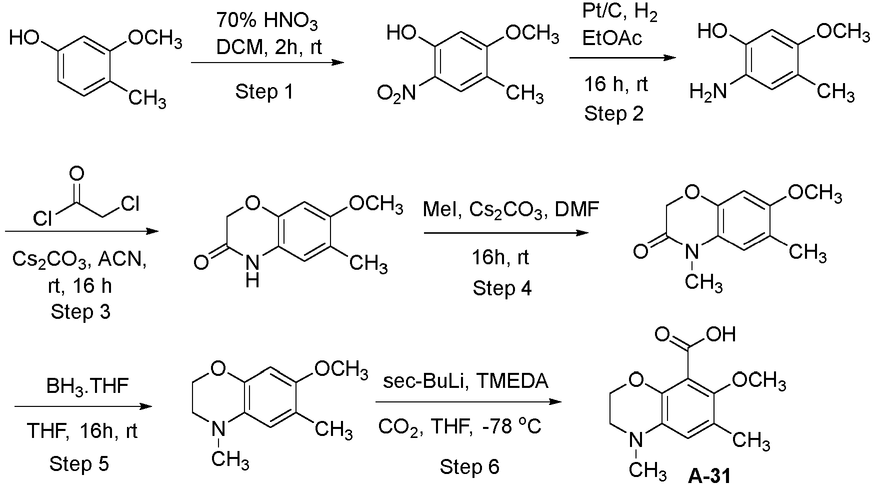

### Step 1: 5-Methoxy-4-methyl-2-nitrophenol

70% Nitric acid (1.3 mL, 20.71 mmol) was added to a solution of 3-methoxy-4-methylphenol (2.6 g, 18.8 mmol) in CH₂Cl₂ (20 mL) at 0°C and the mixture stirred at ambient temperature for 2 h. The reaction mixture was cooled to 0°C, quenched with H₂O (10 mL) and extracted into EtOAc (2 x 25 mL). The combined extracts were dried (Na₂SO₄), concentrated and the residue purified by column chromatography on silica gel eluting with hexane/ EtOAc (0 - 20%) to give the title compound (600 mg, 17%).

¹H NMR (300 MHz, CDCl₃) δ 11.02 (s, 1H), 7.85 (s, 1H), 6.47 (s, 1H), 3.91 (s, 3H), 2.15 (s, 3H).

### Step 2: 2-Amino-5-methoxy-4-methylphenol

5% Pt/C (16.4 mg) was added to a solution of 5-methoxy-4-methyl-2-nitrophenol (600 mg, 3.28 mmol) in EtOAc (12 mL), the reaction mixture stirred under a hydrogen atmosphere at 20 psi for 16 h, filtered through a celite pad which was washed with EtOAc (20 mL). After concentration under reduced pressure to the title compound (490 mg, 98%) was obtained as a dark brown solid.

¹H NMR (300 MHz, DMSO-d₆) δ 8.70 (br s, 1H), 6.38 (s, 1H), 6.33 (s, 1H), 4.01 (br s, 2H), 3.60 (s, 3H), 1.96 (s, 3H).

### Step 3: 7-Methoxy-6-methyl-2H-benzo[b][1,4]oxazin-3(4H)-one

Cs₂CO₃ (3.13 g, 9.60 mmol) was added to a solution of 2-amino-5-methoxy-4-methylphenol (490 mg, 3.20 mmol) in CH₃CN (12 mL) at 0°C followed by addition of 2-chloroacetyl chloride (0.3 mL, 3.36 mmol). The reaction mixture was stirred at ambient temperature for 16 h., filtered through a celite pad which was washed with EtOAc (15 mL). The solvent was removed under reduced pressure, then treated with EtOAc (25 mL) and H₂O (25 mL). The layers were separated and the aqueous layer was extracted with EtOAc (2 x 15 mL). The combined organic layers were dried (Na₂SO₄) and the residue on evaporation triturated with diethyl ether (3 x 4 mL) to provide the title compound (560 mg, 91%).

¹H NMR (300 MHz, DMSO-d₆) δ 7.95 (br s, 1H), 6.57 (s, 1H), 6.51 (s, 1H), 4.57 (s, 2H), 3.78 (s, 3H), 2.14 (s, 3H).

### Step 4: 7-Methoxy-4,6-dimethyl-2H-benzo[b][1,4]oxazin-3(4H)-one

Cs₂CO₃ (1.42 g., 4.35 mmol) and CH₃I (0.73 mL, 14.50 mmol) were added to a solution of 7-methoxy-6-methyl-2*H*-benzo[b][1,4]oxazin-3(4*H*)-one (560 mg, 2.90 mmol) in DMF (12 mL). The reaction mixture was stirred at ambient temperature for 16 h, quenched with H₂O (10 mL) and extracted into EtOAc (2 x 25 mL). The combined extracts were dried (Na₂SO₄) and the residue on evaporation purified by column chromatography on silica gel, eluting with hexane/EtOAc (0 - 20%) to give the title compound (351 mg, 59%).

¹H NMR (300 MHz, CDCl₃) δ 6.73 (s, 1H), 6.50 (s, 1H), 4.55 (s, 2H), 3.77 (s, 3H), 3.31 (s, 3H), 2.17 (s, 3H).

### Step 5: 7-Methoxy-4,6-dimethyl-3,4-dihydro-2H-benzo[b][1,4]oxazine

1M BH₃.THF (60 mL, 59.30 mmol) was added to a solution of 7-methoxy-4,6-dimethyl-2*H*-benzo[*b*][1,4]oxazin-3(4*H*)-one (351 mg, 1.69 mmol) in THF (10 mL) at 0°C. The reaction mixture was stirred at ambient temperature for 16 h., cooled to 0°C, quenched with 1.0 N NaOH (4 mL) before the reaction mixture was then diluted with H₂O (12 mL) and the solvent removed under reduced pressure. The residue was extracted with EtOAc (2 x 25 mL), the combined extracts dried (Na₂SO₄) and concentrated under reduced pressure to provide the title compound (155 mg, 47%).

¹H NMR (300 MHz, CDCl₃) δ 6.53 (s, 1H), 6.39 (s, 1H), 4.31-4.28 (m, 2H), 3.74 (s, 3H), 3.17-3.14 (m, 2H), 2.82 (s, 3H), 2.16 (s, 3H).

### Step 6: 7-Methoxy-4,6-dimethyl-3,4-dihydro-2H-1,4-benzoxazine-8-carboxylic acid

TMEDA (0.48 ml, 3.21 mmol) and sec-BuLi (2.5 M in hexane) (0.32 mL, 0.78 mmol) were introduced to a solution of 7-methoxy-4,6-dimethyl-3,4-dihydro-2*H-*benzo[b][1,4]oxazine (155 mg, 0.80 mmol) in THF (7.0 mL) at -78°C. The reaction mixture was stirred at -78 °C for 1 h., CO₂ gas was bubbled for 40 min., it was slowly brought to ambient temperature, quenched with H₂O (15 mL) and extracted with EtOAc (2 x 20 mL). The aqueous layer was acidified to pH ~2.0 with 1.0 N HCl and extracted with EtOAc (2 x 25 mL). The combined extracts were dried (Na₂SO₄) and concentrated under reduced pressure to afford the title compound (30 mg, 16%).

¹H NMR (300 MHz, CDCl₃) δ 10.29 (br s, 1H), 6.54 (s, 1H), 4.36 - 4.33 (m, 2H), 3.77 (s, 3H), 3.25 - 3.22 (m, 2H), 2.85 (s, 3H), 2.21 (s, 3H).

ES- MS: *m*/*z* 238.7 (M+H).

HPLC: Retention time 7.48 min., Purity: >99% @ 254 nm.

### Example 49: 6-Chloro-5-fluoro-7-methoxy-4-methyl-3,4-dihydro-2H-1,4-benzoxazine-8-carboxylic acid (A-44)

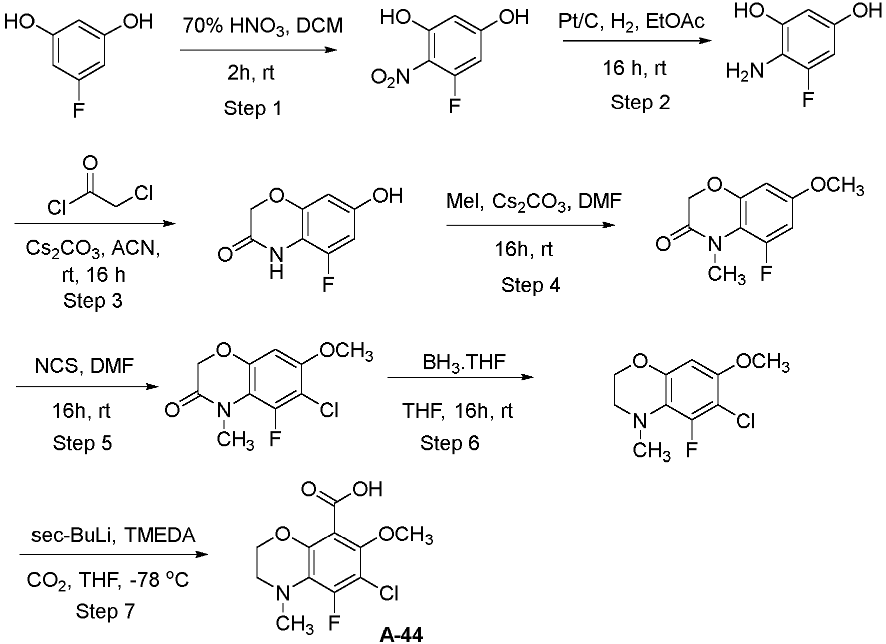

### Step 1: 5-Fluoro-4-nitrobenzene-1,3-diol

To a solution of 5-fluorobenzene-1,3-diol (5 g, 39.03 mmol) in dichloromethane (50 mL) at 0 °C, 70% nitric acid (2.49 ml, 39.03 mmol) was added. The reaction mixture was stirred at ambient temperature for 2 h., cooled to 0°C, quenched with H₂O (10 mL) and extracted with EtOAc (2 x 25 mL). The combined organic layers were dried (Na₂SO₄), concentrated and the residue purified by column chromatography on silica gel eluting with hexane/EtOAc (0 - 20%) to obtain the title compound (2.7 g, 40%).

¹H NMR (300 MHz, CDCl₃) δ 11.13 (s, 1H), 8.43 (s, 1H), 6.36-6.27 (m, 2H).

¹⁹F NMR (300 MHz, CDCl₃) δ -118.86 ppm

### Step 2: 4-Amino-5-fluorobenzene-1,3-diol

5% Pt/C (390 mg) was added to a solution of 5-fluoro-4-nitrobenzene-1,3-diol (2.7 g, 15.60 mmol) in EtOAc (20 mL) and the reaction mixture subjected to a hydrogen atmosphere at 20 psi for 16 h. The reaction mixture was filtered through a celite pad, washed with EtOAc (50 mL) and concentrated under reduced pressure to obtain title compound (1.95 g, 87%) as a solid.

¹H NMR (300 MHz, MeOD) δ 6.15 - 6.13 (m, 1H), 6.09 - 6.04 (m, 1H).

¹⁹F NMR (300 MHz, CDCl₃) δ -134.28 ppm

### Step 3: 5-Fluoro-7-hydroxy-2H-benzo[b][1,4]oxazin-3(4H)-one

Cs₂CO₃ (13.31 g, 40.86 mmol) and 2-chloroacetyl chloride (1.03 mL, 12.94 mmol) were added to a solution of 4-amino-5-fluorobenzene-1,3-diol (1.95 g, 13.62 mmol) in CH₃CN (50 mL) at 0°C then the reaction mixture was stirred at ambient temperature for 16 h. The mixture was filtered through a celite pad, washed with EtOAc (50 mL), the solvent was removed before the residue was treated with EtOAc (75 mL) and H₂O (75 mL). The layers were separated and the aqueous layer was extracted with EtOAc (2 x 50 mL). The combined extracts were dried (Na₂SO₄), concentrated, evaporated and the residue triturated with diethyl ether (3 x 10 mL) to provide the title compound (1.76 g, 71%).

¹H NMR (300 MHz, DMSO-d₆) δ 10.59 (br s, 1H), 9.70 (br s, 1H), 6.31 - 6.23 (m, 2H), 4.52 (s, 2H).

¹⁹F NMR (300 MHz, DMSO-d₆) δ -129.80 ppm

### Step 4: 5-Fluoro-7-methoxy-4-methyl-2H-benzo[b][1,4]oxazin-3(4H)-one

Cs₂CO₃ (4.70 gm, 14.42 mmol) and CH₃I (5.98 mL, 96.10 mmol) were added to a solution of 5-fluoro-7-hydroxy-2*H*-benzo[*b*][1,4]oxazin-3(4*H*)-one (1.76 g, 9.61 mmol) in DMF (30 mL). The reaction was stirred at ambient temperature for 16 h., cooled to 0 °C, quenched with H₂O (20 mL) and the mixture extracted into EtOAc (2 x 50 mL). The combined extracts were dried (Na₂SO₄), concentrated and the residue purified by column chromatography on silica gel, eluting with hexane/EtOAc (0 - 20%) to give title compound (1.4 g, 69%).

¹H NMR (300 MHz, CDCl₃) δ 6.42 - 6.36 (m, 2H), 4.52 (s, 2H), 3.76 (s, 3H), 3.45 - 3.43 (d, 3H).

¹⁹F NMR (300 MHz, CDCl₃) δ -122.23 ppm

### Step 5: 6-Chloro-5-fluoro-7-methoxy-4-methyl-2H-benzo[b][1,4]oxazin-3(4H)-one

NCS (327 mg, 2.45 mmol) was added to a solution of 5-fluoro-7-methoxy-4-methyl-2*H-*benzo[*b*][1,4]oxazin-3(4*H*)-one (518 mg, 2.45 mmol) in DMF (10.0 mL). The reaction was stirred at ambient temperature for 16 h., the mixture was quenched with H₂O (20 mL) and extracted with EtOAc (2 x 50 mL). The combined extracts layers were dried (Na₂SO₄), concentrated and the residue purified by column chromatography on silica gel eluting with hexane/EtOAc (0-50%) to obtain title compound (206 mg, 33%).

¹H NMR (300 MHz, CDCl₃) δ 6.47 (d, 1H), 4.53 (s, 2H), 3.86 (s, 3H), 3.44 (d, 3H).

¹⁹F NMR (300 MHz, CDCl₃) δ -122.98 ppm

### Step 6: 6-Chloro-5-fluoro-7-methoxy-4-methyl-3,4-dihydro-2H-benzo[b][1,4]oxazine

1M BH₃.THF (4.05 mL, 4.05 mmol) was introduced to a solution of 6-chloro-5-fluoro-7-methoxy-4-methyl-2*H*-benzo[*b*][1,4]oxazin-3(4*H*)-one (206 mg, 0.81 mmol) in THF (6 mL) at 0°C and the reaction was stirred at ambient temperature for 16 h. The mixture was cooled to 0°C and carefully quenched with 1.0 N NaOH (4 mL), diluted with H₂O (12 mL) and concentrated under reduced pressure. The residue was extracted into EtOAc (2 x 25 mL) and the combined extracts were dried (Na₂SO₄) and concentrated to yield the title compound (175 mg, 94%).

¹H NMR (300 MHz, CDCl₃) δ 6.28 (d, 1H), 4.14 - 4.12 (m, 2H), 3.81 (s, 3H), 3.12 - 3.10 (s, 2H), 2.82 (s, 3H).

¹⁹F NMR (300 MHz, CDCl₃) δ -123.88 ppm

### Step 7: 6-Chloro-5-fluoro-7-methoxy-4-methyl-3,4-dihydro-2H-1,4-benzoxazine-8-carboxylic acid

TMEDA (0.45 ml, 3.02 mmol) and sec-BuLi (1.4 M in hexanes) (2.16 mL, 3.02 mmol) were added to a solution of 6-chloro-5-fluoro-7-methoxy-4-methyl-3,4-dihydro-2*H-*benzo[*b*][1,4]oxazine (175 mg, 0.76 mmol) in THF (5.0 mL) at -78°C and the mixture was stirred at -78 °C for 1 h. CO₂ gas was bubbled for 40 min. The reaction was slowly brought to ambient temperature, quenched with H₂O (15 mL) and washed with EtOAc (2 x 20 mL). The aqueous layer was acidified to pH ~2.0 with 1.0 N HCl, extracted into EtOAc (2 x 25 mL), then the combined extracts were dried (Na₂SO₄) and concentrated to afford the title compound (100 mg, 48%).

¹H NMR (300 MHz, CDCl₃) δ 9.78 (br s, 1H), 4.24 - 4.21 (m, 2H), 3.91 (s, 3H), 3.21 - 3.18 (m, 2H), 2.90 (s, 3H).

¹⁹F NMR (300 MHz, CDCl₃) δ -119.41 ppm

ES- MS: *m*/*z* 274.6 (M-H).

HPLC: Retention time 8.87 min., Purity: 96% @ 254 nm.

### Example 50: 6-Chloro-7-methoxy-4-[(1,3-oxazol-2-yl)methyl]-3,4-dihydro-2H-1,4-benzoxazine-8-carboxylic acid (A-50)

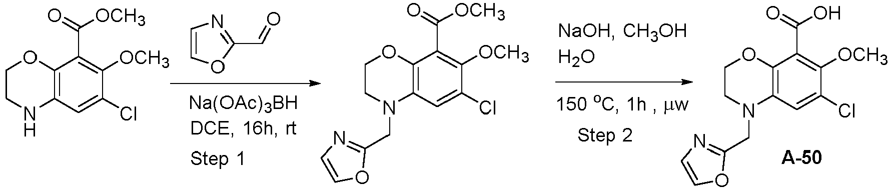

### Step 1: Methyl 6-chloro-7-methoxy-4-(oxazol-2-ylmethyl)-3,4-dihydro-2H-benzo[b][1,4] oxazine-8-carboxylate

Oxazole-2-carbaldehyde (41.2 mg, 0.28 mmol) was added to a solution of methyl 6-chloro-7-methoxy-3,4-dihydro-2H-benzo[b][1,4]oxazine-8-carboxylate (60 mg, 0.23 mmol) in 1,2-DCE (4.0 mL) and the mixture stirred at ambient temperature for 30 min. After 30 min. Na(OAc)₃BH (148.1 mg, 0.70 mmol) was added at 0°C in a single portion and the reaction was stirred at ambient temperature for 16 h. The reaction mixture was diluted with CH₂Cl₂ (15 mL), washed with saturated NaHCO₃ solution (3 x 15 mL), the organic layer dried (Na₂SO₄) and concentrated to provide the title compound which was utilised directly in the next step.

### Step 2: 6-Chloro-7-methoxy-4-[(1,3-oxazol-2-yl)methyl]-3,4-dihydro-2H-1,4-benzoxazine-8-carboxylic acid

NaOH (29.5 mg, 0.74 mmol) was added to a solution of methyl 6-chloro-7-methoxy-4-(oxazol-2-ylmethyl)-3,4-dihydro-2*H*-benzo[b][1,4]oxazine-8-carboxylate (125 mg, 0.37 mmol) in a mixture of CH₃OH and H₂O (3.0 mL/1.0 mL). The reaction was heated at 150°C for 1 h under microwave conditions, the solvent was removed under reduced pressure and the residue was diluted with H₂O (10 mL) and washed with EtOAc (2 x 10 mL). The aqueous layer was acidified to pH ~2.0 using 1.0 N HCl and extracted with EtOAc (2 x 15 mL). The combined organic layers were dried (Na₂SO₄) and concentrated under reduced pressure to afford the title compound (10 mg, 8%).

¹H NMR (300 MHz, CDCl₃) δ 7.63 (s, 1H), 7.11 (s, 1H), 6.86 (s, 1H), 4.54 (s, 2H), 4.35 - 4.29 (m, 2H), 3.86 (s, 3H), 3.53 - 3.50 (m, 2H).

ES-MS: 323.7 [M-1].

HPLC: Retention Time: 7.92 min., purity: 98.3% at 254 nm.

### Example 51: 6-Chloro-7-methoxy-4-[2-(4-methoxyphenyl)ethyl]-3,4-dihydro-2H-1,4-benzoxazine-8-carboxylic acid (A-34)

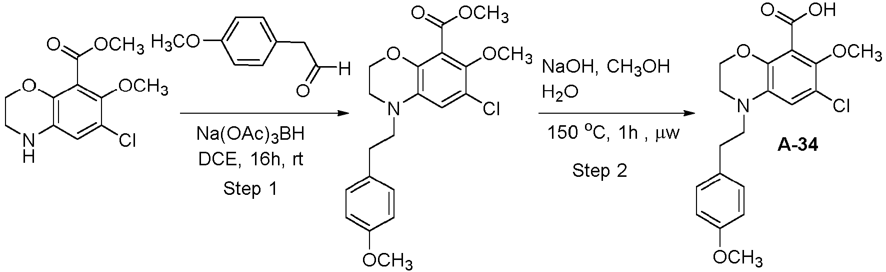

### Step 1: Methyl 6-chloro-7-methoxy-4-(4-methoxyphenethyl)-3,4-dihydro-2H-benzo[b][1,4] oxazine-8-carboxylate

2-(4-Methoxyphenyl)acetaldehyde (0.06 ml, 0.37 mmol) was introduced to a solution of 6-chloro-7-methoxy-3,4-dihydro-2H-benzo[b][1,4]oxazine-8-carboxylate (80 mg, 0.31 mmol) in 1,2-DCE (4.0 mL) and stirred at ambient temperature for 30 min. Na(OAc)₃BH (197 mg, 0.93 mmol) was added at 0°C in one portion and the reaction mixture was stirred at ambient temperature for 16 h. The reaction mixture was diluted with CH₂Cl₂ (15 mL) and washed with aqueous saturated NaHCO₃ (3 x 15 mL). The organic layer was dried (Na₂SO₄) and concentrated under reduced pressure. The crude product was utilised directly in the next stage.

### Step 2: 6-Chloro-7-methoxy-4-[2-(4-methoxyphenyl)ethyl]-3,4-dihydro-2H-1,4-benzoxazine-8-carboxylic acid

To a solution of methyl 6-chloro-7-methoxy-4-(4-methoxyphenethyl)-3,4-dihydro-2*H-*benzo[*b*][1,4] oxazine-8-carboxylate (51 mg, 0.13 mmol) in CH₃OH/H₂O (2.0 mL/1.0 mL), NaOH (10.4 mg, 0.26 mmol) was added. The reaction mixture was heated at 150°C for 1 h. under microwave conditions, the solvent was removed under reduced pressure and the residue diluted with H₂O (10 mL) and washed with washed with EtOAc (2 x 10 mL) to remove impurities. The aqueous layer was acidified to pH ~2.0 using 1.0 N HCl and extracted into EtOAc (2 x 15 mL). The combined organic layers were dried (Na₂SO₄) and concentrated under reduced pressure to provide the title compound (12 mg, 24%).

¹H NMR (300 MHz, CDCl₃) δ 7.19 - 7.16 (m, 2H), 6.92 - 6.90 (m, 2H), 6.71 (s, 1H), 4.25 - 4.22 (m, 2H), 3.92 (s, 3H), 3.86 (s, 3H), 3.51 - 3.46 (m, 2H), 3.27 - 3.24 (m, 2H), 2.88 - 2.84 (m, 2H).

ES-MS: 376.8 [M-1].

HPLC: Retention Time: 11.72 min., purity: >99% at 254 nm.

### Example 52: 7-chloro-6-fluoro-4-methyl-1,2,3,4-tetrahydro-5-quinoxalinecarboxylic acid (A-57)

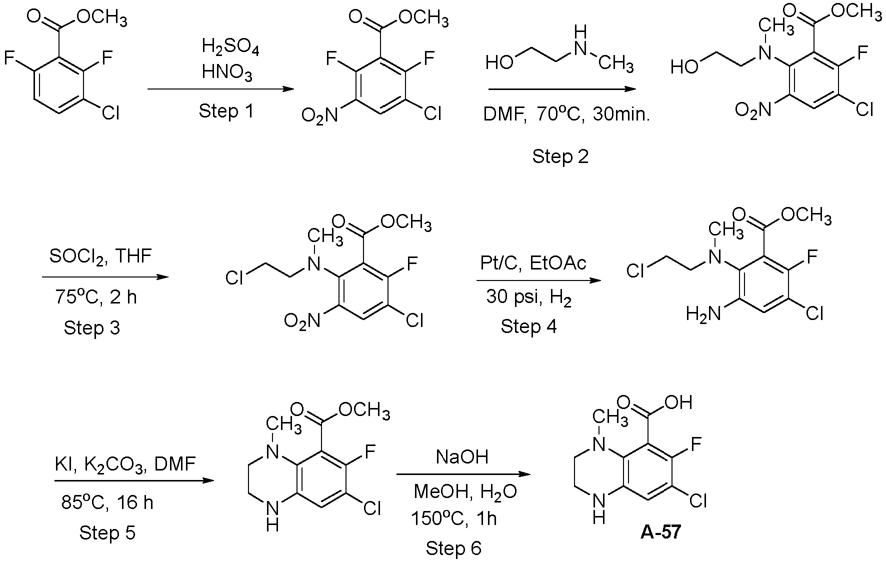

### Step 1: Methyl 3-chloro-2,6-difluoro-5-nitrobenzoate

Nitric acid (70%) (2.26 mL, 35.2 mmol) was added to a solution of methyl 3-chloro-2,6-difluorobenzoate (4.85 g, 23.5 mmol) in sulphuric acid (20 mL) at 0°C. The reaction was stirred at ambient temperature for 30 min. and poured into a mixture of crushed ice and H₂O. After melting, the mixture was extracted into EtOAc (3 x 50 mL), washed with H₂O (3 x 20 mL), brine (20 mL), the combined extracts were dried (Na₂SO₄) and concentrated to obtain the title compound (4.90 g, 83%) as an oil.

¹H NMR (300 MHz, CDCl₃) δ 8.30 (t, 1H), 4.02 (s, 3H).

¹⁹F NMR (300 MHz, CDCl₃); δ -98.95, -114.43 ppm.

### Step 2: Methyl 3-chloro-2-fluoro-6-((2-hydroxyethyl)(methyl)amino)-5-nitrobenzoate

2-(Methylamino)ethanol (0.30 g, 3.98 mmol) was added to a solution of methyl 3-chloro-2,6-difluoro-5-nitrobenzoate (1.0 g, 3.98 mmol) in DMF (5 mL) and heated in a sealed tube at 70°C for 30 min., cooled and extracted into EtOAc (3 x 20 mL) before the combined extracts were washed with H₂O (3 x 20 mL) and brine (20 mL). The solution was dried (Na₂SO₄), concentrated and the resultant residue purified by column chromatography on silica gel eluting with hexane/EtOAc (0 - 50%) to obtain the title compound (1.0 g, 82%) as a yellow solid.

¹H NMR (300 MHz, CDCl₃) δ 8.19 (d, 1H), 4.03 (s, 3H), 3.78 - 3.67 (m, 2H), 3.48 (t, 2H), 3.29 (t, 1H), 2.90 (s, 3H).

¹⁹F NMR (300 MHz, CDCl₃); δ -117.77 ppm.

ES-MS: 277.6 [M+1].

### Step 3: Methyl 3-chloro-6-((2-chloroethyl)(methyl)amino)-2-fluoro-5-nitrobenzoate

SOCl₂ (0.71 mL, 9.80 mmol) was added to a solution of methyl 3-chloro-2-fluoro-6-((2-hydroxyethyl)(methyl)amino)-5-nitrobenzoate (0.30 g, 0.98 mmol) in THF (6 mL) and the reaction mixture was heated at 75°C for 2 h. After cooling and evaporation, H₂O (10 mL) was added, and the mixture extracted into EtOAc (2 x 20 mL). The combined extracts were washed with saturated NaHCO₃ solution (10 mL) and brine (10 mL), dried (Na₂SO₄) and concentrated *in vacuo* to provide the title compound (0.31g, 98%) as a gum.

¹H NMR (300 MHz, CDCl₃) δ 8.18 (d, 1H), 3.99 (s, 3H), 3.62 t, 2H), 3.46 (t, 2H), 2.95 (s, 3H).

¹⁹F NMR (300 MHz, CDCl₃); δ -118.49 ppm.

ES-MS: 325.6 [M+1].

### Step 4: Methyl 3-amino-5-chloro-2-((2-chloroethyl)(methyl)amino)-6-fluorobenzoate

A mixture of methyl 3-chloro-6-((2-chloroethyl)(methyl)amino)-2-fluoro-5-nitrobenzoate (0.305 g, 0.94 mmol), and 5wt% Pt/C (0.03 g, 10%, w/w) in EtOAc (15 mL) was hydrogenated at 30psi for 16 h. at ambient temperature. The mixture was filtered through a pad of Celite^{®} and washed with CH₃OH/EtOAc (20: 80, v/v) and concentrated under reduced pressure. Purification by column chromatography on silica gel eluting with CH₂Cl₂/CH₃OH (0 - 2%) provided the title compound (0.17 g, 61%) as a gum.

¹H NMR (300 MHz, CDCl₃) δ 6.79 (d, 1H), 3.92 (s, 3H), 3.79 (br s, 2H), 3.47 t, 2H), 3.31 (t, 2H), 2.80 (s, 3H).

¹⁹F NMR (300 MHz, CDCl₃); δ - 137.97 ppm.

ES-MS: 295.6 [M+1].

### Step 5: Methyl 7-chloro-6-fluoro-4-methyl-1,2,3,4-tetrahydroquinoxaline-5-carboxylate

K₂CO₃ (0.16 g, 1.15 mmol) and KI (0.191 mL, 1.15 mmol) were added to a solution of methyl 3-amino-5-chloro-2-((2-chloroethyl)(methyl)amino)-6-fluorobenzoate (0.17 g, 0.576 mmol) in DMF (5.0 mL). The mixture was heated at 85°C for 16 h. in a sealed tube, cooled and evaporated. The resultant residue was dissolved in EtOAc (30 mL), the suspension filtered and the filtrate concentrated *in vacuo* to give the title compound which was utilised directly in the next step.

ES-MS: 259.7 [M+1].

### Step 6: 7-Chloro-6-fluoro-4-methyl-1,2,3,4-tetrahydro-5-quinoxalinecarboxylic acid

NaOH (0.015 g, 0.38 mmol) was added to a solution of methyl 7-chloro-6-fluoro-4-methyl-1,2,3,4-tetrahydroquinoxaline-5-carboxylate (0.05 g, 0.15 mmol) in a mixture of CH₃OH (3 mL) and H₂O (1 mL). The reaction mixture was heated under microwave conditions at 150°C for 1 h., cooled, evaporated and the residue purified by semi-prep HPLC eluting with 30 - 100% CH₃CN/0.1% formic acid in H₂O to provide the title compound (12 mg, 33%) as an oil.

¹H NMR (300 MHz, CD₃OD) δ 6.90 (d, 1H), 3.61 (t, 2H), 3.35 (t, 2H), 2.91 (s, 3H).

¹⁹F NMR (300 MHz, CDCl₃); δ - 137.38 ppm.

ES-MS: 245.6 [M+1].

HPLC: Retention time 5.45 min., Purity: 95.3% @ 254 nm.

### Example 53: 1-Benzyl-7-chloro-6-fluoro-4-methyl-1,2,3,4-tetrahydro-5-quinoxalinecarboxylic acid (A-59)

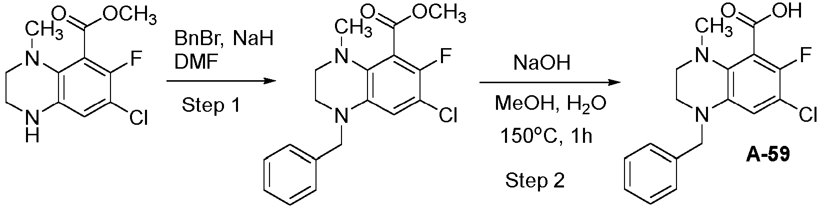

### Step 1: Methyl 1-benzyl-7-chloro-6-fluoro-4-methyl-1,2,3,4-tetrahydroquinoxaline-5-carboxylate

NaH (60% in mineral oil) (30.4 mg, 0.76 mmol) was added to a solution of methyl 7-chloro-6-fluoro-4-methyl-1,2,3,4-tetrahydroquinoxaline-5-carboxylate (50 mg, 0.19 mmol) in DMF (5 mL), and stirred at 0°C for 30 min. Benzyl bromide (39 mg, 0.23 mmol) was introduced at 0°C, the reaction mixture was stirred at ambient temperature for 16 h and H₂O (10 mL) was added. The mixture was extracted into EtOAc (2 x 20 mL), the combined extracts dried (Na₂SO₄), evaporated and the resultant title compound utilised directly in the next step.

### Step 2: 1-Benzyl-7-chloro-6-fluoro-4-methyl-1,2,3,4-tetrahydro-5-quinoxalinecarboxylic acid

NaOH (11 mg, 0.28 mmol) was added to a solution of methyl 1-benzyl-7-chloro-6-fluoro-4-methyl-1,2,3,4-tetrahydroquinoxaline-5-carboxylate (50 mg, 0.14 mmol) in a mixture of CH₃OH (3 mL) and H₂O (1 mL). The reaction mixture was heated under microwave conditions at 150°C for 1 h., cooled and evaporated. and the residue purified by semi-prep HPLC eluting with 30 - 100% CH₃CN/0.1% formic acid in H₂O to provide the title compound (9 mg, 33%) as an oil.

¹H NMR (300 MHz, CD₃OD) δ 7.40 - 7.22 (m, 5H), 6.92 (d, 1H), 4.47 (s, 2H), 3.59 (t, 2H), 3.49 (t, 2H), 2.95 (s, 3H).

¹⁹F NMR (300 MHz, CDCl₃); δ - 135.44 ppm.

ES-MS: 335.6 [M+1].

HPLC: Retention time 8.73 min., Purity: 98.9% @ 254 nm.

### Example 54: 6-Chloro-7-methoxy-4-(propan-2-yl)-3,4-dihydro-2H-1,4-benzoxazine-8-carboxylic acid (A-32)

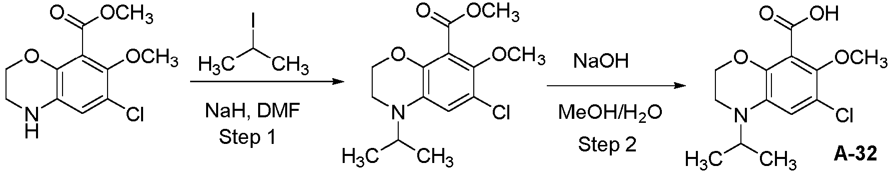

### Step 1: Methyl 6-chloro-4-isopropyl-7-methoxy-3,4-dihydro-2H-benzo[b][1,4]oxazine-8-carboxylate

NaH (60% in mineral oil) (62 mg, 1.55 mmol) was added to a solution of methyl 6-chloro-7-methoxy-3,4-dihydro-2H-benzo[b][1,4]oxazine-8-carboxylate (100 mg, 0.39 mmol) in DMF (5 mL) at 0°C and stirred for 30 min. After a further 30 min., 2-iodopropane (99 mg, 0.582 mmol) was added at 0°C and the mixture stirred at ambient temperature for 16 h., quenched with H₂O (5 mL), extracted into EtOAc (2 x 20 mL), dried (Na₂SO₄) and evaporated. The resultant residue was purified by column chromatography on silica gel eluting with hexane/EtOAc (0 - 30%) to afford the title compound (18 mg, 15%) as a colourless liquid.

¹H NMR (300 MHz, CD₃OD) δ 6.70 (s, 1H), 4.22 (t, 2H), 4.00 - 3.92 (m, 1H), 3.91 (s, 3H), 3.80 (s, 3H), 3.19 (t, 2H), 1.15 (d, 6H).

### Step 2: 6-Chloro-7-methoxy-4-(propan-2-yl)-3,4-dihydro-2H-1,4-benzoxazine-8-carboxylic acid

NaOH (4.8 mg, 0.12 mmol) was added to a solution of methyl 6-chloro-4-isopropyl-7-methoxy-3,4-dihydro-2H-benzo[b][1,4]oxazine-8-carboxylate (18 mg, 0.06 mmol) in MeOH/H₂O (2/1 mL) and the mixture heated under microwave conditions at 150°C for 1h, cooled and evaporated. The resultant residue was was dissolved in H₂O (5 mL) and extracted into CH₂Cl₂ (2 x 5 mL). The aqueous layer was acidified to pH ~2.0 with 1.0 N HCl and extracted into EtOAc (2 x 15 mL). The combined organic layers were dried (Na₂SO₄) and concentrated under reduced pressure to afford the title compound (11 mg, 64%) as a solid.

¹H NMR (300 MHz, CDCl₃) δ 6.78 (s, 1H), 4.28 (t, 2H), 4.02 - 3.90 (m, 1H), 3.86 (s, 3H), 3.23 (t, 2H), 1.17 (d, 6H).

ES- MS: *m*/*z* 284.7 (M-H).

HPLC: Retention time 8.88 min., Purity: 98.7% @ 254 nm.

### Example 55: 6-Chloro-4-[3-(furan-2-yl)propyl]-7-methoxy-3,4-dihydro-2H-1,4-benzoxazine-8-carboxylic acid (A-40)

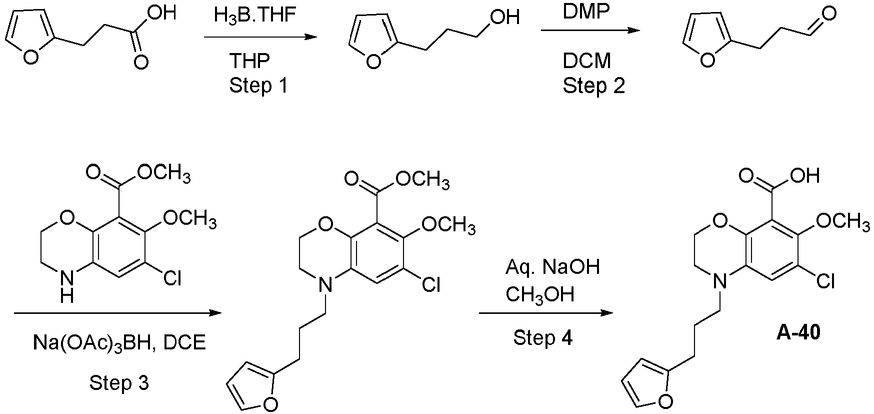

### Step 1: 3-(Furan-2-yl)propan-1-ol

BH₃-THF (28.5 mL, 28.5 mmol) was added to a solution of 3-(furan-2-yl)propanoic acid (2.00 g, 14.3 mmol) in THF (50 mL) at 0°C, and the reaction mixture was stirred at ambient temperature for 24 h. The mixture was cooled to 0°C, slowly quenched with methanol (10 mL) and the solvent was removed. The residue was dissolved in EtOAc (50 mL) and washed with saturated NaHCO₃ (50 mL). The organic layer was dried (Na₂SO₄) and concentrated *in vacuo* to provide the title compound (1.54 g, 86%).

¹H NMR (300 MHz, CDCl₃) δ 7.26 - 7.25 (m, 1H), 6.24 - 6.23 (m, 1H), 5.97 - 5.96 (m, 1H), 3.65 (t, 2H), 2.69 (t, 2H), 1.86 (quint, 2H).

### Step 2: 3-(Furan-2-yl)propanal

To a solution of 3-(furan-2-yl)propan-1-ol (1.50 g, 11.9 mmol) in CH₂Cl₂ (40.0 mL) at 0°C, DMP (6.05 g, 14.3 mmol) was introduced in several portions and the reaction mixture was stirred at ambient temperature for 4 h. The mixture was washed with saturated Na₂S₂O₃ (3 x 50 mL) and aqueous saturated NaHCO₃ (3 x 50 mL). The organic layer was dried (Na₂SO₄) and concentrated under reduced pressure. The residue was triturated with hexanes (3 x 10 mL) and the hexane solution was concentrated to provide the title compound (0.70 g, 47%).

¹H NMR (300 MHz, CDCl₃) δ 9.83 (s, 1H), 7.31 - 7.30 (m, 1H), 6.29 - 6.27 (m, 1H), 6.03 - 6.02 (m, 1H), 2.99 (t, 2H), 2.80 (t, 2H).

### Step 3: Methyl 6-chloro-4-(3-(furan-2-yl)propyl)-7-methoxy-3,4-dihydro-2H-benzo[b][1,4]oxazine-8-carboxylate

A mixture of methyl 6-chloro-7-methoxy-3,4-dihydro-2H-benzo[b][1,4]oxazine-8-carboxylate (4) (80 mg, 0.31 mmol) and 3-(furan-2-yl)propanal (115 mg, 0.93 mmol) in 1,2-DCE (4.0 mL) was stirred at ambient temperature for 30 min. Na(OAc)₃BH (197 mg, 0.93 mmol) was added at 0°C, the reaction mixture was stirred at ambient temperature for 3 h, diluted with CH₂Cl₂ (20 mL) and washed with aqueous saturated NaHCO₃ (3 x 15 mL). The organic layer was dried (Na₂SO₄) and concentrated under reduced pressure and the residue was utilised directly in the next step.

### Step 4: 6-Chloro-4-[3-(furan-2-yl)propyl]-7-methoxy-3,4-dihydro-2H-1,4-benzoxazine-8-carboxylic acid

NaOH (43 mg, 1.08 mmol) was added to a solution of methyl 6-chloro-4-(3-(furan-2-yl)propyl)-7-methoxy-3,4-dihydro-2H-benzo[b][1,4]oxazine-8-carboxylate (118 mg, 0.32 mmol) in a mixture of CH₃OH and H₂O (2.0 mL/1.0 mL) was added and the reaction mixture was heated at 150°C for 1 h under microwave conditions. The solvent was removed, the residue diluted with water (10 mL) and washed with EtOAc (2 x 10 mL). The aqueous layer was acidified to pH ~2.0 using 1.0 N HCl and extracted with EtOAc (2 x 20 mL). The combined extracts were dried (Na₂SO₄) and concentrated under reduced pressure to yield the title compound (32 mg, 28%).

¹H NMR (300 MHz, CDCl₃) δ 7.27 - 7.26 (m, 1H), 6.52 (s, 1H), 6.24 - 6.22 (m, 1H), 6.96 - 6.94 (m, 1H), 4.20 (t, 2H), 3.78 (s, 3H), 3.24 (t, 2H), 3.15 (t, 2H), 2.62 (t, 2H), 1.85 (quint, 2H).

ES-MS: 350.7 [M-1].

HPLC: Retention Time: 11.5 min., purity: 96.7% at 280 nm.

### Example 56: 4-Benzyl-6-fluoro-7-methoxy-3,4-dihydro-2H-1,4-benzoxazine-8-carboxylic acid (A-55)

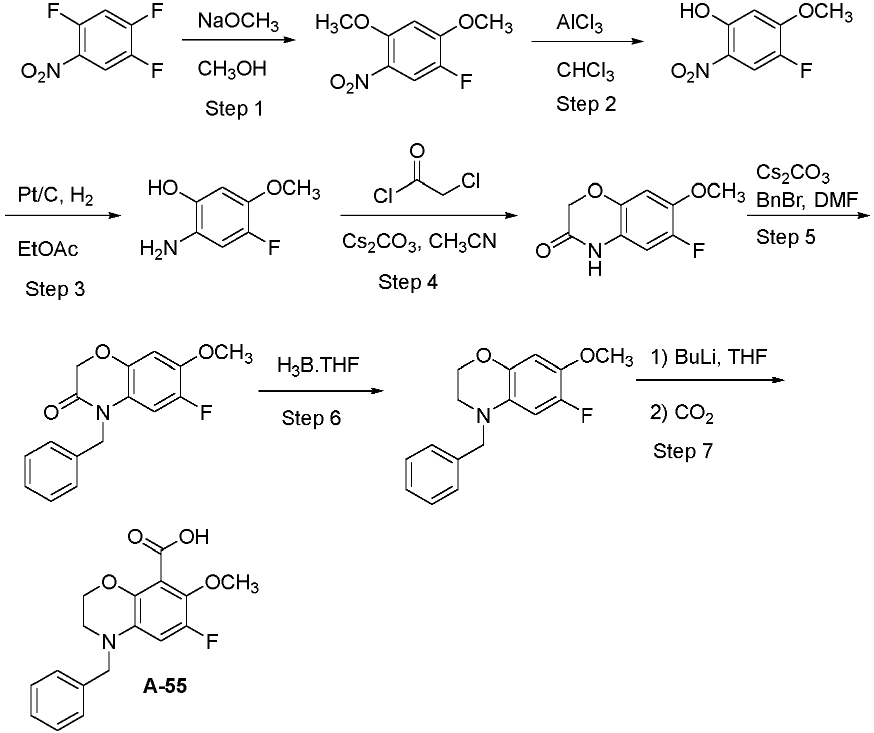

### Step 1: 1-Fluoro-2,4-dimethoxy-5-nitrobenzene

NaOMe (25% in MeOH, 27.0 mL, 124 mmol) was added to a solution of 1,2,4-trifluoro-5-nitrobenzene (10.0 g, 56.4 mmol) in methanol (80 mL) at 0°C, and the reaction mixture was stirred at ambient temperature for 18 h. The solvent was removed, the residue treated with EtOAc (200 mL) and washed with 1.0 N HCl (200 mL). The organic layer was dried (Na₂SO₄) and concentrated under reduced pressure. The residue was triturated with hexanes (3 x 75 mL) to give the title compound (10.2 g, 90%) as a solid. ¹H NMR (300 MHz, CDCl₃) δ 7.83 (d, 1H), 6.60 (s, 1H), 4.00 (s, 3H), 3.98 (s, 3H).

### Step 2: 4-Fluoro-5-methoxy-2-nitrophenol

AlCl₃ (10.1 g, 75.7 mmol) was added to a solution of 1-fluoro-2,4-dimethoxy-5-nitrobenzene (10.2 g, 50.7 mmol) in CHCl₃ (100 mL) at 0°C and the reaction mixture heated at 70°C for 1 h. The mixture was poured into ice water (400 mL), acidified with 1.0 N HCl to pH ~2.0 and extracted into EtOAc (2 x 250 mL), the combined extracts were dried (Na₂SO₄). The residue was triturated with hexanes (2 x 75 mL) to obtain the title compound (9.00 g, 95%) as a yellow solid.

¹H NMR (300 MHz, CDCl₃); δ 10.0 (s, 1H), 7.82 (d, 1H), 6.62 (s, 1H), 3.97 (s, 3H).

### Step 3: 2-Amino-4-fluoro-5-methoxyphenol

5% Pt/C (20% w/w, 600 mg) was added to a solution of 4-fluoro-5-methoxy-2-nitrophenol (3.0 g, 16.0 mmol) in EtOAc (60 mL) and the reaction mixture was stirred for 16 h under an atmosphere of hydrogen at 20 psi. The mixture was filtered through a pad of Celite and the filtrate concentrated to provide the title compound as a solid which was utilised directly in the next step.

¹H NMR (300 MHz, CDCl₃); δ 6.61 (d, 1H), 6.50 (d, 1H), 3.80 (s, 3H).

### Step 4: 6-Fluoro-7-methoxy-2H-benzo[b][1,4]oxazin-3(4H)-one

Chloroacetic chloride (2.00 mL, 24.1 mmol) was added to a suspension of 2-amino-4-fluoro-5-methoxyphenol (2.52 g, 16.0 mmol) and Cs₂CO₃ (13.0 g, 39.9 mmol) in CH₃CN (40 mL) at 0°C. The reaction mixture was stirred at ambient temperature for 16 h and the solvent was removed. The residue was diluted with EtOAc (60 mL) and H₂O (60 mL) and the aqueous layer was extracted with EtOAc (2 x 60 mL). The combined extracts were dried (Na₂SO₄), concentrated under reduced pressure and the residue was triturated with Et₂O (3 x 15 mL) to give the title compound (2.00 g, 63%) as a solid.

¹H NMR (300 MHz, DMSO-d₆) δ 10.5 (br s, 1H), 6.85 (d, 1H), 6.74 (d, 1H), 4.53 (s, 2H), 3.77 (s, 3H).

### Step 5: 4-Benzyl-6-fluoro-7-methoxy-2H-benzo[b][1,4]oxazin-3(4H)-one

Benzyl bromide (0.62 mL, 5.17 mmol) was added to a suspension of 6-fluoro-7-methoxy-2H-benzo[b][1,4]oxazin-3(4H)-one (0.51 g, 2.59 mmol) and Cs₂CO₃ (1.26 g, 3.87 mmol) in DMF (7.0 mL) at 0°C. The reaction mixture was stirred at ambient temperature for 16 h, quenched with H₂O (50 mL) and extracted into EtOAc (2 x 30 mL). The combined extracts were washed with 1.0 N HCl (2 x 30 mL), dried (Na₂SO₄) and the residue purified by column chromatography on silica gel eluting with hexane/EtOAc (10 - 20%) to afford the title compound (0.30 g, 41 %).

¹H NMR (300 MHz, CDCl₃) δ 7.38 - 7.23 (m, 5H), 6.67 (d, 1H), 6.64 (d, 1H), 5.09 (s, 2H), 4.69 (s, 2H), 3.82 (s, 3H).

### Step 6: 4-Benzyl-6-fluoro-7-methoxy-3,4-dihydro-2H-benzo[b][1,4]oxazine

BH₃-THF (1.0 M solution) (6.3 mL, 6.6 mmol) and 4-benzyl-6-fluoro-7-methoxy-2H-benzo[b][1,4]oxazin-3(4H)-one (0.25 g, 1.04 mmol) were combined at 0°C and the reaction mixture stirred at ambient temperature for 1 h. The reaction mixture was cooled to 0°C and quenched with 1.0 N NaOH (10.0 mL) and extracted into EtOAc (2 x 20 mL). The combined extracts were dried (Na₂SO₄) and concentrated to obtain the title compound (260 mg, 91 %).

¹H NMR (300 MHz, CDCl3) δ 7.38 - 7.27 (m, 5H), 6.53 - 6.46 (m, 2H), 4.33 (s, 2H), 4.23 (t, 2H), 3.79 (s, 3H), 3.27 (t, 2H).

### Step 7. 4-Benzyl-6-fluoro-7-methoxy-3,4-dihydro-2H-1,4-benzoxazine-8-carboxylic acid

n-BuLi (2.5 M in hexane) (0.22 mL, 0.52 mmol) was added to a solution of 4-benzyl-6-fluoro-7-methoxy-3,4-dihydro-2H-benzo[b][1,4]oxazine (0.12 g, 0.44 mmol) and TMEDA (0.08 mL, 0.53 mmol) in THF (4.0 mL) at -78°C. The reaction mixture was stirred at that temperature for 1 h., CO₂ (g) was bubbled for 30 min., the mixture was slowly brought to 0°C before quenching with water (7.0 mL) and washed with EtOAc (2 x 20 mL). The aqueous layer was acidified to pH ~2.0 with 1.0 N HCl and extracted with EtOAc (3 x 20 mL). The combined extracts were dried (Na₂SO₄) and concentrated to afford the title compound (5.0 mg, 4%).

¹H NMR (300 MHz, CDCl₃) δ 7.26 - 7.13 (m, 5H), 6.41 (d, *J*_{H-F}= 13.8 Hz, 1H), 4.34 (s, 2H), 4.15 (t, 2H), 3.69 (s, 3H), 3.28 (t, 2H).

¹⁹F NMR (300 MHz, CDCl₃); δ -140.62 ppm.

ES-MS: 316.7 [M-1].

HPLC: Retention Time: 10.6 min., purity: 98.3% at 280 nm.

### Example 57: 4-Benzyl-6,7-dichloro-3,4-dihydro-2H-1,4-benzoxazine-8-carboxylic acid (A-41)

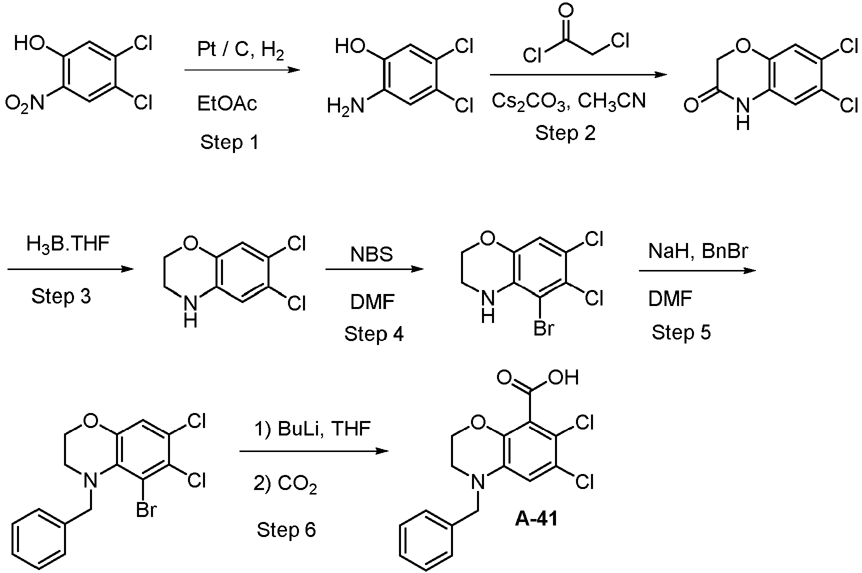

### Step 1. 2-Amino-4,5-dichlorophenol

5% Pt/C (20% w/w, 1000 mg) was added to a solution of 4,5-dichloro-2-nitrophenol (5.00 g, 24.0 mmol) in EtOAc (100 mL) and the reaction mixture was stirred for 20 h under a hydrogen atmosphere at 30 psi. The mixture was filtered through a pad of Celite^{®} which was washed with EtOAc (100 mL). The filtrate was concentrated under reduced pressure to provide the title compound (3.70 g, 86%) which was utilised directly in the next step.

¹H NMR (300 MHz, DMSO-d₆) δ 6.74 (s, 1H), 6.72 (s, 1H), 4.94 (br s, 2H).

### Step 2: 6,7-Dichloro-2H-benzo[b][1,4]oxazin-3(4H)-one

Chloroacetyl chloride (2.50 mL, 31.2 mmol) was added to a suspension of 2-amino-4,5-dichlorophenol (3.70 g, 20.8 mmol) and Cs₂CO₃ (16.9 g, 51.9 mmol) in CH₃CN (70 mL) at 0°C. The reaction mixture was stirred at ambient temperature for 16 h and the solvent was removed under reduced pressure. The residue was treated with EtOAc (200 mL) and water (200 mL), the layers were separated before the aqueous layer was extracted with EtOAc (2 x 100 mL). The combined extracts were dried (Na₂SO₄), concentrated under reduced pressure and the residue was triturated with Et₂O (3 x 20 mL) to give the title compound (1.73 g, 38%).

¹H NMR (300 MHz, DMSO-d₆) δ 7.26 (s, 1H), 7.04 (s, 1H), 4.62 (s, 2H).

### Step 3: 6,7-Dichloro-3,4-dihydro-2H-benzo[b][1,4]oxazine

6,7-Dichloro-2H-benzo[b][1,4]oxazin-3(4H)-one (1.70 g, 7.79 mmol) and BH₃-THF (1.0 M solution) (39.0 mL, 6.6 mmol) were combined 0°C and stirred at ambient temperature for 16 h. The reaction mixture was again cooled to 0°C, quenched with 1.0 N NaOH (50.0 mL) and extracted with EtOAc (2 x 50 mL). The combined extracts were dried (Na₂SO₄) and concentrated to obtain the title compound (1.47 g, 92%).

¹H NMR (300 MHz, CDCl₃) δ 6.84 (s, 1H), 6.64 (s, 1H), 4.21 (t, 2H), 3.82 (br s, 1H), 3.40 (t, 2H).

### Step 4: 5-Bromo-6,7-dichloro-3,4-dihydro-2H-benzo[b][1,4]oxazine

NBS (1.28 g, 7.19 mmol) was added to a solution of 6,7-dichloro-3,4-dihydro-2H-benzo[b][1,4]oxazine (1.40 g, 6.86 mmol) in DMF (14.0 mL) and the mixture was stirred at ambient temperature for 2 h. The reaction mixture was quenched with water (25 mL) and extracted into EtOAc (2 x 50 mL). The combined extracts were washed with 1.0 N HCl (2 x 50 mL), dried (Na₂SO₄) and concentrated. The resultant residue was purified by column chromatography on silica gel eluting with hexane/EtOAc (10-20%) to afford the title compound (0.50 g, 26%).

¹H NMR (300 MHz, CDCl₃) δ 6.90 (s, 1H), 4.45 (br s, 1H), 4.21 (t, 2H), 3.50 (t, 2H).

### Step 5: 4-Benzyl-5-bromo-6,7-dichloro-3,4-dihydro-2H-benzo[b][1,4]oxazine

A 60% oil dispersion of NaH (0.21g, 5.29 mmol) was added to a solution of 5-bromo-6,7-dichloro-3,4-dihydro-2H-benzo[b][1,4]oxazine (0.30 g, 1.06 mmol) in DMF (4.0 mL) at 0°C, and the mixture stirred at ambient temperature for 30 min. Benzyl bromide (0.23 mL, 1.91 mmol) was added and the reaction mixture was stirred at ambient temperature for 72 h. The mixture was cooled to 0°C, quenched with water (50 mL) and extracted into EtOAc (2 x 25 mL). The combined extracts were washed with 1.0 N HCl (2 x 20 mL) and dried (Na₂SO₄). The residue on evaporation was purified by column chromatography on silica gel, eluting with hexane/EtOAc (0 - 10%) to afford the title compound (0.15 g, 38%).

¹H NMR (300 MHz, CDCl₃) δ 7.59 - 7.56 (m, 2H), 7.42 - 7.30 (m, 3H), 7.06 (s, 1H), 4.21 (s, 2H), 4.14 (t, 2H), 2.98 (t, 2H).

### Step 6: 4-Benzyl-6,7-dichloro-3,4-dihydro-2H-1,4-benzoxazine-8-carboxylic acid

n-BuLi (2.5 M in hexane) (0.20 mL, 0.48 mmol) was added to a solution of 4-benzyl-5-bromo-6,7-dichloro-3,4-dihydro-2H-benzo[b][1,4]oxazine (0.15 g, 0.40 mmol) in THF (4.0 mL) at -78°C and the reaction mixture was stirred at that temperature for 1 h. CO₂ (g) was bubbled for 40 min. and the reaction mixture was slowly brought to 0°C. The mixture was quenched with water (10.0 mL) and washed with EtOAc (2 x 20 mL) then the aqueous layer was acidified to pH ~2.0 with 1.0 N HCl and extracted into EtOAc (2 x 20 mL). The combined extracts were dried (Na₂SO₄) and the residue on evaporation purified by semi-prep HPLC eluting with 10 - 100% CH₃CN/0.1% formic acid in H₂O to provide the title compound (7.0 mg, 5%).

Note: during the bromine-lithium exchange step, the anion either rearranged or quenched/deprotonated in the 8-position to give the title product.

¹H NMR (300 MHz, CD₃OD) δ 7.40 - 7.27 (m, 5H), 6.78 (s, 1H), 4.53 (s, 2H), 4.31 (t, 2H), 3.47 (t, 2H).

ES-MS: 338.7 [M+1].

HPLC: Retention Time: 11.6 min., purity: >99% at 280 nm.

### Example 58: 6-Chloro-7-methoxy-2-(methoxymethyl)-4-methyl-3,4-dihydro-2H-1,4-benzoxazine-5-carboxylic acid (A-63)

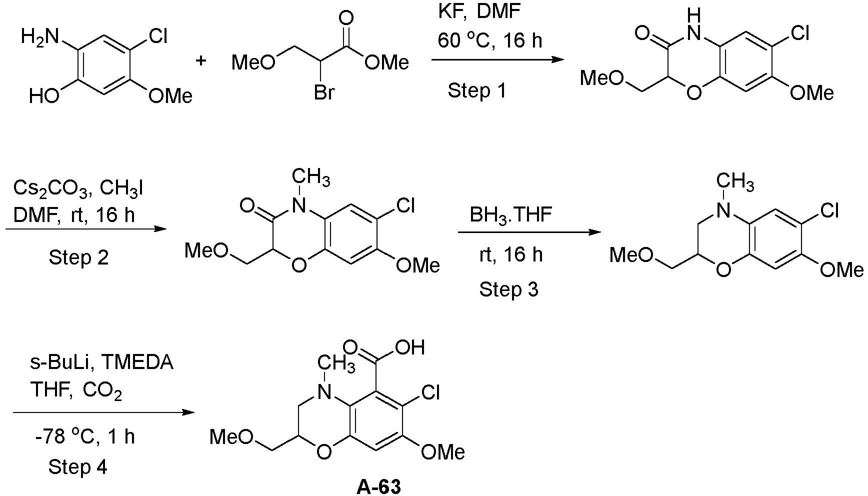

### Step 1: 6-Chloro-7-methoxy-2-(methoxymethyl)-2H-benzo[b][1,4]oxazin-3(4H)-one

KF (0.54 g, 9.22 mmol) and methyl 2-bromo-3-methoxypropanoate (0.91 g, 4.61 mmol) were added to a solution of 2-amino-4-chloro-5-methoxyphenol (0.4 g, 2.30 mmol) in DMF (5 mL). The mixture was stirred at 60°C for 16 h in a sealed tube. The black solution was poured into a mixture of crushed ice and water and the resultant solid was collected by filtration, washed with water (15 mL) and used directly in the next step without purification (0.57 g, 96 %).

¹H NMR (300 MHz, CDCl₃) δ 6.90 (s, 1H), 6.73 (s, 1H), 4.85 - 4.79 (m, 1H), 4.00 - 3.72 (m, 5H), 3.48 (s, 3H).

ES-MS: 256.7 [M-1].

### Step 2: 6-Chloro-7-methoxy-2-(methoxymethyl)-4-methyl-2H-benzo[b][1,4]oxazin-3(4H)-one

Cs₂CO₃ (2.09 g, 6.40 mmol) and CH₃I (0.45 g, 3.20 mmol) were added to a solution of 6-chloro-7-methoxy-2-(methoxymethyl)-2H-benzo[b][1,4]oxazin-3(4H)-one (0.55 g, 2.13 mmol) in DMF (15 mL). The reaction mixture was stirred at ambient temperature for 16 h, diluted with EtOAc (2 x 50 mL), washed with brine solution (3 x 25 mL), dried (Na₂SO₄) and concentrated. The residue was purified by column chromatography on silica gel eluting with hexane/EtOAc (0 - 40%) to obtain the title compound (0.21 g, 36%).

¹H NMR (300 MHz, CDCl₃) δ 6.97 (s, 1H), 6.72 (s, 1H), 4.76 (t, 1H), 3.94 - 3.90 (m, 2H), 3.87 (s, 3H), 3.44 (s, 3H). 3.33 (s, 3H).

### Step 3: 6-Chloro-7-methoxy-2-(methoxymethyl)-4-methyl-3,4-dihydro-2H-benzo[b][1,4]oxazine

A mixture of 6-chloro-7-methoxy-2-(methoxymethyl)-4-methyl-2H-benzo[b][1,4]oxazin-3(4H)-one (0.20 g, 0.74 mmol), and 1M BH₃.THF (2.94 mL, 2.94 mmol) was stirred at ambient temperature for 16 h. The reaction mixture was cooled, and quenched with methanol (10 mL), evaporated and the residue purified by column chromatography on silica gel eluting with hexane/EtOAc (5 - 50%) to obtain the title compound (0.134 g, 70%).

¹H NMR (300 MHz, CDCl₃) δ 6.72 (s, 1H), 6.58 (s, 1H), 4.50 - 4.39 (m, 1H), 3.83 (s, 3H), 3.71 -3.53 (m, 2H), 3.47 (s, 3H), 3.21 (dd, 1H), 3.06 (dd, 1H). 2.84 (s, 3H). ES-MS: 258.7 [M+1].

### Step 4. 6-Chloro-7-methoxy-2-(methoxymethyl)-4-methyl-3,4-dihydro-2H-1,4-benzoxazine-5-carboxylic acid

To a solution of 6-chloro-7-methoxy-2-(methoxymethyl)-4-methyl-3,4-dihydro-2H-benzo[b][1,4]oxazine (130 mg, 0.50 mmol) and TMEDA (0.083 mL, 0.56 mmol) in THF (7 mL) at -78°C, s-BuLi (1.4 M in cyclohexane) (0.40 mL, 0.56 mmol) was introduced. The reaction mixture was stirred at that temperature for 1 h., CO₂ gas was bubbled for 10 min., then the reaction mixture was stirred at -78°C for 1 h., slowly brought to ambient temperature and quenched with 1N NaOH (5 mL). The mixture was washed with EtOAc (2 x 10 mL), the aqueous layer was acidified to pH ~4.0 using 1.0 N HCl and then extracted into EtOAc (2 x 20 mL). The combined extracts were dried (Na₂SO₄), concentrated and the residue was purified by semi-prep HPLC eluting with 30 - 100% CH₃CN/0.1% formic acid in water to provide the title compound (6.3 mg, 4.0 %).

¹H NMR (300 MHz, CD₃OD) δ 6.26 (s, 1H), 4.45-4.34 (m, 1H), 3.77 (s, 3H), 3.72-3.58 (m, 2H), 3.46 (s, 3H), 3.29 - 3.22 (m, 1H), 3.09 - 3.00 (m, 1H), 2.83 (s, 3H).

ES-MS: 300.7 [M-1].

HPLC: Retention Time: 9.90 min., purity: >99% @ 254 nm.

### Example 59: 6-Chloro-7-methoxy-4-[(4-methoxyphenyl)methyl]-3,4-dihydro-2H-1,4-benzoxazine-8-carboxylic acid (A-49)

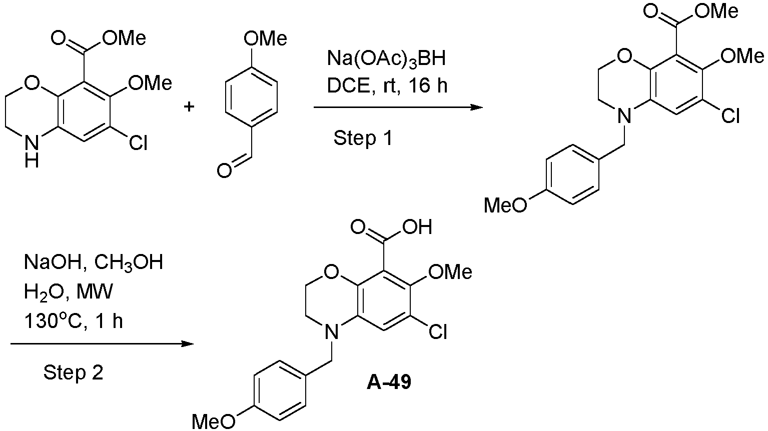

### Step 1: Methyl 6-chloro-7-methoxy-4-(4-methoxybenzyl)-3,4-dihydro-2H-benzo[b][1,4]oxazine-8-carboxylate

Sodium triacetoxyborohydride (147.9 mg, 0.69 mmol) was added to a solution of methyl 6-chloro-7-methoxy-3,4-dihydro-2H-benzo[b][1,4]oxazine-8-carboxylate (60.0 mg, 0.23 mmol) and 4-methoxybenzalyde (94 mg, 0.69 mmol) in 1,2-DCE (4 mL). The reaction mixture was stirred at ambient temperature for 16 h, quenched with water (10 mL), extracted into CH₂Cl₂ (2 x 20 mL), dried (Na₂SO₄) and evaporated. The crude product was purified by column chromatography on silica gel eluting with hexane/EtOAc (0 - 40%) to afford the title compound (73 mg, 84 %).

¹H NMR (300 MHz, CDCl₃) δ 7.27 (d, 2H), 6.97 (d, 2H), 6.79 (s, 1H), 4.42 (s, 2H), 4.35 (t, 2H), 4.03 (s, 3H), 3.91 (s, 3H), 3.90 (s, 3H), 3.38 (t, 2H).

### Step 2: 6-Chloro-7-methoxy-4-[(4-methoxyphenyl)methyl]-3,4-dihydro-2H-1,4-benzoxazine-8-carboxylic acid

NaOH (22.2 mg, 0.56 mmol) was added to a solution of methyl 6-chloro-7-methoxy-4-(4-methoxybenzyl)-3,4-dihydro-2H-benzo[b][1,4]oxazine-8-carboxylate (70.0 mg, 0.18 mmol) in CH₃OH (2.0 mL) and water (1.0 mL) and the reaction mixture was heated under microwave conditions at 130°C. The solvent was removed under reduced pressure and the resultant residue was dissolved in water (10 mL) and washed with CH₂Cl₂ (2 x 10 mL). The water layer was acidified with 1N HCl to pH 4 - 5 and extracted with EtOAc (3 x 25 mL). The combined extracts were dried (Na₂SO₄) to provide the title compound (59 mg, 90 %).

¹H NMR (300 MHz, CD₃OD) δ 7.09 (d, 2H), 6.77 (d, 2H), 6.61 (s, 1H), 4.43 - 4.17 (br s, 2H), 4.14 (t, 2H), 3.66 (s, 6H), 3.23 - 3.15 (m, 2H).

ES-MS: 362.8 [M-1].

HPLC: Retention Time: 11.17 min., purity: >99 % @ 254 nm.

### Example 60: 6-Chloro-7-methoxy-3,4-dihydro-2H-1,4-benzoxazine-8-carboxylic acid (A-14)

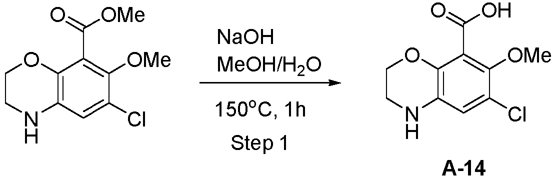

### Step 1: 6-Chloro-7-methoxy-3,4-dihydro-2H-1,4-benzoxazine-8-carboxylic acid

1N solution of NaOH (0.47 mL, 0.47 mmol) was added to a solution of methyl 6-chloro-7-methoxy-3,4-dihydro-2H-benzo[b][1,4]oxazine-8-carboxylate (100 mg, 0.39 mmol) in a mixture of CH₃OH and H₂O (1 mL/ 2 mL), the reaction mixture was subjected to microwave conditions at 150°C for 1 h, cooled then evaporated. The resultant residue was neutralised with 0.1% formic acid solution, extracted into a mixture of CH₂Cl₂ /CH₃OH (19:1) (3 x 10 mL), the combined extracts dried (Na₂SO₄) and concentrated to provide the title compound 6-chloro-7-methoxy-3,4-dihydro-2H-benzo[b][1,4]oxazine-8-carboxylic acid (80 mg, 84%) as a solid.

¹H NMR (300 MHz, CD₃OD) δ 7.50 (s, 1H), 4.56 (t, 2H), 3.93 (s, 3H), 3.77 (t, 2H) ppm. ES-MS: 242.4 M-1].

HPLC: Retention time: 7.13 min., purity >99% at 254 nm.

### Example 61: 6-Chloro-4-ethyl-7-methoxy-3,4-dihydro-2H-1,4-benzoxazine-8-carboxylic acid (A-28)

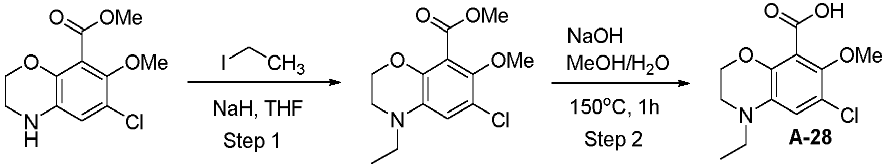

### Step 1: Methyl 6-chloro-4-ethyl-7-methoxy-3,4-dihydro-2H-benzo[b][1,4]oxazine-8-carboxylate

NaH (60% in mineral oil) (155 mg, 3.88 mmol) was added to a solution of methyl 6-chloro-7-methoxy-3,4-dihydro-2H-benzo[b][1,4]oxazine-8-carboxylate (100 mg, 0.388 mmol) in THF (5 mL) at 0°C and stirred for 30 min. lodoethane (0.312 mL, 3.88 mmol) was introduced at 0°C, the reaction mixture stirred at ambient temperature for 16 h., quenched with H₂O (5 mL), extracted into EtOAc (3 x 50 mL), the combined extracts dried (Na₂SO₄), evaporated and purified by column chromatography on silica gel eluting with hexane/EtOAc (10 - 30%) to give the title compound (43 mg, 39%) as an oil.

¹H NMR (300 MHz, CDCl₃); δ 6.62 (s, 1H), 4.23 (m, 2H), 3.90 (s, 3H), 3.79 (s, 3H), 3.28 (m, 4H), 1.14 (t, 3H) ppm.

### Step 2: 6-Chloro-4-ethyl-7-methoxy-3,4-dihydro-2H-1,4-benzoxazine-8-carboxylic acid

1N NaOH (0.28 mL, 0.28 mmol) was added to a solution of methyl 6-chloro-4-ethyl-7-methoxy-3,4-dihydro-2H-benzo[b][1,4]oxazine-8-carboxylate (40 mg, 0.14 mmol) in a mixture of CH₃OH and H₂O (1 mL/ 2 mL). The reaction mixture was subjected to microwave conditions at 150°C for 1 h, cooled and evaporated. The resulting product was neutralised with 0.1% formic acid solution, extracted into a mixture of CH₂Cl₂ /CH₃OH (19:1) (3 x 10 mL), the combined extracts dried (Na₂SO₄) and concentrated to obtain the title compound (35 mg, 92%), as a solid.

¹H NMR (300 MHz, CD₃OD) δ 6.64 (s, 1H), 4.15 (t, 2H), 3.68 (s, 3H), 3.24 (m, 4H), 1.05 (t, 3H),

ES-MS: 270.6 [M-1].

HPLC: Retention time: 9.67 min., purity >99% at 280 nm.

### Example 62: 6-Chloro-7-methoxy-4-(2-phenylethyl)-3,4-dihydro-2H-1,4-benzoxazine-8-carboxylic acid (A-33)

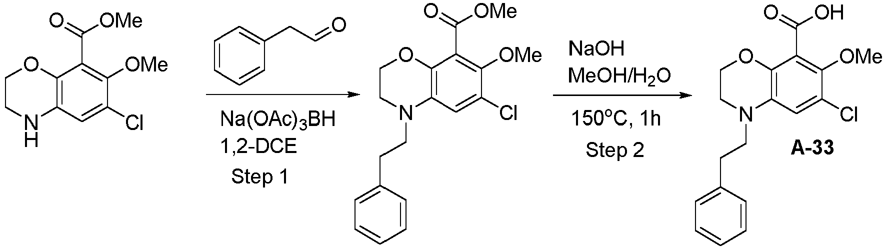

### Step 1: Methyl 6-chloro-7-methoxy-4-phenethyl-3,4-dihydro-2H-benzo[b][1,4]oxazine-8-carboxylate

2-Phenylacetaldehyde (49 mg, 0.41 mmol) was added to a solution of methyl 6-chloro-7-methoxy-3,4-dihydro-2H-benzo[b][1,4]oxazine-8-carboxylate (70 mg, 0.27 mmol) in 1,2-DCE (3 mL) at ambient temperature and stirred for 30 min. Na(OAc)₃BH (144 mg, 0.68 mmol) was introduced and the mixture was stirred at ambient temperature for 16 h. Saturated aqueous NaHCO₃ (10 mL) was added, the mixture stirred for 10 min. The organic layer was separated, aqueous layer extracted into CH₂Cl₂ (3 x 30 mL), the combined extracts dried (Na₂SO₄) and concentrated. The residue was purified by column chromatography on silica gel, eluting with hexane/EtOAc (10 - 30%), to give the title compound as a solid (90 mg, 92% yield).

¹H NMR (300 MHz, CD3OD); δ 7.31 (m, 5H), 6.95 (s, 1H), 4.25 (t, 2H), 3.83 (s, 3H), 3.61 (t, 2H), 3.37 (m, 5H), 2.96 (t, 2H) ppm.

### Step 2: 6-Chloro-7-methoxy-4-(2-phenylethyl)-3,4-dihydro-2H-1,4-benzoxazine-8-carboxylic acid

1N NaOH (0.56 mL, 0.56 mmol) was added to a solution of methyl 6-chloro-7-methoxy-4-phenethyl-3,4-dihydro-2H-benzo[b][1,4]oxazine-8-carboxylate (100 mg, 0.28 mmol) in a mixture of CH₃OH and H₂O (1 mL/ 2 mL). The reaction mixture was subjected to microwave conditions at 150°C for 1 h, cooled then evaporated. The resultant residue was neutralised with 0.1% formic acid, extracted into a mixture of CH₂Cl₂/CH₃OH (19:1) (3 x 10 mL), the combined extracts dried (Na₂SO₄), concentrated and the mixture purified by semi-prep HPLC eluting with 30 - 100% CH₃CN/0.1% formic acid in water to provide the title compound (20 mg, 21%) as a solid.

¹H NMR (300 MHz, CD3OD); δ 7.28 (m, 5H), 6.68 (s, 1H), 4.14 (t, 2H), 3.81 (s, 3H), 3.51 (t, 2H), 3.25 (m, 2H), 2.90 (t, 2H) ppm.

ES-MS: 346.7 [M-1].

HPLC: Retention time: 12.01 min., purity >99% at 280 nm.

### Example 63: 6-Chloro-7-methoxy-4-[(thiophen-3-yl)methyl]-3,4-dihydro-2H-1,4-benzoxazine-8-carboxylic acid (A-36)

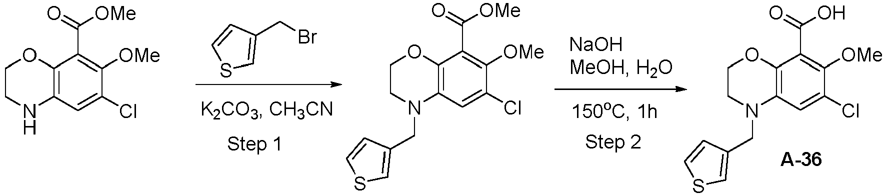

### Step 1: Methyl 6-chloro-7-methoxy-4-(thiophen-3-ylmethyl)-3,4-dihydro-2H-benzo[b][1,4]oxazine-8-carboxylate

K₂CO₃ (75 mg, 0.543 mmol) and 3-(bromomethyl)thiophene (72 mg, 0.41 mmol) were added to a solution of methyl 6-chloro-7-methoxy-3,4-dihydro-2H-benzo[b][1,4]oxazine-8-carboxylate (70 mg, 0.272 mmol) in CH₃CN (2 mL). The reaction mixture was stirred at ambient temperature for 16 h., quenched with saturated NaHCO₃ solution (5 mL), extracted into EtOAc (3 x 30 mL), the combined extracts dried (Na₂SO₄) and evaporated to give the title compound (90 mg, 94 %) as a solid which was utilised directly in the next step.

¹H NMR (300 MHz, CDCl3); δ 7.38 (m, 1H), 7.16 (m, 1H), 7.03 (m, 1H), 6.78 (s, 1H), 4.43 (s, 2H), 4.30 (m, 2H), 3.96 (s, 3H), 3.87 (s, 3H), 2.36 (t, 2H) ppm.

### Step 2: 6-Chloro-7-methoxy-4-[(thiophen-3-yl)methyl]-3,4-dihydro-2H-1,4-benzoxazine-8-carboxylic acid

To a solution of methyl 6-chloro-7-methoxy-4-(thiophen-3-ylmethyl)-3,4-dihydro-2H-benzo[b][1,4]oxazine-8-carboxylate (90 mg, 0.25 mmol) in a mixture of CH₃OH and H₂O (1 mL/ 1 mL). The reaction mixture was subjected to microwave conditions at 150°C for 1 h, cooled and evaporated. The resultant residue was neutralised with 0.1% formic acid solution, extracted into a mixture of CH₂Cl₂/CH₃OH (19:1) (3 x 10 mL), the combined extracts dried (Na₂SO₄) then concentrated afford the title compound (75 mg, 88%) as a solid.

¹H NMR (300 MHz, CD3OD); δ 7.40 (m, 1H), 7.23 (m, 1H), 7.03 (dd, 1H), 6.81 (s, 1H), 4.44 (s, 2H), 4.27(t, 2H), 3.87 (s, 3H), 2.37 (t, 2H) ppm.

ES-MS: 339.0 [M-1].

HPLC: Retention time: 10.91 min., purity >99% at 280 nm.

### Example 64: 6-Chloro-7-methoxy-4-[(1,3-thiazol-2-yl)methyl]-3,4-dihydro-2H-1,4-benzoxazine-8-carboxylic acid (A-43)

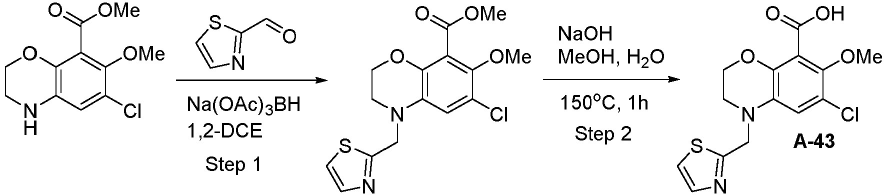

### Step 1: Methyl 6-chloro-7-methoxy-4-(thiazol-2-ylmethyl)-3,4-dihydro-2H-benzo[b][1,4]oxazine-8-carboxylate

Thiazole-2-carbaldehyde (36 mg, 0.32 mmol) was added to a solution of methyl 6-chloro-7-methoxy-3,4-dihydro-2H-benzo[b][1,4]oxazine-8-carboxylate (68 mg, 0.264 mmol) in 1,2-DCE (2 mL) and the mixture stirred at ambient temperature for 30 min. Na(OAc)₃BH (168 mg, 0.792 mmol) was introduced, the reaction mixture stirred at ambient temperature for a further 16 h, then saturated NaHCO₃ (10 mL) was added and stirring continued for 10 min. The organic layer was separated, the aqueous layer extracted into CH₂Cl₂ (3 x 30 mL), the combined extracts dried (Na₂SO₄) and concentrated. The crude product was purified by column chromatography on silica gel eluting with hexane/EtOAc (0 - 10%) to provide the title compound as an oil (55 mg, 59%).

¹H NMR (300 MHz, CDCl3); δ 7.65 (m, 1H), 7.29 (m, 1H), 6.64 (s, 1H), 4.72 (s, 2H), 4.25 (t, 2H), 3.99 (s, 3H), 3.82 (s, 3H), 3.38 (t, 2H) ppm.

### Step 2: 6-Chloro-7-methoxy-4-[(1,3-thiazol-2-yl)methyl]-3,4-dihydro-2H-1,4-benzoxazine-8-carboxylic acid

A 1N solution of NaOH (0.29 mL, 0.29 mmol) was added to a solution of methyl 6-chloro-7-methoxy-4-(thiazol-2-ylmethyl)-3,4-dihydro-2H-benzo[b][1,4]oxazine-8-carboxylate (52 mg, 0.146 mmol) in a mixture of CH₃OH and H₂O (1 mL/ 2 mL). The reaction mixture was then subjected to microwave conditions at 150°C for 1 h, cooled, then evaporated. The resultant product was neutralised with 0.1% formic acid, extracted into a mixture of CH₂Cl₂/CH₃OH (19:1) (3 x 10 mL), the combined extracts dried (Na₂SO₄) and concentrated under reduced pressure to provide the title compound (40 mg, 80%) as a solid.

¹H NMR (300 MHz, CD3OD); δ 7.75 (d, 1H), 7.49 (d, 1H), 6.68 (s, 1H), 4.68 (s, 2H), 4.26 (t, 2H), 3.70 (s, 3H), 3.35 (t, 2H) ppm.

ES-MS: 341.6 [M+1].

HPLC: Retention time: 8.48 min., purity >99% at 280 nm.

### Example 65. 6-Chloro-7-methoxy-4-[3-(1,3-thiazol-2-yl)propyl]-3,4-dihydro-2H-1,4-benzoxazine-8-carboxylic acid (A-47)

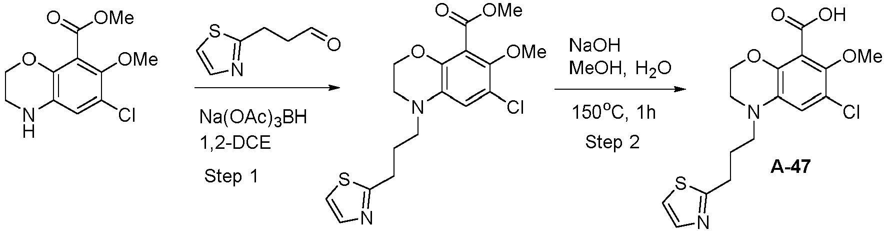

### Step 1: Methyl 6-chloro-7-methoxy-4-(3-(thiazol-2-yl)propyl)-3,4-dihydro-2H-benzo[b][1,4]oxazine-8-carboxylate

3-(Thiazol-2-yl)propanal (40 mg, 0.283 mmol) was added to a solution of methyl 6-chloro-7-methoxy-3,4-dihydro-2H-benzo[b][1,4]oxazine-8-carboxylate (73 mg, 0.283 mmol) in 1,2-DCE (5 mL), at ambient temperature and the mixture stirred for 30 min. Na(OAc)₃BH (150 mg, 0.708 mmol) was added and the mixture stirred at ambient temperature for 16 h. Saturated aqueous NaHCO₃ (10 mL) was added and stirring continued for a further 10 min. The organic layer was separated, the aqueous layer extracted into CH₂Cl₂ (3 x 30 mL), the combined extracts dried (Na₂SO₄) and concentrated. The crude product was purified by column chromatography on silica gel eluting with EtOAc/hexane (10 - 30%) to give the title compound as a white solid (103 mg, 99%).

¹H NMR (300 MHz, CDCl3); δ 7.36 (m, 1H), 7.22 (m, 1H), 6.64 (s, 1H), 4.26 (m, 2H), 3.93 (s, 3H), 3.82 (s, 3H), 3.34 (m, 2H), 3.20 (t, 2H), 3.10 (t, 2H), 2.15 (m, 2H) ppm.

### Step 2: 6-Chloro-7-methoxy-4-[3-(1,3-thiazol-2-yl)propyl]-3,4-dihydro-2H-1,4-benzoxazine-8-carboxylic acid

A 1N solution of NaOH (0.52 mL, 0.52 mmol) was added to a solution of methyl 6-chloro-7-methoxy-4-(3-(thiazol-2-yl)propyl)-3,4-dihydro-2H-benzo[b][1,4]oxazine-8-carboxylate (100 mg, 0.26 mmol) in a mixture of CH₃OH and H₂O (1 mL/1 mL). The reaction mixture was subjected to microwave conditions at 150°C for 1 h., cooled and evaporated. The resultant product was neutralised with 0.1% formic acid and extracted into a mixture of CH₂Cl₂/CH₃OH (19:1) (3 x 10 mL). The combined extracts were dried (Na₂SO₄) then concentrated under reduced pressure to provide the title compound (13 mg, 14%) as a solid.

¹H NMR (300 MHz, CD3OD); δ 7.76 (d, 1H), 7.53 (d, 1H), 6.69 (s, 1H), 4.24 (m, 2H), 3.80 (s, 3H), 3.36 (m, 4H), 3.14 (t, 2H), 2.09 (t, 2H) ppm.

ES-MS: 369.6 [M+1].

HPLC: Retention time: 9.22 min., purity 95.5 % at 280 nm.

### Example 66: 4-Benzyl-6-chloro-7-methoxy-3,4-dihydro-2H-1,4-benzoxazine-8-carboxylic acid (A-52)

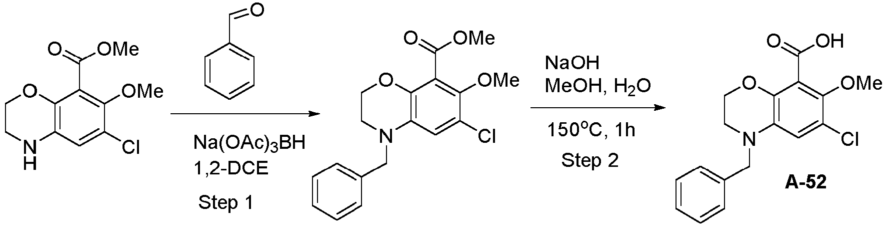

### Step 1: Methyl 4-benzyl-6-chloro-7-methoxy-3,4-dihydro-2H-benzo[b][1,4]oxazine-8-carboxylate

Benzaldehyde (0.076 mL 0.745 mmol) was added to a solution of methyl 6-chloro-7-methoxy-3,4-dihydro-2H-benzo[b][1,4]oxazine-8-carboxylate (64 mg, 0.248 mmol) in 1,2-DCE (10 mL) at ambient temperature and the mixture stirred for 30 min. Na(OAc)₃BH (158 mg, 0.745 mmol) was introduced, the reaction was stirred at ambient temperature for 16 h. and quenched with aqueous saturated NaHCO₃ (10 mL). The aqueous layer was extracted into EtOAc (2 x 20 mL), the combined extracts dried (Na₂SO₄), evaporated and the residue purified by column chromatography on silica gel eluting with EtOAc/hexane (0 - 30%) to provide the title compound (84 mg, 97%) as an oil.

¹H NMR (300 MHz, CDCl₃) δ 7.25 -7.00 (m, 5H), 6.52 (s, 1H), 4.42 (s, 2H), 4.21 (t, 2H), 3.91 (s, 3H), 3.80 (s, 3H), 3.22 (t, 2H).

### Step 2: 4-Benzyl-6-chloro-7-methoxy-3,4-dihydro-2H-1,4-benzoxazine-8-carboxylic acid

NaOH (29 mg, 0.72 mmol) was added to a solution of methyl 4-benzyl-6-chloro-7-methoxy-3,4-dihydro-2H-benzo[b][1,4]oxazine-8-carboxylate (84 mg, 0.24 mmol) in a mixture of CH₃OH and H₂O (2 mL/1 mL). The reaction mixture was heated under microwave conditions at 130°C for 1 h., cooled and evaporated. The residue was dissolved in H₂O (5 mL), washed with CH₂Cl₂ (2 x 5 mL). The aqueous layer was acidified to pH ~2.0 with 1.0 N HCl and extracted into EtOAc (2 x 15 mL). The combined extracts dried (Na₂SO₄) and evaporated under reduced pressure to afford the title compound (67 mg, 84%) as a solid.

¹H NMR (300 MHz, CDCl₃) δ 7.30 -7.10 (m, 5H), 6.54 (s, 1H), 4.32 (s, 2H), 4.20 (t, 2H), 3.72 (s, 3H), 3.25 (t, 2H).

ES- MS: *m*/*z* 234.7 (M-H).

HPLC: Retention time 11.27 min., Purity: 97.2% @ 280 nm.

### Example 67: Electrophysiological measurement of compound inhibition of CIC-1 in rat muscle

The investigatory goal of these experiments was to evaluate whether compounds inhibit CIC-1 channels in native tissue of rat skeletal muscle fibres. Apparent CIC-1 affinity was reported by the concentration of compound at which 50% of the compound's full inhibition of CIC-1 was observed (EC₅₀) (see also WO2019/115777).

Experimentally, Gₘ was measured in individual fibres of whole rat soleus muscles using a three micro-electrodes technique described in this example and in full detail elsewhere (Riisager et al., Determination of cable parameters in skeletal muscle fibres during repetitive firing of action potentials. Journal of Physiology, 2014, 592, 4417-4429). Briefly, intact rat soleus muscles were dissected out from 12-14 week old Wistar rats and placed in an experimental chamber that was perfused with a standard Krebs Ringer solution containing 122 mM NaCl, 25 mM NaHCO₃, 2.8 mM KCI, 1.2 mM KH₂PO₄, 1.2 mM MgSO₄, 1.3 mM CaCl₂, 5.0 mM D-glucose. During experiments, the solution was kept at approx. 30°C and continuously equilibrated with a mixture of 95% O₂ and 5% CO₂, pH -7.4. The experimental chamber was placed in Nikon upright microscope that was used to visualize individual muscle fibres and the three electrodes (glass pipettes filled with 2 M potassium citrate). For Gₘ measurements, the electrodes were inserted into the same fibre with known inter-electrode distances of 0.35 - 0.5 mm (V1-V2, X1) and 1.1-1.5 mm (V1-V3, X3). The membrane potential of the impaled muscle fibre was recorded by all electrodes. Two of the electrodes were furthermore used to inject 50 ms current pulses of -30 nA. Given the positions of the electrodes, three different inter-electrode distances could be identified (X1-X2, X1-X3, X2-X3) and hence the membrane potential responses to the current injections could be obtained at three distances from the point of current injection. The steady state voltage deflection at each distance was divided by the magnitude of current injected (-30 nA) and the resulting transfer resistances were plotted against inter-electrode distance and the data was fitted to a mono-exponential function from which Gₘ could be calculated using linear cable theory.

To establish a dose response relationship, Gₘ was first determined in 10 muscle fibres in the absence of compound and then at four increasing compound concentrations with Gₘ determinations in 5-10 fibres at each concentration. The average Gₘ values at each concentration were plotted against compound concentration and the data was fitted to sigmoidal function to obtain an EC₅₀ value. Table 2 shows the EC₅₀ values for a range of compounds with n values referring to number of experiments that each reflect recordings from around 50 fibres.

**Table 2: Inhibition of CIC-1 ion channel using compounds of the disclosure**

| **Compound investigated** | **Potency Range** |
|---|---|
| A-1 | A |
| A-2 | C |
| A-3 | B |
| A-4 | B |
| A-5 | A |
| A-6 | A |
| A-7 | C |
| A-8 | B |
| A-9 | B |
| A-13 | B |
| A-17 | A |
| A-18 | B |
| A-19 | B |
| A-21 | B |
| A-28 | C |
| A-29 | C |
| A-30 | C |
| A-36 | B |
| A-54 | C |
| A-58 | C |
| A-60 | C |

Potency range: A means an EC₅₀ between 5 and 15 µM; B means an EC₅₀ between 1 and 5 µM; C means an EC₅₀ between 0.2 and 1.0 µM.

In conclusion, this example demonstrates that the compounds of the present disclosure inhibit the CIC-1 channel.

### Example 68: Measurement of force in an in vitro model

The current disclosure relates to compounds that inhibit CIC-1 ion channels and increase muscle excitability and thereby improve muscle function in clinical conditions where muscle activation is failing. Such conditions result in loss of contractile function of skeletal muscle, weakness and excessive fatigue. In this series of experiments the compounds were tested for their ability to restore contractile function of isolated rat muscle when the neuromuscular transmission had been compromised akin to neuromuscular disorders.

Experimentally, soleus muscles from 4-5 wk old rats were isolated with the motor nerve remaining attached. The nerve-muscle preparations were mounted in experimental setups that enabled electrical stimulation of the motor nerve. Stimulation of the motor nerve led to activation of the muscle fibres and ensuing force production that was recorded. The nerve-muscle preparations were also in these experiments incubated in the standard Krebs Ringer (see example 15) and the solution was heated to 30°C and continuously equilibrated with a mixture of 95% O₂ and 5% CO₂, pH ~7.4.

After mounting the nerve-muscle preparation in the experimental setup, the contractile function of the muscle was initially assessed under the control conditions. Sub-maximal concentration of tubocurarine (115 nM), an acetylcholine receptor antagonist, was then added to the experimental bath to impose partial inhibition of the ability of the motor nerve to activate the muscle fibres. The experimental condition mimics the failing neuromuscular transmission in a range of neuromuscular disorders. After addition of tubocurarine the contractile force declined over the next 90 minutes to 10-50 % of the control force. The test compound was then added to obtain the required compound concentration in the bath and the contractile force recovered was measured. To quantify the ability of the compound to restore force the percentage of the initial force that was restored was determined after 40 mins of compound exposure and the point increase is reported in Tables 3 to 7.

**Table 3: Percentage increase of initial force that was restored when using 100 µM of test compound**

| **Compound investigated** | **Point increase (%)** |
|---|---|
| A-22 | 15 |
| A-41 | 28 |
| A-43 | 14 |
| A-47 | 14 |

**Table 4: Percentage increase of initial force that was restored when using 50 µM of test compound**

| **Compound investigated** | **Point increase (%)** |
|---|---|
| A-1 | 31 |
| A-5 | 44 |
| A-11 | 23 |
| A-12 | 54 |
| A-15 | 51 |
| A-16 | 49 |
| A-17 | 29 |
| A-20 | 22 |
| A-24 | 34 |
| A-31 | 32 |
| A-32 | 39 |
| A-33 | 25 |
| A-35 | 21 |
| A-37 | 51 |
| A-38 | 17 |
| A-39 | 35 |

**Table 5: Percentage increase of initial force that was restored when using 25 µM of test compound**

| **Compound investigated** | **Point increase (%)** |
|---|---|
| A-14 | 28 |
| A-21 | 29 |
| A-26 | 28 |
| A-28 | 36 |
| A-36 | 36 |
| A-40 | 14 |
| A-42 | 30 |
| A-44 | 35 |
| A-46 | 8 |
| A-48 | 23 |
| A-52 | 14 |
| A-53 | 30 |

**Table 6: Percentage increase of initial force that was restored when using 35 µM of test compound**

| **Compound investigated** | **Point increase (%)** |
|---|---|
| A-56 | 48 |

**Table 7: Percentage increase of initial force that was restored when using 10 µM of test compound**

| **Compound investigated** | **Point increase (%)** |
|---|---|
| A-2 | 32 |
| A-3 | 41 |
| A-4 | 36 |
| A-6 | 19 |
| A-8 | 26 |
| A-9 | 37 |
| A-10 | 36 |
| A-13 | 30 |
| A-18 | 34 |
| A-19 | 28 |
| A-29 | 43 |
| A-30 | 39 |
| A-45 | 43 |
| A-54 | 28 |
| A-58 | 35 |
| A-60 | 17 |
| A-61 | 16 |
| A-62 | 28 |

In conclusion, this example demonstrates that the compounds of the present disclosure are able to increase muscle excitability and thereby improve muscle function in clinical conditions.

## Claims

1. A compound of Formula (I): 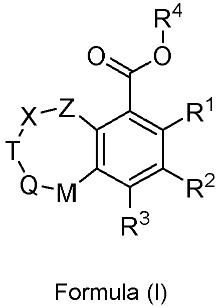 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
when R⁵ and R⁶ are individually C₁₋₅ alkyl optionally substituted with one or more, identical or different, substituents R¹⁰, then R⁵ and R⁶ are optionally joined together to form a ring;
or a pharmaceutically acceptable salt, hydrate, polymorph, tautomer, or solvate thereof provided that:
- only one of M, Q, T, X and Z is O; and
- when M is O, Q and T are CH₂, X is absent, Z is NH, R¹ is Cl, R² is H and R³ is H then R⁴ is not H.

2. The compound according to claim 1, wherein compound is of formula (II): 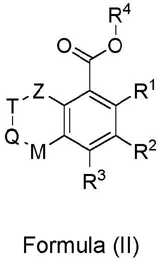
- M is NR⁷, O or S;
- Q is CR⁵R⁶;
- T is CR⁵R⁶;
- Z is NR⁹, O or S;
- R¹ is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
when R⁵ and R⁶ are individually C₁₋₅ alkyl optionally substituted with one or more, identical or different, substituents R¹⁰, then R⁵ and R⁶ are optionally joined together to form a ring;
or a pharmaceutically acceptable salt, hydrate, polymorph, tautomer, or solvate thereof provided that:
- only one of M and Z is O; and
- when M is O, Q and T are CH₂, Z is NH, R' is Cl, R² is H and R³ is H then R⁴ is not H.

3. The compound according to claim 1, wherein the compound is selected from the group consisting of Formula (III), Formula (IV), Formula (V), Formula (VI), and Formula (VII): 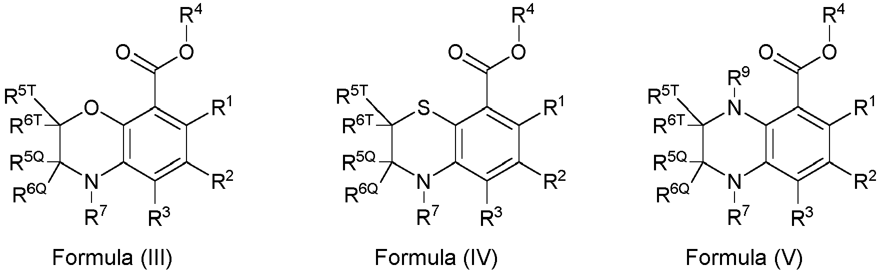 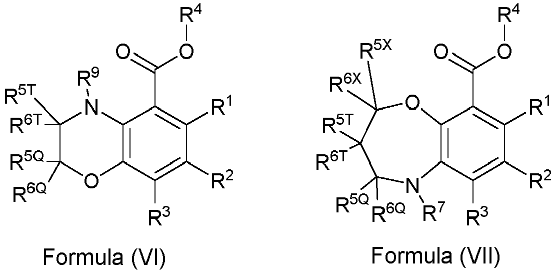 wherein:
- R' is selected from the group consisting of F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; and C₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; **phenyl optionally** substituted with one or more, identical or different, substituents R¹¹; and benzyl optionally substituted with one or more, identical or different, substituents R¹¹;
- R^{5Q}, R^{5T} and R^{5X} are independently selected from the group consisting of H, deuterium, F and C₁₋₅ alkyl optionally substituted with one or more, identical or different, substituents R¹⁰;
- R^{6Q}, R^{6T} and R^{6X} are independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
**when** R^{5Q} and R^{6Q} are individually C₁₋₅ alkyl optionally substituted with one or more, identical or different, substituents R¹⁰, then R^{5Q} and R^{6Q} are optionally joined together to form a ring;
or when R^{5T} and R^{6T} are individually C₁₋₅ alkyl optionally substituted with one or more, identical or different, substituents R¹⁰, then R^{5T} and R^{6T} are optionally joined together to form a ring;
or when R^{5X} and R^{6X} are individually C₁₋₅ alkyl optionally substituted with one or more, identical or different, substituents R¹⁰, then R^{5X} and R^{6X} are optionally joined together to form a ring;
or a pharmaceutically acceptable salt, hydrate, polymorph, tautomer, or solvate thereof.

4. The compound according to any of claims 1 to 3, wherein R¹ is selected from the group consisting of F, Cl, Me, OMe, OEt, OCHMe₂ and SMe.

5. The compound according to any of claims 1 to 4, wherein R² is selected from the group consisting of H, F, Cl and Me.

6. The compound according to any of claims 1 to 5, wherein R⁴ is H.

7. The compound according to any of claims 1 to 6, wherein R¹ is OMe and R² is F or Cl.

8. The compound according to any one of claims 1 to 7, wherein the compound is selected from the group consisting of:
6-chloro-7-methoxy-4-methyl-3,4-dihydro-2H-1,4-benzoxazine-8-carboxylic acid;
7-chloro-6-fluoro-4-methyl-3,4-dihydro-2H-1,4-benzoxazine-5-carboxylic acid;
7-chloro-6-fluoro-4-methyl-3,4-dihydro-2H-1,4-benzoxazine-8-carboxylic acid;
5-chloro-7-fluoro-3,4-dihydro-2H-1,4-benzoxazine-8-carboxylic acid;
6-chloro-7-fluoro-3,4-dihydro-2H-1,4-benzoxazine-8-carboxylic acid;
6-chloro-7-fluoro-4-methyl-3,4-dihydro-2H-1,4-benzoxazine-8-carboxylic acid;
7-chloro-6-fluoro-1,4-dimethyl-1,2,3,4-tetrahydroquinoxaline-5-carboxylic acid;
6-chloro-7-fluoro-2,2,4-trimethyl-3,4-dihydro-2H-1,4-benzoxazine-8-carboxylic acid;
7-chloro-6-methoxy-4-methyl-3,4-dihydro-2H-1,4-benzoxazine-5-carboxylic acid;
7-chloro-6-fluoro-3,4-dihydro-2H-1,4-benzoxazine-5-carboxylic acid;
7-chloro-8-methoxy-5-methyl-2,3,4,5-tetrahydro-1,5-benzoxazepine-9-carboxylic acid;
7-chloro-8-fluoro-5-methyl-2,3,4,5-tetrahydro-1,5-benzoxazepine-9-carboxylic acid;
6-chloro-7-methoxy-3,4-dihydro-2H-1,4-benzoxazine-8-carboxylic acid;
7-chloro-8-fluoro-2,3,4,5-tetrahydro-1,5-benzoxazepine-9-carboxylic acid;
7-chloro-8-ethoxy-5-methyl-2,3,4,5-tetrahydro-1,5-benzoxazepine-9-carboxylic acid;
6-chloro-7-fluoro-4-(3-methylbutyl)-3,4-dihydro-2H-1,4-benzoxazine-8-carboxylic acid;
6-chloro-4-ethyl-7-fluoro-3,4-dihydro-2H-1,4-benzoxazine-8-carboxylic acid;
6-chloro-7-fluoro-4-methyl-3,4-dihydro-2H-1,4-benzothiazine-8-carboxylic acid;
6-chloro-4-(cyclopropylmethyl)-7-fluoro-3,4-dihydro-2H-1,4-benzoxazine-8-carboxylic acid;
6-chloro-7-fluoro-4-propyl-3,4-dihydro-2H-1,4-benzoxazine-8-carboxylic acid;
4-benzyl-6-chloro-7-fluoro-3,4-dihydro-2H-1,4-benzoxazine-8-carboxylic acid;
6-chloro-7-fluoro-4-[3-(4-fluorophenyl)propyl]-3,4-dihydro-2H-1,4-benzoxazine-8-carboxylic acid;
6-chloro-4-methyl-7-(propan-2-yloxy)-3,4-dihydro-2H-1,4-benzoxazine-8-carboxylic acid;
6-chloro-7-fluoro-4-[2-(4-fluorophenyl)ethyl]-3,4-dihydro-2H-1,4-benzoxazine-8-carboxylic acid;
6-chloro-4-methyl-7-(methylsulfanyl)-3,4-dihydro-2H-1,4-benzoxazine-8-carboxylic acid;
6-chloro-7-fluoro-4-(3-methoxypropyl)-3,4-dihydro-2H-1,4-benzoxazine-8-carboxylic acid;
6-chloro-4-ethyl-7-methoxy-3,4-dihydro-2H-1,4-benzoxazine-8-carboxylic acid;
6-chloro-7-methoxy-4-methyl-3,4-dihydro-2H-1,4-benzothiazine-8-carboxylic acid;
6-fluoro-7-methoxy-4-methyl-3,4-dihydro-2H-1,4-benzoxazine-8-carboxylic acid;
7-methoxy-4,6-dimethyl-3,4-dihydro-2H-1,4-benzoxazine-8-carboxylic acid;
6-chloro-7-methoxy-4-(propan-2-yl)-3,4-dihydro-2H-1,4-benzoxazine-8-carboxylic acid;
6-chloro-7-methoxy-4-(2-phenylethyl)-3,4-dihydro-2H-1,4-benzoxazine-8-carboxylic acid;
6-chloro-7-methoxy-4-[2-(4-methoxyphenyl)ethyl]-3,4-dihydro-2H-1,4-benzoxazine-8-carboxylic acid;
6-chloro-7-methoxy-2-(methoxymethyl)-4-methyl-3,4-dihydro-2H-1,4-benzoxazine-8-carboxylic acid;
6-chloro-7-methoxy-4-[(thiophen-3-yl)methyl]-3,4-dihydro-2H-1,4-benzoxazine-8-carboxylic acid;
6-chloro-7-methoxy-2,2,4-trimethyl-3,4-dihydro-2H-1,4-benzoxazine-8-carboxylic acid;
6-chloro-7-fluoro-4-(2-methoxyethyl)-3,4-dihydro-2H-1,4-benzoxazine-8-carboxylic acid;
6-chloro-7-methoxy-4-methyl-3,4-dihydrospiro[1,4-benzoxazine-2,1'-cyclobutane]-8-carboxylic acid;
6-chloro-4-[3-(furan-2-yl)propyl]-7-methoxy-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(1,3-thiazol-2-yl)methyl]-3,4-dihydro-2H-1,4-benzoxazine-8-carboxylic acid;
6-chloro-5-fluoro-7-methoxy-4-methyl-3,4-dihydro-2H-1,4-benzoxazine-8-carboxylic acid;
4-benzyl-7-chloro-6-fluoro-3,4-dihydro-2H-1,4-benzoxazine-5-carboxylic acid;
6-chloro-4-(cyclobutylmethyl)-7-fluoro-3,4-dihydro-2H-1,4-benzoxazine-8-carboxylic acid;
6-chloro-7-methoxy-4-[3-(1,3-thiazol-2-yl)propyl]-3,4-dihydro-2H-1,4-benzoxazine-8-carboxylic acid;
6-chloro-4,7-dimethyl-3,4-dihydro-2H-1,4-benzoxazine-8-carboxylic acid;
6-chloro-7-methoxy-4-[(4-methoxyphenyl)methyl]-3,4-dihydro-2H-1,4-benzoxazine-8-carboxylic acid;
6-chloro-7-methoxy-4-[(1,3-oxazol-2-yl)methyl]-3,4-dihydro-2H-1,4-benzoxazine-8-carboxylic acid;
7-chloro-6-fluoro-4-(3-methylbutyl)-3,4-dihydro-2H-1,4-benzoxazine-5-carboxylic acid;
4-benzyl-6-chloro-7-methoxy-3,4-dihydro-2H-1,4-benzoxazine-8-carboxylic acid;
6-chloro-4-[2-(3,5-difluorophenyl)ethyl]-7-methoxy-3,4-dihydro-2H-1,4-benzoxazine-8-carboxylic acid;
7-chloro-6-fluoro-4-[(thiophen-3-yl)methyl]-3,4-dihydro-2H-1,4-benzoxazine-5-carboxylic acid;
4-benzyl-6-fluoro-7-methoxy-3,4-dihydro-2H-1,4-benzoxazine-8-carboxylic acid;
methyl 6-chloro-7-methoxy-3,4-dihydro-2H-1,4-benzoxazine-8-carboxylate;
ethyl 6-chloro-7-fluoro-3,4-dihydro-2H-1,4-benzoxazine-8-carboxylate;
methyl 7-chloro-6-fluoro-3,4-dihydro-2H-1,4-benzoxazine-5-carboxylate;
methyl 6-chloro-7-fluoro-3,4-dihydro-2H-1,4-benzoxazine-8-carboxylate;
7-chloro-6-fluoro-4-propyl-3,4-dihydro-2H-1,4-benzoxazine-5-carboxylic acid;
7-chloro-6-fluoro-4-methyl-1,2,3,4-tetrahydro-5-quinoxalinecarboxylic acid;
7-chloro-6-fluoro-4-[(p-methoxyphenyl)methyl]-3,4-dihydro-2H-1,4-benzoxazine-5-carboxylic acid;
1-benzyl-7-chloro-6-fluoro-4-methyl-1,2,3,4-tetrahydro-5-quinoxalinecarboxylic acid;
7-chloro-6-fluoro-4-[(p-tolyl)methyl]-3,4-dihydro-2H-1,4-benzoxazine-5-carboxylic acid;
7-chloro-6-fluoro-4-[(1-naphthyl)methyl]-3,4-dihydro-2H-1,4-benzoxazine-5-carboxylic acid;
7-chloro-6-fluoro-4-[(o-tolyl)methyl]-3,4-dihydro-2H-1,4-benzoxazine-5-carboxylic acid; and
4-benzyl-6,7-dichloro-3,4-dihydro-2H-1,4-benzoxazine-8-carboxylic acid.

9. A composition comprising the compound according to any one of claims 1 to 8 and a pharmaceutically acceptable carrier.

10. The compound according to any one of claims 1 to 8 or composition according to claim 9 for use as a medicament.

11. The compound according to any one of claims 1 to 8 or composition according to claim 9 for use in the treatment of an indication selected from the group consisting of myasthenia gravis, autoimmune myasthenia gravis, congenital myasthenic syndrome, seronegative myasthenia gravis, muscle specific kinase myasthenia gravis (MuSK-MG), Lambert-Eaton Syndrome, critical illness myopathy, amyotrophic lateral sclerosis (ALS), spinal muscular atrophy (SMA), critical illness myopathy (CIM), Charcot-Marie Tooth disease, diabetic polyneuropathy, periodic paralysis, hypokalemic periodic paralysis, hyperkalemic periodic paralysis, myotubular myopathy, Duchenne muscular dystrophy, Guillain-Barré syndrome, poliomyelitis, post-polio syndrome, chronic fatigue syndrome, critical illness polyneuropathy, metabolic myopathy, Kennedy's disorder, multiple sclerosis and multifocal motor neuropathy.

12. The compound according to any one of claims 1 to 8 or composition according to claim 9 for use in the symptomatic treatment of sarcopenia.

13. The compound according to any one of claims 1 to 8 or composition according to claim 9 for use in reversing and/or ameliorating a neuromuscular blockade.

14. A compound of Formula (I): 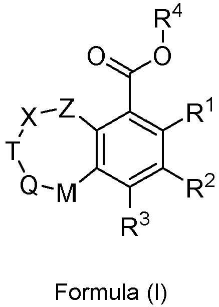 wherein:
- M is CR⁵R⁶, NR⁷, O or S;
- Q is CR⁵R⁶, NR⁸, O or S;
- T is CR⁵R⁶, NR⁸, O or S;
- X is absent, CR⁵R⁶, NR⁸, O or S;
- Z is CR⁵R⁶, NR⁹, O or S;
- two or more of M, Q, T, X and Z are NR⁷, NR⁸, NR⁹, O or S;
- R' is selected from the group consisting of H; F; Cl; Br; C₁₋₃ alkyl optionally substituted with one or more, identical or different, substituents R¹⁰; C₂₋₃ alkenyl optionally substituted with one or more, identical or different, substituents R¹⁰; -OC₁₋₃ alkyl optionally substituted with one or more, identical or different, substituents R¹⁰; -OC₃₋₅ cycloalkyl optionally substituted with one or more, identical or different, substituents R¹⁰; - SC₁₋₃ alkyl optionally substituted with one or more, identical or different, substituents R¹⁰ and -SC₃₋₅ cycloalkyl optionally substituted with one or more, identical or different, substituents R¹⁰;
- R² is selected from the group consisting of H; F; Cl; Br; C₁₋₃ alkyl optionally substituted with one or more, identical or different, substituents R¹⁰ and OC₁₋₃ alkyl optionally substituted with one or more, identical or different, substituents R¹⁰;
- R³ is selected from the group consisting of H, F and Cl;
- R⁴ is selected from the group consisting of H; C₁₋₅ alkyl optionally substituted with one or more, identical or different, substituents R¹⁰; C₂₋₅ alkenyl optionally substituted with one or more, identical or different, substituents R¹⁰; C₂₋₅ alkynyl optionally substituted with one or more, identical or different, substituents R¹⁰; C₃₋₆ cycloalkyl optionally substituted with one or more, identical or different, substituents R¹⁰; phenyl optionally substituted with one or more, identical or different, substituents R¹¹; and benzyl optionally substituted with one or more, identical or different, substituents R¹¹;
- R⁵ is independently selected from the group consisting of H, deuterium, F and C₁₋₅ alkyl optionally substituted with one or more, identical or different, substituents R¹⁰;
- R⁶ is independently selected from the group consisting of H, deuterium, F and C₁₋₅ alkyl optionally substituted with one or more, identical or different, substituents R¹⁰;
- R⁷ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with C₅ heteroaryl optionally substituted with one or more, identical or different, substituents R¹¹; and C₃₋₅ cycloalkyl optionally substituted with one or more, identical or different, substituents R¹⁰;
- R⁸ is independently selected from benzyl optionally substituted with one or more, identical or different, substituents R¹¹;
- R⁹ is independently selected from the group consisting of H, C₁₋₅ alkyl optionally substituted with one or more, identical or different, substituents R¹⁰; C₁₋₃ alkyl substituted with C₃₋₅ cycloalkyl optionally substituted with one or more, identical or different, substituents R¹⁰; C₁₋₃ alkyl substituted with phenyl optionally substituted with one or more, identical or different, substituents R¹¹; C₁₋₃ alkyl substituted with naphthyl optionally substituted with one or more, identical or different, substituents R¹¹; C₁₋₃ alkyl substituted with a 5- to 10-membered heteroaryl optionally substituted with one or more, identical or different, substituents R¹¹; and C₃₋₅ cycloalkyl optionally substituted with one or more, identical or different, substituents R¹⁰;
- R¹⁰ is independently selected from the group consisting of H, deuterium, F, Cl and -OC₁₋₃ alkyl; and
- R¹¹ is independently selected from the group consisting of deuterium, methoxy, -OCF₃, Me, CF₃, CF₂Cl, CF₂H, CFH₂, CD₃, cyclopropyl, NH₂, -NHAc, -C(=O)-NH₂, nitro, cyano, CI, Br, I, and F;
when R⁵ and R⁶ are individually C₁₋₅ alkyl optionally substituted with one or more, identical or different, substituents R¹⁰, then R⁵ and R⁶ are optionally joined together to form a ring;
or a pharmaceutically acceptable salt, hydrate, polymorph, tautomer, or solvate thereof
for use in treating, ameliorating and/or preventing a neuromuscular disorder, and/or for use in reversing and/or ameliorating a neuromuscular blockade.

15. The compound for use according to claim 14, wherein the compound is selected from the group consisting of:
7-chloro-2,3-dihydro-1,4-benzodioxine-5-carboxylic acid;
7-chloro-6-methoxy-2,3-dihydro-1,4-benzodioxine-5-carboxylic acid;
7-chloro-6-methyl-2,3-dihydro-1,4-benzodioxine-5-carboxylic acid;
7,8-dichloro-6-methyl-2,3-dihydro-1,4-benzodioxine-5-carboxylic acid;
6-chloro-7-methoxy-4-methyl-3,4-dihydro-2H-1,4-benzoxazine-8-carboxylic acid;
7-chloro-6-fluoro-4-methyl-3,4-dihydro-2H-1,4-benzoxazine-5-carboxylic acid;
7-chloro-6-fluoro-4-methyl-3,4-dihydro-2H-1,4-benzoxazine-8-carboxylic acid;
5-chloro-7-fluoro-3,4-dihydro-2H-1,4-benzoxazine-8-carboxylic acid;
6-chloro-7-fluoro-3,4-dihydro-2H-1,4-benzoxazine-8-carboxylic acid;
7-chloro-6-methoxy-4-methyl-3,4-dihydro-2H-1,4-benzoxazine-8-carboxylic acid;
6-chloro-7-fluoro-4-methyl-3,4-dihydro-2H-1,4-benzoxazine-8-carboxylic acid;
7-chloro-6-fluoro-1,4-dimethyl-1,2,3,4-tetrahydroquinoxaline-5-carboxylic acid;
6-chloro-7-fluoro-2,2,4-trimethyl-3,4-dihydro-2H-1,4-benzoxazine-8-carboxylic acid;
7-chloro-6-methoxy-4-methyl-3,4-dihydro-2H-1,4-benzoxazine-5-carboxylic acid;
7-chloro-6-fluoro-3,4-dihydro-2H-1,4-benzoxazine-5-carboxylic acid;
7-chloro-8-methoxy-5-methyl-2,3,4,5-tetrahydro-1,5-benzoxazepine-9-carboxylic acid;
7-chloro-8-fluoro-5-methyl-2,3,4,5-tetrahydro-1,5-benzoxazepine-9-carboxylic acid;
6-chloro-7-methoxy-3,4-dihydro-2H-1,4-benzoxazine-8-carboxylic acid;
7-chloro-8-fluoro-2,3,4,5-tetrahydro-1,5-benzoxazepine-9-carboxylic acid;
7-chloro-8-ethoxy-5-methyl-2,3,4,5-tetrahydro-1,5-benzoxazepine-9-carboxylic acid;
6-chloro-7-fluoro-4-(3-methylbutyl)-3,4-dihydro-2H-1,4-benzoxazine-8-carboxylic acid;
6-chloro-4-ethyl-7-fluoro-3,4-dihydro-2H-1,4-benzoxazine-8-carboxylic acid;
6-chloro-7-fluoro-4-methyl-3,4-dihydro-2H-1,4-benzothiazine-8-carboxylic acid;
6-chloro-4-(cyclopropylmethyl)-7-fluoro-3,4-dihydro-2H-1,4-benzoxazine-8-carboxylic acid;
6-chloro-7-fluoro-4-propyl-3,4-dihydro-2H-1,4-benzoxazine-8-carboxylic acid;
4-benzyl-6-chloro-7-fluoro-3,4-dihydro-2H-1,4-benzoxazine-8-carboxylic acid;
7-fluoro-6-methoxy-4-methyl-3,4-dihydro-2H-1,4-benzoxazine-8-carboxylic acid;
6-chloro-7-fluoro-4-[3-(4-fluorophenyl)propyl]-3,4-dihydro-2H-1,4-benzoxazine-8-carboxylic acid;
6-chloro-4-methyl-7-(propan-2-yloxy)-3,4-dihydro-2H-1,4-benzoxazine-8-carboxylic acid;
6-chloro-7-fluoro-4-[2-(4-fluorophenyl)ethyl]-3,4-dihydro-2H-1,4-benzoxazine-8-carboxylic acid;
6-chloro-4-methyl-7-(methylsulfanyl)-3,4-dihydro-2H-1,4-benzoxazine-8-carboxylic acid;
6-chloro-7-fluoro-4-(3-methoxypropyl)-3,4-dihydro-2H-1,4-benzoxazine-8-carboxylic acid;
6-chloro-4-ethyl-7-methoxy-3,4-dihydro-2H-1,4-benzoxazine-8-carboxylic acid;
6-chloro-7-methoxy-4-methyl-3,4-dihydro-2H-1,4-benzothiazine-8-carboxylic acid;
6-fluoro-7-methoxy-4-methyl-3,4-dihydro-2H-1,4-benzoxazine-8-carboxylic acid;
7-methoxy-4,6-dimethyl-3,4-dihydro-2H-1,4-benzoxazine-8-carboxylic acid;
6-chloro-7-methoxy-4-(propan-2-yl)-3,4-dihydro-2H-1,4-benzoxazine-8-carboxylic acid;
6-chloro-7-methoxy-4-(2-phenylethyl)-3,4-dihydro-2H-1,4-benzoxazine-8-carboxylic acid;
6-chloro-7-methoxy-4-[2-(4-methoxyphenyl)ethyl]-3,4-dihydro-2H-1,4-benzoxazine-8-carboxylic acid;
6-chloro-7-methoxy-2-(methoxymethyl)-4-methyl-3,4-dihydro-2H-1,4-benzoxazine-8-carboxylic acid;
6-chloro-7-methoxy-4-[(thiophen-3-yl)methyl]-3,4-dihydro-2H-1,4-benzoxazine-8-carboxylic acid;
6-chloro-7-methoxy-2,2,4-trimethyl-3,4-dihydro-2H-1,4-benzoxazine-8-carboxylic acid;
6-chloro-7-methoxy-2-(methoxymethyl)-4-methyl-3,4-dihydro-2H-1,4-benzoxazine-5-carboxylic acid;
6-chloro-7-fluoro-4-(2-methoxyethyl)-3,4-dihydro-2H-1,4-benzoxazine-8-carboxylic acid;
6-chloro-7-methoxy-4-methyl-3,4-dihydrospiro[1,4-benzoxazine-2,1'-cyclobutane]-8-carboxylic acid;
6-chloro-4-[3-(furan-2-yl)propyl]-7-methoxy-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(thiophen-2-yl)methyl]-3,4-dihydro-2H-1,4-benzoxazine-8-carboxylic acid;
6-chloro-7-methoxy-4-[(1,3-thiazol-2-yl)methyl]-3,4-dihydro-2H-1,4-benzoxazine-8-carboxylic acid;
6-chloro-5-fluoro-7-methoxy-4-methyl-3,4-dihydro-2H-1,4-benzoxazine-8-carboxylic acid;
4-benzyl-7-chloro-6-fluoro-3,4-dihydro-2H-1,4-benzoxazine-5-carboxylic acid;
6-chloro-4-(cyclobutylmethyl)-7-fluoro-3,4-dihydro-2H-1,4-benzoxazine-8-carboxylic acid;
6-chloro-7-methoxy-4-[3-(1,3-thiazol-2-yl)propyl]-3,4-dihydro-2H-1,4-benzoxazine-8-carboxylic acid;
6-chloro-4,7-dimethyl-3,4-dihydro-2H-1,4-benzoxazine-8-carboxylic acid;
6-chloro-7-methoxy-4-[(4-methoxyphenyl)methyl]-3,4-dihydro-2H-1,4-benzoxazine-8-carboxylic acid;
6-chloro-7-methoxy-4-[(1,3-oxazol-2-yl)methyl]-3,4-dihydro-2H-1,4-benzoxazine-8-carboxylic acid;
7-chloro-6-fluoro-4-(3-methylbutyl)-3,4-dihydro-2H-1,4-benzoxazine-5-carboxylic acid;
4-benzyl-6-chloro-7-methoxy-3,4-dihydro-2H-1,4-benzoxazine-8-carboxylic acid;
6-chloro-4-[2-(3,5-difluorophenyl)ethyl]-7-methoxy-3,4-dihydro-2H-1,4-benzoxazine-8-carboxylic acid;
7-chloro-6-fluoro-4-[(thiophen-3-yl)methyl]-3,4-dihydro-2H-1,4-benzoxazine-5-carboxylic acid;
4-benzyl-6-fluoro-7-methoxy-3,4-dihydro-2H-1,4-benzoxazine-8-carboxylic acid;
methyl 6-chloro-7-methoxy-3,4-dihydro-2H-1,4-benzoxazine-8-carboxylate;
ethyl 6-chloro-7-fluoro-3,4-dihydro-2H-1,4-benzoxazine-8-carboxylate;
methyl 7-chloro-6-fluoro-3,4-dihydro-2H-1,4-benzoxazine-5-carboxylate;
methyl 6-chloro-7-fluoro-3,4-dihydro-2H-1,4-benzoxazine-8-carboxylate;
7-chloro-6-fluoro-4-propyl-3,4-dihydro-2H-1,4-benzoxazine-5-carboxylic acid;
7-chloro-6-fluoro-4-methyl-1,2,3,4-tetrahydro-5-quinoxalinecarboxylic acid;
7-chloro-6-fluoro-4-[(p-methoxyphenyl)methyl]-3,4-dihydro-2H-1,4-benzoxazine-5-carboxylic acid;
1-benzyl-7-chloro-6-fluoro-4-methyl-1,2,3,4-tetrahydro-5-quinoxalinecarboxylic acid;
7-chloro-6-fluoro-4-[(p-tolyl)methyl]-3,4-dihydro-2H-1,4-benzoxazine-5-carboxylic acid;
7-chloro-6-fluoro-4-[(1-naphthyl)methyl]-3,4-dihydro-2H-1,4-benzoxazine-5-carboxylic acid;
7-chloro-6-fluoro-4-[(o-tolyl)methyl]-3,4-dihydro-2H-1,4-benzoxazine-5-carboxylic acid; and
4-benzyl-6,7-dichloro-3,4-dihydro-2H-1,4-benzoxazine-8-carboxylic acid.

## Patentansprüche

1. Verbindung der Formel (I): 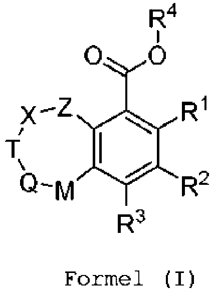 wobei:
- M CR⁵R⁶, NR⁷, O oder S ist;
- Q CR⁵R⁶, NR⁸, O oder S ist;
- T CR⁵R⁶, NR⁸, O oder S ist;
- X abwesend, CR⁵R⁶, NR⁸, O oder S ist;
- Z CR⁵R⁶, NR⁹, O oder S ist;
- zwei oder mehr von M, Q, T, X und Z NR⁷, NR⁸, NR⁹, O oder S sind;
- R¹ ausgewählt ist aus der Gruppe bestehend aus F; Cl; Br; C₁₋₃-Alkyl, optional substituiert mit einem oder mehreren gleichen oder unterschiedlichen Substituenten R¹⁰; C₂₋₃-Alkenyl, optional substituiert mit einem oder mehreren gleichen oder unterschiedlichen Substituenten R¹⁰; -OC₁₋₃-Alkyl, optional substituiert mit einem oder mehreren gleichen oder unterschiedlichen Substituenten R¹⁰; -OC₃₋₅-Cycloalkyl, optional substituiert mit einem oder mehreren gleichen oder unterschiedlichen Substituenten R¹⁰; -SC₁₋₃-Alkyl, optional substituiert mit einem oder mehreren gleichen oder unterschiedlichen Substituenten R¹⁰, und -SC₃₋₅-Cycloalkyl, optional substituiert mit einem oder mehreren gleichen oder unterschiedlichen Substituenten R¹⁰;
- R² ausgewählt ist aus der Gruppe bestehend aus H; F; Cl; Br; und C₁₋₃-Alkyl, optional substituiert mit einem oder mehreren gleichen oder unterschiedlichen Substituenten R¹⁰;
- R³ ausgewählt ist aus der Gruppe bestehend aus H, F und Cl;
- R⁴ ausgewählt ist aus der Gruppe bestehend aus H; C₁₋₅-Alkyl, optional substituiert mit einem oder mehreren gleichen oder unterschiedlichen Substituenten R¹⁰; C₂₋₅-Alkenyl, optional substituiert mit einem oder mehreren gleichen oder unterschiedlichen Substituenten R¹⁰; C₂₋₅-Alkinyl, optional substituiert mit einem oder mehreren gleichen oder unterschiedlichen Substituenten R¹⁰; C₃₋₆-Cycloalkyl, optional substituiert mit einem oder mehreren gleichen oder unterschiedlichen Substituenten R¹⁰; Phenyl, optional substituiert mit einem oder mehreren gleichen oder unterschiedlichen Substituenten R¹¹; und Benzyl, optional substituiert mit einem oder mehreren gleichen oder unterschiedlichen Substituenten R¹¹;
- R⁵ unabhängig ausgewählt ist aus der Gruppe bestehend aus H, Deuterium, F und C₁₋₅-Alkyl, optional substituiert mit einem oder mehreren gleichen oder unterschiedlichen Substituenten R¹⁰;
- R⁶ unabhängig ausgewählt ist aus der Gruppe bestehend aus H, Deuterium, F und C₁₋₅-Alkyl, optional substituiert mit einem oder mehreren gleichen oder unterschiedlichen Substituenten R¹⁰;
- R⁷ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit C₅-Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R⁸ unabhängig ausgewählt ist aus Benzyl, optional substituiert mit einem oder mehreren gleichen oder unterschiedlichen Substituenten R¹¹;
- R⁹ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit Naphthyl, optional substituiert mit einem oder mehreren gleichen oder unterschiedlichen Substituenten R¹¹; C₁₋₃-Alkyl, substituiert mit einem 5- bis 10-gliedrigen Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R¹⁰ unabhängig ausgewählt ist aus der Gruppe bestehend aus H, Deuterium, F, Cl und -OC₁₋₃-Alkyl; und
- R¹¹ unabhängig ausgewählt ist aus der Gruppe bestehend aus Deuterium, Methoxy, -OCF₃, Me, CF₃, CF₂Cl, CF₂H, CFH₂, CD₃, Cyclopropyl, NH₂, -NHAc, -C(=O)-NH₂, Nitro, Cyano, Cl, Br, I und F;
wenn R⁵ und R⁶ jeweils C₁₋₅-Alkyl, optional substituiert mit einem oder mehreren gleichen oder unterschiedlichen Substituenten R¹⁰, sind, dann sind R⁵ und R⁶ optional verbunden, um einen Ring auszubilden;
oder ein pharmazeutisch verträgliches Salz, Hydrat, Polymorph, Tautomer oder Solvat davon, mit der Maßgabe, dass:
- nur eines von M, Q, T, X und Z O ist; und
- wenn M O ist, Q und T CH₂ sind, X abwesend ist, Z NH ist, R¹ Cl ist, R² H ist und R³ H ist, R⁴ dann nicht H ist.

2. Verbindung nach Anspruch 1, wobei die Verbindung von Formel (II) ist: 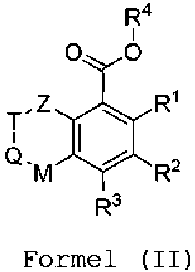
- M NR⁷, O oder S ist;
- Q CR⁵R⁶ ist;
- T CR⁵R⁶ ist;
- Z NR⁹, O oder S ist;
- R¹ ausgewählt ist aus der Gruppe bestehend aus F; Cl; Br; C₁₋₃-Alkyl, optional substituiert mit einem oder mehreren gleichen oder unterschiedlichen Substituenten R¹⁰; C₂₋₃-Alkenyl, optional substituiert mit einem oder mehreren gleichen oder unterschiedlichen Substituenten R¹⁰; -OC₁₋₃-Alkyl, optional substituiert mit einem oder mehreren gleichen oder unterschiedlichen Substituenten R¹⁰; -OC₃₋₅-Cycloalkyl, optional substituiert mit einem oder mehreren gleichen oder unterschiedlichen Substituenten R¹⁰; -SC₁₋₃-Alkyl, optional substituiert mit einem oder mehreren gleichen oder unterschiedlichen Substituenten R¹⁰, und -SC₃₋₅-Cycloalkyl, optional substituiert mit einem oder mehreren gleichen oder unterschiedlichen Substituenten R¹⁰;
- R² ausgewählt ist aus der Gruppe bestehend aus H; F; Cl; Br; und C₁₋₃-Alkyl, optional substituiert mit einem oder mehreren gleichen oder unterschiedlichen Substituenten R¹⁰;
- R³ ausgewählt ist aus der Gruppe bestehend aus H, F und Cl;
- R⁴ ausgewählt ist aus der Gruppe bestehend aus H; C₁₋₅-Alkyl, optional substituiert mit einem oder mehreren gleichen oder unterschiedlichen Substituenten R¹⁰; C₂₋₅-Alkenyl, optional substituiert mit einem oder mehreren gleichen oder unterschiedlichen Substituenten R¹⁰; C₂₋₅-Alkinyl, optional substituiert mit einem oder mehreren gleichen oder unterschiedlichen Substituenten R¹⁰; C₃₋₆-Cycloalkyl, optional substituiert mit einem oder mehreren gleichen oder unterschiedlichen Substituenten R¹⁰; Phenyl, optional substituiert mit einem oder mehreren gleichen oder unterschiedlichen Substituenten R¹¹; und Benzyl, optional substituiert mit einem oder mehreren gleichen oder unterschiedlichen Substituenten R¹¹;
- R⁵ unabhängig ausgewählt ist aus der Gruppe bestehend aus H, Deuterium, F und C₁₋₅-Alkyl, optional substituiert mit einem oder mehreren gleichen oder unterschiedlichen Substituenten R¹⁰;
- R⁶ unabhängig ausgewählt ist aus der Gruppe bestehend aus H, Deuterium, F und C₁₋₅-Alkyl, optional substituiert mit einem oder mehreren gleichen oder unterschiedlichen Substituenten R¹⁰;
- R⁷ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit C₅-Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R⁸ unabhängig ausgewählt ist aus Benzyl, optional substituiert mit einem oder mehreren gleichen oder unterschiedlichen Substituenten R¹¹;
- R⁹ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit Naphthyl, optional substituiert mit einem oder mehreren gleichen oder unterschiedlichen Substituenten R¹¹; C₁₋₃-Alkyl, substituiert mit einem 5- bis 10-gliedrigen Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R¹⁰ unabhängig ausgewählt ist aus der Gruppe bestehend aus H, Deuterium, F, Cl und -OC₁₋₃-Alkyl; und
- R¹¹ unabhängig ausgewählt ist aus der Gruppe bestehend aus Deuterium, Methoxy, -OCF₃, Me, CF₃, CF₂Cl, CF₂H, CFH₂, CD₃, Cyclopropyl, NH₂, -NHAc, -C(=O)-NH₂, Nitro, Cyano, Cl, Br, I und F;
wenn R⁵ und R⁶ jeweils C₁₋₅-Alkyl, optional substituiert mit einem oder mehreren gleichen oder unterschiedlichen Substituenten R¹⁰, sind, dann sind R⁵ und R⁶ optional verbunden, um einen Ring auszubilden;
oder ein pharmazeutisch verträgliches Salz, Hydrat, Polymorph, Tautomer oder Solvat davon, mit der Maßgabe, dass:
- nur eines von M und Z O ist; und
- wenn M O ist, Q und T CH₂ sind, Z NH ist, R¹ Cl ist, R² H ist und R³ H ist, R⁴ dann nicht H ist.

3. Verbindung nach Anspruch 1, wobei die Verbindung ausgewählt ist aus der Gruppe bestehend aus Formel (III), Formel (IV), Formel (V), Formel (VI) und Formel (VII): 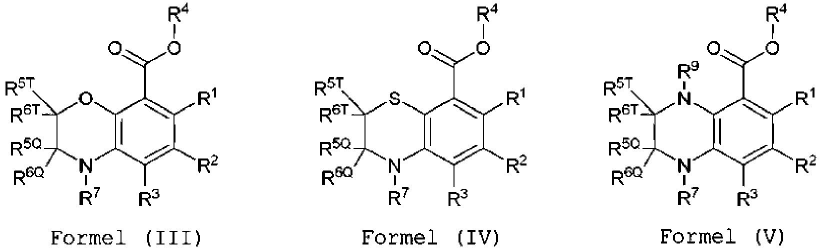 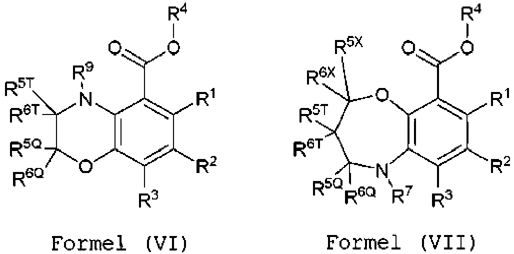 wobei:
- R¹ ausgewählt ist aus der Gruppe bestehend aus F; Cl; Br; C₁₋₃-Alkyl, optional substituiert mit einem oder mehreren gleichen oder unterschiedlichen Substituenten R¹⁰; C₂₋₃-Alkenyl, optional substituiert mit einem oder mehreren gleichen oder unterschiedlichen Substituenten R¹⁰; -OC₁₋₃-Alkyl, optional substituiert mit einem oder mehreren gleichen oder unterschiedlichen Substituenten R¹⁰; -OC₃₋₅-Cycloalkyl, optional substituiert mit einem oder mehreren gleichen oder unterschiedlichen Substituenten R¹⁰; -SC₁₋₃-Alkyl, optional substituiert mit einem oder mehreren gleichen oder unterschiedlichen Substituenten R¹⁰, und -SC₃₋₅-Cycloalkyl, optional substituiert mit einem oder mehreren gleichen oder unterschiedlichen Substituenten R¹⁰;
- R² ausgewählt ist aus der Gruppe bestehend aus H; F; Cl; Br; und C₁₋₃-Alkyl, optional substituiert mit einem oder mehreren gleichen oder unterschiedlichen Substituenten R¹⁰;
- R³ ausgewählt ist aus der Gruppe bestehend aus H, F und Cl;
- R⁴ ausgewählt ist aus der Gruppe bestehend aus H; C₁₋₅-Alkyl, optional substituiert mit einem oder mehreren gleichen oder unterschiedlichen Substituenten R¹⁰; C₂₋₅-Alkenyl, optional substituiert mit einem oder mehreren gleichen oder unterschiedlichen Substituenten R¹⁰; C₂₋₅-Alkinyl, optional substituiert mit einem oder mehreren gleichen oder unterschiedlichen Substituenten R¹⁰; C₃₋₆-Cycloalkyl, optional substituiert mit einem oder mehreren gleichen oder unterschiedlichen Substituenten R¹⁰; Phenyl, optional substituiert mit einem oder mehreren gleichen oder unterschiedlichen Substituenten R¹¹; und Benzyl, optional substituiert mit einem oder mehreren gleichen oder unterschiedlichen Substituenten R¹¹;
- R^{5Q}, R^{5T} und R^{5X} unabhängig ausgewählt sind aus der Gruppe bestehend aus H, Deuterium, F und C₁₋₅-Alkyl, optional substituiert mit einem oder mehreren gleichen oder unterschiedlichen Substituenten R¹⁰;
- R^{6Q}, R^{6T} und R^{6X} unabhängig ausgewählt sind aus der Gruppe bestehend aus H, Deuterium, F und C₁₋₅-Alkyl, optional substituiert mit einem oder mehreren gleichen oder unterschiedlichen Substituenten R¹⁰;
- R⁷ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit C₅-Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R⁹ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit Naphthyl, optional substituiert mit einem oder mehreren gleichen oder unterschiedlichen Substituenten R¹¹; C₁₋₃-Alkyl, substituiert mit einem 5- bis 10-gliedrigen Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R¹⁰ unabhängig ausgewählt ist aus der Gruppe bestehend aus H, Deuterium, F, Cl und -OC₁₋₃-Alkyl; und
- R¹¹ unabhängig ausgewählt ist aus der Gruppe bestehend aus Deuterium, Methoxy, -OCF₃, Me, CF₃, CF₂Cl, CF₂H, CFH₂, CD₃, Cyclopropyl, NH₂, -NHAc, -C(=O)-NH₂, Nitro, Cyano, Cl, Br, I und F;
wenn R^{5Q} und R^{6Q} jeweils C₁₋₅-Alkyl, optional substituiert mit einem oder mehreren gleichen oder unterschiedlichen Substituenten R¹⁰, sind, dann sind R^{5Q} und R^{6Q} optional verbunden, um einen Ring auszubilden;
oder, wenn R^{5T} und R^{6T} jeweils C₁₋₅-Alkyl, optional substituiert mit einem oder mehreren gleichen oder unterschiedlichen Substituenten R¹⁰, sind, dann sind R^{5T} und R^{6T} optional verbunden um einen Ring auszubilden;
oder, wenn R^{5X} und R^{6X} jeweils C₁₋₅-Alkyl, optional substituiert mit einem oder mehreren gleichen oder unterschiedlichen Substituenten R¹⁰, sind, dann sind R^{5X} und R^{6X} optional verbunden, um einen Ring auszubilden;
oder ein pharmazeutisch verträgliches Salz, Hydrat, Polymorph, Tautomer oder Solvat davon.

4. Verbindung nach einem der Ansprüche 1 bis 3, wobei R¹ ausgewählt ist aus der Gruppe bestehend aus F, Cl, Me, OMe, OEt, OCHMe₂ und SMe.

5. Verbindung nach einem der Ansprüche 1 bis 4, wobei R² ausgewählt ist aus der Gruppe bestehend aus H, F, Cl und Me.

6. Verbindung nach einem der Ansprüche 1 bis 5, wobei R⁴ H ist.

7. Verbindung nach einem der Ansprüche 1 bis 6, wobei R¹ OMe ist und R² F oder Cl ist.

8. Verbindung nach einem der Ansprüche 1 bis 7, wobei die Verbindung ausgewählt ist aus der Gruppe bestehend aus:
6-Chlor-7-methoxy-4-methyl-3,4-dihydro-2H-1,4-benzoxazin-8-Carbonsäure;
7-Chlor-6-fluor-4-methyl-3,4-dihydro-2H-1,4-benzoxazin-5-Carbonsäure;
7-Chlor-6-fluor-4-methyl-3,4-dihydro-2H-1,4-benzoxazin-8-Carbonsäure;
5-Chlor-7-fluor-3,4-dihydro-2H-1,4-benzoxazin-8-Carbonsäure;
6-Chlor-7-fluor-3,4-dihydro-2H-1,4-benzoxazin-8-Carbonsäure;
6-Chlor-7-fluor-4-methyl-3,4-dihydro-2H-1,4-benzoxazin-8-Carbonsäure;
7-Chlor-6-fluor-1,4-dimethyl-1,2,3,4-tetrahydrochinoxalin-5-Carbonsäure;
6-Chlor-7-fluor-2,2,4-trimethyl-3,4-dihydro-2H-1,4-benzoxazin-8-Carbonsäure;
7-Chlor-6-methoxy-4-methyl-3,4-dihydro-2H-1,4-benzoxazin-5-Carbonsäure;
7-Chlor-6-fluor-3,4-dihydro-2H-1,4-benzoxazin-5-Carbonsäure;
7-Chlor-8-methoxy-5-methyl-2,3,4,5-tetrahydro-1,5-benzoxazepin-9-Carbonsäure;
7-Chlor-8-fluor-5-methyl-2,3,4,5-tetrahydro-1,5-benzoxazepin-9-Carbonsäure;
6-Chlor-7-methoxy-3,4-dihydro-2H-1,4-benzoxazin-8-Carbonsäure;
7-Chlor-8-fluor-2,3,4,5-tetrahydro-1,5-benzoxazepin-9-Carbonsäure;
7-Chlor-8-ethoxy-5-methyl-2,3,4,5-tetrahydro-1,5-benzoxazepin-9-Carbonsäure;
6-Chlor-7-fluor-4-(3-methylbutyl)-3,4-dihydro-2H-1,4-benzoxazin-8-Carbonsäure;
6-Chlor-4-ethyl-7-fluor-3,4-dihydro-2H-1,4-benzoxazin-8-Carbonsäure;
6-Chlor-7-fluor-4-methyl-3,4-dihydro-2H-1,4-benzothiazin-8-Carbonsäure;
6-Chlor-4-(cyclopropylmethyl)-7-fluor-3,4-dihydro-2H-1,4-benzoxazin-8-Carbonsäure;
6-Chlor-7-fluor-4-propyl-3,4-dihydro-2H-1,4-benzoxazin-8-Carbonsäure;
4-Benzyl-6-chlor-7-fluor-3,4-dihydro-2H-1,4-benzoxazin-8-Carbonsäure;
6-Chlor-7-fluor-4-[3-(4-fluorphenyl)propyl]-3,4-dihydro-2H-1,4-benzoxazin-8-Carbonsäure;
6-Chlor-4-methyl-7-(propan-2-yloxy)-3,4-dihydro-2H-1,4-benzoxazin-8-Carbonsäure;
6-Chlor-7-fluor-4-[2-(4-fluorphenyl)ethyl]-3,4-dihydro-2H-1,4-benzoxazin-8-Carbonsäure;
6-Chlor-4-methyl-7-(methylsulfanyl)-3,4-dihydro-2H-1,4-benzoxazin-8-Carbonsäure;
6-Chlor-7-fluor-4-(3-methoxypropyl)-3,4-dihydro-2H-1,4-benzoxazin-8-Carbonsäure;
6-Chlor-4-ethyl-7-methoxy-3,4-dihydro-2H-1,4-benzoxazin-8-Carbonsäure;
6-Chlor-7-methoxy-4-methyl-3,4-dihydro-2H-1,4-benzothiazin-8-Carbonsäure;
6-Fluor-7-methoxy-4-methyl-3,4-dihydro-2H-1,4-benzoxazin-8-Carbonsäure;
7-Methoxy-4,6-dimethyl-3,4-dihydro-2H-1,4-benzoxazin-8-Carbonsäure;
6-Chlor-7-methoxy-4-(propan-2-yl)-3,4-dihydro-2H-1,4-benzoxazin-8-Carbonsäure;
6-Chlor-7-methoxy-4-(2-phenylethyl)-3,4-dihydro-2H-1,4-benzoxazin-8-Carbonsäure;
6-Chlor-7-methoxy-4-[2-(4-methoxyphenyl)ethyl]-3,4-dihydro-2H-1,4-benzoxazin-8-Carbonsäure;
6-Chlor-7-methoxy-2-(methoxymethyl)-4-methyl-3,4-dihydro-2H-1,4-benzoxazin-8-Carbonsäure;
6-Chlor-7-methoxy-4-[(thiophen-3-yl)methyl]-3,4-dihydro-2H-1,4-benzoxazin-8-Carbonsäure;
6-Chlor-7-methoxy-2,2,4-trimethyl-3,4-dihydro-2H-1,4-benzoxazin-8-Carbonsäure;
6-Chlor-7-fluor-4-(2-methoxyethyl)-3,4-dihydro-2H-1,4-benzoxazin-8-Carbonsäure;
6-Chlor-7-methoxy-4-methyl-3,4-dihydrospiro[1,4-benzoxazin-2,1'-cyclobutan]-8-Carbonsäure;
6-Chlor-4-[3-(furan-2-yl)propyl]-7-methoxy-3,4-dihydro-2H-1,4-benzoxazin-8-Carbonsäure;
6-Chlor-7-methoxy-4-[(thiophen-2-yl)methyl]-3,4-dihydro-2H-1,4-benzoxazin-8-Carbonsäure;
6-Chlor-7-methoxy-4-[(1,3-thiazol-2-yl)methyl]-3,4-dihydro-2H-1,4-benzoxazin-8-Carbonsäure;
6-Chlor-5-fluor-7-methoxy-4-methyl-3,4-dihydro-2H-1,4-benzoxazin-8-Carbonsäure;
4-Benzyl-7-chlor-6-fluor-3,4-dihydro-2H-1,4-benzoxazin-5-Carbonsäure;
6-Chlor-4-(cyclobutylmethyl)-7-fluor-3,4-dihydro-2H-1,4-benzoxazin-8-Carbonsäure;
6-Chlor-7-methoxy-4-[3-(1,3-thiazol-2-yl)propyl]-3,4-dihydro-2H-1,4-benzoxazin-8-Carbonsäure;
6-Chlor-4,7-dimethyl-3,4-dihydro-2H-1,4-benzoxazin-8-Carbonsäure;
6-Chlor-7-methoxy-4-[(4-methoxyphenyl)methyl]-3,4-dihydro-2H-1,4-benzoxazin-8-Carbonsäure;
6-Chlor-7-methoxy-4-[(1,3-oxazol-2-yl)methyl]-3,4-dihydro-2H-1,4-benzoxazin-8-Carbonsäure;
7-Chlor-6-fluor-4-(3-methylbutyl)-3,4-dihydro-2H-1,4-benzoxazin-5-Carbonsäure;
4-Benzyl-6-chlor-7-methoxy-3,4-dihydro-2H-1,4-benzoxazin-8-Carbonsäure;
6-Chlor-4-[2-(3,5-difluorphenyl)ethyl]-7-methoxy-3,4-dihydro-2H-1,4-benzoxazin-8-Carbonsäure;
7-Chlor-6-fluor-4-[(thiophen-3-yl)methyl]-3,4-dihydro-2H-1,4-benzoxazin-5-Carbonsäure;
4-Benzyl-6-fluor-7-methoxy-3,4-dihydro-2H-1,4-benzoxazin-8-Carbonsäure;
Methyl-6-chlor-7-methoxy-3,4-dihydro-2H-1,4-benzoxazin-8-Carboxylat;
Ethyl-6-chlor-7-fluor-3,4-dihydro-2H-1,4-benzoxazin-8-Carboxylat;
Methyl-7-chlor-6-fluor-3,4-dihydro-2H-1,4-benzoxazin-5-Carboxylat;
Methyl-6-chlor-7-fluor-3,4-dihydro-2H-1,4-benzoxazin-8-Carboxylat;
7-Chlor-6-fluor-4-propyl-3,4-dihydro-2H-1,4-benzoxazin-5-Carbonsäure;
7-Chlor-6-fluor-4-methyl-1,2,3,4-tetrahydro-5-Chinoxalincarbonsäure;
7-Chlor-6-fluor-4-[(p-methoxyphenyl)methyl]-3,4-dihydro-2H-1,4-benzoxazin-5-Carbonsäure;
1-Benzyl-7-chlor-6-fluor-4-methyl-1,2,3,4-tetrahydro-5-Chinoxalincarbonsäure;
7-Chlor-6-fluor-4-[(p-tolyl)methyl]-3,4-dihydro-2H-1,4-benzoxazin-5-Carbonsäure;
7-Chlor-6-fluor-4-[(1-naphthyl)methyl]-3,4-dihydro-2H-1,4-benzoxazin-5-Carbonsäure;
7-Chlor-6-fluor-4-[(o-tolyl)methyl]-3,4-dihydro-2H-1,4-benzoxazin-5-Carbonsäure; und
4-Benzyl-6,7-dichlor-3,4-dihydro-2H-1,4-benzoxazin-8-Carbonsäure.

9. Zusammensetzung, umfassend die Verbindung nach einem der Ansprüche 1 bis 8 und einen pharmazeutisch verträglichen Träger.

10. Verbindung nach einem der Ansprüche 1 bis 8 oder Zusammensetzung nach Anspruch 9 zur Verwendung als ein Medikament.

11. Verbindung nach einem der Ansprüche 1 bis 8 oder Zusammensetzung nach Anspruch 9 zur Verwendung bei der Behandlung einer Indikation, die ausgewählt ist aus der Gruppe bestehend aus Myasthenia gravis, autoimmuner Myasthenia gravis, kongenitalem myasthenischem Syndrom, seronegativer Myasthenia gravis, muskelspezifischer Kinase-Myasthenia gravis (MuSK-MG), Lambert-Eaton-Syndrom, kritischer Myopathie, amyotropher Lateralsklerose (ALS), spinaler Muskelatrophie (SMA), kritischer Myopathie (CIM), Charcot-Marie-Tooth-Krankheit, diabetischer Polyneuropathie, periodischer Lähmung, hypokaliämischer periodischer Lähmung, hyperkaliämischer periodischer Lähmung, myotubulärer Myopathie, Duchenne-Muskeldystrophie, Guillain-Barré-Syndrom, Poliomyelitis, Post-Polio-Syndrom, chronischem Müdigkeitssyndrom, kritischer Polyneuropathie, metabolischer Myopathie, Kennedy-Erkrankung, Multipler Sklerose und multifokaler motorischer Neuropathie.

12. Verbindung nach einem der Ansprüche 1 bis 8 oder Zusammensetzung nach Anspruch 9 zur Verwendung bei der symptomatischen Behandlung von Sarkopenie.

13. Verbindung nach einem der Ansprüche 1 bis 8 oder Zusammensetzung nach Anspruch 9 zur Verwendung beim Rückbilden und/oder Lindern einer neuromuskulären Blockade.

14. Verbindung der Formel (I): 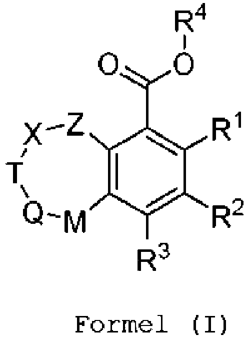 wobei:
- M CR⁵R⁶, NR⁷, O oder S ist;
- Q CR⁵R⁶, NR⁸, O oder S ist;
- T CR⁵R⁶, NR⁸, O oder S ist;
- X abwesend, CR⁵R⁶, NR⁸, O oder S ist;
- Z CR⁵R⁶, NR⁹, O oder S ist;
- zwei oder mehr von M, Q, T, X und Z NR⁷, NR⁸, NR⁹, O oder S sind;
- R¹ ausgewählt ist aus der Gruppe bestehend aus H; F; Cl; Br; C₁₋₃-Alkyl, optional substituiert mit einem oder mehreren gleichen oder unterschiedlichen Substituenten R¹⁰; C₂₋₃-Alkenyl, optional substituiert mit einem oder mehreren gleichen oder unterschiedlichen Substituenten R¹⁰; -OC₁₋₃-Alkyl, optional substituiert mit einem oder mehreren gleichen oder unterschiedlichen Substituenten R¹⁰; -OC₃₋₅-Cycloalkyl, optional substituiert mit einem oder mehreren gleichen oder unterschiedlichen Substituenten R¹⁰; -SC₁₋₃-Alkyl, optional substituiert mit einem oder mehreren gleichen oder unterschiedlichen Substituenten R¹⁰, und -SC₃₋₅-Cycloalkyl, optional substituiert mit einem oder mehreren gleichen oder unterschiedlichen Substituenten R¹⁰;
- R² ausgewählt ist aus der Gruppe bestehend aus H; F; Cl; Br; C₁₋₃-Alkyl, optional substituiert mit einem oder mehreren gleichen oder unterschiedlichen Substituenten R¹⁰ und OC₁₋₃-Alkyl, optional substituiert mit einem oder mehreren gleichen oder unterschiedlichen Substituenten R¹⁰;
- R³ ausgewählt ist aus der Gruppe bestehend aus H, F und Cl;
- R⁴ ausgewählt ist aus der Gruppe bestehend aus H; C₁₋₅-Alkyl, optional substituiert mit einem oder mehreren gleichen oder unterschiedlichen Substituenten R¹⁰; C₂₋₅-Alkenyl, optional substituiert mit einem oder mehreren gleichen oder unterschiedlichen Substituenten R¹⁰; C₂₋₅-Alkinyl, optional substituiert mit einem oder mehreren gleichen oder unterschiedlichen Substituenten R¹⁰; C₃₋₆-Cycloalkyl, optional substituiert mit einem oder mehreren gleichen oder unterschiedlichen Substituenten R¹⁰; Phenyl, optional substituiert mit einem oder mehreren gleichen oder unterschiedlichen Substituenten R¹¹; und Benzyl, optional substituiert mit einem oder mehreren gleichen oder unterschiedlichen Substituenten R¹¹;
- R⁵ unabhängig ausgewählt ist aus der Gruppe bestehend aus H, Deuterium, F und C₁₋₅-Alkyl, optional substituiert mit einem oder mehreren gleichen oder unterschiedlichen Substituenten R¹⁰;
- R⁶ unabhängig ausgewählt ist aus der Gruppe bestehend aus H, Deuterium, F und C₁₋₅-Alkyl, optional substituiert mit einem oder mehreren gleichen oder unterschiedlichen Substituenten R¹⁰;
- R⁷ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit C₅-Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R⁸ unabhängig ausgewählt ist aus Benzyl, optional substituiert mit einem oder mehreren gleichen oder unterschiedlichen Substituenten R¹¹;
- R⁹ unabhängig ausgewählt ist aus der Gruppe bestehend aus H, C₁₋₅-Alkyl, optional substituiert mit einem oder mehreren gleichen oder unterschiedlichen Substituenten R¹⁰; C₁₋₃-Alkyl, substituiert mit C₃₋₅-Cycloalkyl, optional substituiert mit einem oder mehreren gleichen oder unterschiedlichen Substituenten R¹⁰; C₁₋₃-Alkyl, substituiert mit Phenyl, optional substituiert mit einem oder mehreren gleichen oder unterschiedlichen Substituenten R¹¹; C₁₋₃-Alkyl, substituiert mit Naphthyl, optional substituiert mit einem oder mehreren gleichen oder unterschiedlichen Substituenten R¹¹; C₁₋₃-Alkyl, substituiert mit einem 5- bis 10-gliedrigen Heteroaryl, optional substituiert mit einem oder mehreren gleichen oder unterschiedlichen Substituenten R¹¹; und C₃₋₅-Cycloalkyl, optional substituiert mit einem oder mehreren gleichen oder unterschiedlichen Substituenten R¹⁰;
- R¹⁰ unabhängig ausgewählt ist aus der Gruppe bestehend aus H, Deuterium, F, Cl und -OC₁₋₃-Alkyl; und
- R¹¹ unabhängig ausgewählt ist aus der Gruppe bestehend aus Deuterium, Methoxy, -OCF₃, Me, CF₃, CF₂Cl, CF₂H, CFH₂, CD₃, Cyclopropyl, NH₂, -NHAc, -C(=O)-NH₂, Nitro, Cyano, Cl, Br, I und F;
wenn R⁵ und R⁶ jeweils C₁₋₅-Alkyl, optional substituiert mit einem oder mehreren gleichen oder unterschiedlichen Substituenten R¹⁰, sind, dann sind R⁵ und R⁶ optional verbunden, um einen Ring auszubilden;
oder ein pharmazeutisch verträgliches Salz, Hydrat, Polymorph, Tautomer oder Solvat davon
zur Verwendung beim Behandeln, Lindern und/oder Vorbeugen einer neuromuskulären Erkrankung und/oder zur Verwendung beim Rückbilden und/oder Lindern einer neuromuskulären Blockade.

15. Verbindung zur Verwendung nach Anspruch 14, wobei die Verbindung ausgewählt ist aus der Gruppe bestehend aus:
7-Chlor-2,3-dihydro-1,4-benzodioxin-5-Carbonsäure;
7-Chlor-6-methoxy-2,3-dihydro-1,4-benzodioxin-5-Carbonsäure;
7-Chlor-6-methyl-2,3-dihydro-1,4-benzodioxin-5-Carbonsäure;
7,8-Dichlor-6-methyl-2,3-dihydro-1,4-benzodioxin-5-Carbonsäure;
6-Chlor-7-methoxy-4-methyl-3,4-dihydro-2H-1,4-benzoxazin-8-Carbonsäure;
7-Chlor-6-fluor-4-methyl-3,4-dihydro-2H-1,4-benzoxazin-5-Carbonsäure;
7-Chlor-6-fluor-4-methyl-3,4-dihydro-2H-1,4-benzoxazin-8-Carbonsäure;
5-Chlor-7-fluor-3,4-dihydro-2H-1,4-benzoxazin-8-Carbonsäure;
6-Chlor-7-fluor-3,4-dihydro-2H-1,4-benzoxazin-8-Carbonsäure;
7-Chlor-6-methoxy-4-methyl-3,4-dihydro-2H-1,4-benzoxazin-8-Carbonsäure;
6-Chlor-7-fluor-4-methyl-3,4-dihydro-2H-1,4-benzoxazin-8-Carbonsäure;
7-Chlor-6-fluor-1,4-dimethyl-1,2,3,4-tetrahydrochinoxalin-5-Carbonsäure;
6-Chlor-7-fluor-2,2,4-trimethyl-3,4-dihydro-2H-1,4-benzoxazin-8-Carbonsäure;
7-Chlor-6-methoxy-4-methyl-3,4-dihydro-2H-1,4-benzoxazin-5-Carbonsäure;
7-Chlor-6-fluor-3,4-dihydro-2H-1,4-benzoxazin-5-Carbonsäure;
7-Chlor-8-methoxy-5-methyl-2,3,4,5-tetrahydro-1,5-benzoxazepin-9-Carbonsäure;
7-Chlor-8-fluor-5-methyl-2,3,4,5-tetrahydro-1,5-benzoxazepin-9-Carbonsäure;
6-Chlor-7-methoxy-3,4-dihydro-2H-1,4-benzoxazin-8-Carbonsäure;
7-Chlor-8-fluor-2,3,4,5-tetrahydro-1,5-benzoxazepin-9-Carbonsäure;
7-Chlor-8-ethoxy-5-methyl-2,3,4,5-tetrahydro-1,5-benzoxazepin-9-Carbonsäure;
6-Chlor-7-fluor-4-(3-methylbutyl)-3,4-dihydro-2H-1,4-benzoxazin-8-Carbonsäure;
6-Chlor-4-ethyl-7-fluor-3,4-dihydro-2H-1,4-benzoxazin-8-Carbonsäure;
6-Chlor-7-fluor-4-methyl-3,4-dihydro-2H-1,4-benzothiazin-8-Carbonsäure;
6-Chlor-4-(cyclopropylmethyl)-7-fluor-3,4-dihydro-2H-1,4-benzoxazin-8-Carbonsäure;
6-Chlor-7-fluor-4-propyl-3,4-dihydro-2H-1,4-benzoxazin-8-Carbonsäure;
4-Benzyl-6-chlor-7-fluor-3,4-dihydro-2H-1,4-benzoxazin-8-Carbonsäure;
7-Fluor-6-methoxy-4-methyl-3,4-dihydro-2H-1,4-benzoxazin-8-Carbonsäure;
6-Chlor-7-fluor-4-[3-(4-fluorphenyl)propyl]-3,4-dihydro-2H-1,4-benzoxazin-8-Carbonsäure;
6-Chlor-4-methyl-7-(propan-2-yloxy)-3,4-dihydro-2H-1,4-benzoxazin-8-Carbonsäure;
6-Chlor-7-fluor-4-[2-(4-fluorphenyl)ethyl]-3,4-dihydro-2H-1,4-benzoxazin-8-Carbonsäure;
6-Chlor-4-methyl-7-(methylsulfanyl)-3,4-dihydro-2H-1,4-benzoxazin-8-Carbonsäure;
6-Chlor-7-fluor-4-(3-methoxypropyl)-3,4-dihydro-2H-1,4-benzoxazin-8-Carbonsäure;
6-Chlor-4-ethyl-7-methoxy-3,4-dihydro-2H-1,4-benzoxazin-8-Carbonsäure;
6-Chlor-7-methoxy-4-methyl-3,4-dihydro-2H-1,4-benzothiazin-8-Carbonsäure;
6-Fluor-7-methoxy-4-methyl-3,4-dihydro-2H-1,4-benzoxazin-8-Carbonsäure;
7-Methoxy-4,6-dimethyl-3,4-dihydro-2H-1,4-benzoxazin-8-Carbonsäure;
6-Chlor-7-methoxy-4-(propan-2-yl)-3,4-dihydro-2H-1,4-benzoxazin-8-Carbonsäure;
6-Chlor-7-methoxy-4-(2-phenylethyl)-3,4-dihydro-2H-1,4-benzoxazin-8-Carbonsäure;
6-Chlor-7-methoxy-4-[2-(4-methoxyphenyl)ethyl]-3,4-dihydro-2H-1,4-benzoxazin-8-Carbonsäure;
6-Chlor-7-methoxy-2-(methoxymethyl)-4-methyl-3,4-dihydro-2H-1,4-benzoxazin-8-Carbonsäure;
6-Chlor-7-methoxy-4-[(thiophen-3-yl)methyl]-3,4-dihydro-2H-1,4-benzoxazin-8-Carbonsäure;
6-Chlor-7-methoxy-2,2,4-trimethyl-3,4-dihydro-2H-1,4-benzoxazin-8-Carbonsäure;
6-Chlor-7-methoxy-2-(methoxymethyl)-4-methyl-3,4-dihydro-2H-1,4-benzoxazin-5-Carbonsäure;
6-Chlor-7-fluor-4-(2-methoxyethyl)-3,4-dihydro-2H-1,4-benzoxazin-8-Carbonsäure;
6-Chlor-7-methoxy-4-methyl-3,4-dihydrospiro[1,4-benzoxazin-2,1'-cyclobutan]-8-Carbonsäure;
6-Chlor-4-[3-(furan-2-yl)propyl]-7-methoxy-3,4-dihydro-2H-1,4-benzoxazin-8-Carbonsäure;
6-Chlor-7-methoxy-4-[(thiophen-2-yl)methyl]-3,4-dihydro-2H-1,4-benzoxazin-8-Carbonsäure;
6-Chlor-7-methoxy-4-[(thiophen-2-yl)methyl]-3,4-dihydro-2H-1,4-benzoxazin-8-Carbonsäure;
6-Chlor-7-methoxy-4-[(1,3-thiazol-2-yl)methyl]-3,4-dihydro-2H-1,4-benzoxazin-8-Carbonsäure;
5 6-Chlor-5-fluor-7-methoxy-4-methyl-3,4-dihydro-2H-1,4-benzoxazin-8-Carbonsäure;
4-Benzyl-7-chlor-6-fluor-3,4-dihydro-2H-1,4-benzoxazin-5-Carbonsäure;
6-Chlor-4-(cyclobutylmethyl)-7-fluor-3,4-dihydro-2H-1,4-benzoxazin-8-Carbonsäure;
10 6-Chlor-7-methoxy-4-[3-(1,3-thiazol-2-yl)propyl]-3,4-dihydro-2H-1,4-benzoxazin-8-Carbonsäure;
6-Chlor-4,7-dimethyl-3,4-dihydro-2H-1,4-benzoxazin-8-Carbonsäure;
6-Chlor-7-methoxy-4-[(4-methoxyphenyl)methyl]-3,4-dihydro-2H-1,4-benzoxazin-8-Carbonsäure;
6-Chlor-7-methoxy-4-[(1,3-oxazol-2-yl)methyl]-3,4-dihydro-2H-1,4-benzoxazin-8-Carbonsäure;
7-Chlor-6-fluor-4-(3-methylbutyl)-3,4-dihydro-2H-1,4-benzoxazin-5-Carbonsäure;
4-Benzyl-6-chlor-7-methoxy-3,4-dihydro-2H-1,4-benzoxazin-8-Carbonsäure;
6-Chlor-4-[2-(3,5-difluorphenyl)ethyl]-7-methoxy-3,4-dihydro-2H-1,4-benzoxazin-8-Carbonsäure;
7-Chlor-6-fluor-4-[(thiophen-3-yl)methyl]-3,4-dihydro-2H-1,4-benzoxazin-5-Carbonsäure;
4-Benzyl-6-fluor-7-methoxy-3,4-dihydro-2H-1,4-benzoxazin-8-Carbonsäure;
Methyl-6-chlor-7-methoxy-3,4-dihydro-2H-1,4-benzoxazin-8-Carboxylat;
Ethyl-6-chlor-7-fluor-3,4-dihydro-2H-1,4-benzoxazin-8-Carboxylat;
Methyl-7-chlor-6-fluor-3,4-dihydro-2H-1,4-benzoxazin-5-Carboxylat;
Methyl-6-chlor-7-fluor-3,4-dihydro-2H-1,4-benzoxazin-8-Carboxylat;
7-Chlor-6-fluor-4-propyl-3,4-dihydro-2H-1,4-benzoxazin-5-Carbonsäure;
7-Chlor-6-fluor-4-methyl-1,2,3,4-tetrahydro-5-Chinoxalincarbonsäure;
7-Chlor-6-fluor-4-[(p-methoxyphenyl)methyl]-3,4-dihydro-2H-1,4-benzoxazin-5-Carbonsäure;
1-Benzyl-7-chlor-6-fluor-4-methyl-1,2,3,4-tetrahydro-5-Chinoxalincarbonsäure;
7-Chlor-6-fluor-4-[(p-tolyl)methyl]-3,4-dihydro-2H-1,4-benzoxazin-5-Carbonsäure;
7-Chlor-6-fluor-4-[(1-naphthyl)methyl]-3,4-dihydro-2H-1,4-benzoxazin-5-Carbonsäure;
7-Chlor-6-fluor-4-[(o-tolyl)methyl]-3,4-dihydro-2H-1,4-benzoxazin-5-Carbonsäure; und
4-Benzyl-6,7-dichlor-3,4-dihydro-2H-1,4-benzoxazin-8-Carbonsäure.

## Revendications

1. Composé de Formule (I) : 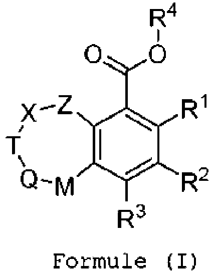 dans lequel :
- M est CR⁵R⁶, NR⁷, O ou S ;
- Q est CR⁵R⁶, NR⁸, O ou S ;
- T est CR⁵R⁶, NR⁸, O ou S ;
- X est absent, CR⁵R⁶, NR⁸, O ou S ;
- Z est CR⁵R⁶, NR⁹, O ou S ;
- deux ou plus parmi M, Q, T, X et Z sont NR⁷, NR⁸, NR⁹, O ou S ;
- R¹ est choisi dans le groupe constitué de F ; Cl ; Br ; alkyle en C₁₋₃ éventuellement substitué par un ou plusieurs substituants R¹⁰ identiques ou différents ; alcényle en C₂₋₃ éventuellement substitué par un ou plusieurs substituants R¹⁰ identiques ou différents ; -Oalkyle en C₁₋₃ éventuellement substitué par un ou plusieurs substituants R¹⁰ identiques ou différents ; -Ocycloalkyle en C₃₋₅ éventuellement substitué par un ou plusieurs substituants R¹⁰ identiques ou différents ; - Salkyle en C₁₋₃ éventuellement substitué par un ou plusieurs substituants R¹⁰ identiques ou différents et -Scycloalkyle en C₃₋₅ éventuellement substitué par un ou plusieurs substituants R¹⁰ identiques ou différents ;
- R² est choisi dans le groupe constitué de H ; F ; Cl ; Br ; et alkyle en C₁₋₃ éventuellement substitué par un ou plusieurs substituants R¹⁰ identiques ou différents ;
- R³ est choisi dans le groupe constitué de H, F et Cl ;
- R⁴ est choisi dans le groupe constitué de H ; alkyle en C₁₋₅ éventuellement substitué par un ou plusieurs substituants R¹⁰ identiques ou différents ; alcényle en C₂₋₅ éventuellement substitué par un ou plusieurs substituants R¹⁰ identiques ou différents ; alcynyle en C₂₋₅ éventuellement substitué par un ou plusieurs substituants R¹⁰ identiques ou différents ; cycloalkyle en C₃₋₆ éventuellement substitué par un ou plusieurs substituants R¹⁰ identiques ou différents ; phényle éventuellement substitué par un ou plusieurs substituants R¹¹ identiques ou différents ; et benzyle éventuellement substitué par un ou plusieurs substituants R¹¹ identiques ou différents ;
- R⁵ est choisi indépendamment dans le groupe constitué de H, deutérium, F et alkyle en C₁₋₅ éventuellement substitué par un ou plusieurs substituants R¹⁰ identiques ou différents ;
- R⁶ est choisi indépendamment dans le groupe constitué de H, deutérium, F et alkyle en C₁₋₅ éventuellement substitué par un ou plusieurs substituants R¹⁰ identiques ou différents ;
- R⁷ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par hétéroaryle en C₅ éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R⁸ est choisi indépendamment parmi benzyle éventuellement substitué par un ou plusieurs substituants R¹¹ identiques ou différents ;
- R⁹ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par naphtyle éventuellement substitué par un ou plusieurs substituants R¹¹ identiques ou différents ; alkyle en C₁₋₃ substitué par hétéroaryle à 5 à 10 chaînons éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R¹⁰ est choisi indépendamment dans le groupe constitué de H, deutérium, F, Cl et -Oalkyle en C₁₋₃ ; et
- R¹¹ est choisi indépendamment dans le groupe constitué de deutérium, méthoxy, -OCF₃, Me, CF₃, CF₂Cl, CF₂H, CFH₂, CD₃, cyclopropyle, NH₂, -NHAc, -C(=O)-NH₂, nitro, cyano, Cl, Br, I et F ;
lorsque R⁵ et R⁶ sont individuellement alkyle en C₁₋₅ éventuellement substitué par un ou plusieurs substituants R¹⁰ identiques ou différents, alors R⁵ et R⁶ sont éventuellement liés ensemble pour former un cycle ;
ou un sel, hydrate, polymorphe, tautomère ou solvate pharmaceutiquement acceptable de celui-ci à condition que :
- seul un parmi M, Q, T, X et Z est O ; et
- lorsque M est O, Q et T sont CH₂, X est absent, Z est NH, R¹ est Cl, R² est H et R³ est H, alors R⁴ n'est pas H.

2. Composé selon la revendication 1, dans lequel le composé est de Formule (II) : 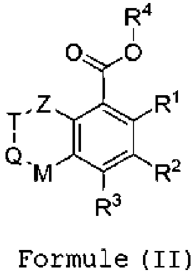
- M est NR⁷, O ou S ;
- Q est CR⁵R⁶ ;
- T est CR⁵R⁶ ;
- Z est NR⁹, O ou S ;
- R¹ est choisi dans le groupe constitué de F ; Cl ; Br ; alkyle en C₁₋₃ éventuellement substitué par un ou plusieurs substituants R¹⁰ identiques ou différents ; alcényle en C₂₋₃ éventuellement substitué par un ou plusieurs substituants R¹⁰ identiques ou différents ; -Oalkyle en C₁₋₃ éventuellement substitué par un ou plusieurs substituants R¹⁰ identiques ou différents ; -Ocycloalkyle en C₃₋₅ éventuellement substitué par un ou plusieurs substituants R¹⁰ identiques ou différents ; - Salkyle en C₁₋₃ éventuellement substitué par un ou plusieurs substituants R¹⁰ identiques ou différents et -Scycloalkyle en C₃₋₅ éventuellement substitué par un ou plusieurs substituants R¹⁰ identiques ou différents ;
- R² est choisi dans le groupe constitué de H ; F ; Cl ; Br ; et alkyle en C₁₋₃ éventuellement substitué par un ou plusieurs substituants R¹⁰ identiques ou différents ;
- R³ est choisi dans le groupe constitué de H, F et Cl ;
- R⁴ est choisi dans le groupe constitué de H ; alkyle en C₁₋₅ éventuellement substitué par un ou plusieurs substituants R¹⁰ identiques ou différents ; alcényle en C₂₋₅ éventuellement substitué par un ou plusieurs substituants R¹⁰ identiques ou différents ; alcynyle en C₂₋₅ éventuellement substitué par un ou plusieurs substituants R¹⁰ identiques ou différents ; cycloalkyle en C₃₋₆ éventuellement substitué par un ou plusieurs substituants R¹⁰ identiques ou différents ; phényle éventuellement substitué par un ou plusieurs substituants R¹¹ identiques ou différents ; et benzyle éventuellement substitué par un ou plusieurs substituants R¹¹ identiques ou différents ;
- R⁵ est choisi indépendamment dans le groupe constitué de H, deutérium, F et alkyle en C₁₋₅ éventuellement substitué par un ou plusieurs substituants R¹⁰ identiques ou différents ;
- R⁶ est choisi indépendamment dans le groupe constitué de H, deutérium, F et alkyle en C₁₋₅ éventuellement substitué par un ou plusieurs substituants R¹⁰ identiques ou différents ;
- R⁷ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par hétéroaryle en C₅ éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R⁸ est choisi indépendamment parmi benzyle éventuellement substitué par un ou plusieurs substituants R¹¹ identiques ou différents ;
- R⁹ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par naphtyle éventuellement substitué par un ou plusieurs substituants R¹¹ identiques ou différents ; alkyle en C₁₋₃ substitué par hétéroaryle à 5 à 10 chaînons éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R¹⁰ est choisi indépendamment dans le groupe constitué de H, deutérium, F, Cl et -Oalkyle en C₁₋₃ ; et
- R¹¹ est choisi indépendamment dans le groupe constitué de deutérium, méthoxy, -OCF₃, Me, CF₃, CF₂Cl, CF₂H, CFH₂, CD₃, cyclopropyle, NH₂, -NHAc, -C(=O)-NH₂, nitro, cyano, Cl, Br, I et F ;
lorsque R⁵ et R⁶ sont individuellement alkyle en C₁₋₅ éventuellement substitué par un ou plusieurs substituants R¹⁰ identiques ou différents, alors R⁵ et R⁶ sont éventuellement liés ensemble pour former un cycle ;
ou un sel, hydrate, polymorphe, tautomère ou solvate pharmaceutiquement acceptable de celui-ci à condition que :
- seul un parmi M et Z est O ; et
- lorsque M est O, Q et T sont CH₂, Z est NH, R¹ est Cl, R² est H et R³ est H, alors R⁴ n'est pas H.

3. Composé selon la revendication 1, dans lequel le composé est choisi parmi le groupe constitué de la Formule (III), de la Formule (IV), de la Formule (V), de la Formule (VI) et de la Formule (VII) : 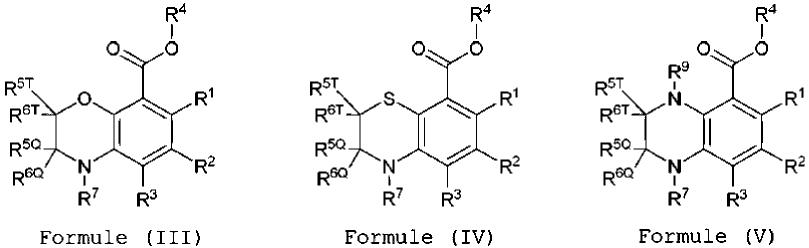 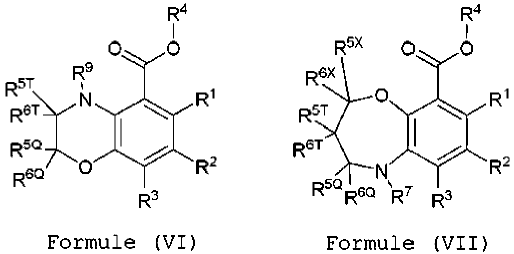 dans lequel :
- R¹ est choisi dans le groupe constitué de F ; Cl ; Br ; alkyle en C₁₋₃ éventuellement substitué par un ou plusieurs substituants R¹⁰ identiques ou différents ; alcényle en C₂₋₃ éventuellement substitué par un ou plusieurs substituants R¹⁰ identiques ou différents ; -Oalkyle en C₁₋₃ éventuellement substitué par un ou plusieurs substituants R¹⁰ identiques ou différents ; -Ocycloalkyle en C₃₋₅ éventuellement substitué par un ou plusieurs substituants R¹⁰ identiques ou différents ; - Salkyle en C₁₋₃ éventuellement substitué par un ou plusieurs substituants R¹⁰ identiques ou différents et -Scycloalkyle en C₃₋₅ éventuellement substitué par un ou plusieurs substituants R¹⁰ identiques ou différents ;
- R² est choisi dans le groupe constitué de H ; F ; Cl ; Br ; et alkyle en C₁₋₃ éventuellement substitué par un ou plusieurs substituants R¹⁰ identiques ou différents ;
- R³ est choisi dans le groupe constitué de H, F et Cl ;
- R⁴ est choisi dans le groupe constitué de H ; alkyle en C₁₋₃ éventuellement substitué par un ou plusieurs substituants R¹⁰ identiques ou différents ; alcényle en C₂₋₅ éventuellement substitué par un ou plusieurs substituants R¹⁰ identiques ou différents ; alcynyle en C₂₋₅ éventuellement substitué par un ou plusieurs substituants R¹⁰ identiques ou différents ; cycloalkyle en C₃₋₆ éventuellement substitué par un ou plusieurs substituants R¹⁰ identiques ou différents ; phényle éventuellement substitué par un ou plusieurs substituants R¹¹ identiques ou différents ; et benzyle éventuellement substitué par un ou plusieurs substituants R¹¹ identiques ou différents ;
- R^{5Q}, R^{5T} et R^{5X} sont choisis indépendamment dans le groupe constitué de H, deutérium, F et alkyle en C₁₋₅ éventuellement substitué par un ou plusieurs substituants R¹⁰ identiques ou différents ;
- R^{6Q}, R^{6T} et R^{6X} sont choisis indépendamment dans le groupe constitué de H, deutérium, F et alkyle en C₁₋₅ éventuellement substitué par un ou plusieurs substituants R¹⁰ identiques ou différents ;
- R⁷ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par hétéroaryle en C₅ éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R⁹ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par naphtyle éventuellement substitué par un ou plusieurs substituants R¹¹ identiques ou différents ; alkyle en C₁₋₃ substitué par hétéroaryle à 5 à 10 chaînons éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R¹⁰ est choisi indépendamment dans le groupe constitué de H, deutérium, F, Cl et -Oalkyle en C₁₋₃ ; et
- R¹¹ est choisi indépendamment dans le groupe constitué de deutérium, méthoxy, -OCF₃, Me, CF₃, CF₂Cl, CF₂H, CFH₂, CD₃, cyclopropyle, NH₂, -NHAc, -C(=O)-NH₂, nitro, cyano, Cl, Br, I et F ;
lorsque R^{5Q} et R^{6Q} sont individuellement alkyle en C₁₋₅ éventuellement substitué par un ou plusieurs substituants R¹⁰ identiques ou différents, alors R^{5Q} et R^{6Q} sont éventuellement liés ensemble pour former un cycle ;
ou lorsque R^{5T} et R^{6T} sont individuellement alkyle en C₁₋₅ éventuellement substitué par un ou plusieurs substituants R¹⁰ identiques ou différents, alors R^{5T} et R^{6T} sont éventuellement liés ensemble pour former un cycle ;
ou lorsque R^{5X} et R^{6X} sont individuellement alkyle en C₁₋₅ éventuellement substitué par un ou plusieurs substituants R¹⁰ identiques ou différents, alors R^{5X} et R^{6X} sont éventuellement liés ensemble pour former un cycle ;
ou un sel, hydrate, polymorphe, tautomère ou solvate pharmaceutiquement acceptable de celui-ci.

4. Composé selon l'une quelconque des revendications 1 à 3, dans lequel R¹ est choisi dans le groupe constitué de F, Cl, Me, OMe, OEt, OCHMe₂ et SMe.

5. Composé selon l'une quelconque des revendications 1 à 4, dans lequel R² est choisi dans le groupe constitué de H, F, Cl et Me.

6. Composé selon l'une quelconque des revendications 1 à 5, dans lequel R⁴ est H.

7. Composé selon l'une quelconque des revendications 1 à 6, dans lequel R¹ est OMe et R² est F ou Cl.

8. Composé selon l'une quelconque des revendications 1 à 7, dans lequel le composé est choisi dans le groupe constitué de :
acide 6-chloro-7-méthoxy-4-méthyl-3,4-dihydro-2H-1,4-benzoxazine-8-carboxylique ;
acide 7-chloro-6-fluoro-4-méthyl-3,4-dihydro-2H-1,4-benzoxazine-5-carboxylique ;
acide 7-chloro-6-fluoro-4-méthyl-3,4-dihydro-2H-1,4-benzoxazine-8-carboxylique ;
acide 5-chloro-7-fluoro-3,4-dihydro-2H-1,4-benzoxazine-8-carboxylique ;
acide 6-chloro-7-fluoro-3,4-dihydro-2H-1,4-benzoxazine-8-carboxylique ;
acide 6-chloro-7-fluoro-4-méthyl-3,4-dihydro-2H-1,4-benzoxazine-8-carboxylique ;
acide 7-chloro-6-fluoro-1,4-diméthyl-1,2,3,4-tétrahydroquinoxaline-5-carboxylique ;
acide 6-chloro-7-fluoro-2,2,4-triméthyl-3,4-dihydro-2H-1,4-benzoxazine-8-carboxylique ;
acide 7-chloro-6-méthoxy-4-méthyl-3,4-dihydro-2H-1,4-benzoxazine-5-carboxylique ;
acide 7-chloro-6-fluoro-3,4-dihydro-2H-1,4-benzoxazine-5-carboxylique ;
acide 7-chloro-8-méthoxy-5-méthyl-2,3,4,5-tétrahydro-1,5-benzoxazépine-9-carboxylique ;
acide 7-chloro-8-fluoro-5-méthyl-2,3,4,5-tétrahydro-1,5-benzoxazépine-9-carboxylique ;
acide 6-chloro-7-méthoxy-3,4-dihydro-2H-1,4-benzoxazine-8-carboxylique ;
acide 7-chloro-8-fluoro-2,3,4,5-tétrahydro-1,5-benzoxazépine-9-carboxylique ;
acide 7-chloro-8-éthoxy-5-méthyl-2,3,4,5-tétrahydro-1,5-benzoxazépine-9-carboxylique ;
acide 6-chloro-7-fluoro-4-(3-méthylbutyl)-3,4-dihydro-2H-1,4-benzoxazine-8-carboxylique ;
acide 6-chloro-4-éthyl-7-fluoro-3,4-dihydro-2H-1,4-benzoxazine-8-carboxylique ;
acide 6-chloro-7-fluoro-4-méthyl-3,4-dihydro-2H-1,4-benzothiazine-8-carboxylique ;
acide 6-chloro-4-(cyclopropylméthyl)-7-fluoro-3,4-dihydro-2H-1,4-benzoxazine-8-carboxylique ;
acide 6-chloro-7-fluoro-4-propyl-3,4-dihydro-2H-1,4-benzoxazine-8-carboxylique ;
acide 4-benzyl-6-chloro-7-fluoro-3,4-dihydro-2H-1,4-benzoxazine-8-carboxylique ;
acide 6-chloro-7-fluoro-4-[3-(4-fluorophényl)propyl]-3,4-dihydro-2H-1,4-benzoxazine-8-carboxylique ;
acide 6-chloro-4-méthyl-7-(propan-2-yloxy)-3,4-dihydro-2H-1,4-benzoxazine-8-carboxylique ;
acide 6-chloro-7-fluoro-4-[2-(4-fluorophényl)éthyl]-3,4-dihydro-2H-1,4-benzoxazine-8-carboxylique ;
acide 6-chloro-4-méthyl-7-(méthylsulfanyl)-3,4-dihydro-2H-1,4-benzoxazine-8-carboxylique ;
acide 6-chloro-7-fluoro-4-(3-méthoxypropyl)-3,4-dihydro-2H-1,4-benzoxazine-8-carboxylique ;
acide 6-chloro-4-éthyl-7-méthoxy-3,4-dihydro-2H-1,4-benzoxazine-8-carboxylique ;
acide 6-chloro-7-méthoxy-4-méthyl-3,4-dihydro-2H-1,4-benzothiazine-8-carboxylique ;
acide 6-fluoro-7-méthoxy-4-méthyl-3,4-dihydro-2H-1,4-benzoxazine-8-carboxylique ;
acide 7-méthoxy-4,6-diméthyl-3,4-dihydro-2H-1,4-benzoxazine-8-carboxylique ;
acide 6-chloro-7-méthoxy-4-(propan-2-yl)-3,4-dihydro-2H-1,4-benzoxazine-8-carboxylique ;
acide 6-chloro-7-méthoxy-4-(2-phényléthyl)-3,4-dihydro-2H-1,4-benzoxazine-8-carboxylique ;
acide 6-chloro-7-méthoxy-4-[2-(4-méthoxyphényl)éthyl]-3,4-dihydro-2H-1,4-benzoxazine-8-carboxylique ;
acide 6-chloro-7-méthoxy-2-(méthoxyméthyl)-4-méthyl-3,4-dihydro-2H-1,4-benzoxazine-8-carboxylique ;
acide 6-chloro-7-méthoxy-4-[(thiophèn-3-yl)méthyl]-3,4-dihydro-2H-1,4-benzoxazine-8-carboxylique ;
acide 6-chloro-7-méthoxy-2,2,4-triméthyl-3,4-dihydro-2H-1,4-benzoxazine-8-carboxylique ;
acide 6-chloro-7-fluoro-4-(2-méthoxyéthyl)-3,4-dihydro-2H-1,4-benzoxazine-8-carboxylique ;
acide 6-chloro-7-méthoxy-4-méthyl-3,4-dihydrospiro[1,4-benzoxazine-2,1'-cyclobutane]-8-carboxylique ;
acide 6-chloro-4-[3-(furan-2-yl)propyl]-7-méthoxy-3,4-dihydro-2H-1,4-benzoxazine-8-carboxylique ;
acide 6-chloro-7-méthoxy-4-[(thiophèn-2-yl)méthyl]-3,4-dihydro-2H-1,4-benzoxazine-8-carboxylique ;
acide 6-chloro-7-méthoxy-4-[(1,3-thiazol-2-yl)méthyl]-3,4-dihydro-2H-1,4-benzoxazine-8-carboxylique ;
acide 6-chloro-5-fluoro-7-méthoxy-4-méthyl-3,4-dihydro-2H-1,4-benzoxazine-8-carboxylique ;
acide 4-benzyl-7-chloro-6-fluoro-3,4-dihydro-2H-1,4-benzoxazine-5-carboxylique ;
acide 6-chloro-4-(cyclobutylméthyl)-7-fluoro-3,4-dihydro-2H-1,4-benzoxazine-8-carboxylique ;
acide 6-chloro-7-méthoxy-4-[3-(1,3-thiazol-2-yl)propyl]-3,4-dihydro-2H-1,4-benzoxazine-8-carboxylique ;
acide 6-chloro-4,7-diméthyl-3,4-dihydro-2H-1,4-benzoxazine-8-carboxylique ;
acide 6-chloro-7-méthoxy-4-[(4-méthoxyphényl)méthyl]-3,4-dihydro-2H-1,4-benzoxazine-8-carboxylique ;
acide 6-chloro-7-méthoxy-4-[(1,3-oxazol-2-yl)méthyl]-3,4-dihydro-2H-1,4-benzoxazine-8-carboxylique ;
acide 7-chloro-6-fluoro-4-(3-méthylbutyl)-3,4-dihydro-2H-1,4-benzoxazine-5-carboxylique ;
acide 4-benzyl-6-chloro-7-méthoxy-3,4-dihydro-2H-1,4-benzoxazine-8-carboxylique ;
acide 6-chloro-4-[2-(3,5-difluorophényl)éthyl]-7-méthoxy-3,4-dihydro-2H-1,4-benzoxazine-8-carboxylique ;
acide 7-chloro-6-fluoro-4-[(thiophèn-3-yl)méthyl]-3,4-dihydro-2H-1,4-benzoxazine-5-carboxylique ;
acide 4-benzyl-6-fluoro-7-méthoxy-3,4-dihydro-2H-1,4-benzoxazine-8-carboxylique ;
6-chloro-7-méthoxy-3,4-dihydro-2H-1,4-benzoxazine-8-carboxylate de méthyle ;
6-chloro-7-fluoro-3,4-dihydro-2H-1,4-benzoxazine-8-carboxylate d'éthyle ;
7-chloro-6-fluoro-3,4-dihydro-2H-1,4-benzoxazine-5-carboxylate de méthyle ;
6-chloro-7-fluoro-3,4-dihydro-2H-1,4-benzoxazine-8-carboxylate de méthyle ;
acide 7-chloro-6-fluoro-4-propyl-3,4-dihydro-2H-1,4-benzoxazine-5-carboxylique ;
acide 7-chloro-6-fluoro-4-méthyl-1,2,3,4-tétrahydro-5-quinoxalinecarboxylique ;
acide 7-chloro-6-fluoro-4-[(p-méthoxyphényl)méthyl]-3,4-dihydro-2H-1,4-benzoxazine-5-carboxylique ;
acide 1-benzyl-7-chloro-6-fluoro-4-méthyl-1,2,3,4-tétrahydro-5-quinoxalinecarboxylique ;
acide 7-chloro-6-fluoro-4-[(p-tolyl)méthyl]-3,4-dihydro-2H-1,4-benzoxazine-5-carboxylique ;
acide 7-chloro-6-fluoro-4-[(1-naphtyl)méthyl]-3,4-dihydro-2H-1,4-benzoxazine-5-carboxylique ;
acide 7-chloro-6-fluoro-4-[(o-tolyl)méthyl]-3,4-dihydro-2H-1,4-benzoxazine-5-carboxylique ; et
acide 4-benzyl-6,7-dichloro-3,4-dihydro-2H-1,4-benzoxazine-8-carboxylique.

9. Composition comprenant le composé selon l'une quelconque des revendications 1 à 8 et un support pharmaceutiquement acceptable.

10. Composé selon l'une quelconque des revendications 1 à 8 ou composition selon la revendication 9 pour une utilisation en tant que médicament.

11. Composé selon l'une quelconque des revendications 1 à 8 ou composition selon la revendication 9 pour une utilisation dans le traitement d'une indication choisie dans le groupe constitué de la myasthénie grave, de la myasthénie grave autoimmune, du syndrome myasthénique congénital, de la myasthénie grave séronégative, de la myasthénie grave à kinase spécifique du muscle (MuSK-MG), du syndrome de Lambert-Eaton, de la myopathie des maladies graves, de la sclérose latérale amyotrophique (SLA), de l'amyotrophie spinale (SMA), de la myopathie des maladies graves (CIM), de la maladie de Charcot-Marie-Tooth, de la polyneuropathie diabétique, de la paralysie périodique, de la paralysie périodique hypokaliémique, de la paralysie périodique hyperkaliémique, de la myopathie myotubulaire, de la dystrophie musculaire de Duchenne, du syndrome de Guillain-Barré, de la poliomyélite, du syndrome post-polio, du syndrome de fatigue chronique, de la polyneuropathie des maladies graves, de la myopathie métabolique, de la maladie de Kennedy, de la sclérose en plaques et de la neuropathie motrice multifocale.

12. Composé selon l'une quelconque des revendications 1 à 8 ou a composition selon la revendication 9 pour une utilisation dans le traitement symptomatique de la sarcopénie.

13. Composé selon l'une quelconque des revendications 1 à 8 ou composition selon la revendication 9 pour une utilisation dans l'inversion et/ou l'amélioration d'un blocage neuromusculaire.

14. Composé de Formule (I) : 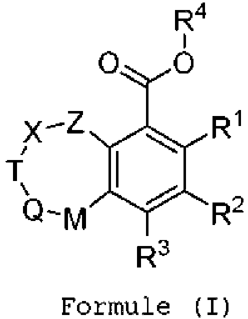 dans lequel :
- M est CR⁵R⁶, NR⁷, O ou S ;
- Q est CR⁵R⁶, NR⁸, O ou S ;
- T est CR⁵R⁶, NR⁸, O ou S ;
- X est absent, CR⁵R⁶, NR⁸, O ou S ;
- Z est CR⁵R⁶, NR⁹, O ou S ;
- deux ou plus parmi M, Q, T, X et Z sont NR⁷, NR⁸, NR⁹, O ou S ;
- R¹ est choisi dans le groupe constitué de H ; F ; Cl ; Br ; alkyle en C₁₋₃ éventuellement substitué par un ou plusieurs substituants R¹⁰ identiques ou différents ; alcényle en C₂₋₃ éventuellement substitué par un ou plusieurs substituants R¹⁰ identiques ou différents ; -Oalkyle en C₁₋₃ éventuellement substitué par un ou plusieurs substituants R¹⁰ identiques ou différents ; -Ocycloalkyle en C₃₋₅ éventuellement substitué par un ou plusieurs substituants R¹⁰ identiques ou différents ; - Salkyle en C₁₋₃ éventuellement substitué par un ou plusieurs substituants R¹⁰ identiques ou différents et -Scycloalkyle en C₃₋₅ éventuellement substitué par un ou plusieurs substituants R¹⁰ identiques ou différents ;
- R² est choisi dans le groupe constitué de H ; F ; Cl ; Br ; alkyle en C₁₋₃ éventuellement substitué par un ou plusieurs substituants R¹⁰ identiques ou différents et Oalkyle en C₁₋₃ éventuellement substitué par un ou plusieurs substituants R¹⁰ identiques ou différents ;
- R³ est choisi dans le groupe constitué de H, F et Cl ;
- R⁴ est choisi dans le groupe constitué de H ; alkyle en C₁₋₅ éventuellement substitué par un ou plusieurs substituants R¹⁰ identiques ou différents ; alcényle en C₂₋₅ éventuellement substitué par un ou plusieurs substituants R¹⁰ identiques ou différents ; alcynyle en C₂₋₅ éventuellement substitué par un ou plusieurs substituants R¹⁰ identiques ou différents ; cycloalkyle en C₃₋₆ éventuellement substitué par un ou plusieurs substituants R¹⁰ identiques ou différents ; phényle éventuellement substitué par un ou plusieurs substituants R¹¹ identiques ou différents ; et benzyle éventuellement substitué par un ou plusieurs substituants R¹¹ identiques ou différents ;
- R⁵ est choisi indépendamment dans le groupe constitué de H, deutérium, F et alkyle en C₁₋₅ éventuellement substitué par un ou plusieurs substituants R¹⁰ identiques ou différents ;
- R⁶ est choisi indépendamment dans le groupe constitué de H, deutérium, F et alkyle en C₁₋₅ éventuellement substitué par un ou plusieurs substituants R¹⁰ identiques ou différents ;
- R⁷ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par hétéroaryle en C₅ éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R⁸ est choisi indépendamment parmi benzyle éventuellement substitué par un ou plusieurs substituants R¹¹ identiques ou différents ;
- R⁹ est choisi indépendamment dans le groupe constitué de H, alkyle en C₁₋₅ éventuellement substitué par un ou plusieurs substituants R¹⁰ identiques ou différents ; alkyle en C₁₋₃ substitué par cycloalkyle en C₃₋₅ éventuellement substitué par un ou plusieurs substituants R¹⁰ identiques ou différents ; alkyle en C₁₋₃ substitué par phényle éventuellement substitué par un ou plusieurs substituants R¹¹ identiques ou différents ; alkyle en C₁₋₃ substitué par naphtyle éventuellement substitué par un ou plusieurs substituants R¹¹ identiques ou différents ; alkyle en C₁₋₃ substitué par hétéroaryle à 5 à 10 chaînons éventuellement substitué par un ou plusieurs substituants R¹¹ identiques ou différents ; et cycloalkyle en C₃₋₅ éventuellement substitué par un ou plusieurs substituants R¹⁰ identiques ou différents ;
- R¹⁰ est choisi indépendamment dans le groupe constitué de H, deutérium, F, Cl et -Oalkyle en C₁₋₃ ; et
- R¹¹ est choisi indépendamment dans le groupe constitué de deutérium, méthoxy, -OCF₃, Me, CF₃, CF₂Cl, CF₂H, CFH₂, CD₃, cyclopropyle, NH₂, -NHAc, -C(=O)-NH₂, nitro, cyano, Cl, Br, I et F ;
lorsque R⁵ et R⁶ sont individuellement alkyle en C₁₋₅ éventuellement substitué par un ou plusieurs substituants R¹⁰ identiques ou différents, alors R⁵ et R⁶ sont éventuellement liés ensemble pour former un cycle ;
ou un sel, hydrate, polymorphe, tautomère ou solvate pharmaceutiquement acceptable de celui-ci
pour une utilisation dans le traitement, l'amélioration et/ou la prévention d'un trouble neuromusculaire, et/ou pour une utilisation dans l'inversion et/ou l'amélioration d'un blocage neuromusculaire.

15. Composé pour une utilisation selon la revendication 14, dans lequel le composé est choisi dans le groupe constitué de :
acide 7-chloro-2,3-dihydro-1,4-benzodioxine-5-carboxylique ;
acide 7-chloro-6-méthoxy-2,3-dihydro-1,4-benzodioxine-5-carboxylique ;
acide 7-chloro-6-méthyl-2,3-dihydro-1,4-benzodioxine-5-carboxylique ;
acide 7,8-dichloro-6-méthyl-2,3-dihydro-1,4-benzodioxine-5-carboxylique ;
acide 6-chloro-7-méthoxy-4-méthyl-3,4-dihydro-2H-1,4-benzoxazine-8-carboxylique ;
acide 7-chloro-6-fluoro-4-méthyl-3,4-dihydro-2H-1,4-benzoxazine-5-carboxylique ;
acide 7-chloro-6-fluoro-4-méthyl-3,4-dihydro-2H-1,4-benzoxazine-8-carboxylique ;
acide 5-chloro-7-fluoro-3,4-dihydro-2H-1,4-benzoxazine-8-carboxylique ;
acide 6-chloro-7-fluoro-3,4-dihydro-2H-1,4-benzoxazine-8-carboxylique ;
acide 7-chloro-6-méthoxy-4-méthyl-3,4-dihydro-2H-1,4-benzoxazine-8-carboxylique ;
acide 6-chloro-7-fluoro-4-méthyl-3,4-dihydro-2H-1,4-benzoxazine-8-carboxylique ;
acide 7-chloro-6-fluoro-1,4-diméthyl-1,2,3,4-tétrahydroquinoxaline-5-carboxylique ;
acide 6-chloro-7-fluoro-2,2,4-triméthyl-3,4-dihydro-2H-1,4-benzoxazine-8-carboxylique ;
acide 7-chloro-6-méthoxy-4-méthyl-3,4-dihydro-2H-1,4-benzoxazine-5-carboxylique ;
acide 7-chloro-6-fluoro-3,4-dihydro-2H-1,4-benzoxazine-5-carboxylique ;
acide 7-chloro-8-méthoxy-5-méthyl-2,3,4,5-tétrahydro-1,5-benzoxazépine-9-carboxylique ;
acide 7-chloro-8-fluoro-5-méthyl-2,3,4,5-tétrahydro-1,5-benzoxazépine-9-carboxylique ;
acide 6-chloro-7-méthoxy-3,4-dihydro-2H-1,4-benzoxazine-8-carboxylique ;
acide 7-chloro-8-fluoro-2,3,4,5-tétrahydro-1,5-benzoxazépine-9-carboxylique ;
acide 7-chloro-8-éthoxy-5-méthyl-2,3,4,5-tétrahydro-1,5-benzoxazépine-9-carboxylique ;
acide 6-chloro-7-fluoro-4-(3-méthylbutyl)-3,4-dihydro-2H-1,4-benzoxazine-8-carboxylique ;
acide 6-chloro-4-éthyl-7-fluoro-3,4-dihydro-2H-1,4-benzoxazine-8-carboxylique ;
acide 6-chloro-7-fluoro-4-méthyl-3,4-dihydro-2H-1,4-benzothiazine-8-carboxylique ;
acide 6-chloro-4-(cyclopropylméthyl)-7-fluoro-3,4-dihydro-2H-1,4-benzoxazine-8-carboxylique ;
acide 6-chloro-7-fluoro-4-propyl-3,4-dihydro-2H-1,4-benzoxazine-8-carboxylique ;
acide 4-benzyl-6-chloro-7-fluoro-3,4-dihydro-2H-1,4-benzoxazine-8-carboxylique ;
acide 7-fluoro-6-méthoxy-4-méthyl-3,4-dihydro-2H-1,4-benzoxazine-8-carboxylique ;
acide 6-chloro-7-fluoro-4-[3-(4-fluorophényl)propyl]-3,4-dihydro-2H-1,4-benzoxazine-8-carboxylique ;
acide 6-chloro-4-méthyl-7-(propan-2-yloxy)-3,4-dihydro-2H-1,4-benzoxazine-8-carboxylique ;
acide 6-chloro-7-fluoro-4-[2-(4-fluorophényl)éthyl]-3,4-dihydro-2H-1,4-benzoxazine-8-carboxylique ;
acide 6-chloro-4-méthyl-7-(méthylsulfanyl)-3,4-dihydro-2H-1,4-benzoxazine-8-carboxylique ;
acide 6-chloro-7-fluoro-4-(3-méthoxypropyl)-3,4-dihydro-2H-1,4-benzoxazine-8-carboxylique ;
acide 6-chloro-4-éthyl-7-méthoxy-3,4-dihydro-2H-1,4-benzoxazine-8-carboxylique ;
acide 6-chloro-7-méthoxy-4-méthyl-3,4-dihydro-2H-1,4-benzothiazine-8-carboxylique ;
acide 6-fluoro-7-méthoxy-4-méthyl-3,4-dihydro-2H-1,4-benzoxazine-8-carboxylique ;
acide 7-méthoxy-4,6-diméthyl-3,4-dihydro-2H-1,4-benzoxazine-8-carboxylique ;
acide 6-chloro-7-méthoxy-4-(propan-2-yl)-3,4-dihydro-2H-1,4-benzoxazine-8-carboxylique ;
acide 6-chloro-7-méthoxy-4-(2-phényléthyl)-3,4-dihydro-2H-1,4-benzoxazine-8-carboxylique ;
acide 6-chloro-7-méthoxy-4-[2-(4-méthoxyphényl)éthyl]-3,4-dihydro-2H-1,4-benzoxazine-8-carboxylique ;
acide 6-chloro-7-méthoxy-2-(méthoxyméthyl)-4-méthyl-3,4-dihydro-2H-1,4-benzoxazine-8-carboxylique ;
acide 6-chloro-7-méthoxy-4-[(thiophèn-3-yl)méthyl]-3,4-dihydro-2H-1,4-benzoxazine-8-carboxylique ;
acide 6-chloro-7-méthoxy-2,2,4-triméthyl-3,4-dihydro-2H-1,4-benzoxazine-8-carboxylique ;
acide 6-chloro-7-méthoxy-2-(méthoxyméthyl)-4-méthyl-3,4-dihydro-2H-1,4-benzoxazine-5-carboxylique ;
acide 6-chloro-7-fluoro-4-(2-méthoxyéthyl)-3,4-dihydro-2H-1,4-benzoxazine-8-carboxylique ;
acide 6-chloro-7-méthoxy-4-méthyl-3,4-dihydrospiro[1,4-benzoxazine-2,1'-cyclobutane]-8-carboxylique ;
acide 6-chloro-4-[3-(furan-2-yl)propyl]-7-méthoxy-3,4-dihydro-2H-1,4-benzoxazine-8-carboxylique ;
acide 6-chloro-7-méthoxy-4-[(thiophèn-2-yl)méthyl]-3,4-dihydro-2H-1,4-benzoxazine-8-carboxylique ;
acide 6-chloro-7-méthoxy-4-[(thiophèn-2-yl)méthyl]-3,4-dihydro-2H-1,4-benzoxazine-8-carboxylique ;
acide 6-chloro-7-méthoxy-4-[(1,3-thiazol-2-yl)méthyl]-3,4-dihydro-2H-1,4-benzoxazine-8-carboxylique ;
acide 6-chloro-5-fluoro-7-méthoxy-4-méthyl-3,4-dihydro-2H-1,4-benzoxazine-8-carboxylique ;
acide 4-benzyl-7-chloro-6-fluoro-3,4-dihydro-2H-1,4-benzoxazine-5-carboxylique ;
acide 6-chloro-4-(cyclobutylméthyl)-7-fluoro-3,4-dihydro-2H-1,4-benzoxazine-8-carboxylique ;
acide 6-chloro-7-méthoxy-4-[3-(1,3-thiazol-2-yl)propyl]-3,4-dihydro-2H-1,4-benzoxazine-8-carboxylique ;
acide 6-chloro-4,7-diméthyl-3,4-dihydro-2H-1,4-benzoxazine-8-carboxylique ;
acide 6-chloro-7-méthoxy-4-[(4-méthoxyphényl)méthyl]-3,4-dihydro-2H-1,4-benzoxazine-8-carboxylique ;
acide 6-chloro-7-méthoxy-4-[(1,3-oxazol-2-yl)méthyl]-3,4-dihydro-2H-1,4-benzoxazine-8-carboxylique ;
acide 7-chloro-6-fluoro-4-(3-méthylbutyl)-3,4-dihydro-2H-1,4-benzoxazine-5-carboxylique ;
acide 4-benzyl-6-chloro-7-méthoxy-3,4-dihydro-2H-1,4-benzoxazine-8-carboxylique ;
acide 6-chloro-4-[2-(3,5-difluorophényl)éthyl]-7-méthoxy-3,4-dihydro-2H-1,4-benzoxazine-8-carboxylique ;
acide 7-chloro-6-fluoro-4-[(thiophèn-3-yl)méthyl]-3,4-dihydro-2H-1,4-benzoxazine-5-carboxylique ;
acide 4-benzyl-6-fluoro-7-méthoxy-3,4-dihydro-2H-1,4-benzoxazine-8-carboxylique ;
6-chloro-7-méthoxy-3,4-dihydro-2H-1,4-benzoxazine-8-carboxylate de méthyle ;
6-chloro-7-fluoro-3,4-dihydro-2H-1,4-benzoxazine-8-carboxylate d'éthyle ;
7-chloro-6-fluoro-3,4-dihydro-2H-1,4-benzoxazine-5-carboxylate de méthyle ;
6-chloro-7-fluoro-3,4-dihydro-2H-1,4-benzoxazine-8-carboxylate de méthyle ;
acide 7-chloro-6-fluoro-4-propyl-3,4-dihydro-2H-1,4-benzoxazine-5-carboxylique ;
acide 7-chloro-6-fluoro-4-méthyl-1,2,3,4-tétrahydro-5-quinoxalinecarboxylique ;
acide 7-chloro-6-fluoro-4-[(p-méthoxyphényl)méthyl]-3,4-dihydro-2H-1,4-benzoxazine-5-carboxylique ;
acide 1-benzyl-7-chloro-6-fluoro-4-méthyl-1,2,3,4-tétrahydro-5-quinoxalinecarboxylique ;
acide 7-chloro-6-fluoro-4-[(p-tolyl)méthyl]-3,4-dihydro-2H-1,4-benzoxazine-5-carboxylique ;
acide 7-chloro-6-fluoro-4-[(1-naphtyl)méthyl]-3,4-dihydro-2H-1,4-benzoxazine-5-carboxylique ;
acide 7-chloro-6-fluoro-4-[(o-tolyl)méthyl]-3,4-dihydro-2H-1,4-benzoxazine-5-carboxylique ; et
acide 4-benzyl-6,7-dichloro-3,4-dihydro-2H-1,4-benzoxazine-8-carboxylique.
